# EUROPEAN PATENT APPLICATION

(11) **EP 4 276 200 A2**
(43) Date of publication of application: **15.11.2023**
(21) Application number: 22194193.3
(22) Date of filing: 24.07.2013
(51) Int. Cl.: C12Q 1/6886

(54) **FGFR-TACC FUSION PROTEINS AND METHODS THEREOF**

(30) Priority: 24.07.2012 US 201261675006 P
(62) Divisional of application: 13823252.5
(71) Applicant: The Trustees of Columbia University in the City of New York, New York, NY 10027 (US)
(72) Inventor: IAVARONE, Antonio, New York, NY 10021 (US); LASORELLA, Anna, New York, NY 10021 (US); RABANDAN, Raul, New York, NY 10027 (US)
(74) Representative: CH Kilger Anwaltspartnerschaft mbB

(57) **Abstract**

The invention discloses oncogenic fusion proteins. The inventions provides methods for treating gene-fusion based cancers.

## Description

This application claims the benefit of and priority to U.S. Provisional Patent Application No. 61/675,006, filed on July 24, 2012, the content of which is hereby incorporated by reference in its entirety.

All patents, patent applications and publications cited herein are hereby incorporated by reference in their entirety. The disclosures of these publications in their entireties are hereby incorporated by reference into this application.

This patent disclosure contains material that is subject to copyright protection. The copyright owner has no objection to the facsimile reproduction by anyone of the patent document or the patent disclosure as it appears in the U.S. Patent and Trademark Office patent file or records, but otherwise reserves any and all copyright rights.

### GOVERNMENT SUPPORT

This invention was made with government support under Grant Nos. R01CA101644 and R01CA085628 awarded by the National Cancer Institute. The Government has certain rights in the invention.

### BACKGROUND OF THE INVENTION

Glioblastoma multiforme (GBM) is the most common form of brain cancer and among the most incurable and lethal of all human cancers. The current standard of care includes surgery, chemotherapy, and radiation therapy. However, the prognosis of GBM remains uniformly poor. There are few available targeted therapies and none that specifically target GBM.

The target population of GBM patients who may carry FGFR-TACC gene fusions and would benefit from targeted inhibition of FGFR kinase activity is estimated to correspond to 6,000 patients per year world-wide.

### SUMMARY OF THE INVENTION

The invention is based, at least in part, on the discovery of a highly expressed class of gene fusions in GBM, which join the tyrosine kinase domain of FGFR genes to the TACC domain of TACC1 or TACC3. The invention is based, at least in part, on the finding that FGFR-TACC fusions identify a subset of GBM patients who will benefit from targeted inhibition of the tyrosine kinase activity of FGFR. Identification of fusions of FGFR and TACC genes in glioblastoma patients and other subjects afflicted with a gene-fusion associated cancer (such as an epithelial cancer) are useful therapeutic targets.

An aspect of the invention provides for a purified fusion protein comprising a tyrosine kinase domain of an FGFR protein fused to a polypeptide that constitutively activates the tyrosine kinase domain of the FGFR protein. In one embodiment, the FGFR protein is FGFR1, FGFR2, FGFR3, or FGR4. In another embodiment, the purified fusion protein is essentially free of other human proteins.

An aspect of the invention provides for a purified fusion protein comprising a transforming acidic coiled-coil (TACC) domain fused to a polypeptide with a tyrosine kinase domain, wherein the TACC domain constitutively activates the tyrosine kinase domain. In one embodiment, the TACC protein is TACC1, TACC2, or TACC3. In another embodiment, the purified fusion protein is essentially free of other human proteins.

An aspect of the invention provides for a purified fusion protein comprising the tyrosine kinase domain of an FGFR protein fused 5' to the TACC domain of a transforming acidic coiled-coil-containing (TACC) protein. In one embodiment, the FGFR protein is FGFR1, FGFR2, FGFR3, or FGR4. In another embodiment, the TACC protein is TACC1, TACC2, or TACC3. In another embodiment, the purified fusion protein is essentially free of other human proteins.

An aspect of the invention provides for a purified fusion protein encoded by an FGFR1-TACC1 nucleic acid, wherein FGFR1-TACC1 comprises a combination of exons 1-17 of FGFR1 located on human chromosome 8p11 spliced 5' to a combination of exons 7-13 of TACC1 located on human chromosome 8p11, wherein a genomic breakpoint occurs in any one of exons 1-17 of FGFR1 and any one of exons 7-13 of TACC1. In another embodiment, the purified fusion protein is essentially free of other human proteins.

An aspect of the invention provides for a purified fusion protein encoded by an FGFR2-TACC2 nucleic acid, wherein FGFR2-TACC2 comprises a combination of any exons 1-18 of FGFR2 located on human chromosome 10q26 spliced 5' to a combination of any exons 1-23 of TACC2 located on human chromosome 10q26. In another embodiment, the purified fusion protein is essentially free of other human proteins.

An aspect of the invention provides for a purified fusion protein encoded by an FGFR3-TACC3 nucleic acid, wherein FGFR3-TACC3 comprises a combination of exons 1-16 of FGFR3 located on human chromosome 4p16 spliced 5' to a combination of exons 8-16 of TACC3 located on human chromosome 4p16, wherein a genomic breakpoint occurs in any one of exons 1-16 of FGFR3 and any one of exons 8-16 of TACC3. In another embodiment, the purified fusion protein is essentially free of other human proteins.

An aspect of the invention provides for a purified fusion protein encoded by an FGFR3-TACC3 nucleic acid, wherein FGFR3-TACC3 comprises a combination of exons 1-16 of FGFR3 located on human chromosome 4p16 spliced 5' to a combination of exons 8-16 of TACC3 located on human chromosome 4p16, wherein a genomic breakpoint occurs in any one of ***introns*** 1-16 of FGFR3 and any one of exons 8-16 of TACC3. In another embodiment, the purified fusion protein is essentially free of other human proteins.

An aspect of the invention provides for a purified fusion protein encoded by an FGFR3-TACC3 nucleic acid, wherein FGFR3-TACC3 comprises a combination of exons 1-16 of FGFR3 located on human chromosome 4p16 spliced 5' to a combination of exons 8-16 of TACC3 located on human chromosome 4p16, wherein a genomic breakpoint occurs in any one of exons 1-16 of FGFR3 and any one of ***introns*** 7-16 of TACC3. In another embodiment, the purified fusion protein is essentially free of other human proteins.

An aspect of the invention provides for a purified fusion protein encoded by an FGFR3-TACC3 nucleic acid, wherein FGFR3-TACC3 comprises a combination of exons 1-16 of FGFR3 located on human chromosome 4p16 spliced 5' to a combination of exons 8-16 of TACC3 located on human chromosome 4p16, wherein a genomic breakpoint occurs in any one of ***introns*** 1-16 of FGFR3 and any one of ***introns*** 7-16 of TACC3. In another embodiment, the purified fusion protein is essentially free of other human proteins.

An aspect of the invention provides for a synthetic nucleic acid encoding the fusion proteins described above.

An aspect of the invention provides for a purified FGFR3-TACC3 fusion protein comprising SEQ ID NO: 79, 158, 159, 160, or 161. In another embodiment, the purified fusion protein is essentially free of other human proteins.

An aspect of the invention provides for a purified FGFR3-TACC3 fusion protein having a genomic breakpoint comprising at least 3 consecutive amino acids from amino acids 730-758 of SEQ ID NO: 90 and comprising at least 3 consecutive amino acids from amino acids 549-838 of SEQ ID NO: 92. In another embodiment, the purified fusion protein is essentially free of other human proteins.

An aspect of the invention provides for a purified FGFR3-TACC3 fusion protein having a genomic breakpoint comprising SEQ ID NO: 78. In another embodiment, the purified fusion protein is essentially free of other human proteins.

An aspect of the invention provides for a purified FGFR3-TACC3 fusion protein having a genomic breakpoint comprising any one of SEQ ID NOS: 85, 86, 87, or 89. In another embodiment, the purified fusion protein is essentially free of other human proteins.

An aspect of the invention provides for a purified FGFR1-TACC1 fusion protein comprising SEQ ID NO: 150. In another embodiment, the purified fusion protein is essentially free of other human proteins.

An aspect of the invention provides for a purified FGFR1-TACC1 fusion protein having a genomic breakpoint comprising at least 3 consecutive amino acids from amino acids 746-762 of SEQ ID NO: 146 and comprising at least 3 consecutive amino acids from amino acids 572-590 of SEQ ID NO: 148. In another embodiment, the purified fusion protein is essentially free of other human proteins.

An aspect of the invention provides for a purified FGFR1-TACC1 fusion protein having a genomic break-point comprising SEQ ID NO: 88. In another embodiment, the purified fusion protein is essentially free of other human proteins.

An aspect of the invention provides for a purified DNA encoding an FGFR3-TACC3 fusion protein comprising SEQ ID NO: 94. In another embodiment, the purified fusion protein is essentially free of other human proteins.

An aspect of the invention provides for a synthetic nucleic acid encoding an FGFR3-TACC3 fusion protein having a genomic breakpoint comprising at least 9 consecutive in-frame nucleotides from nucleotides 2443-2530 of SEQ ID NO: 91 and comprising at least 9 consecutive in-frame nucleotides from nucleotides 1800-2847 of SEQ ID NO:93.

An aspect of the invention provides for a synthetic nucleic acid encoding an FGFR3-TACC3 fusion protein having a genomic breakpoint comprising any one of SEQ ID NOS: 1-77.

An aspect of the invention provides for a synthetic nucleic acid encoding an FGFR1-TACC1 fusion protein comprising SEQ ID NO: 151.

An aspect of the invention provides for a synthetic nucleic acid encoding an FGFR1-TACC1 fusion protein having a genomic breakpoint comprising at least 9 consecutive in-frame nucleotides from nucleotides 3178-3228 of SEQ ID NO: 147 and comprising at least 9 consecutive in-frame nucleotides from nucleotides 2092-2794 of SEQ ID NO: 149.

An aspect of the invention provides for a synthetic nucleic acid encoding an FGFR1-TACC1 fusion protein having a genomic breakpoint comprising SEQ ID NO: 83.

An aspect of the invention provides for an antibody or antigen-binding fragment thereof, that specifically binds to a purified fusion protein comprising a tyrosine kinase domain of an FGFR protein fused to a polypeptide that constitutively activates the tyrosine kinase domain of the FGFR protein. In one embodiment, the FGFR protein is FGFR1, FGFR2, FGFR3, or FGFR4. In another embodiment, the fusion protein is an FGFR-TACC fusion protein. In a further embodiment, the FGFR-TACC fusion protein is FGFR1-TACC1, FGFR2-TACC2, or FGFR3-TACC3. In some embodiments, the FGFR1-TACC1 fusion protein comprises the amino acid sequence of SEQ ID NO: 150. In other embodiments, the FGFR3-TACC3 fusion protein comprises the amino acid sequence of SEQ ID NO: 79, 158, 159, 150, or 161.

An aspect of the invention provides for a composition for decreasing in a subject the expression level or activity of a fusion protein comprising the tyrosine kinase domain of an FGFR protein fused to a polypeptide that constitutively activates the tyrosine kinase domain of the FGFR protein, the composition in an admixture of a pharmaceutically acceptable carrier comprising an inhibitor of the fusion protein. In one embodiment, the fusion protein is an FGFR-TACC fusion protein. In another embodiment, the inhibitor comprises an antibody that specifically binds to a FGFR-TACC fusion protein or a fragment thereof; a small molecule that specifically binds to a FGFR protein; a small molecule that specifically binds to a TACC protein; an antisense RNA or antisense DNA that decreases expression of a FGFR-TACC fusion polypeptide; a siRNA that specifically targets a FGFR-TACC fusion gene; or a combination of the listed inhibitors. In a further embodiment, the FGFR protein is FGFR1, FGFR2, FGFR3, or FGFR4. In some embodiments, the FGFR-TACC fusion protein is FGFR1-TACC1, FGFR2-TACC2, or FGFR3-TACC3. In other embodidments, the small molecule that specifically binds to a FGFR protein comprises AZD4547, NVP-BGJ398, PD173074, NF449, TK1258, BIBF-1120, BMS-582664, AZD-. 2171, TSU68, AB1010, AP24534, E-7080, LY2874455, or a combination of the listed small molecules.

An aspect of the invention provides for a method for decreasing in a subject in need thereof the expression level or activity of a fusion protein comprising the tyrosine kinase domain of an FGFR protein fused to a polypeptide that constitutively activates the tyrosine kinase domain of the FGFR protein. In one embodiment, the method comprises administering to the subject a therapeutic amount of a composition for decreasing the expression level or activity in a subject of a fusion protein comprising the tyrosine kinase domain of an FGFR protein fused to a polypeptide that constitutively activates the tyrosine kinase domain of the FGFR protein. In one embodiment, the method comprises obtaining a sample from the subject to determine the level of expression of an FGFR fusion molecule in the subject. In some embodiments, the sample is incubated with an agent that binds to an FGFR. fusion molecule, such as an antibody, a probe, a nucleic acid primer, and the like. In one embodiment, the detection or determining comprises nucleic acid sequencing, selective hybridization, selective amplification, gene expression analysis, or a combination thereof. In another embodiment, the detection or determination comprises protein expression analysis, for example by western blot analysis, ELISA, or other antibody detection methods. In a further embodiment, the method comprises determining whether the fusion protein expression level or activity is decreased compared to fusion protein expression level or activity prior to administration of the composition, thereby decreasing the expression level or activity of the fusion protein. In one embodiment, the fusion protein is an FGFR-TACC fusion protein. In a further embodiment, the FGFR protein is FGFR1, FGFR2, FGFR3, or FGFR4. In some embodiments, the FGFR-TACC fusion protein is FGFR1-TACC1, FGFR2-TACC2, or FGFR3-TACC3. In one embodiment, the composition for decreasing the expression level or activity of a fusion protein comprises an antibody that specifically binds to a FGFR-TACC fusion protein or a fragment thereof; a small molecule that specifically binds to a FGFR protein; a small molecule that specifically binds to a TACC protein; an antisense RNA or antisense DNA that decreases expression of a FGFR-TACC fusion polypeptide; a siRNA that specifically targets a FGFR-TACC fusion gene; or a combination of the listed inhibitors. In a further embodiment, the FGFR protein is FGFR1, FGFR2, FGFR3, or FGFR4. In some embodiments, the FGFR-TACC fusion protein is FGFR1-TACC1, FGFR2-TACC2, or FGFR3-TACC3. In other embodiments, the small molecule that specifically binds to a FGFR protein comprises AZD4547, NVP-BGJ398, PD173074, NF449, TK1258, BIBF-1120, BMS-582664, AZD-2171, TSU68, AB1010, AP24534, E-7080, LY2874455, or a combination of the small molecules listed.

An aspect of the invention provides for a method for treating a gene-fusion associated cancer in a subject in need thereof, the method comprising administering to the subject an effective amount of a FGFR fusion molecule inhibitor. In one embodiment, the gene-fusion associated cancer comprises an epithelial cancer. In one embodiment, the gene-fusion associated cancer comprises glioblastoma multiforme, breast cancer, lung cancer, prostate cancer, or colorectal carcinoma. In one embodiment, the method comprises obtaining a sample from the subject to determine the level of expression of an FGFR fusion molecule in the subject. In some embodiments, the sample is incubated with an agent that binds to an FGFR fusion molecule, such as an antibody, a probe, a nucleic acid primer, and the like. In one embodiment, the detection or determining comprises nucleic acid sequencing, selective hybridization, selective amplification, gene expression analysis, or a combination thereof. In another embodiment, the detection or determination comprises protein expression analysis, for example by western blot analysis, ELISA, or other antibody detection methods. In another embodiment, the FGFR fusion protein comprises an FGFR protein fused to a polypeptide that constitutively activates the tyrosine kinase domain of the FGFR protein. In one embodiment, the fusion protein is an FGFR-TACC fusion protein. In another embodiment, the inhibitor comprises an antibody that specifically binds to a FGFR-TACC fusion protein or a fragment thereof; a small molecule that specifically binds to a FGFR protein; a small molecule that specifically binds to a TACC protein; an antisense RNA or antisense DNA that decreases expression of a FGFR-TACC fusion polypeptide; a siRNA that specifically targets a FGFR-TACC fusion gene; or a combination of the listed inhibitors. In a further embodiment, the FGFR protein is FGFR1, FGFR2, FGFR3, or FGFR4. In some embodiments, the FGFR-TAC'C fusion protein is FGFR1-TACC1, FGFR2-TACC2, or FGFR3-TACC3. In other embodiments, the small molecule that specifically binds to a FGFR protein comprises AZD4547, NVP-BGJ398, PD173074, NF449, TK1258, BIBF-1120, BMS-582664, AZD-2171, TSU68, AB 10 10, AP24534, E-7080, LY2874455, or a combination of the small molecules listed.

An aspect of the invention provides for a method of decreasing growth of a solid tumor in a subject in need thereof, the method comprising administering to the subject an effective amount of a FGFR fusion molecule inhibitor, wherein the inhibitor decreases the size of the solid tumor. In one embodiment, the solid tumor comprises glioblastoma multiforme, breast cancer, lung cancer, prostate cancer, or colorectal carcinoma. In one embodiment, the method comprises obtaining a sample from the subject to determine the level of expression of an FGFR fusion molecule in the subject. In some embodiments, the sample is incubated with an agent that binds to an FGFR fusion molecule, such as an antibody, a probe, a nucleic acid primer, and the like. In one embodiment, the detection or determining comprises nucleic acid sequencing, selective hybridization, selective amplification, gene expression analysis, or a combination thereof. In another embodiment, the detection or determination comprises protein expression analysis, for example by western blot analysis, ELISA, or other antibody detection methods. In another embodiment, the FGFR fusion protein comprises an FGFR protein fused to a polypeptide that constitutively activates the tyrosine kinase domain of the FGFR protein. In one embodiment, the fusion protein is an FGFR-TACC fusion protein. In another embodiment, the inhibitor comprises an antibody that specifically binds to a FGFR-TACC fusion protein or a fragment thereof; a small molecule that specifically binds to a FGFR protein; a small molecule that specifically binds to a TACC protein; an antisense RNA or antisense DNA that decreases expression of a FGFR-TACC fusion polypeptide; a siRNA that specifically targets a FGFR-TACC fusion gene; or a combination of the listed inhibitors. In a further embodiment, the FGFR protein is FGFR1, FGFR2, FGFR3, or FGFR4. In some embodiments, the FGFR-TACC fusion protein is FGFR1-TACC1, FGFR2-TACC2, or FGFR3-TACC3. In other embodiments, the small molecule that specifically binds to a FGFR protein comprises AZD4547, NVP-BGJ398, PD173074, NF449, TK1258, BIBF-1120, BMS-582664, AZD-2171, TSU68, AB1010, AP24534, E-7080, LY2874455, or a combination of the small molecules listed.

An aspect of the invention provides for a diagnostic kit for determining whether a sample from a subject exhibits a presence of a FGFR fusion, the kit comprising at least one oligonucleotide that specifically hybridizes to a FGFR fusion, or a portion thereof. In one embodiment, the oligonucleotides comprise a set of nucleic acid primers or *in situ* hybridization probes. In another embodiment, the oligonucleotide comprises SEQ ID NO: 162, 163, 164,165, 166, 167, 168, 169, or a combination of the listed oligonucleotides. In one embodiment, the primers prime a polymerase reaction only when a FGFR fusion is present. In another embodiment, the determining comprises gene sequencing, selective hybridization, selective amplification, gene expression analysis, or a combination thereof. In a further embodiment, the FGFR-fusion is an FGFR-TACC fusion. In some embodiments, the FGFR is FGFR1, FGFR2, FGFR3, or FGFR4. In other embodiments, the FGFR-TACC fusion is FGFR1-TACC1, FGFR2-TACC2, or FGFR3-TACC3.

An aspect of the invention provides for a diagnostic kit for determining whether a sample from a subject exhibits a presence of a FGFR fusion protein, the kit comprising an antibody that specifically binds to a FGFR fusion protein comprising SEQ ID NO: 79, 85, 86, 87, 88, 89, 150, 158, 159, 160, or 161, wherein the antibody will recognize the protein only when a FGFR fusion protein is present. In one embodiment, the antibody directed to and FGFR fusion comprising SEQ ID NO: 79, 85, 86, 87, 88, 89, 150, 158, 159, 160, or 161. In a further embodiment, the FGFR-fusion is an FGFR-TACC fusion. In some embodiments, the FGFR is FGFR1, FGFR2, FGFR3, or FGFR4. In other embodiments, the FGFR-TACC fusion is FGFR1-TACC1, FGFR2-TACC2, or FGFR3-TACC3.

An aspect of the invention provides for a method for detecting the presence of a FGFR fusion in a human subject. In one embodiment, the method comprises obtaining a biological sample from the human subject. In some embodiments, the sample is incubated with an agent that binds to an FGFR fusion molecule, such as an antibody. In another embodiment, the detection or determination comprises protein expression analysis, for example by western blot analysis, ELISA, or other antibody detection methods. In some embodiments, the method further comprises assessing whether to administer a FGFR fusion molecule inhibitor based on the expression pattern of the subject. In further embodiments, the method comprises administering a FGFR fusion molecule inhibitor to the subject. In another embodiment, the method comprises detecting whether or not there is a FGFR fusion present in the subject. In one embodiment, the detecting comprises measuring FGFR fusion protein levels by ELISA using an antibody directed to SEQ ID NO: 79, 85, 86, 87, 88, 89, 150, 158, 159, 160, or 161; western blot using an antibody directed to SEQ ID NO: 79, 85, 86, 87, 88, 89, 150, 158, 159, 160, or 161; mass spectroscopy, isoelectric focusing, or a combination of the listed methods. In some embodiments, the FGFR-fusion is an FGFR-TACC fusion. In other embodiments, the FGFR is FGFR1, FGFR2, FGFR3, or FGFR4. In other embodiments, the FGFR-TACC fusion is FGFR1-TACC1, FGFR2-TACC2, or FGFR3-TACC3.

An aspect of the invention provides for a method for detecting the presence of a FGFR. fusion in a human subject. In one embodiment, the method comprises obtaining a biological sample from a human subject. In some embodiments, the sample is incubated with an agent that binds to an FGFR fusion molecule, such as a probe, a nucleic acid primer, and the like. In other embodiments, the detection or determination comprises nucleic acid sequencing, selective hybridization, selective amplification, gene expression analysis, or a combination thereof. In some embodiments, the method further comprises assessing whether to administer a FGFR fusion molecule inhibitor based on the expression pattern of the subject. In further embodiments, the method comprises administering a FGFR fusion molecule inhibitor to the subject. In another embodiment, the method comprises detecting whether or not there is a nucleic acid sequence encoding a FGFR fusion protein in the subject. In one embodiment, the nucleic acid sequence comprises any one of SEQ ID NOS: 1-77, 80-84, or 95-145. In another embodiment, the detecting comprises using hybridization, amplification, or sequencing techniques to detect a FGFR fusion. In a further embodiment, the amplification uses primers comprising SEQ ID NO: 162, 163, 164,165, 166, 167, 168, or 169. In some embodiments, the FGFR-fusion is an FGFR-TACC fusion. In other embodiments, the FGFR is FGFR1, FGFR2, FGFR3, or FGFR4. In other embodiments, the FGFR-TACC fusion is FGFR1-TACC1, FGFR2-TACC2, or FGFR3-TACC3.

An aspect of the invention provides for a method of initiating oncogenic transformation *in vitro.* The method comprises (a) transducing cells cultured *in vitro* with FGFR-TACC fusion DNA; and (b) determining whether the cells acquire the ability to grow in anchorage-independent conditions, form multi-layered foci, or a combination thereof.

An aspect of the invention provides for a method of initiating oncogenic transformation *in vivo.* The method comprises (a) transducing cells cultured *in vitro* with FGFR-TACC fusion DNA.; (b) injecting a mouse with the transduced cells; and (c) determining whether a tumor grows in the mouse. In one embodiment, the injecting is a subcutaneous or intracranial injection.

An aspect of the invention provides a method of identifying a compound that decreases the oncogenic activity of a FGFR-TACC fusion. The method comprises (a) transducing a cell cultured *in vitro* with FGFR-TACC DNA (b) contacting a cell with a ligand source for an effective period of time; and (c) determining whether the cells acquire the ability to grow in anchorage-independent conditions, form multi-layered foci, or a combination thereof, compared to cells cultured in the absence of the test compound.

### BRIEF DESCRIPTION OF THE FIGURES

To conform to the requirements for PCT patent applications, many of the figures presented herein are black and white representations of images originally created in color. In the below descriptions and the examples, the colored plots, and images are described in terms of its appearance in black and white. The original color versions can be viewed in Singh et al., Science (2012), 337(6099):1231-5 (including the accompanying Supplementary Information available in the on-line version of the manuscript available on the Science web site). For the purposes of the PCT, the contents of Singh et al., Science (2012), 337(6099):1231-5, including the accompanying "Supplementary Information," are herein incorporated by reference.

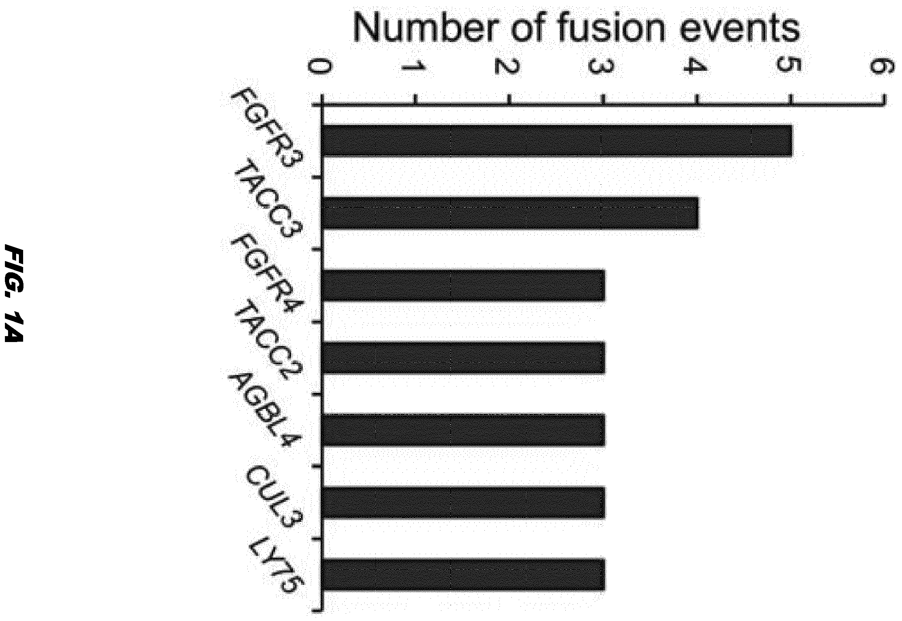

**FIG. 1A** is a graph that shows genes recurrently involved in gene fusions in TCGA. Only genes involved in at least three gene fusions across different samples are displayed.

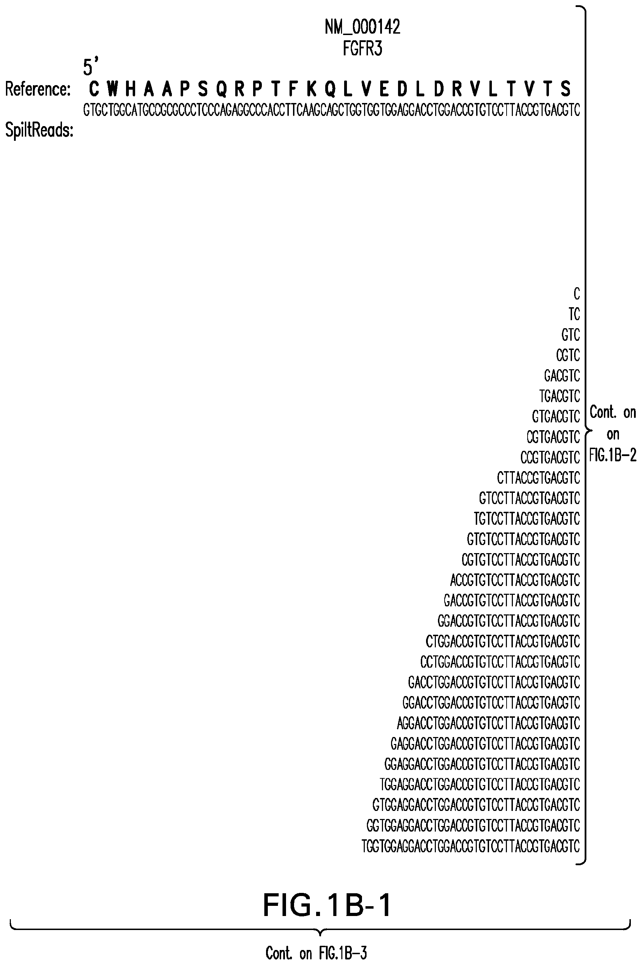

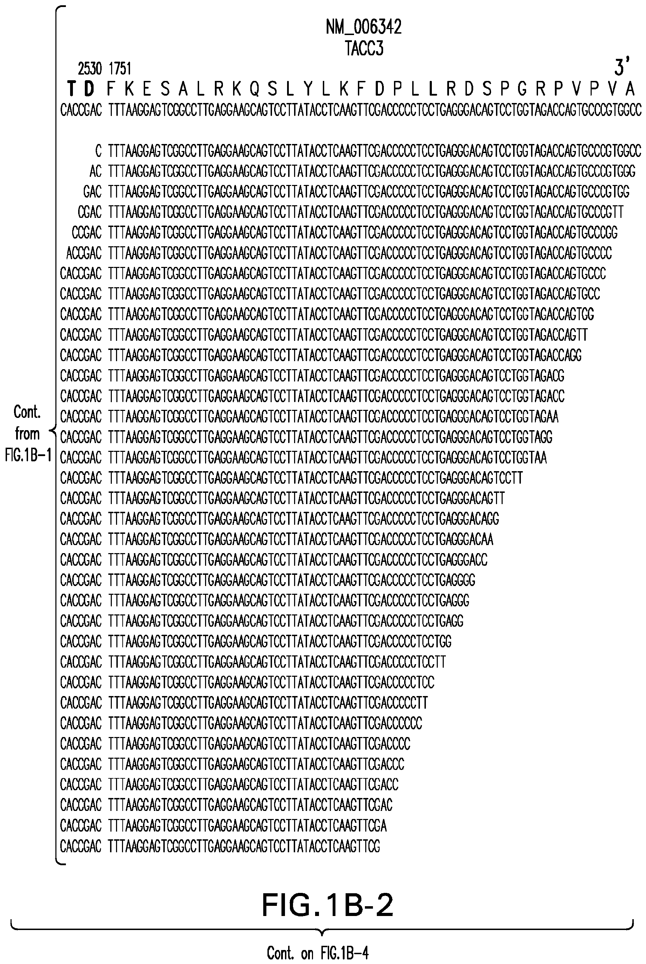

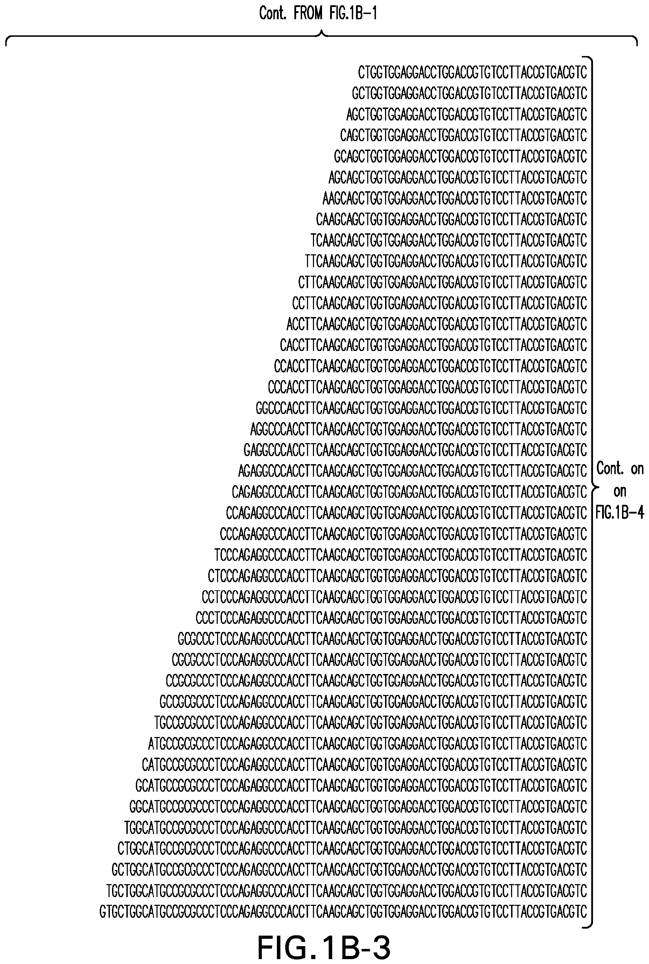

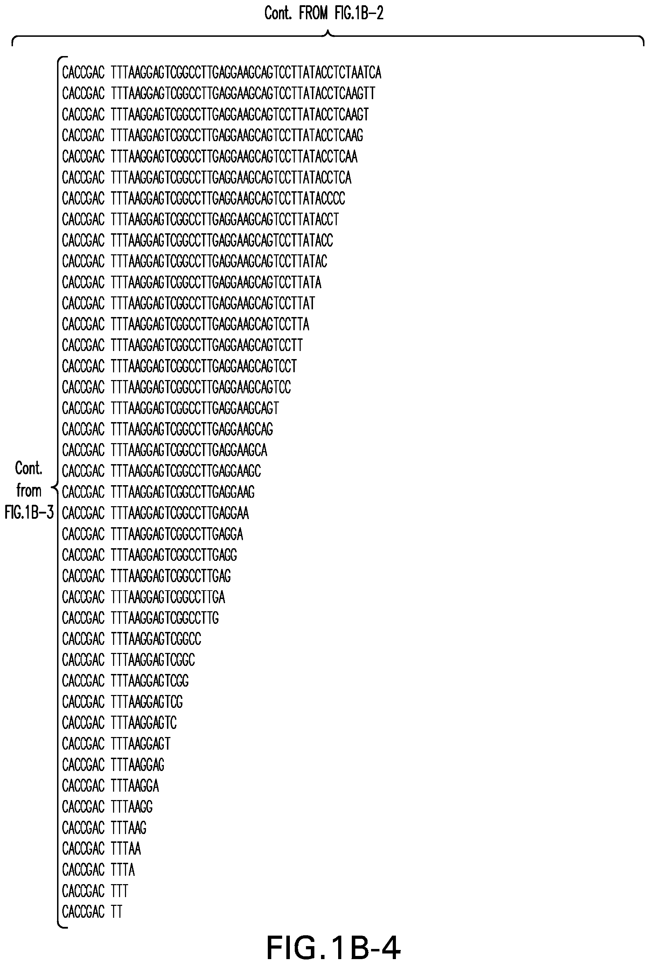

FIG. 1B shows an *FGFR3-TACC3* gene fusion identified by whole transcriptome sequencing of GSCs. 76 split-reads (SEQ ID NOS: 2-77, respectively) are shown aligning on the breakpoint. The predicted reading frame at the breakpoint is shown at the top (FGFR3 nucleotide sequence in purple (left) and TACC3 nucleotide sequence in green (right); SEQ ID NO: 1) with FGFR3 sequences below the predicted reading frame in red (left) and TACC3 in blue (right). The putative amino acid sequence (SEQ ID NO: 78) corresponding to SEQ ID NO: 1 is shown above the predicted reading frame.

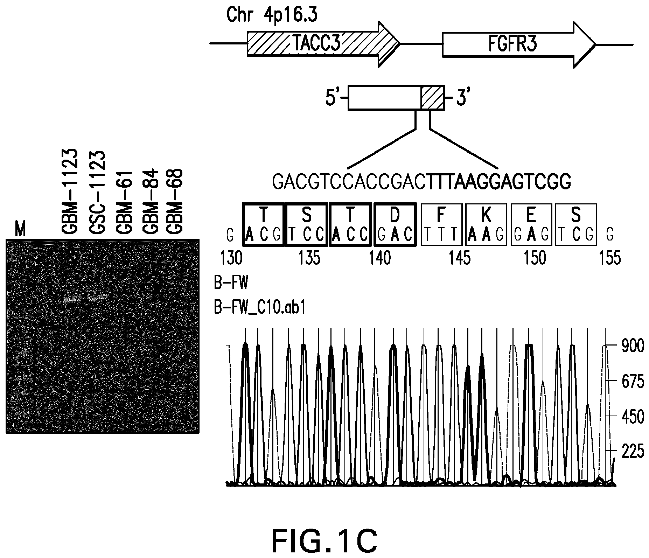

**FIG. 1C** shows an *FGFR3-TACC3* gene fusion identified by whole transcriptome sequencing of GSCs. On the left, *FGFR3-TACC3-*specific PCR from cDNA derived from GSCs and GBM is shown. On the right, Sanger sequencing chromatogram shows the reading frame at the breakpoint (SEQ ID NO: 80) and putative translation of the fusion protein (SEQ ID NO: 85) in the positive samples.

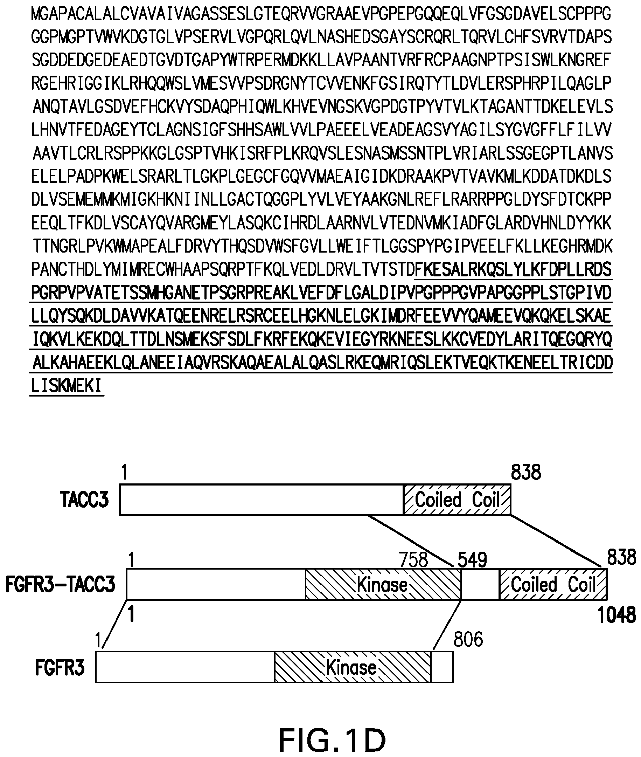

**FIG. 1D** shows an *FGFR3-TACC3* gene fusion identified by whole transcriptome sequencing of GSCs. Amino acid sequence of the FGFR3-TACC3 protein is shown (SEQ ID NO: 79). Residues corresponding to FGFR3 or to TACC3 are shown in green or red (underlined), respectively. The fusion protein joins the tyrosine kinase domain of FGFR3 to the TACC domain of TACC3.

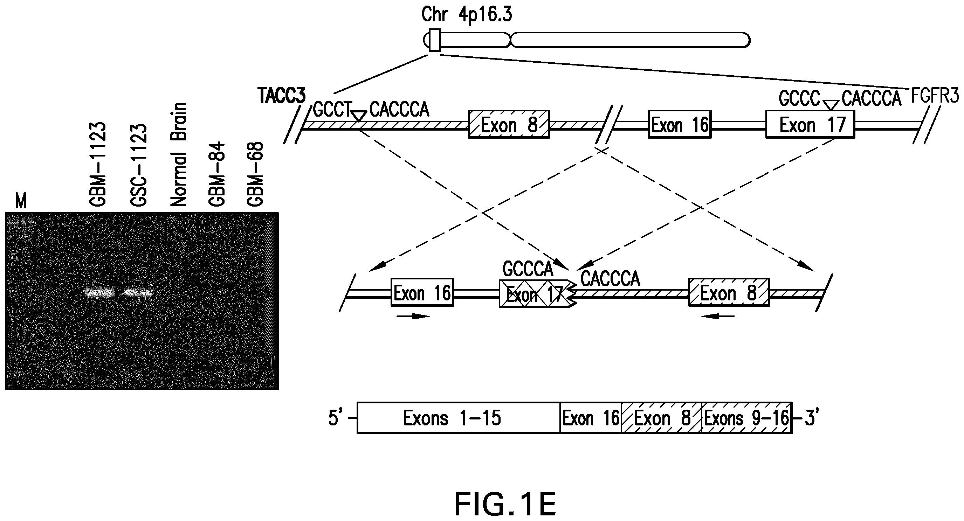

**FIG. 1E** shows an *FGFR3-TACC3* gene fusion identified by whole transcriptome sequencing of GSCs. Genomic fusion of *FGFR3* exon 17 with intron 7 of *TACC3* is shown. In the fused mRNA, exon 16 of *FGFR3* is spliced 5' to exon 8 of *TACC3.* Filled arrows indicate the position of the fusion-genome primers, which generate fusion-specific PCR products in GSC-1123 and GBM-1123.

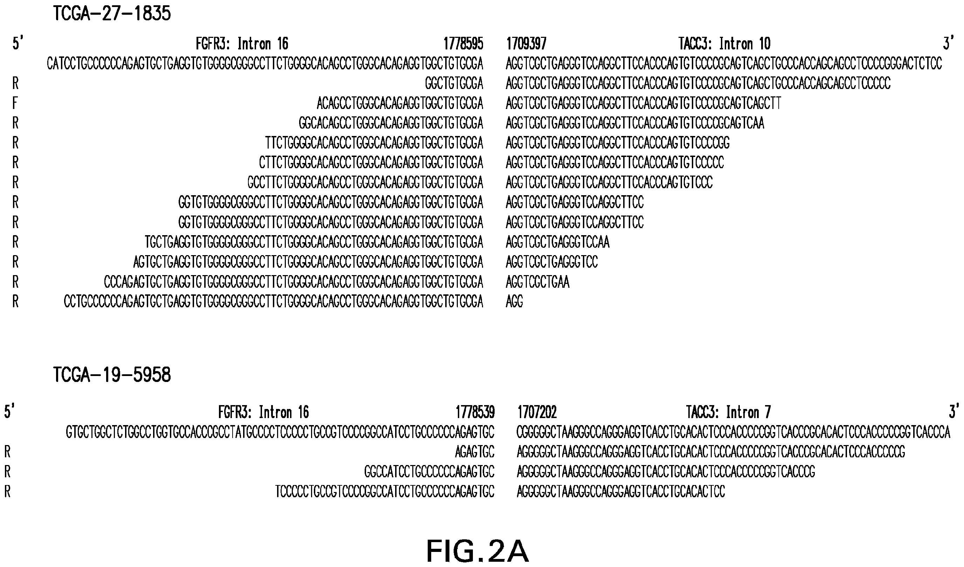

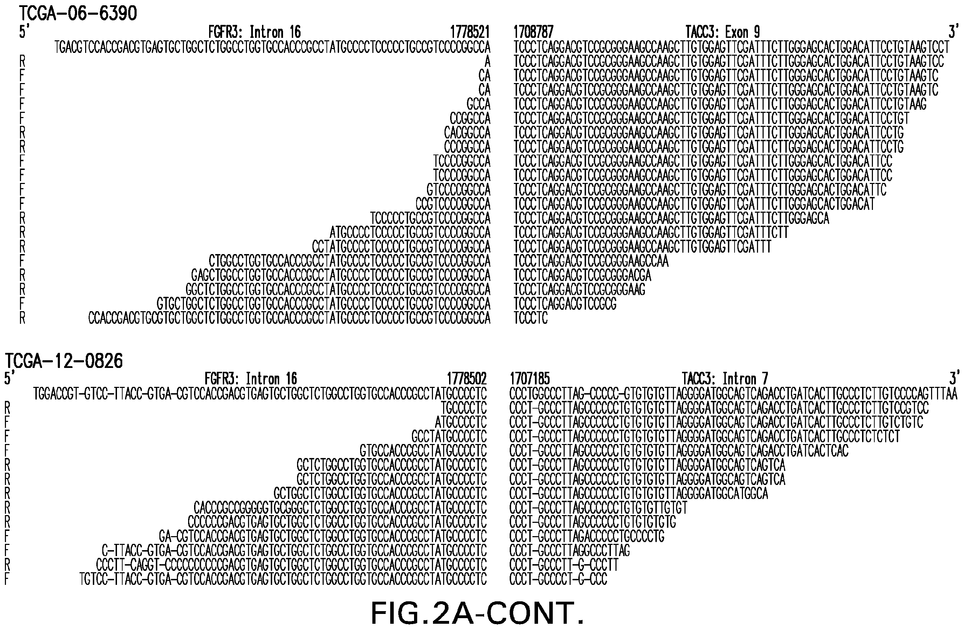

**FIG. 2A** shows recurrent gene fusions between *FGFR* and *TACC* genes in GBM. Specifically, *FGFR3-TACC3* gene fusions are shown that were identified by exome sequencing analysis. Split-reads are shown aligning the genomic breakpoints of *FGFR3* and *TACC3* genes in the four TCGA GBM samples. For TCGA-27-1835, SEQ ID NO: 95 shows the reading frame at the breakpoint (bold), while SEQ ID NOS: 96-107, respectively, show alignments of the genomic breakpoints. For TCGA-19-5958, SEQ ID NO: 108 shows the reading frame at the breakpoint (bold), while SEQ ID NOS: 109-111, respectively, show alignments of the genomic breakpoints. For TCGA-06-6390, SEQ ID NO: 112 shows the reading frame at the breakpoint (bold), while SEQ ID NOS: 113-131, respectively, show alignments of the genomic breakpoints. For TCGA-12-0826, SEQ ID NO: 132. shows the reading frame at the breakpoint (bold), while SEQ ID NOS: 133-145, respectively, show alignments of the genomic breakpoints.

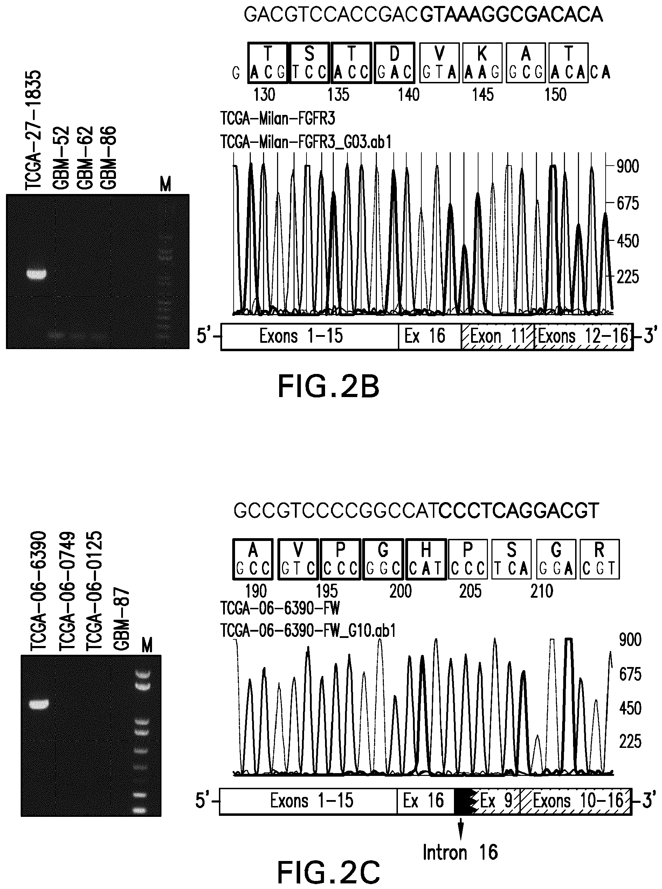

**FIG. 2B** shows recurrent gene fusions between *FCFR* and *TACC* genes in GBM. On the left, a gel of *FGFR-TACC*-specific PCR is shown for *FGFR3-TACC3* from a GBM cDNA sample. On the right, Sanger sequencing chromatograms show the reading frame at. the breakpoint (SEQ ID NO: 81) and putative translation of the fusion protein (SEQ ID NO: 86) in the positive samples.
**FIG. 2C** shows recurrent gene fusions between *FGFR* and *TACC* genes in GBM. On the left, a gel of *FCFR-TACC-*specific PCR is shown for *FGFR3-TACC3* from a GBM cDNA sample. On the right, Sanger sequencing chromatograms show the reading frame at the breakpoint (SEQ ID NO: 82) and putative translation of the fusion protein (SEQ ID NO: 87) in the positive samples.

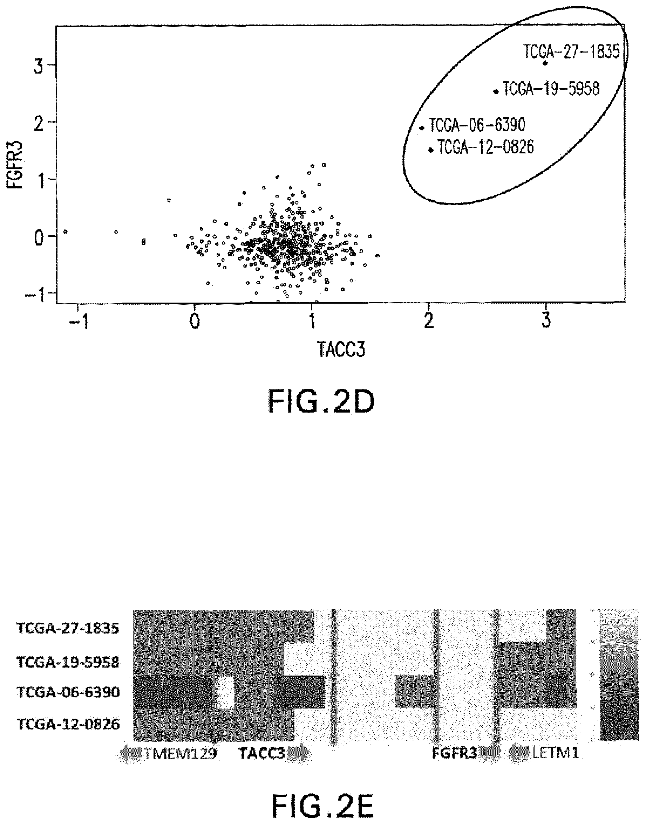

**FIG. 2D** shows recurrent gene fusions between *FGFR* and *TACC* genes in GBM. Co-outlier expression of *FGFR3* and *TACC3* in four GBM tumors from Atlas-TCGA is shown in the plot.
**FIG. 2E** shows recurrent gene fusions between *FGFR* and *TACC* genes in GBM. CNV analysis shows micro-amplifications of the rearranged portions of the *FGFR3* and *TACC3* genes in the same four Atlas-TCGA GBM samples.

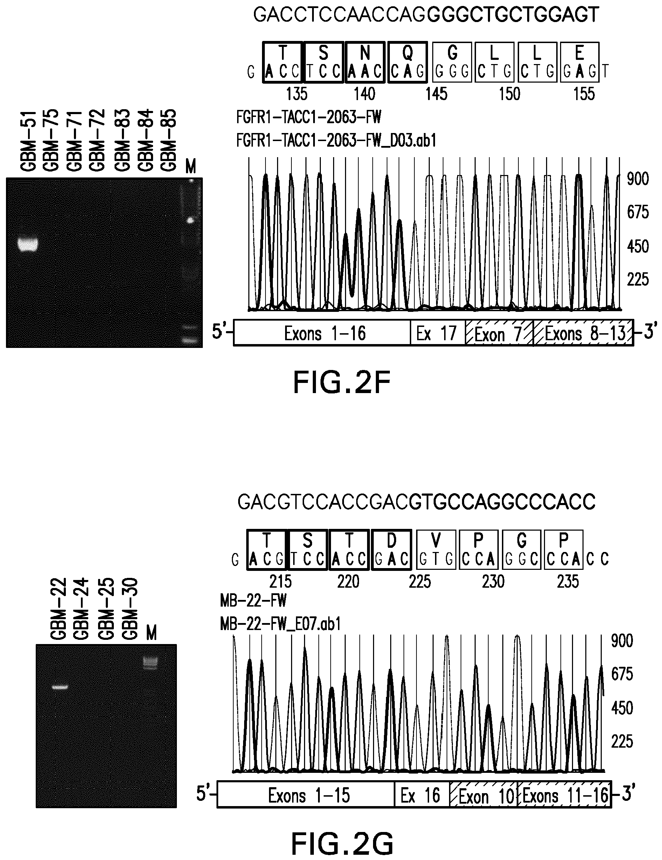

**FIG. 2F** shows recurrent gene fusions between *FGFR* and *TACC* genes in GBM. On the left, a gel of *FGFR-TACC*-specific PCR is shown for *FGFR1-TACC1* from a GBM cDNA sample. On the right, Sanger sequencing chromatograms show the reading frame at the breakpoint (SEQ ID NO: 83) and putative translation of the fusion protein (SEQ ID NO: 88) in the positive samples.
**FIG. 2G** shows recurrent gene fusions between *FGFR* and *TACC* genes in GBM. On the left, a gel of *FGFR-TACC*-specific PCR is shown for *FGFR3-TACC3* from a GBM cDNA sample. On the right, Sanger sequencing chromatograms show the reading frame at the breakpoint (SEQ ID NO: 84) and putative translation of the fusion protein (SEQ ID NO: 89) in the positive samples.

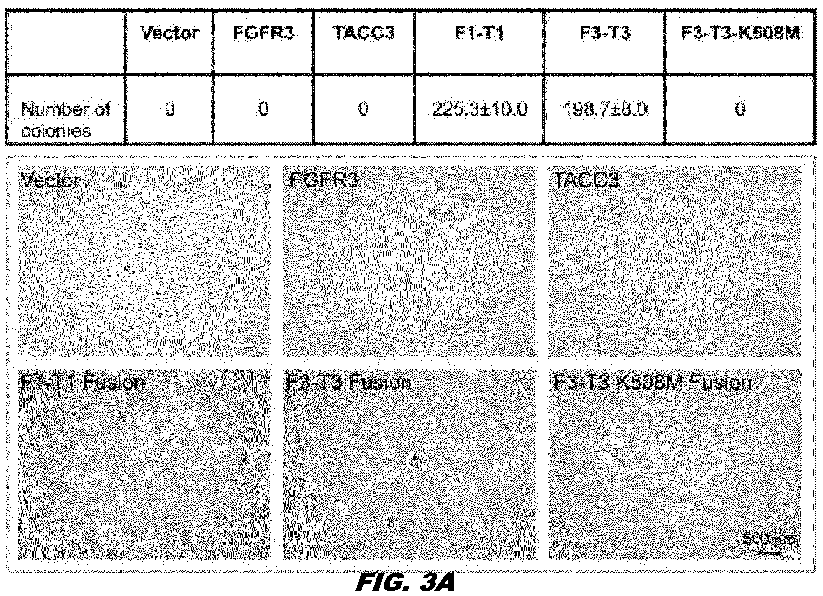

**FIG. 3A** shows transforming activity of FGFR-TACC fusion proteins. FGFR1-TACC1 and FGFR3-TACC3 induce anchorage-independent growth in Rail A fibroblasts. The number of soft agar colonies was scored from triplicate samples 14 days after plating. Representative microphotographs are shown.

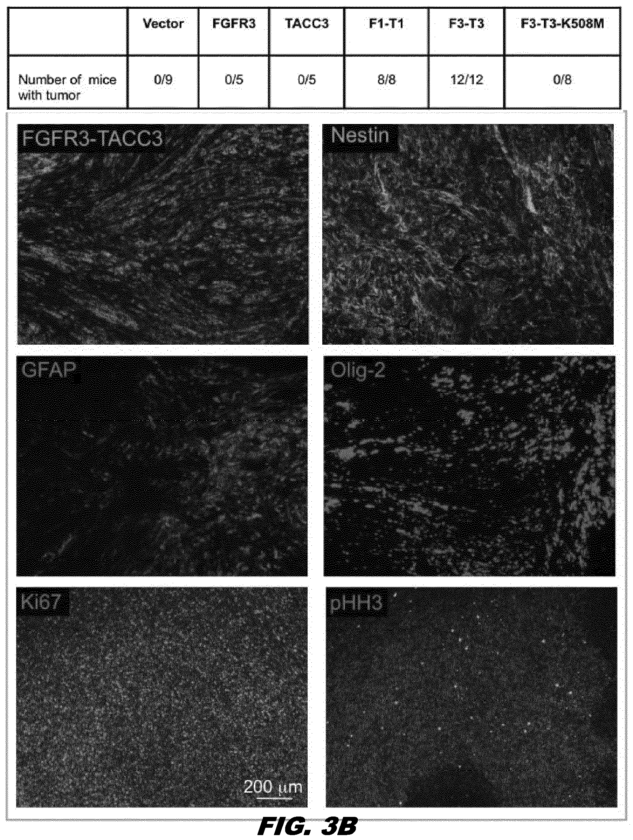

**FIG. 3B** are photomicrographs showing of immunofluoresence staining of tumors from mice injected with *Ink4A;Arf-*/*-* astrocytes expressing FGFR3-TACC3 showing positivity for glioma-specific (Nestin, Oig2 and GFAP) and proliferation markers (Ki67 and pHH3). Sub-cutaneous tumors were generated by *Ink4A;Arf-*/*-* astrocytes expressing FGFR-TACC fusions.

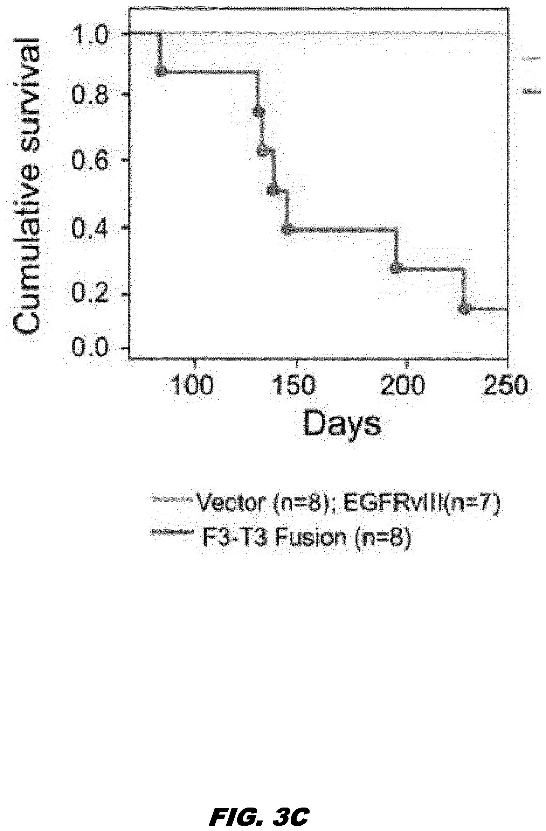

**FIG. 3C** shows Kaplan-Meier survival curves of mice injected intracranially with pTomo-shp53 (n = 8) or pTomo-EGFRvIII-shp53 (n= 7) (green line; "light grey" in black and white image) and pTomo-FGFR3-TACC3-shp53 (n = 8, red line; "dark grey" in black and white image). Points on the curves indicate deaths (log-rank test, p = 0.00001, pTomo-shp53 vs. pTomo-FGFR3-TACC3-shp53).

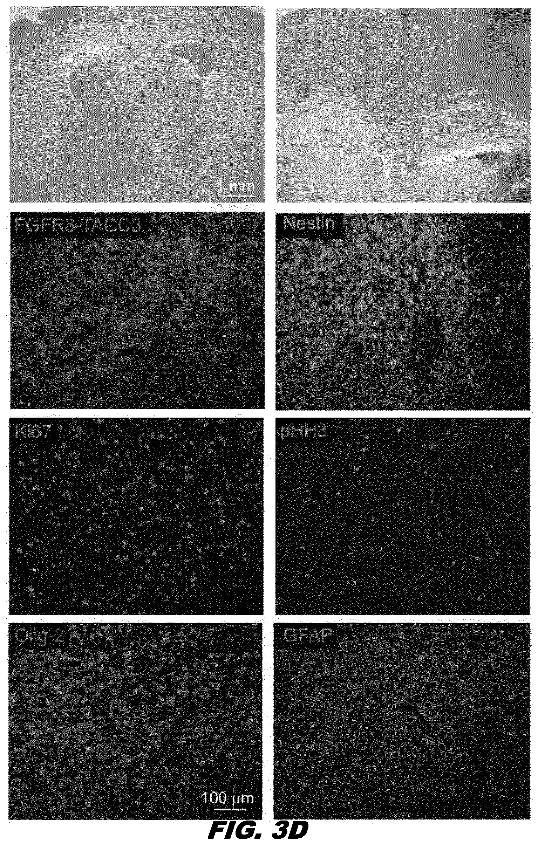

**FIG. 3D** shows representative photomicrographs of Hematoxylin and Eosin staining of advanced FGFR3-TACC3-shp53 generated tumors showing histological features of high-grade glioma. Of note is the high degree of infiltration of the normal brain by the tumor cells. Immunofluorescence staining shows that glioma and stem cell markers (Nestin, Olig2 and GFAP), the proliferation markers (Ki67 and pHH3) and the FGFR3-TACC3 protein are widely expressed in the FGFR3-TACC3-shp53 brain tumors. F1-T1: FGFR1-TACC1; F3-T3: FGFR3-TACC3; F3-T3-K508M: FGFR3-TACC3-K508M.

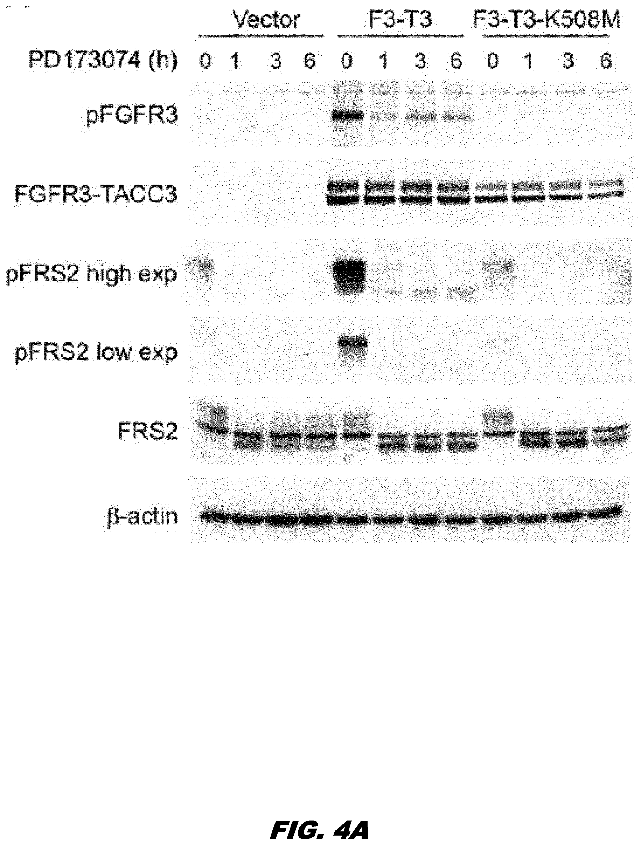

**FIG. 4A** shows that FGFR3-TACC3 localizes to spindle poles, delays mitotic progression and induces chromosome segregation defects and aneuploidy Constitutive auto-phosphorylation of FGFR3-TACC3 fusion. *Ink4A;Arf-*/*-* astrocytes transduced with empty lentivirus or a lentivirus expressing FGFR3-TACC3 or FGFR3-TACC3-K508M were left untreated (0) or treated with 1 00 nM of the FGFR inhibitor PD173074 for the indicated times. Phospho-proteins and total proteins were analyzed by Western blot using the indicated antibodies.

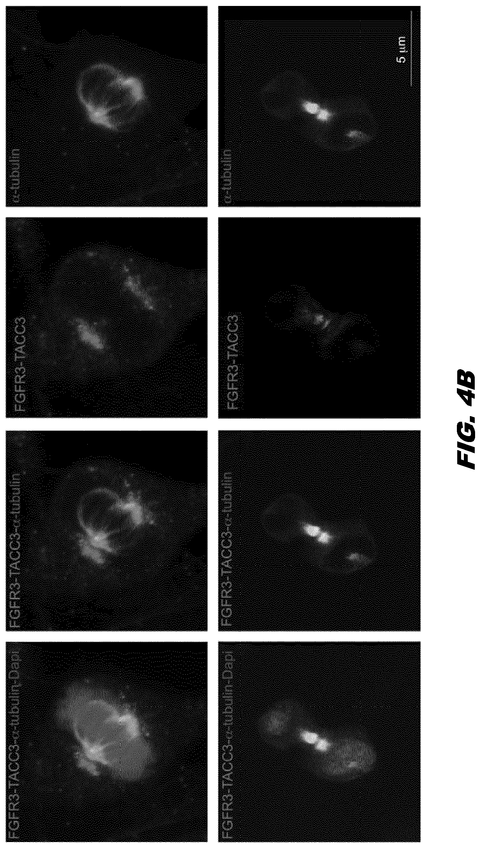

**FIG. 4B** shows that FGFR3-TACC3 localizes to spindle poles, delays mitotic progression and induces chromosome segregation defects. Photomicrographs are shown of confocal microscopy analysis of FGFR3-TACC3 in *Ink4A;Arf-*/*-* astrocytes. Maximun intensity projection of z-stacked images shows FGFR3-TACC3 (red; "dark grey" in black and white image) coating the spindle poles of a representative mitotic cell (upper panels). In telophase (lower panels) FGFR3-TACC3 localizes to the mid-body. α-tubulin (green; "grey" in black and white image), DNA (DAPI, blue; "light grey" in black and white image).

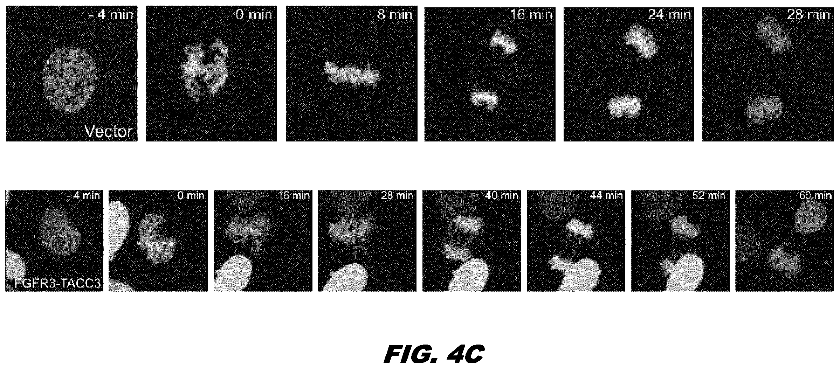

**FIG. 4C** shows representative fluorescence video-microscopy for cells transduced with vector or FGFR3-TACC3.

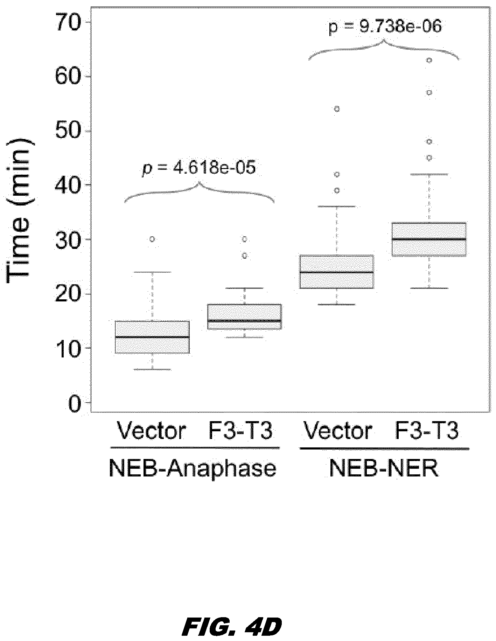

**FIG. 4D** shows a Box and Whisker plot representing the analysis of the time from nuclear envelope breakdown (NEB) to anaphase onset and from NEB to nuclear envelope reconstitution (NER). The duration of mitosis was measured by following 50 mitoses for each condition by time-lapse microscopy.

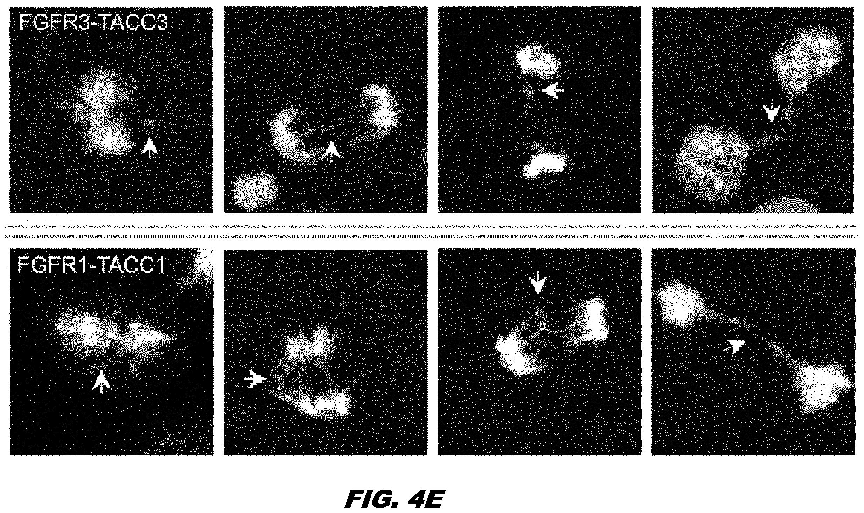

**FIG. 4E** shows that FGFR3- TACC3 localizes to spindle poles, delays mitotic progression and induces chromosome segregation defects. Representative images are shown of cells with chromosome missegregation. Arrows point to chromosome misalignments, lagging chromosomes, and chromosome bridges.

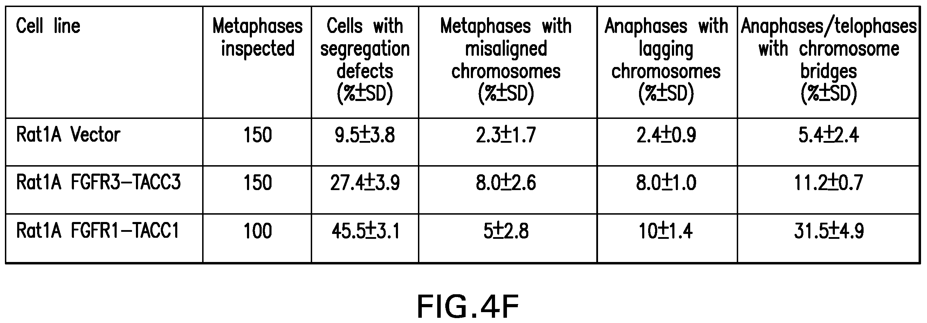

**FIG. 4F** shows quantitative analysis of segregation defects in Rat1A expressing FGFR1-TACC1 and FGFR3-TACC3. F3-T3: FGFR3-TACC3; F3-T3-K508M: FGFR3-TACC3-K508M.

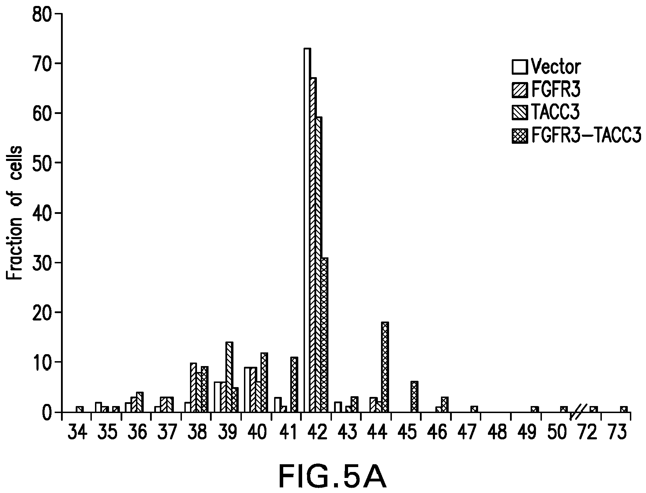

**FIG. 5A** shows karyotype analysis of Rat1A cells transduced with control, FGFR3, TACC3 or FGFR3-TACC3 expressing lentivirus. Distribution of chromosome counts of cells arrested in mitosis and analyzed for karyotypes using DAPI. Chromosomes were counted in 100 metaphase cells for each condition to determine the ploidy and the diversity of chromosome counts within the cell population. FGFR3-TACC3 fusion induces aneuploidy.

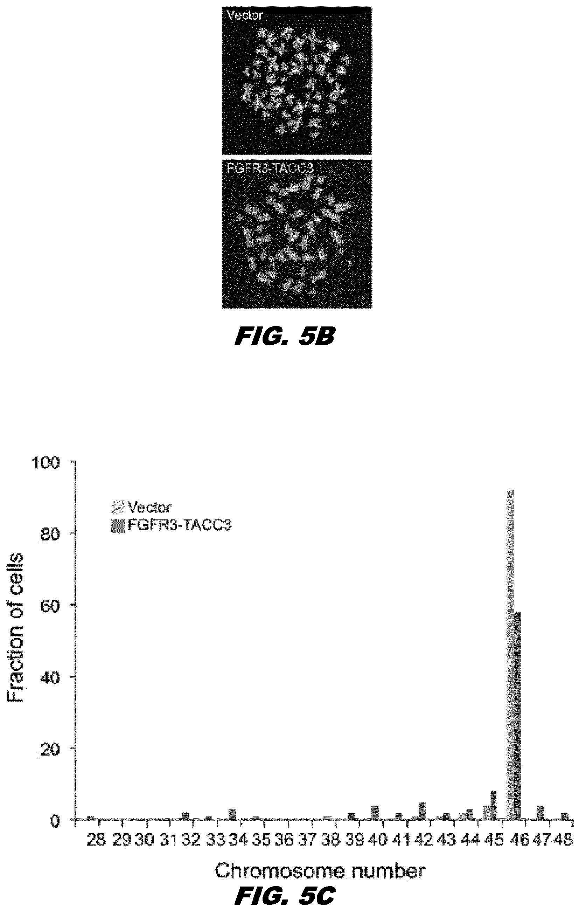

**FIG. 5B** shows representative karyotypes and FIG. 5C shows distribution of chromosome counts of human astrocytes transduced with control or FGFR3-TACC3 expressing lentivirus. Chromosomes were counted in 100 metaphase cells for each condition to determine the ploidy and the diversity of chromosome counts within the cell population.

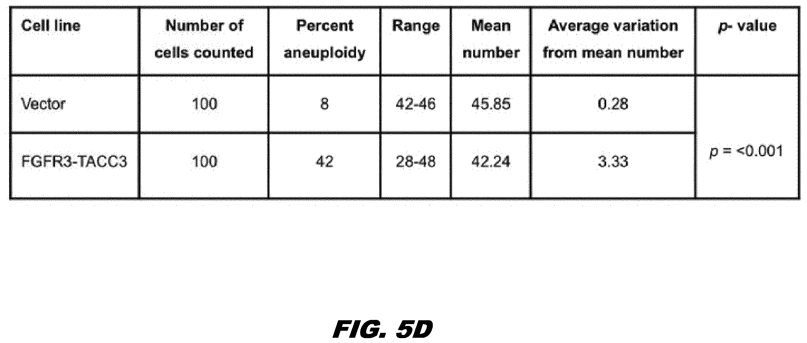

**FIG. 5D** shows quantitative analysis of chromosome number in 100 metaphase cells for each condition to determine the ploidy and the diversity of chromosome counts within the cell population. (n=3 independent experiments).

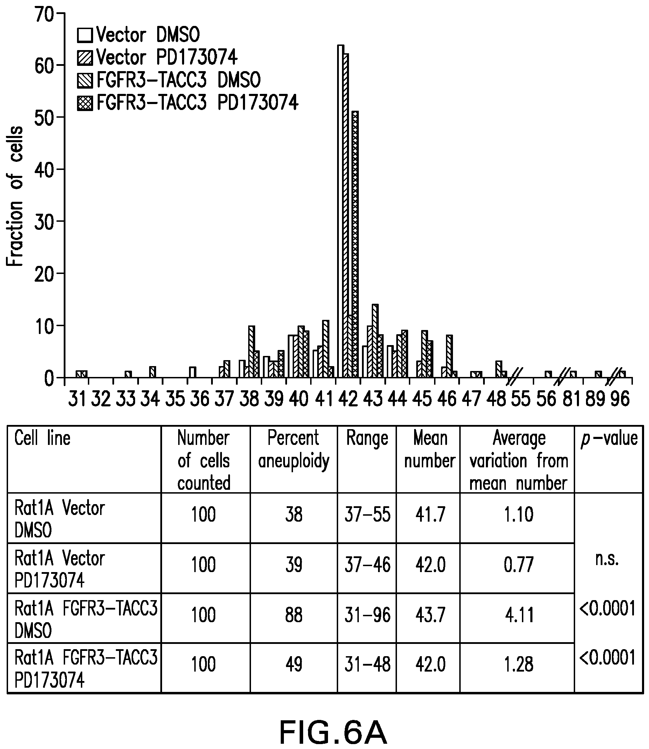

**FIG. 6A** shows inhibition of FGFR-TK activity corrects the aneuploidy initiated by FGFR3-TACC3. The upper panel is a karyotype analysis of Rat1A cells transduced with control or FGFR3-TACC3 lentivirus and treated with vehicle (DMSO) or PD173470 (100 nM) for five days. The lower panel shows the ploidy and the diversity of chromosome counts within the cell population were determined by quantitative analysis of chromosome number in 100 metaphase cells for each condition.

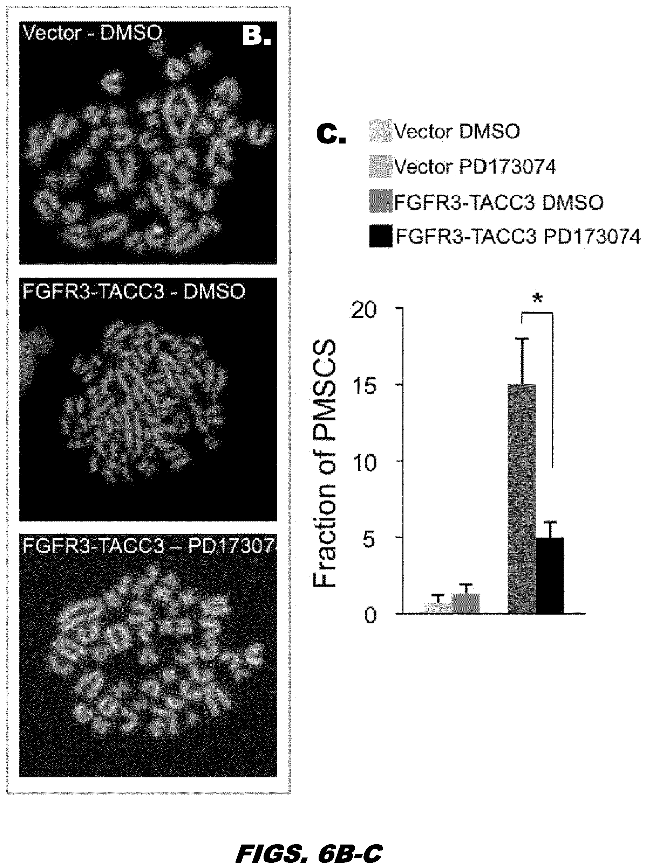

**FIG. 6B** shows inhibition of FGFR-TK activity corrects the aneuploidy initiated by FGFR3-TACC3. Correction of premature sister chromatid separation (PMSCS) by PD173470 in cells expressing FGFR3-TACC3. Panels show representative metaphase spreads. DNA was stained by DAPI. **FIG. 6C** shows quantitative analysis of metaphases with loss of sister chromatid cohesion (n=3; p = 0.001, FGFR3-TACC3 treated with DMSO vs. FGFR3-TACC3 treated with PD173470).

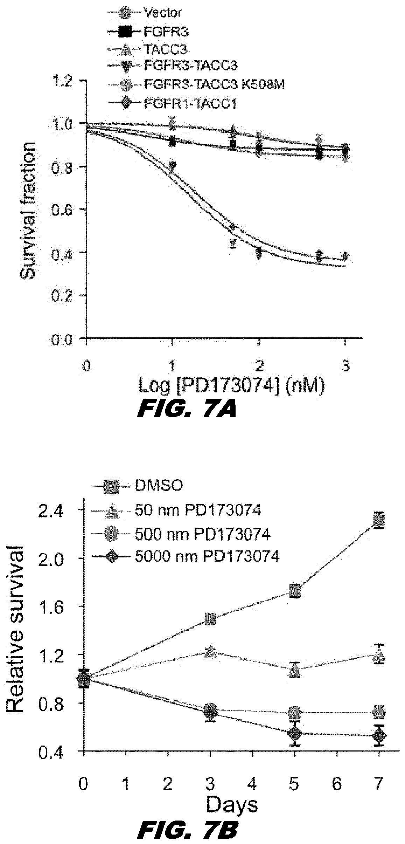

**FIG. 7A** shows inhibition of FGFR-TK activity suppresses tumor growth initiated by FGFR3-TACC3. Growth rate of Rat1A transduced with the indicated lentiviruses and treated for three days with increasing concentrations of PD1 73074. Cell growth was determined by the MTT assay. Data are presented as the means±standard error (n=4).
**FIG. 7B** shows the growth rate of GSC-1123 treated with PD173470 at the indicated concentrations for the indicated times. Cell growth was determined by the MTT assay. Data are presented as the means±standard error (n=4).

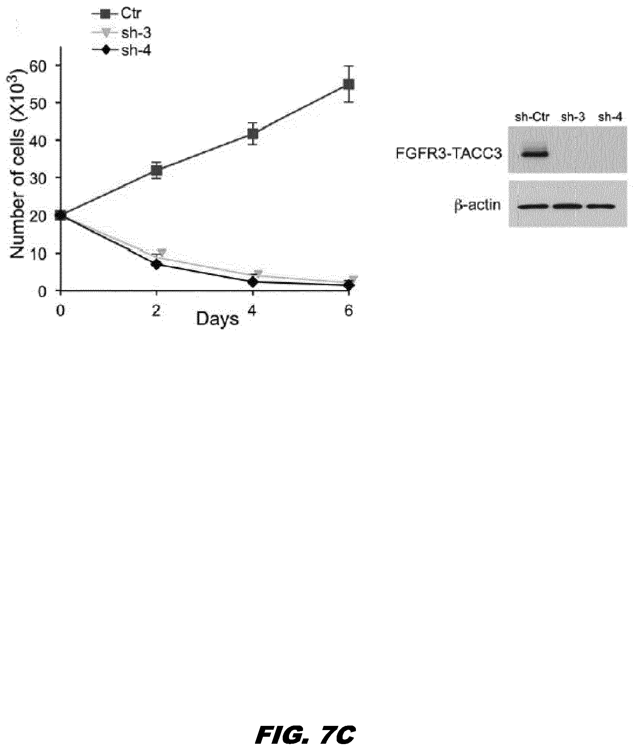

**FIG. 7C** shows the growth inhibitory effect of silencing FGFR3-TACC3 fusion. At the left, parallel cultures of GSC-1123 cells were transduced in triplicate. Rat1A cells expressing FGFR3-TACC3 fusion were transduced with lentivirus expressing a non-targeting shRNA (Ctr) or shRNA sequences targeting FGFR3 (sh2, sh3, sh4). Five days after infection cells were plated at density of 2×10⁴ cells/well in triplicate and the number of trypan blue excluding cells was scored at the indicated times. Infection with lentivirus expressing sh-3 and sh-4, the most efficient FGFR3 silencing sequences reverted the growth rate of FGFR3-TACC3 expressing cultures to levels comparable to those of Rat1A transduced with empty vector. Values are the means ± standard deviation (n = 3). At th right sided figure, GSC-. 1123 cells were transduced with lentivirus expressing a non-targeting shRNA (sh-Ctr) or lentivirus expressing sh-3 and sh-4 sequences targeting FGFR3. Western Blot analysis was performed on parallel cultures using the FGFR3 antibody to the detect FGFT3-TACC3 fusion protein. β-actin is shown as a control for loading.

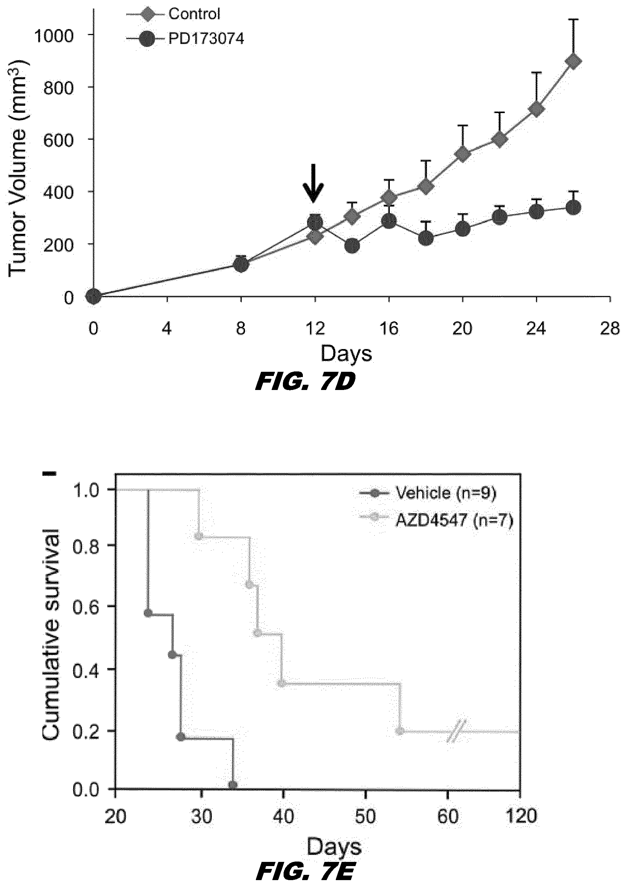

**FIG. 7D** shows that the FGFR inhibitor PD173074 suppresses tumor growth of glioma sub-cutaneous xenografts generated by *Ink4A;Arf-*/*-* astrocytes expressing FGFR3-TACC3. After tumor establishment (200-300 mm³, arrow) mice were treated with vehicle or PD173074 (50 mg/kg) for 14 days. Values are mean tumor volumes ± standard error (n = 7 mice per group).
**FIG. 7E** is a Kaplan-Meier analysis of glioma-bearing mice following orthotopic implantation of *Ink4A;Arf-*/*-* astrocytes transduced with FGFR3-TACC3. After tumor engraftment mice were treated with vehicle (n=9) or AZD4547 (50 mg/kg) (n=7) for 20 days (*p* = 0.001).

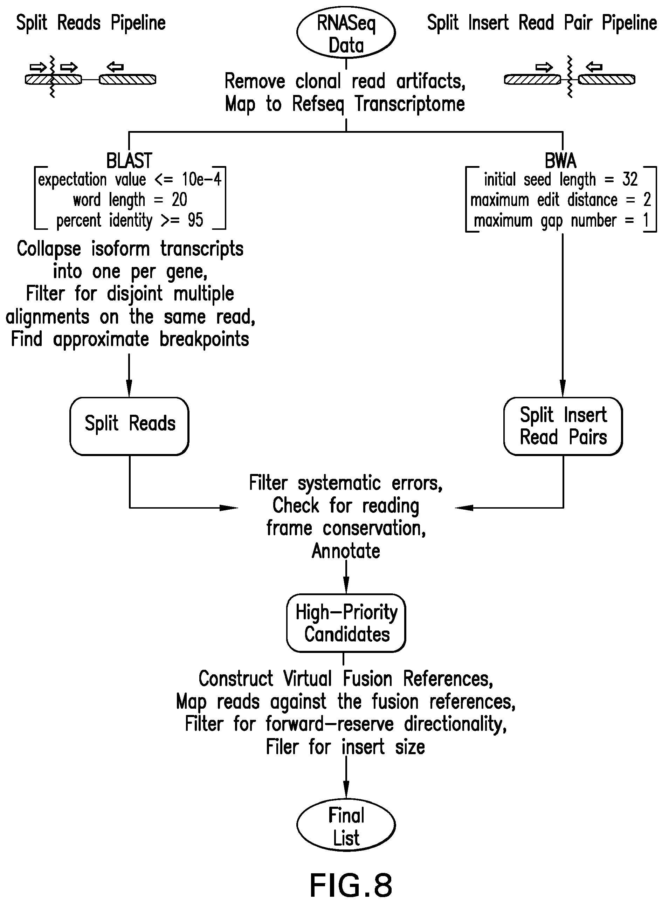

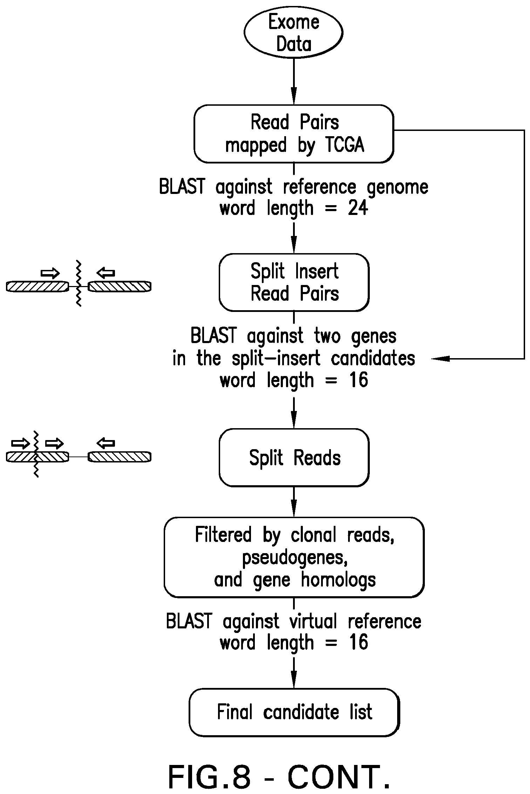

**FIG. 8** shows a schematic of the TX-Fuse pipeline for the identification of fusion transcripts from RNA-Seq data generated from nine GSC cultures. The continued figure shows a schematic of the Exome-Fuse pipeline for the identification of gene fusion rearrangements from DNA exome sequences of 84 GBM TCGA tumor samples.

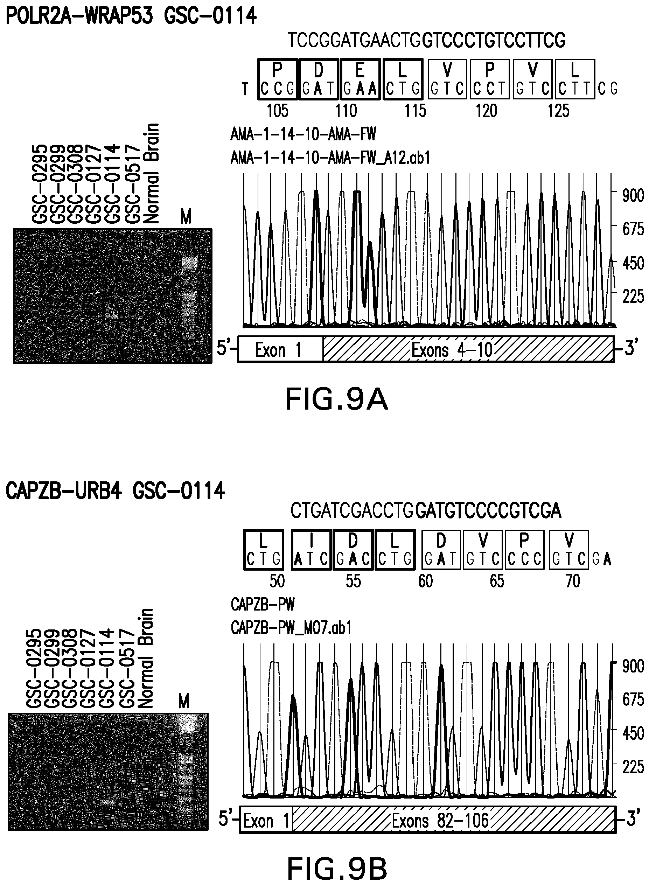

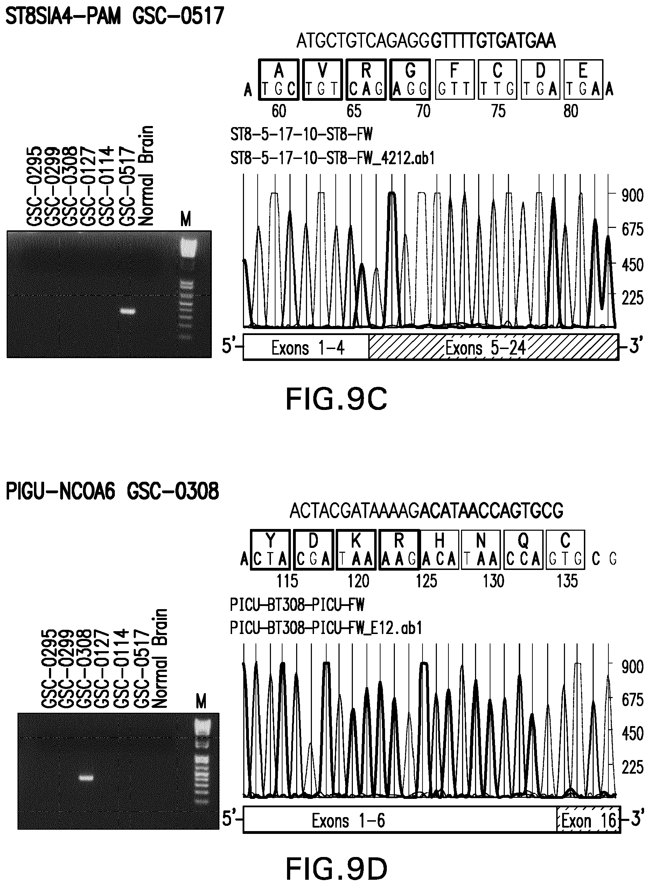

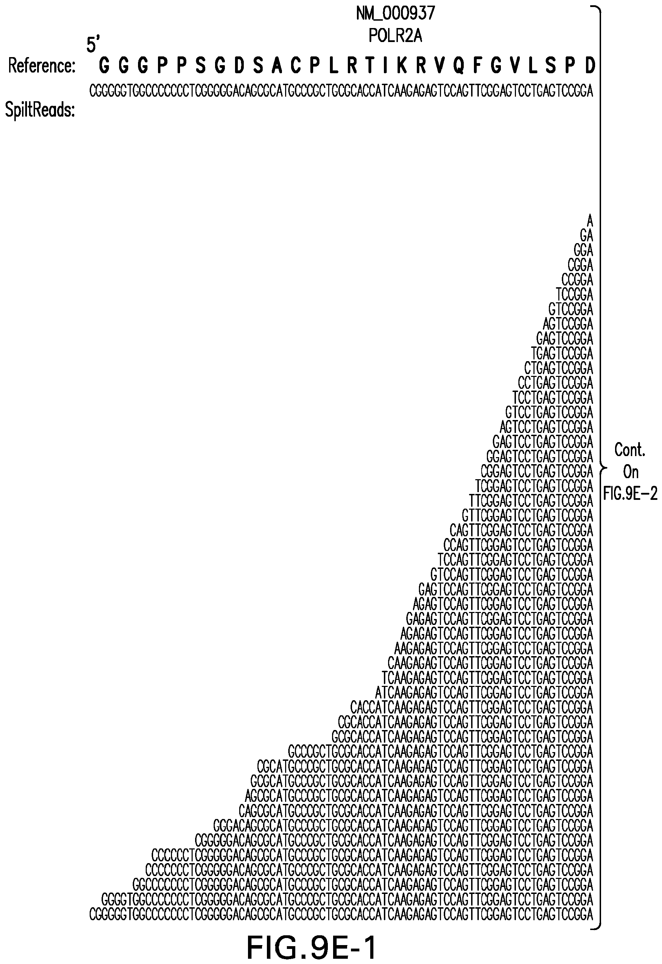

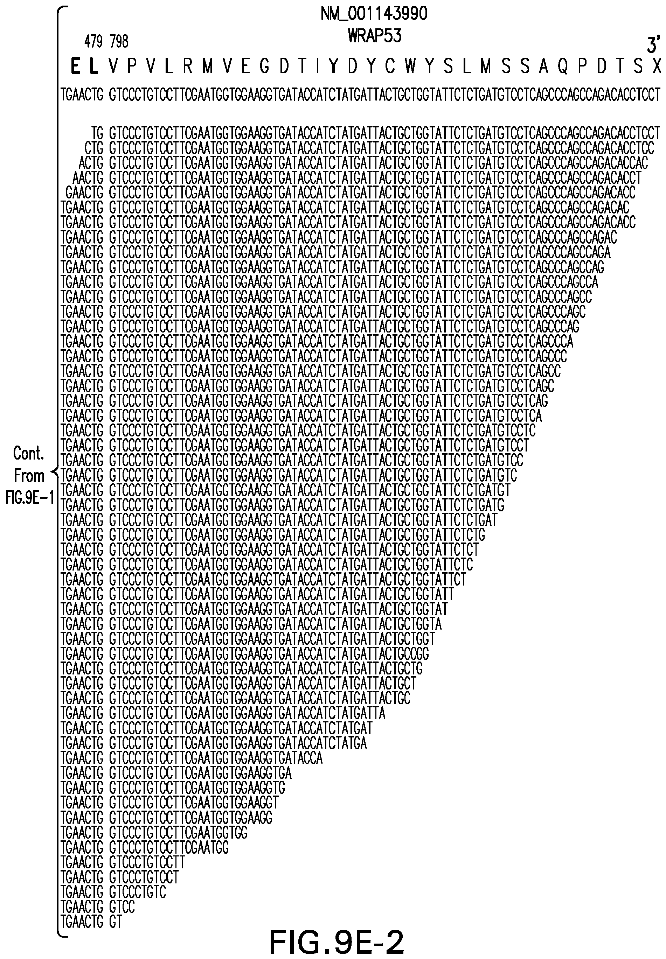

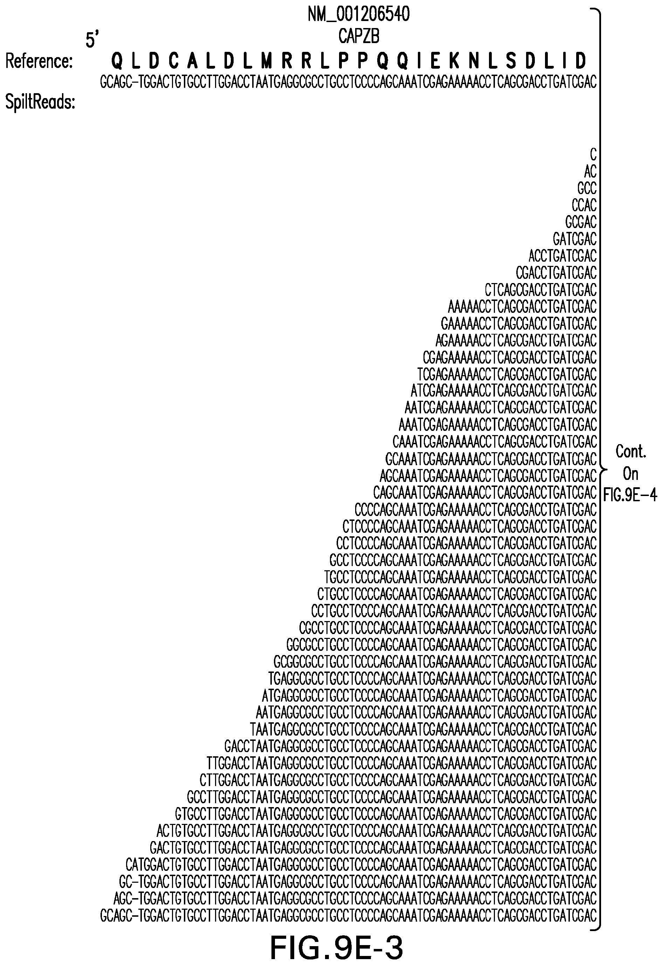

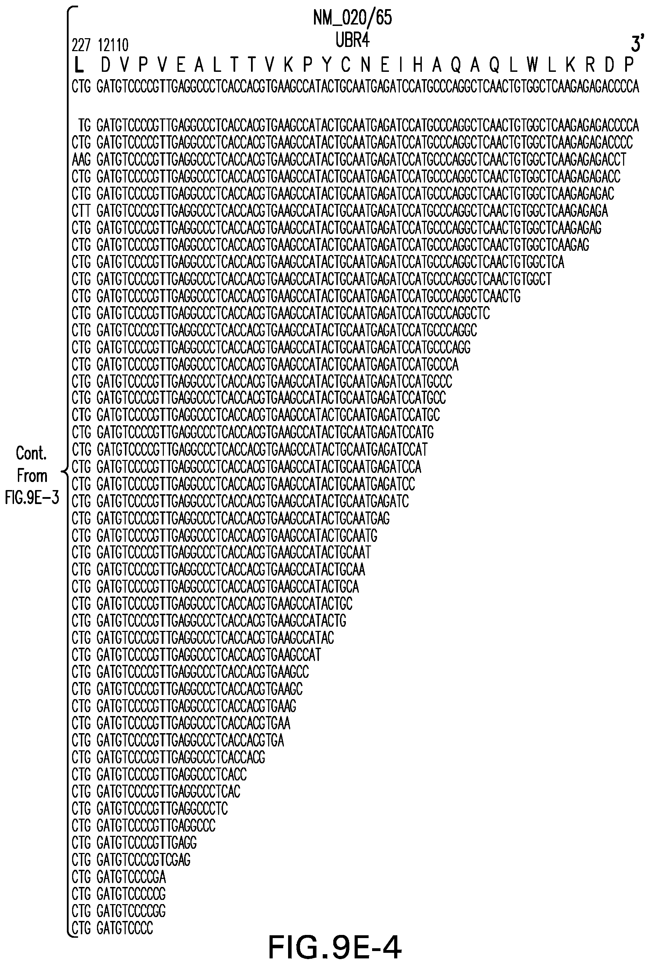

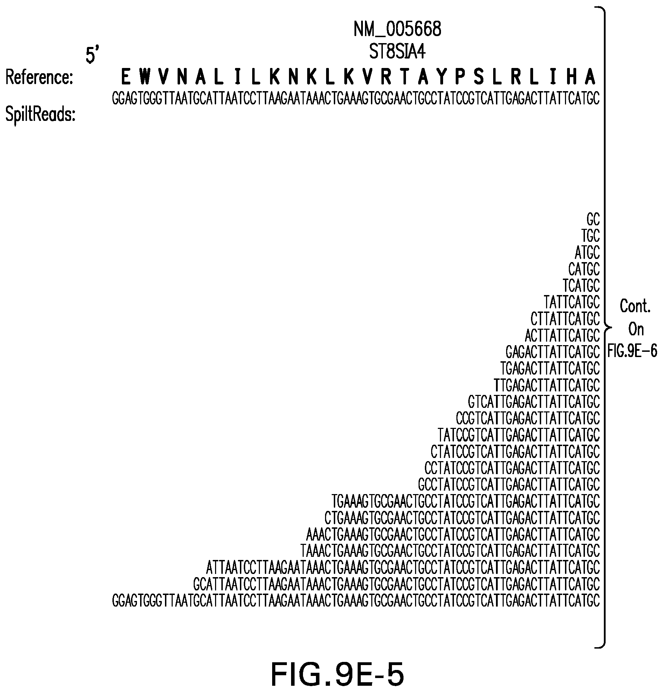

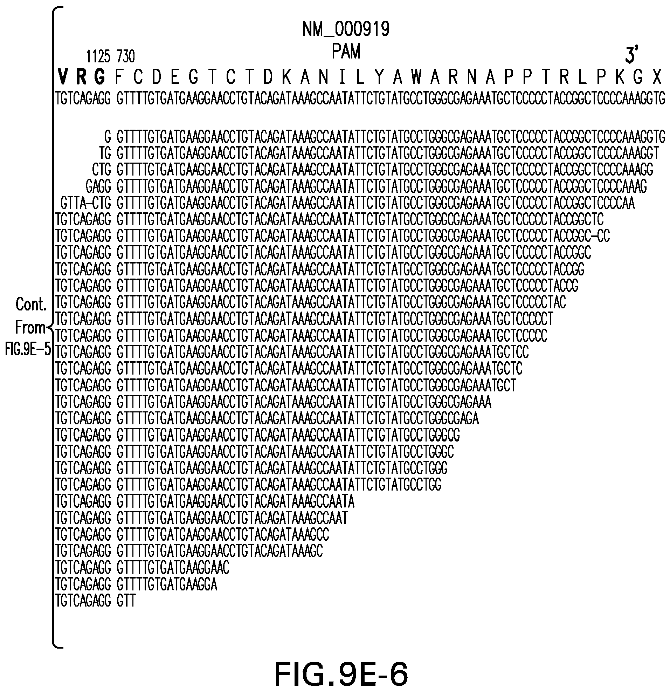

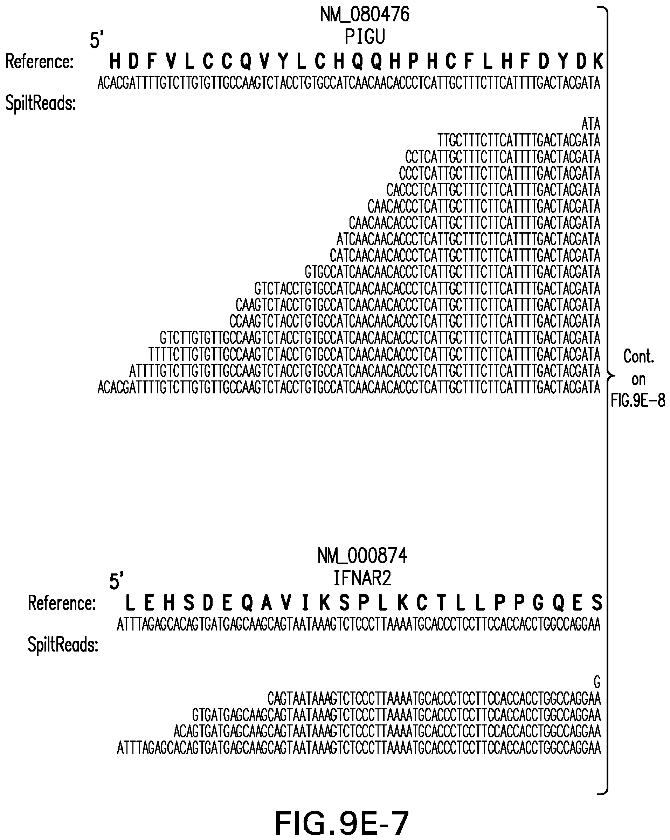

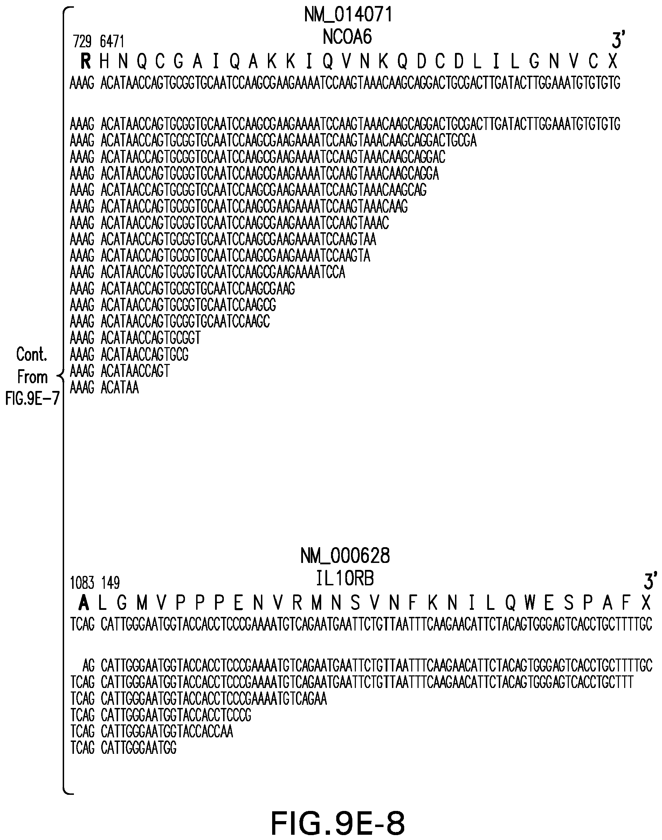

**FIG. 9** shows the validation of fusion transcripts identified by RNA-seq of nine GSCs. Sanger sequencing chromatograms show the reading frames at the breakpoint and putative translation of the fusion proteins in the positive samples (right side). The left side shows gels of RT-PCR conducted. (**A**) POLR2A-WRAP53. (**B**) CAPZB-UBR4. (C) ST8SIA4-PAM. (**D**) PIGU-NCOA6.
FIG. 9E shows the fusion transcripts identified by whole transcriptome sequencing of nine GSCs. 54 split-reads are shown aligning on the breakpoint of the POLR2A-WRAP53 fusion. The predicted reading frame at the breakpoint is shown at the top with POLR2A sequences in red (left) and WRAP53 in blue (right). On the continued page, 48 split-reads are shown aligning on the breakpoint of the CAPZB-UBR4 fusion. The predicted reading frame at the breakpoint is shown at the top with CAPZB sequences in red (left) and UBR4 in blue (right). On the continued page after, 29 split-reads are shown aligning on the breakpoint of the ST8SIA4--PAM fusion. The predicted reading frame at the breakpoint is shown at the top with ST8SIA4 sequences in red (left) and PAM in blue (right). On the subsequent continued page, 17 split-reads are shown (top) aligning on the breakpoint of the PIGU-NCOA6 fusion. The predicted reading frame at the breakpoint is shown at the top with PIGU sequences in red (left) and NCOA6 in blue (right). Also (below), 6 split-reads are shown aligning on the breakpoint of the IFNAR2-IL1 0RB fusion. The predicted reading frame at the breakpoint is shown at the top with IFNAR2 sequences in red (left) and IL10RD in blue (right).

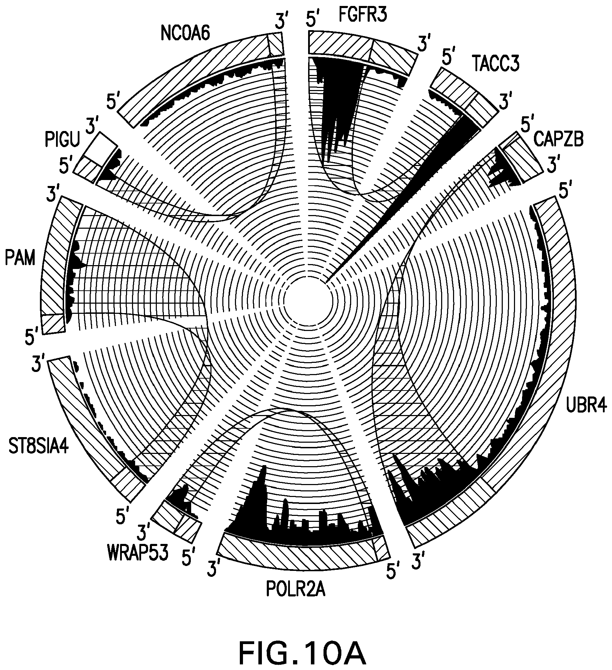

**FIG. 10A** shows the analysis and validation of the expression of fused transcripts in GSCs and GBM sample. Expression measured by read depth from RNA-seq data. Light grey arcs indicate predicted components of transcripts fused together. Overall read depth (blue; "grey" in black and white image) and split insert depth (red; "dark grey" in black and white image) are depicted in the graph, with a 50-read increment and a maximum range of 1800 reads. Note the very high level of expression in the regions of the genes implicated in the fusion events, particularly for FGFR3-TACC3.

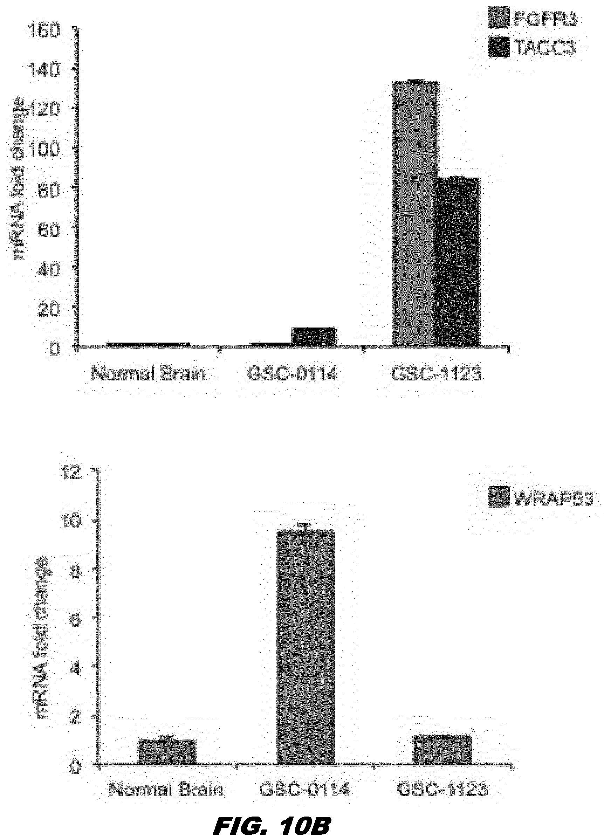

**FIG. 10B** shows the analysis and validation of the expression of fused transcripts in GSCs and GBM sample. Top panel, qRT-PCR showing the very high expression of FGFR3 and TACC3 mRNA sequences included in the FGFR3-TACC3 fusion transcript in GSC-1123. Bottom panel, for comparison the expression of sequences of WRAP53 mRNA included in the POLR2A-WPLAP53 fusion in GSC-0114 is also shown.
**FIG. 10C** shows the expression of the FGFR3-TACC3 protein in GSC-1123 and GBM-1123. Western blot analysis with a monoclonal antibody, which recognizes the N-terminal region of human FGFR3 shows expression of a∼150 kD protein in GSC-1123 but not in the GSC cultures GSC-0331 and GSC-01 14, which lack the FGFR3-TACC3 rearrangement.

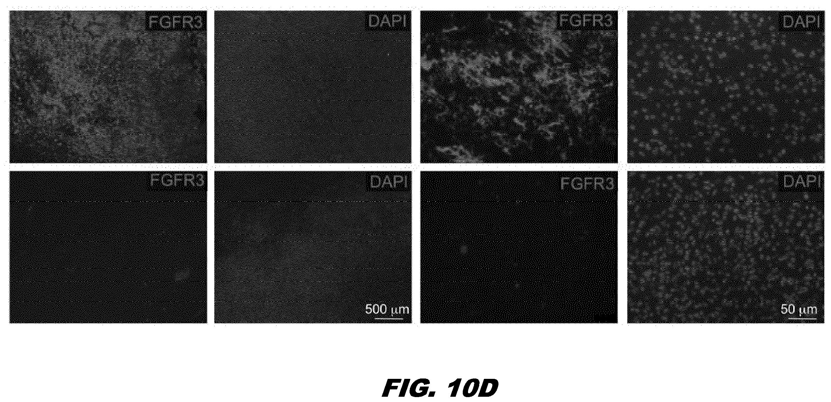

**FIG. 10D** shows the analysis and validation of the expression of fused transcripts in GSCs and GBM sample. Immunostaining analysis with the FGFR3 antibody of the tumor CiBM-1 123 (top panel) and a GBM tumor lacking the FGFR3-TACC3 rearrangement. FGFR3 (red; "light grey" in black and white image), DNA (DAPI, blue; "grey" in black and white image). The pictures were taken at low (left) and high (right) magnification.

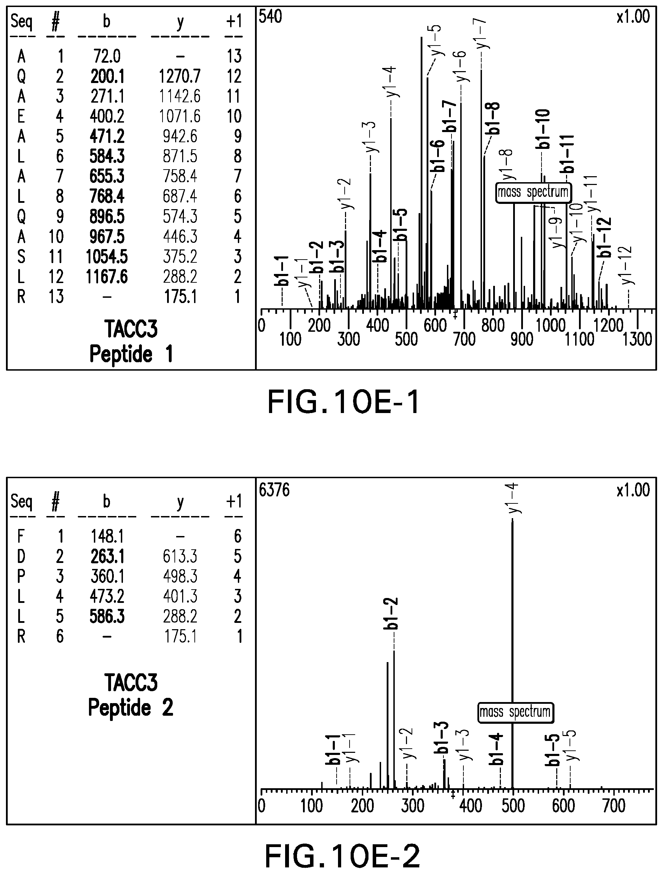

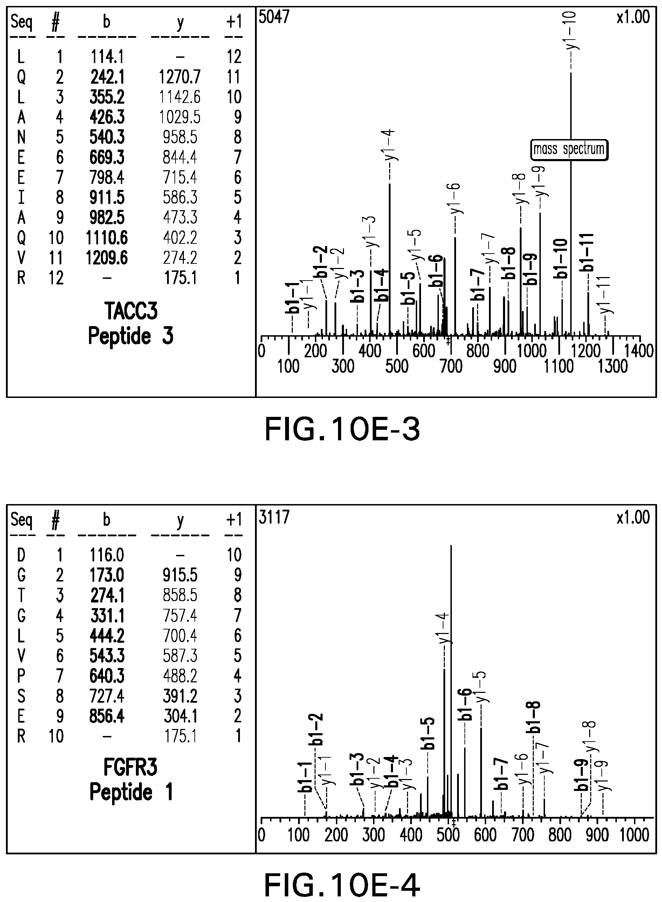

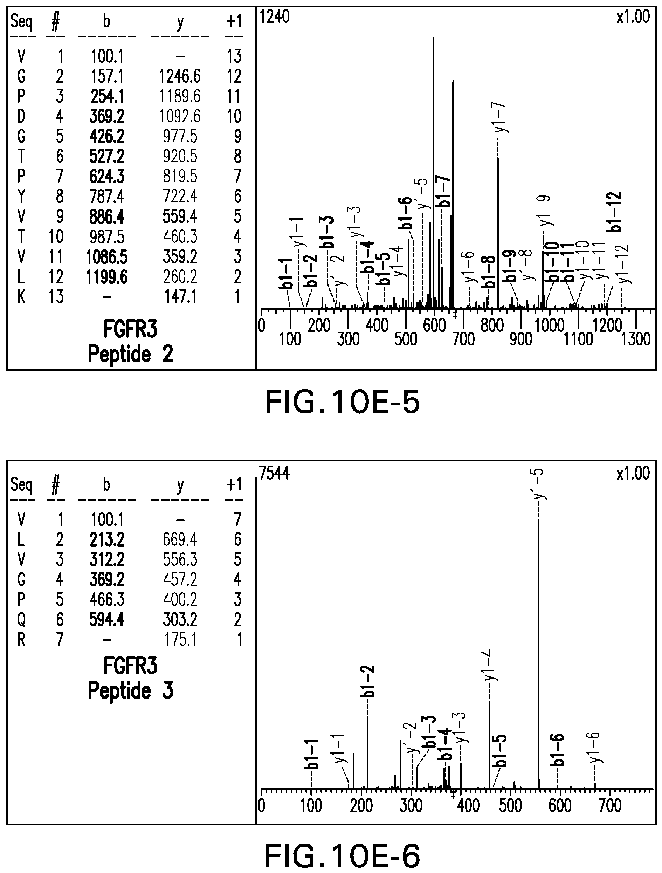

FIG. 10E shows MS/MS analysis of the ∼150 kD fusion protein immunoprecipitated by the monoclonal anti-FGFR3 antibody from GSC-1123, identifying three unique peptides mapping to the FGFR3 (FGFR3 Peptide 1, 2, and 3) and three peptides mapping to the C-terminal region of TACC3 (TACC Peptide 1, 2, and 3).

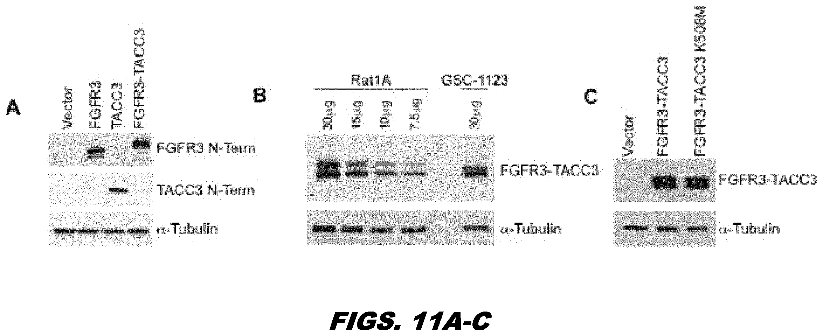

**FIGS. 11A****∼C** shows Rat1A cells transduced with control lentivirus or letivurus expressing FGFR3, TACC3, FGFR3-TACC3 (**FIG. 11A**) that were analyzed by Western blot with an antibody recognizing the N-terminus of FGFR3 (included in the FGFR3-TACC3 fusion protein) or the N-terminus of TACC3 (not included in the FGFR3-TACC3 fusion protein). **FIG. 11B** shows quantitative Western blot analysis of endogenous FGFR3-TACC3 in GSC-1123 compared with lentivirally expressed FGFR3-TACC3 in Rat1A **FIG. 11C** shows Western blot analysis of FGFR3-TACC3 and FGFR3-TACC3-K508M in Rat1A. α-tubulin is shown as a control for loading.

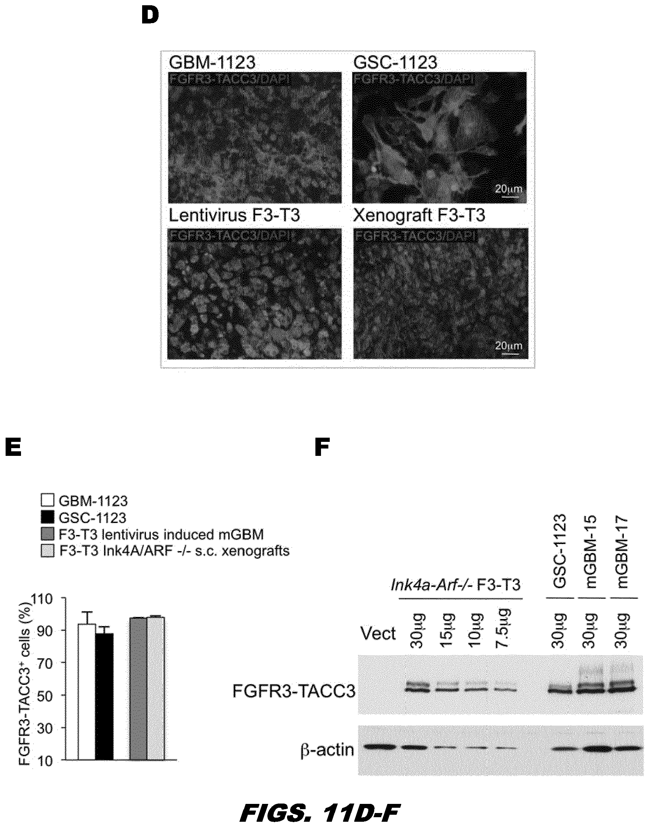

FIGS. 11D-F shows expression analyses of FGFR3-TACC3 fusion construct (**FIG. 11D**) FGFR3 immunostaining of GBM-1123 (left, upper panel), BTSC1123 (right, upper panel), mouse GBM induced by FGFR3-TACC3 expressing lentivirus (left, lower panel), and sub-cutaneous xenograft of mouse astrocytes transformed by FGFR3-TACC3 fusion (right, lower panel); FGFR3-TACC3, red ("light grey" in black and white image); DNA (DAPI), blue ("grey" in black and white image). **FIG. 11E** shows quantification of FGFR3-TACC3 positive cells in the tumors and cultures of cells shown in **FIG. 11D. FIG. 11F** shows a quantitative Western blot analysis of ectopic FGFR3-TACC3 fusion protein in mouse astrocytes and FGFR3-TACC3 induced mouse GBM (mGBM-15 and mGBM-17) compared with the endogenous expression in GBM1123. β-actin is shown as a control for loading. F3-T3: FGFR3-TACC3. α-tubulin or β-actin is shown as a control for loading.

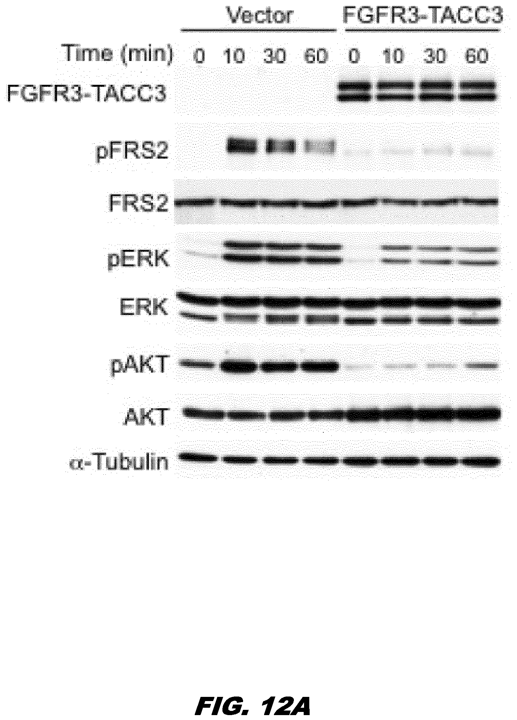

**FIG. 12A** shows a western blot. *Ink4A;Arf-*/*-* astrocytes transduced with empty lentivirus or a lentivirus expressing FGFR3-TACC3 were starved of mitogens and left untreated (time 0) or treated with FGF-2 at concentration of 50 ng/ml for the indicated times. Phospho-proteins and total proteins were analyzed by Western blot using the indicated antibodies, α-tubulin is shown as a control for loading.

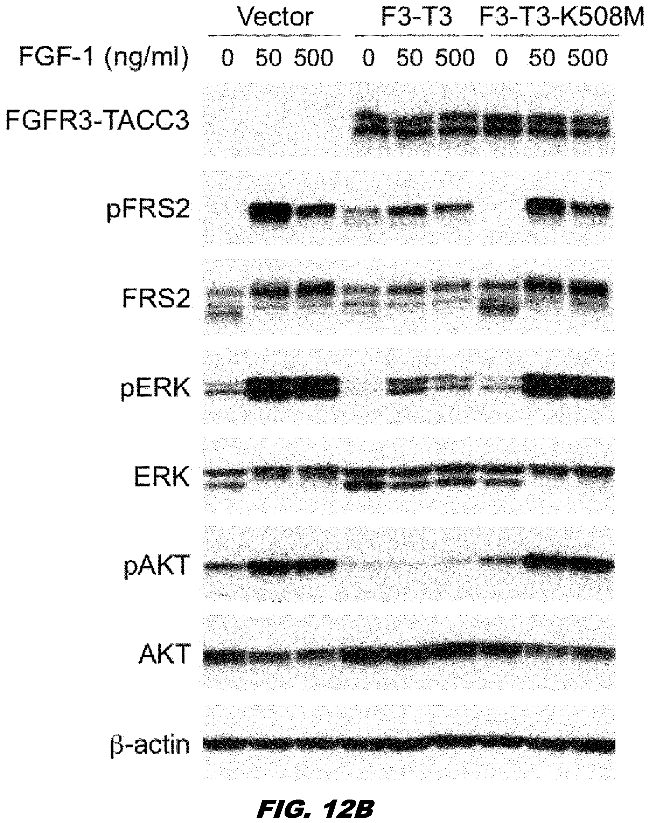

**FIGS. 12B** show western blots. *Ink4A;Arf-*/*-* astrocytes transduced with empty lentivirus or a lentivirus expressing FGFR3-TACC3 or FGFR3-TACC3-K508M were starved of mitogens and left untreated (time 0) or treated for 10 min with FGF-1 at the indicated concentrations. Phospho-proteins and total proteins were analyzed by Western blot using the indicated antibodies. β-actin is shown as a control for loading.

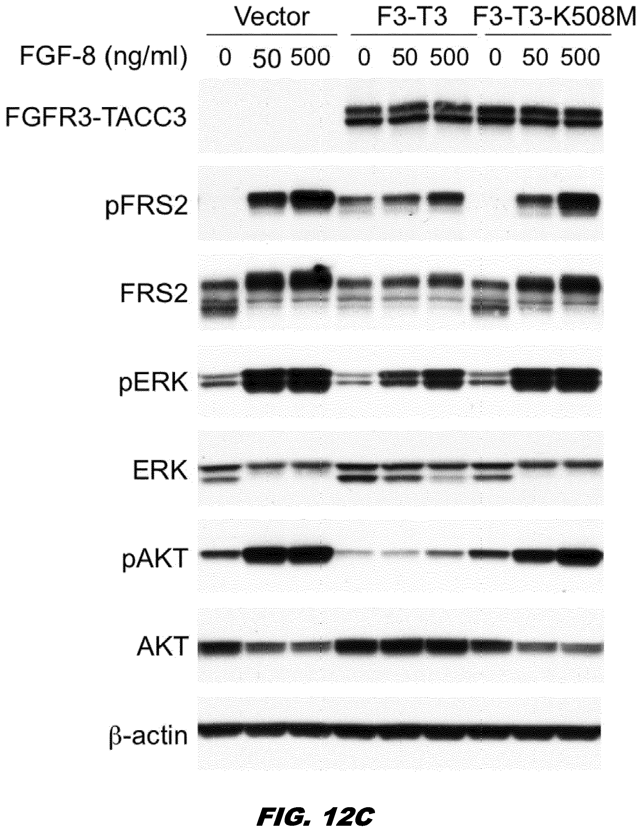

**FIG. 12C** show western blots. *Ink4A;Arf-*/*-* astrocytes transduced with empty lentivirus or a lentivirus expressing FGFR3-TACC3 or FGFR3-TACC3-K508M were starved of mitogens and left untreated (time 0) or treated for 10 min with FGF-8 at the indicated concentrations. Phospho-proteins and total proteins were analyzed by Western blot using the indicated antibodies, β-actin is shown as a control for loading.

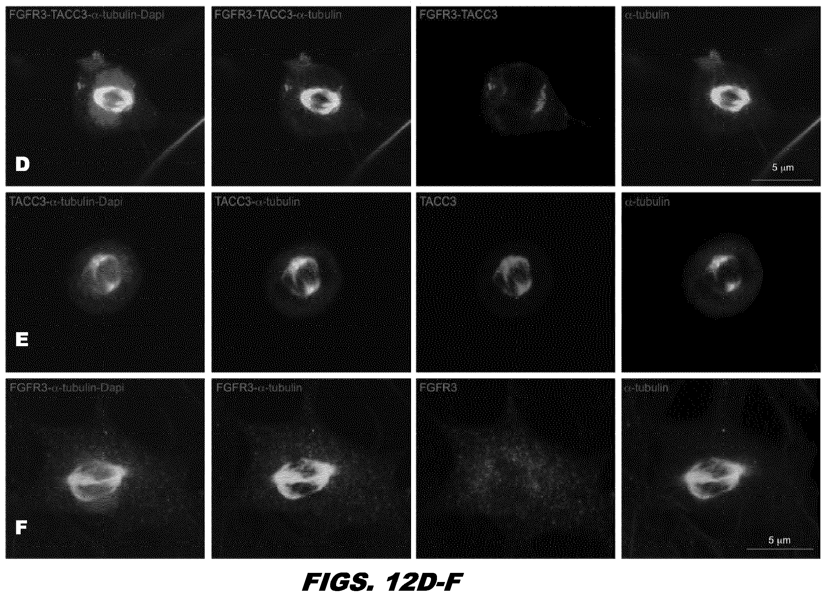

FIGS. 12D-F shows mitotic localization of FGFR3-TACC3 fusion protein. **FIG. 12D** shows maximum intensity projection confocal image of a representative FGFR3-TACC3 expressing *Ink4A;Arf-*/*-* mouse astrocyte at metaphase immunostained using the FGFR3 antibody (red; "dark grey" in black and white image). FGFR3-TACC3 displays asymmetric localization on top of one spindle pole. **FIG. 12E** shows maximum intensity projection confocal image of a representative TACC3 expressing *Ink4A;Arf-*/*-* mouse astrocyte at metaphase immunostained with the TACC3 antibody (red; ("dark grey" in black and white image). TACC3 staining coincides with the spindle microtubules. **FIG. 12F** shows maximum intensity projection confocal image of a representative FGFR3 expressing *Ink4A;Arf-*/*-* mouse astrocyte at metaphase immunostained with the FGFR3 antibody (red; ("dark grey" in black and white image). FGFR3 does not show a specific staining pattern in mitosis. Cells were co-immunostained using α-tubulin (green; ("light grey" in black and white image) to visualize the mitotic spindle. DNA was counterstained with DAPI (blue; ("grey" in black and white image). Images were acquired at 0.250 um intervals. Endogenous levels of FGFR3 or TACC3 were undetectable under the applied experimental conditions. F3-T3: FGFR3-TACC3.

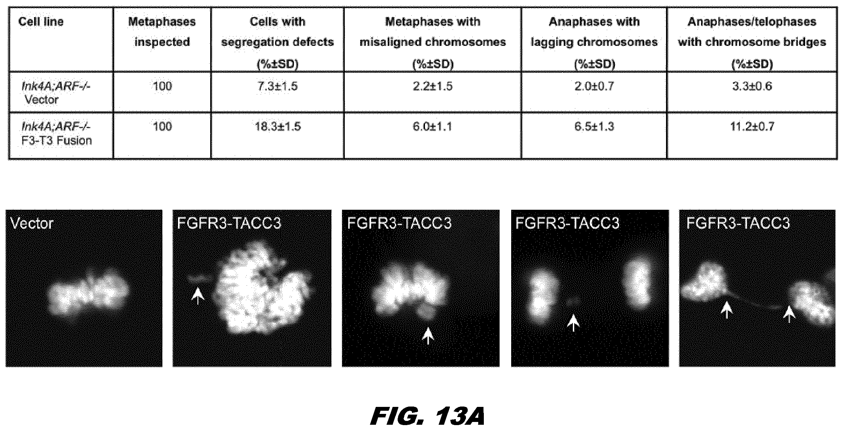

**FIG. 13A** shows sthat the FGFR3-TACC3 protein induces chromosomal missegregation, chromatid cohesion defects and defective spindle checkpoint. Quantitative analysis of metaphase spreads for chromosome segregation defects in *Ink4A;ARF-*/*-* astrocytes expressing vector control or FGFR3-TACC3 (upper panel). Microscope imaging analysis of chromosome segregation defects in *Ink4A;Arf-*/*-* astrocytes expressing FGFR3-TACC3 or vector control. Representative images of cells with chromosome missegregation. Arrows point to chromosome misalignments, lagging chromosomes and chromosome bridges.

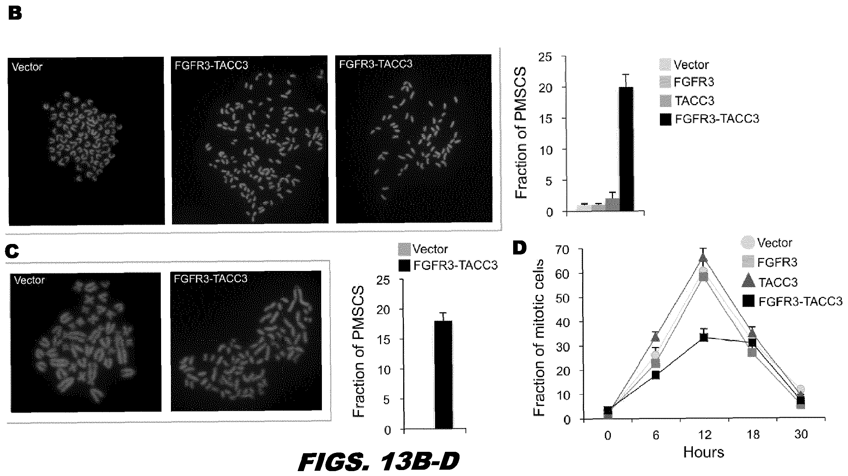

FIGS. 13B-D shows representative images of premature sister chromatid separation (PMSCS) in *Ink4A;Arf-*/*-* astrocytes (**FIG. 13B**) and Rat1A cells (**FIG. 13C**) expressing FGFR3-TACC3. Left, panels show representative metaphase spreads. Right, quantitative analysis of metaphases with loss of sister chromatid cohesion. The number of mitosis with PMSCS in *Ink4A;Arf-*/*-* astrocytes was scored in at least 100 methaphases for each condition in three independent experiments. The number of mitosis with PMSCS was scored in triplicate samples of Rat1A cells. **FIG. 13D** is a graph showing nocodazole was added for the indicated durations to Rat1A-H2B-GFP cells transduced with the specified lentiviruses. The mitotic index at each time point was determined by quantitating the H2B-GFP-positive cells in mitosis at each time point. Data are presented as average and standard deviation (n= 3). F3-T3: FGFR3-TACC3.

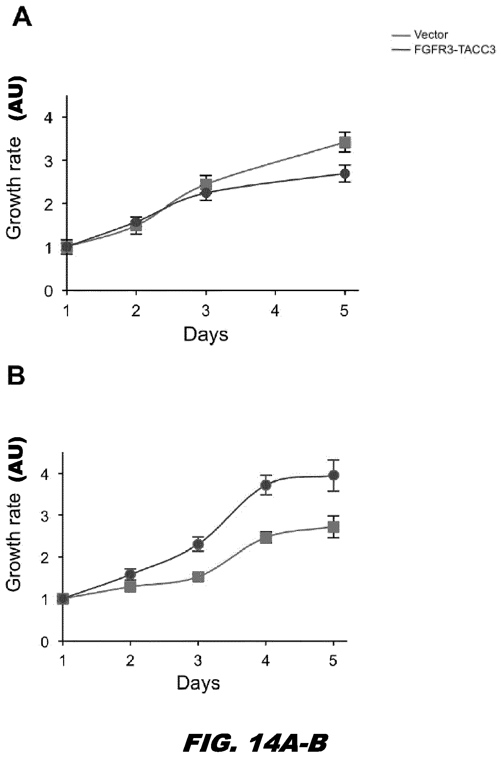

FIGS. 14A-B shows growth curves of human primary astrocytes transduced with lentivirus expressing FGFR3-TACC3 fusion or the empty vector. An analysis was conducted of FGFR3-TACC3 fusion mediated growth alteration and specific effect of RTK inhibitors on cells carrying FGFR-TACC fusions. **FIG. 14A** is a graph that shows cell proliferation of human primary astrocytes transduced with lentivirus expressing FGFR3-TACC3 fusion or the empty vector was determined by the MTT assay 7 days after infection (passage 1). Values are the means ± standard deviation (n = 4). *p*-value: 0.0033. **FIG. 14B** is a graph that shows cell proliferation of human primary astrocytes transduced with lentivirus expressing FGFR3-TACC3 fusion or the empty vector was determined by the MTT assay six weeks after the infection (passage 10). Values are the means±standard deviation (n=4). *p*-value: 0.0018.

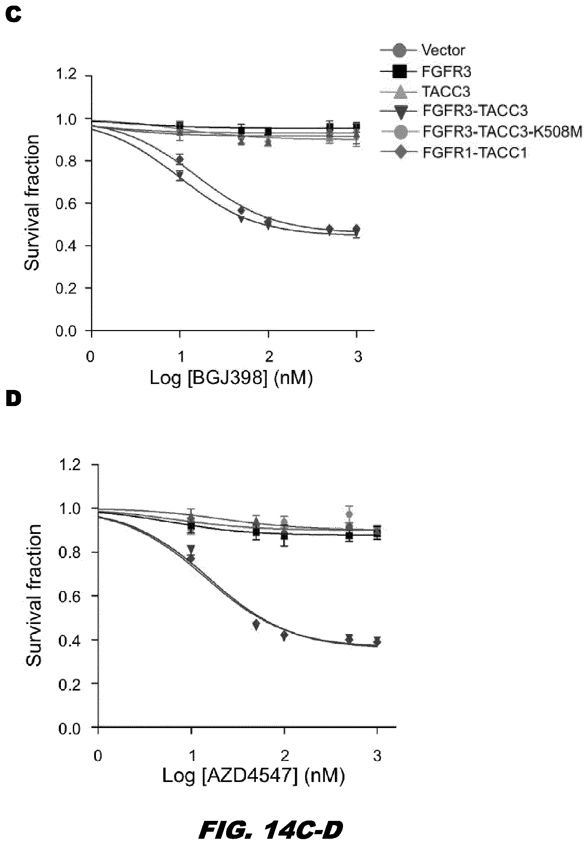

FIGS. 14C-D shows specific growth inhibitory effect by FGFR inhibitors on FGFR-TACC fusion expressing cells. Cell growth was determined by MTT assay. Rat1A cells transduced with the indicated lentivirus were treated for three days with BGJ398 (**FIG. 14C**) or AZD4547 (**FIG. 14D**) at the indicated concentration. Values are the means ± standard error (n = 4).

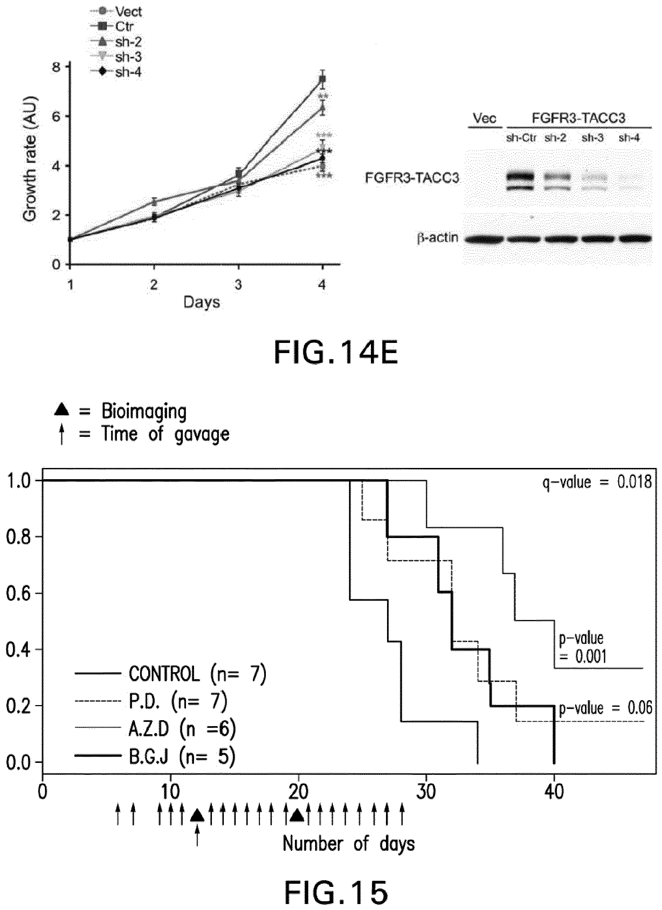

**FIG. 14E** shows the growth inhibitory effect of silencing FGFR3-TACC3 fusion. **(left)** GSC-1123 cells were transduced in triplicate with lentivirus expressing a non-targeting shRNA (Ctr) or lentivirus expressing sh-3 and sh-4 sequences targeting FGFR3, Five days after infection cells were plated at density of 2×10⁴ cells/well in triplicate and the number of trypan blue excluding cells was scored at the indicated times. Values are the means ± standard deviation (n = 3). **(right)** Western Blot analysis was performed on parallel cultures collected five days after infection using the FGFR3 antibody to the detect FGFT3-TACC3 fusion protein. β-actin is shown as a control for loading. (^{∗∗}: *p*-value = < 0.005; ^{∗∗∗}: *p*-value - < 0.0001).
**FIG. 15** shows a survival plot of cells treated with PD173074, NVP-BGJ398, or AZD4547.

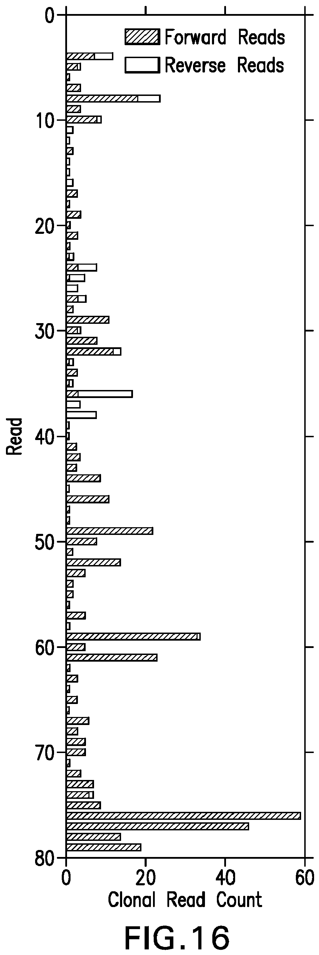

**FIG. 16** shows an *FGFR3-TACC3* gene fusion identified by whole transcriptome sequencing of GSCs. The histogram describes the absolute frequency of each forward and reverse sequence read spanning the breakpoint.

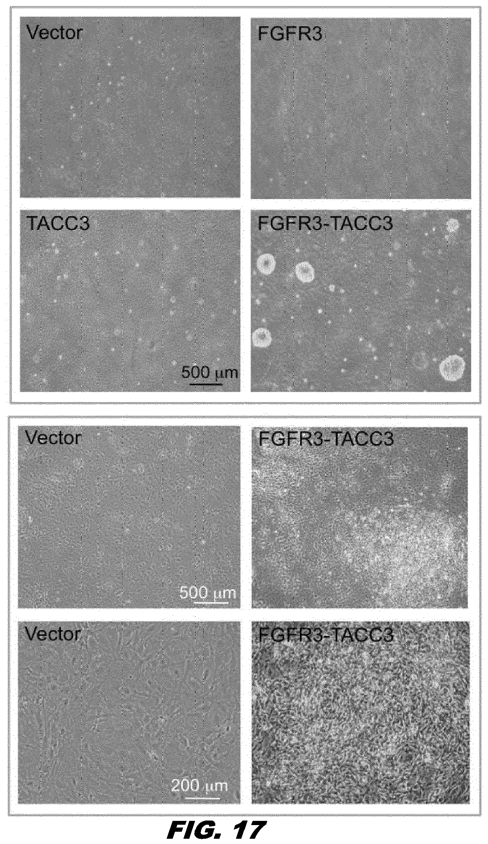

**FIG. 17** shows transforming activity of FGFR3-TACC3. FGFR3-TACC3 induces anchorage-independent growth in Rat1A fibroblasts (top panels) and a transformed phenotype in *Ink4A;Arf-*/*-* primary astrocytes (bottom panels).

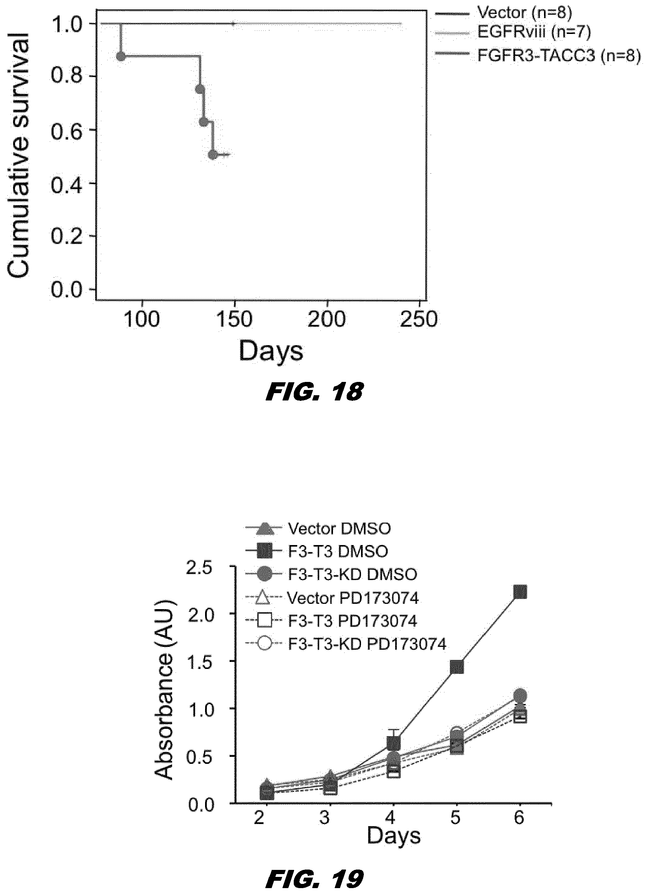

**FIG. 18** shows transforming activity of FGFR3-TACC3. Kaplan-Meier survival curves of mice injected intracranially with pTomo-shp53 (n = 8), pTomo-FGFR3-TACC3-shp53 (n = 8) and pTomo-EGFRvIII-shp53 (n = 7) are shown. Points on the curves indicate deaths (log-rank test, p = 0.025, pTomo-shp53 vs. pTomo-FGFR3-TACC3-shp53).
**FIG. 19** shows that inhibition of FGFR-TK activity corrects the aneuploidy and suppresses tumor growth initiated by FGFR3-TACC3. Short-term growth inhibition assays are shown of Rat1A transduced with the indicated lentivirus and treated with PD173470 at the indicated concentrations. Cells were treated for three days. Cell viability was determined by the MTT assay. Error bars show means ± standard error (n = 4).

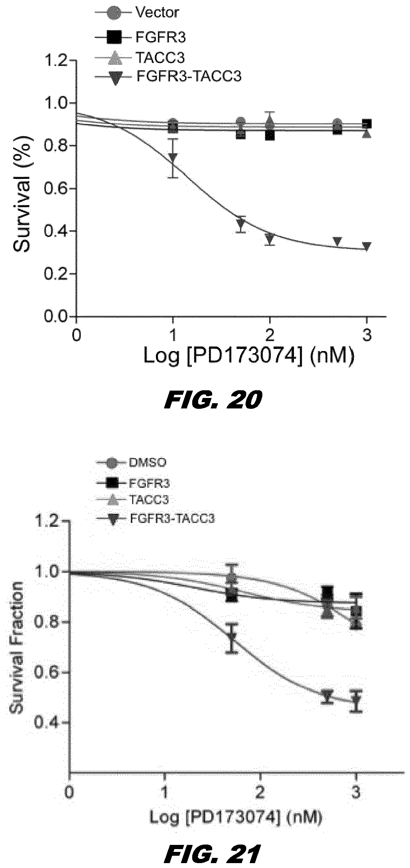

**FIG. 20** is a growth inhibition assay of human astrocytes transduced with the indicated lentivirus and treated for four days with PD173470 at the indicated concentration. Cell viability was determined by the MTT assay. Error bars show means ± standard error (n = 4).
**FIG. 21** is a graph showing a growth inhibition assay of human astrocytes transduced with the indicated lentivirus and treated for four days with PD173470 at the indicated concentration. Cell viability was determined by the MTT assay. Error bars show means ± standard error (n = 4).

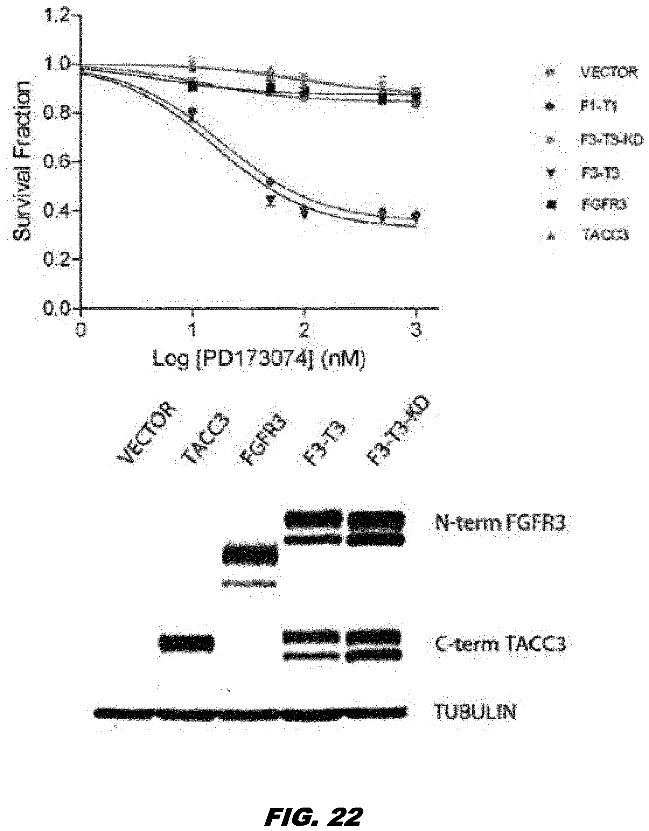

**FIG. 22** shows graphs of the survival of Rat1A cells in short-term growth inhibition assays. (**Top graph**) Rat1A cells were transduced with the indicated ptomo constructs and treated with PD173074 at the indicated concentrations . Cells were treated for three days. Cell viability was determined by the MTT assay. Error bars show means ± standard error (n = 4). In the bottom panel, a western blot photograph is shown.

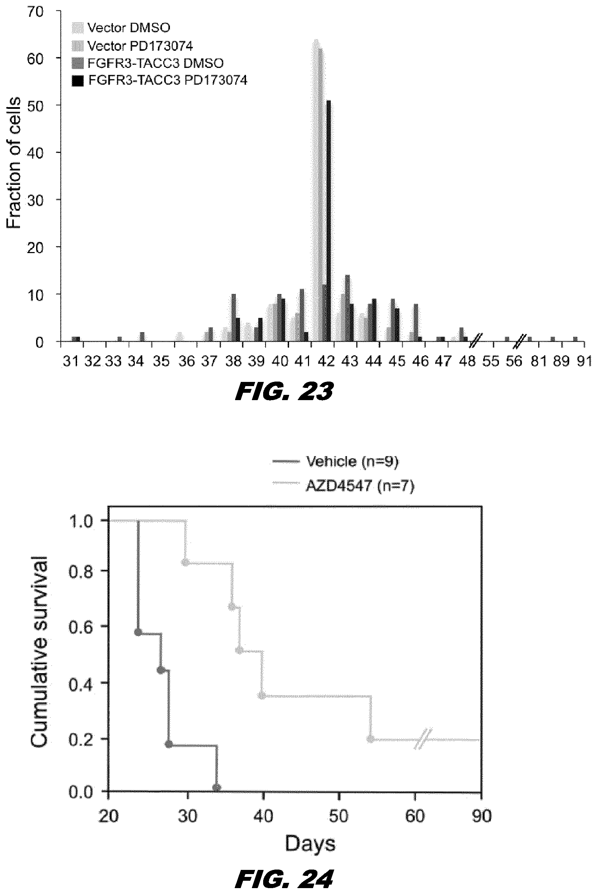

**FIG. 23** shows that inhibition of FGFR-TK activity corrects the aneuploidy and suppresses tumor growth initiated by FGFR3-TACC3. A plot is shown of karyotype analysis of Rat1A cells transduced with control or FGFR3-TACC3 lentivirus and treated with vehicle (DMSO) or PD173470 (100 nM.) for five days.
**FIG. 24** shows Survival of glioma-bearing mice was tracked following intracranial implantation of *Ink4A;Arf-*/*-* astrocytes transduced with FGFR3-TACC3. After tumor engraftment mice were treated with vehicle or AZD4547 (50 mg/kg) for 20 days (vehicle, n = 7; AZD4547, n = 6; p = 0.001).

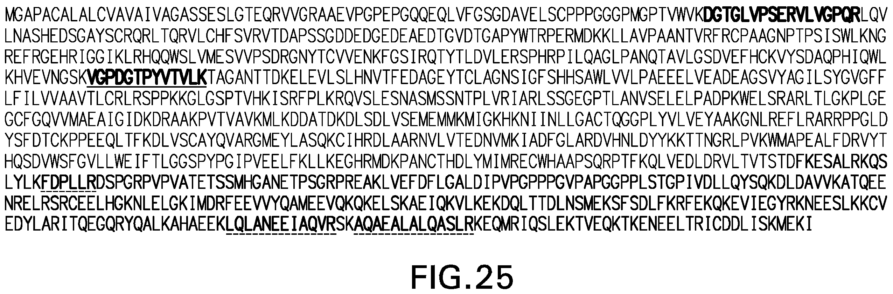

**FIG. 25** shows the position of the peptides from FIG. 10E in the amino acid sequence of the FGFR3-TACC3 fusion protein, which are highlighted in pink (FGFR3; underlined) and blue (TACC3; dotted lines).

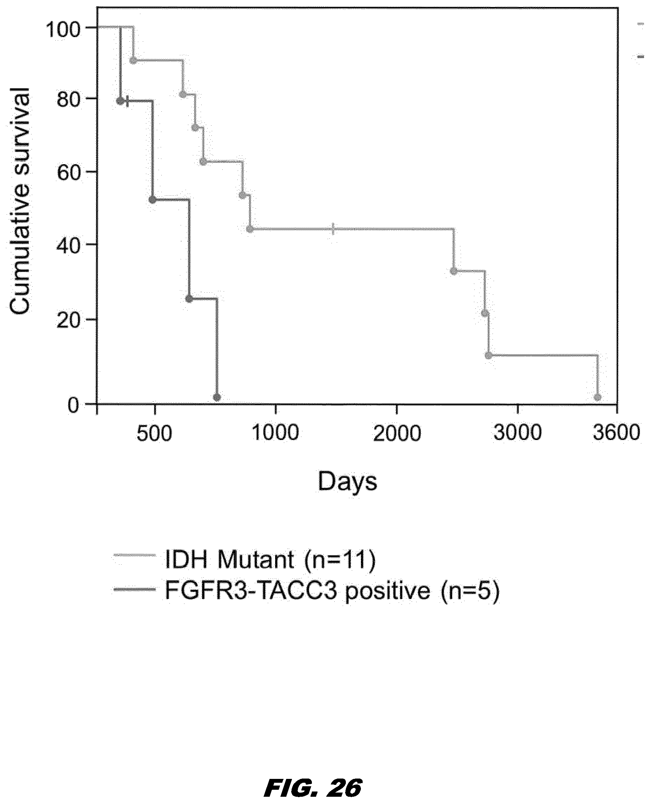

**FIG. 26** shows Kaplan-Meier analysis of 1DH mutant and *FGFR3-TACC3* positive human GBM. Log rank test *p*-value: 0.0169.

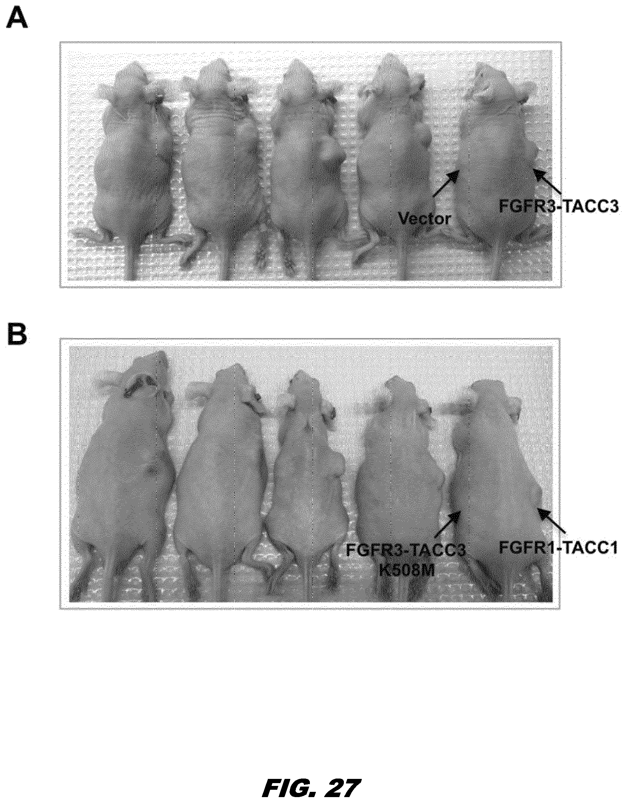

**FIG. 27** is a picture that shows tumor xenografts that were induced following sub-cutaneous injection of *Ink4A;Arf-*/*-* mouse astrocytes transduced with lentivirus expressing FGFR3-TACC3 (upper panel A, right flank) or FGFR1-TACC1 (lower panel B, right flank) fusion, but not with the empty vector (upper panel, left flank) or FGFR3-TACC3 carrying a K508M mutation in the kinase domain (FGFR3-TACC3-K508M; lower panel, left flank).

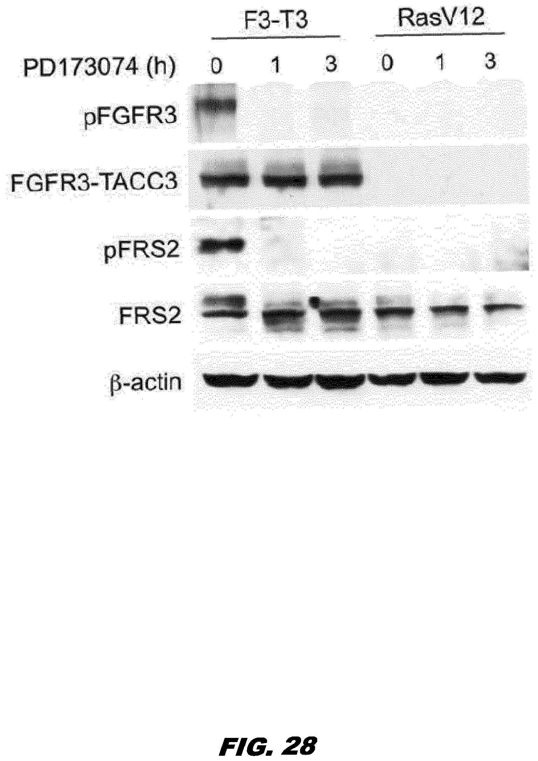

**FIG. 28** shows constitutive auto-phosphorylation of FGFR3 -TACC3 fusion. BTSC derived from FGFR3-TACC3 or RasV12 induced mouse GBM were left untreated or treated with 500nM PD1 73074 for the indicated times. Phospho-proteins and total proteins were analyzed by Western blot using the indicated antibodies. β-actin is shown as a control for loading.

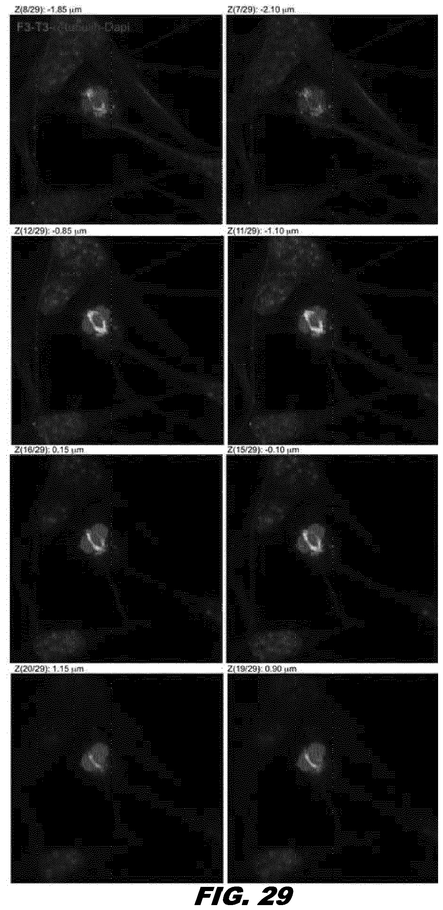

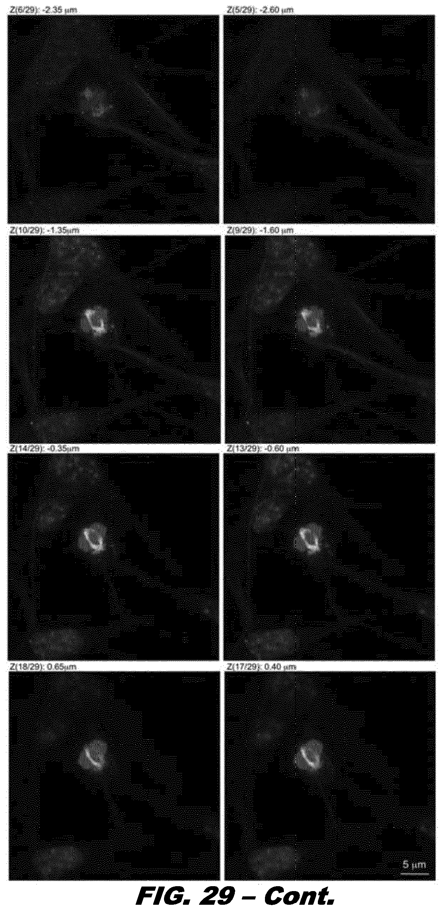

**FIG. 29** shows Z-stacked confocal images of the representative FGFR3-TACC3 expressing *Ink4A;Arf-*/*-* mouse astrocyte shown as a maximum intensity projection. Cells were immunostained using FGFR3 (red; "dark grey" in black and white image) and α-tubulin (green; ("light grey" in black and white image). DNA was counterstained with DAPI (blue; ("grey" in black and white image). Images were acquired at 0.250 µm intervals. Coordinates of the image series are indicated. F3-T3: FGFR3-TACC3.

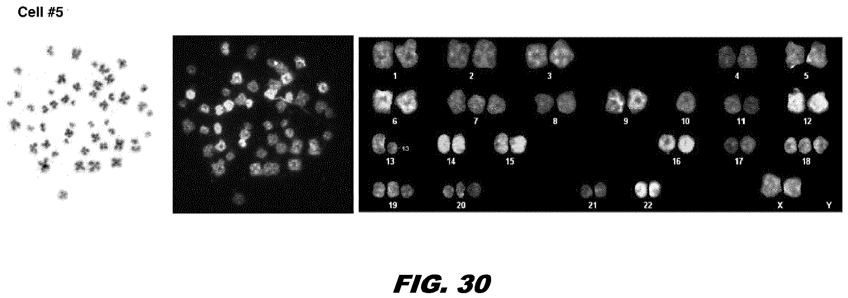

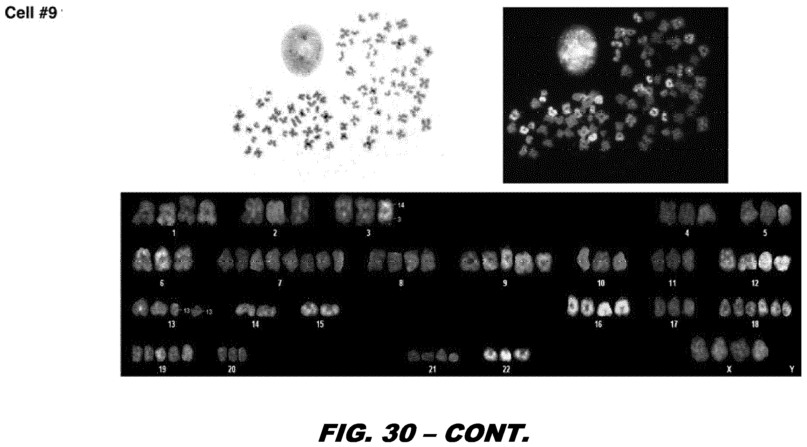

**FIG. 30** shows examples of SKY karyotype analysis painting two different cells from the same culture of GSC-1 123, illustrating the ongoing CLN and aneuploidy. Details of the karyotype analysis of 20 cells are reported in Table 6.

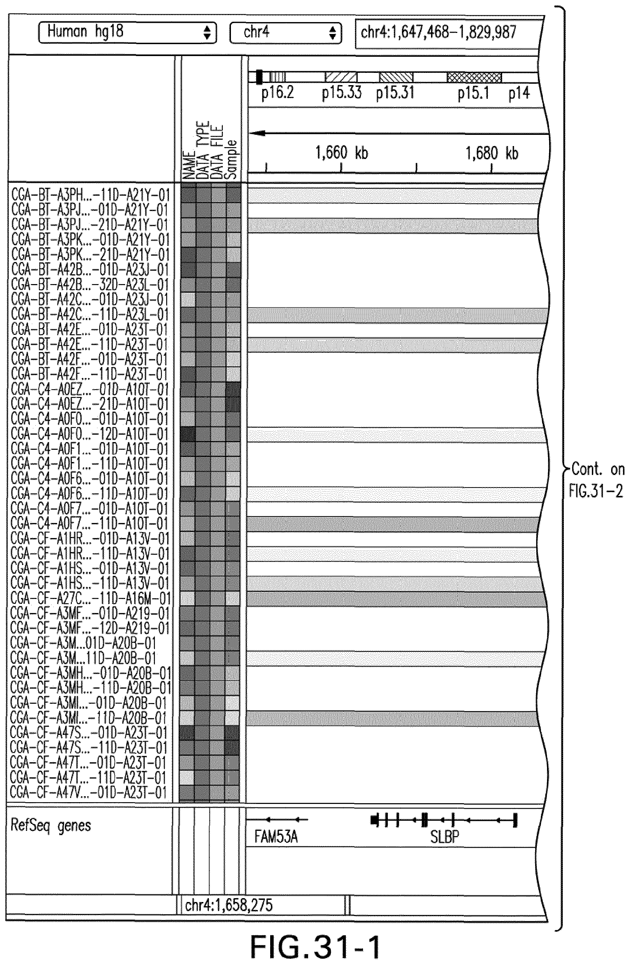

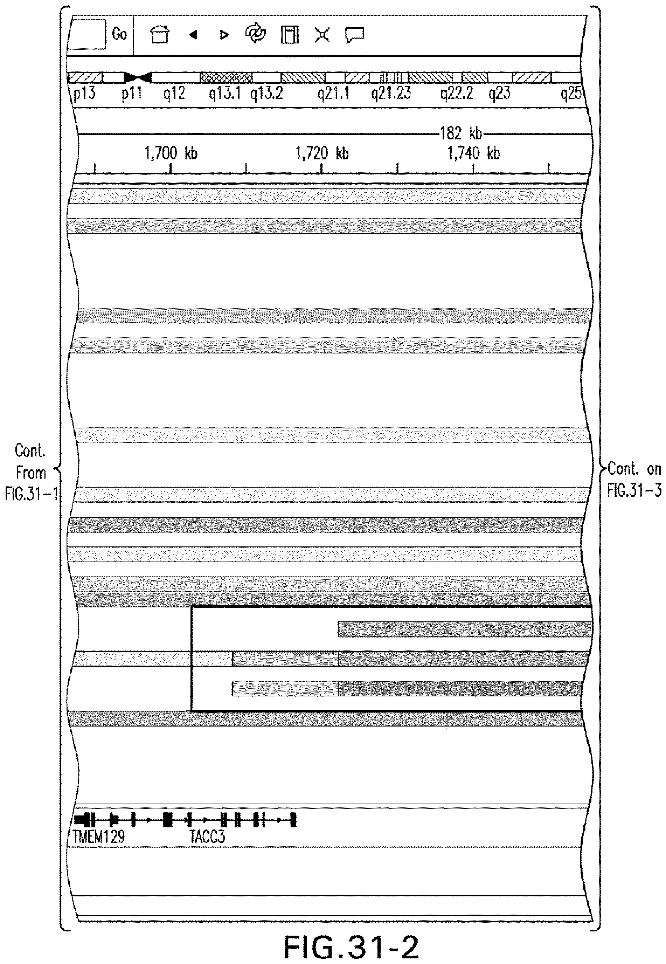

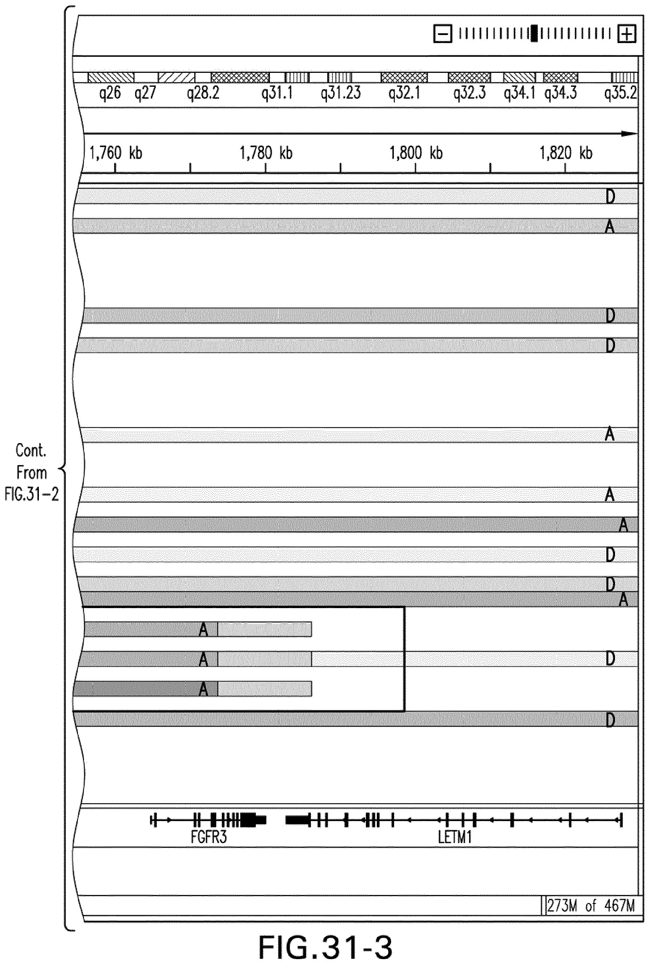

**FIG. 31** is a graphical representation of segmented CNVs data visualized using the Integrated Genomic Viewers software. Three bladder Urothelial Carcinoma harbor FGFR3-TACC3 gene fusions (black box). Red indicates amplification (A), blue indicates deletion (D).

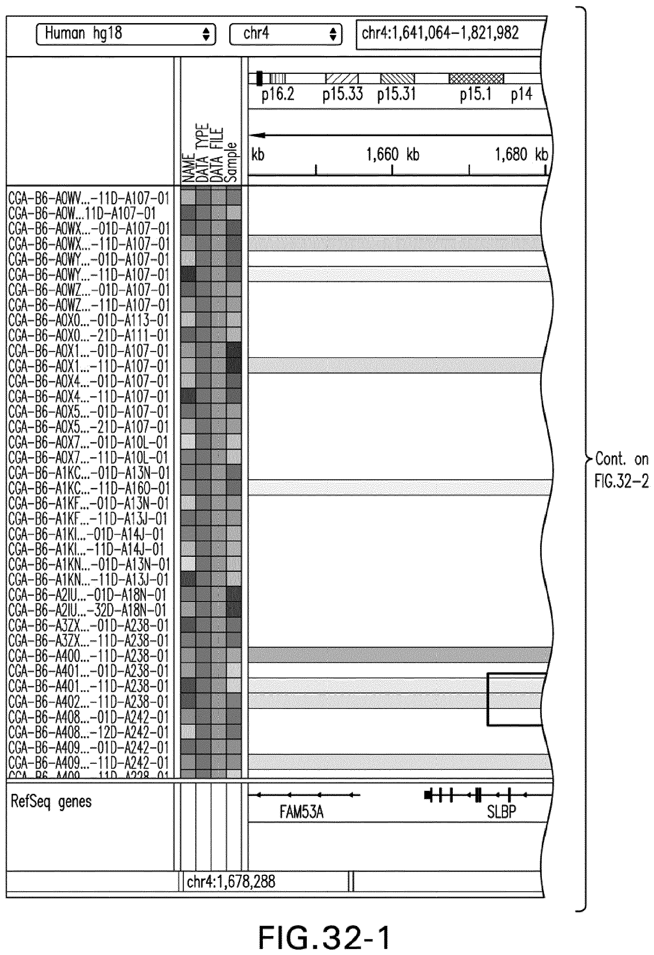

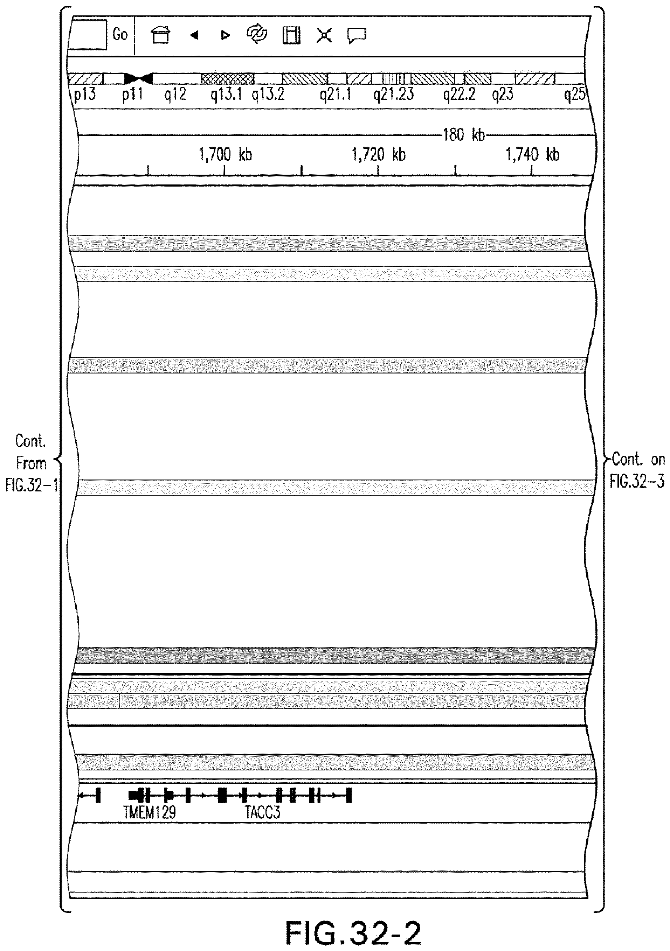

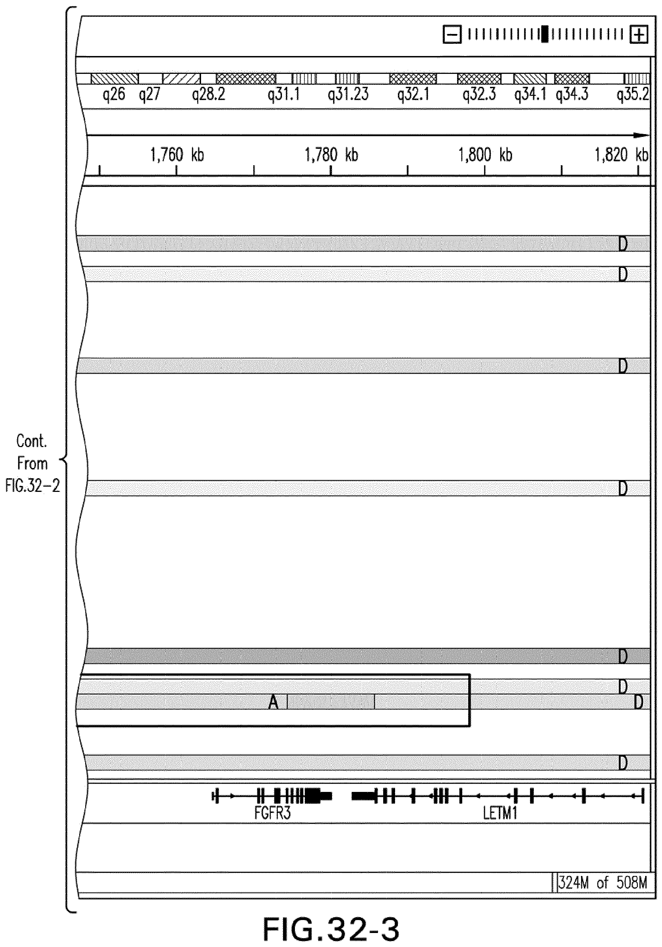

**FIG. 32** is a graphical representation of segmented CNVs data visualized using the Integrated Genomic Viewers software. One Breast Carcinoma harbors FGFR3-TACC3 gene fusions (black box). Red indicates amplification (A), blue indicates deletion (D).

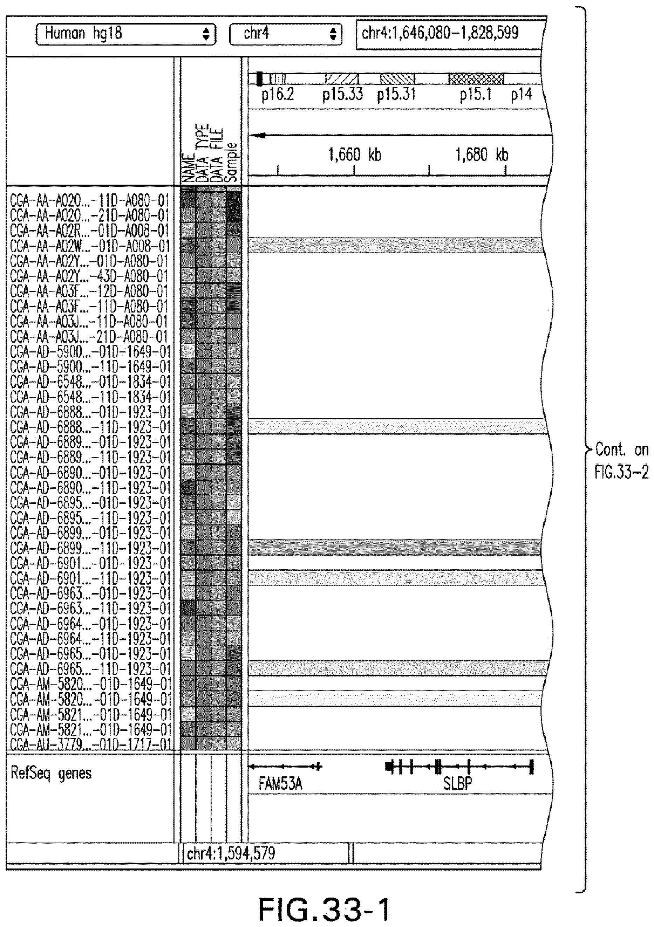

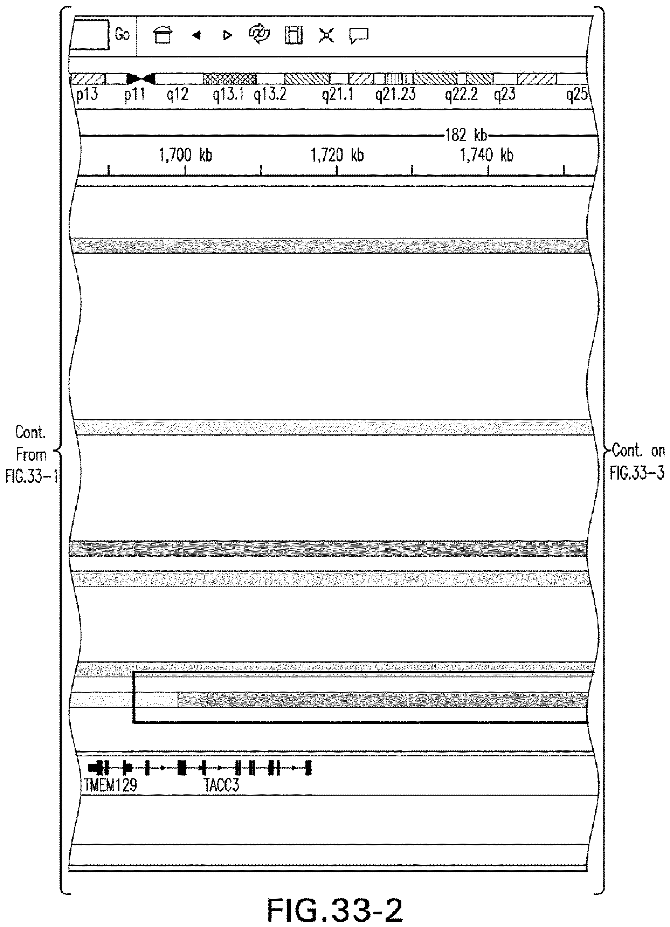

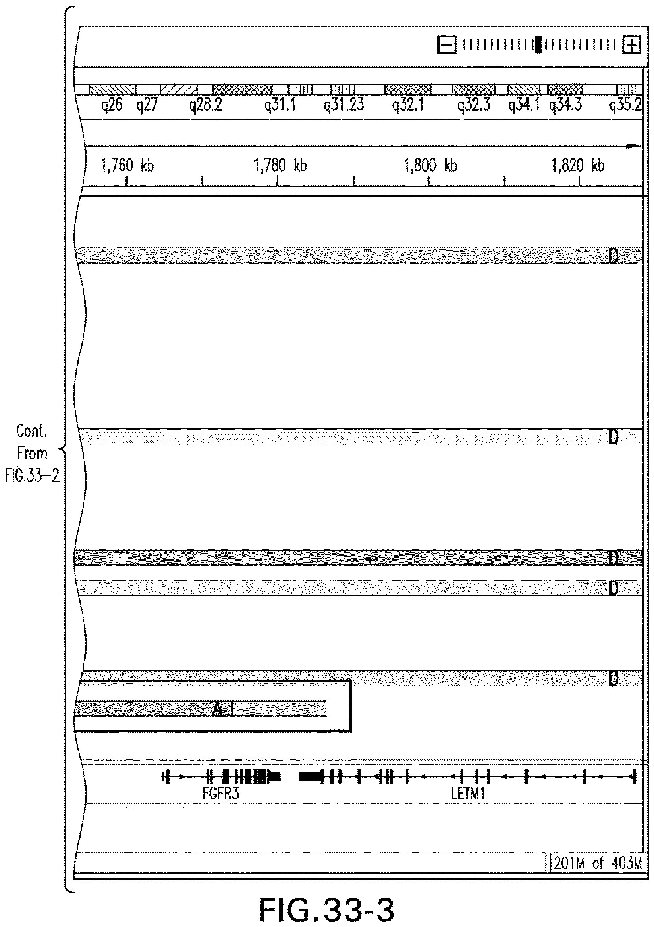

**FIG. 33** is a graphical representation of segmented CNVs data visualized using the Integrated Genomic Viewers software. One Colorectal Carcinoma harbors FGFR3-TACC3 gene fusions (black box). Red indicates amplification (A), blue indicates deletion (D).

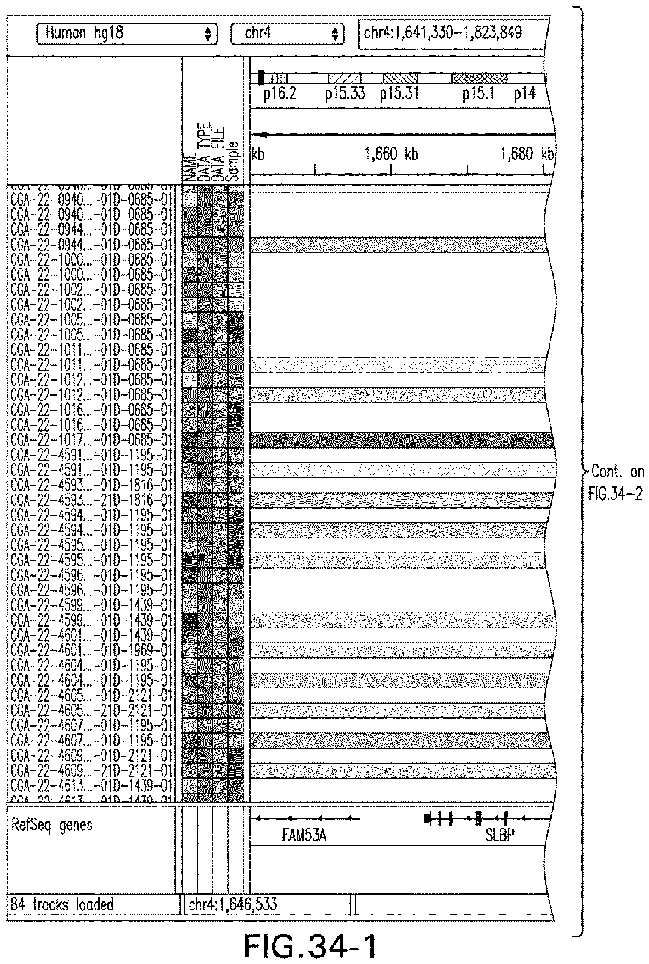

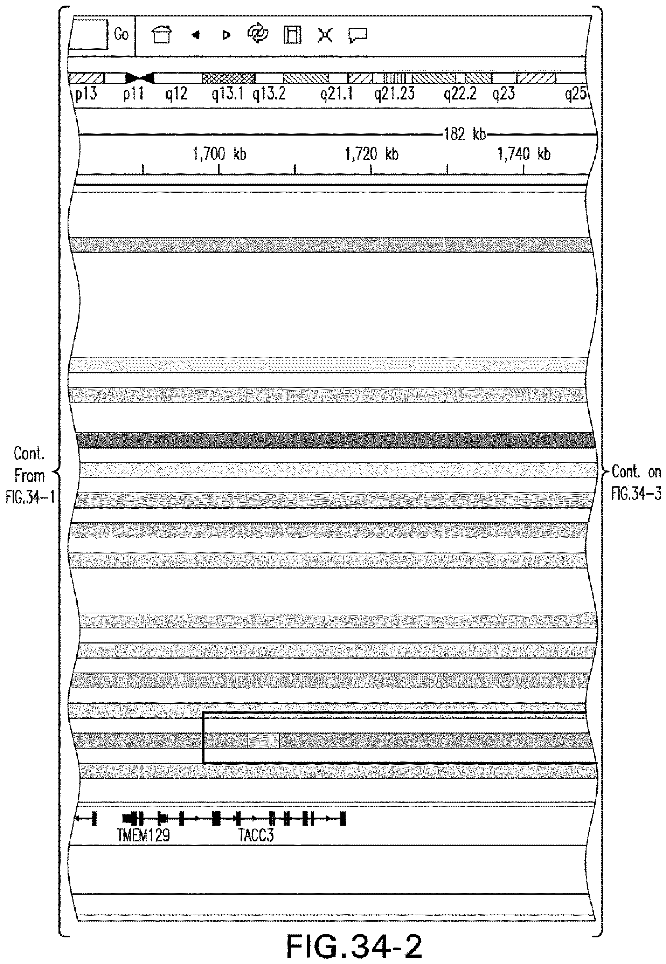

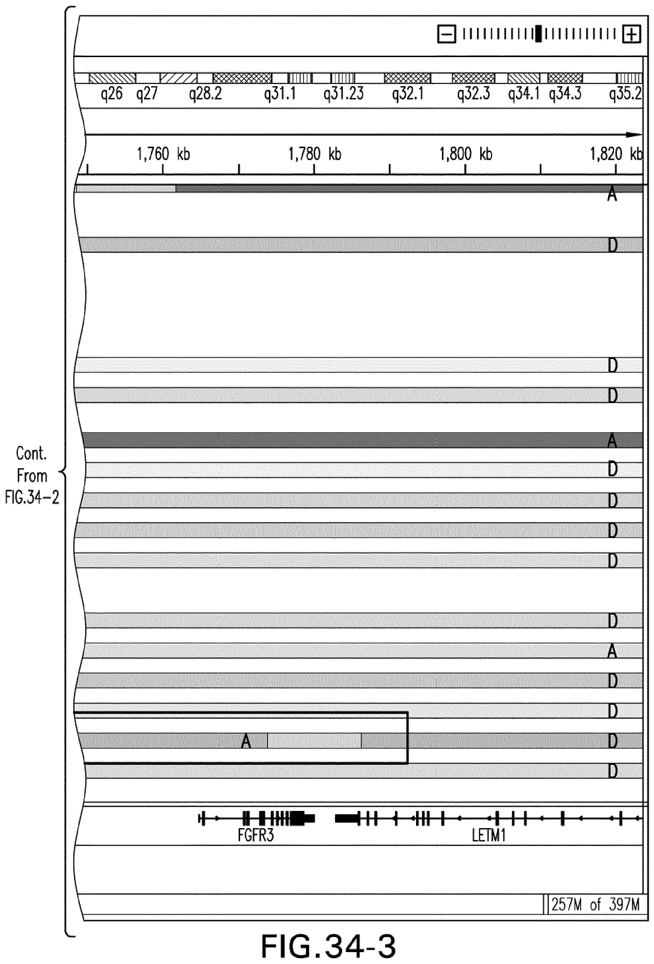

**FIG. 34** is a graphical representation of segmented CNVs data visualized using the Integrated Genomic Viewers software. One Lung Squamous Cell Carcinoma harbors FGFR3-TACC3 gene fusions (black box). Red indicates amplification (A), blue indicates deletion (D).

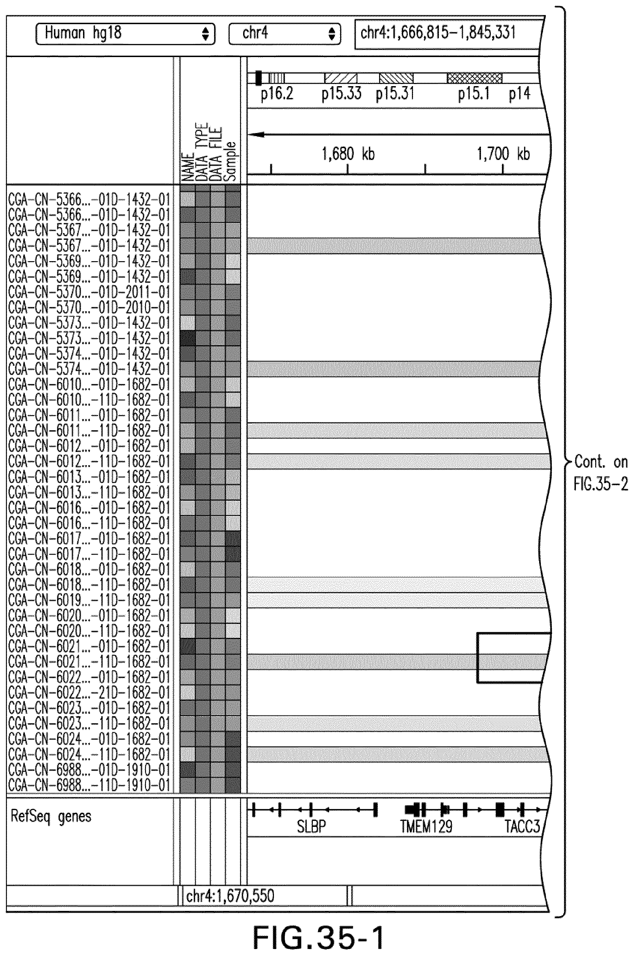

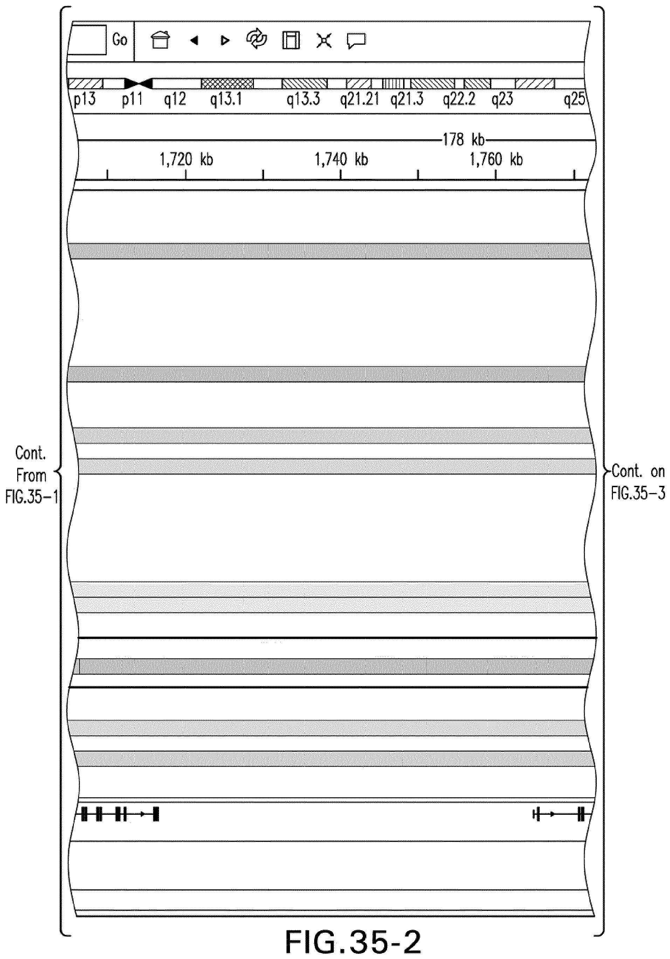

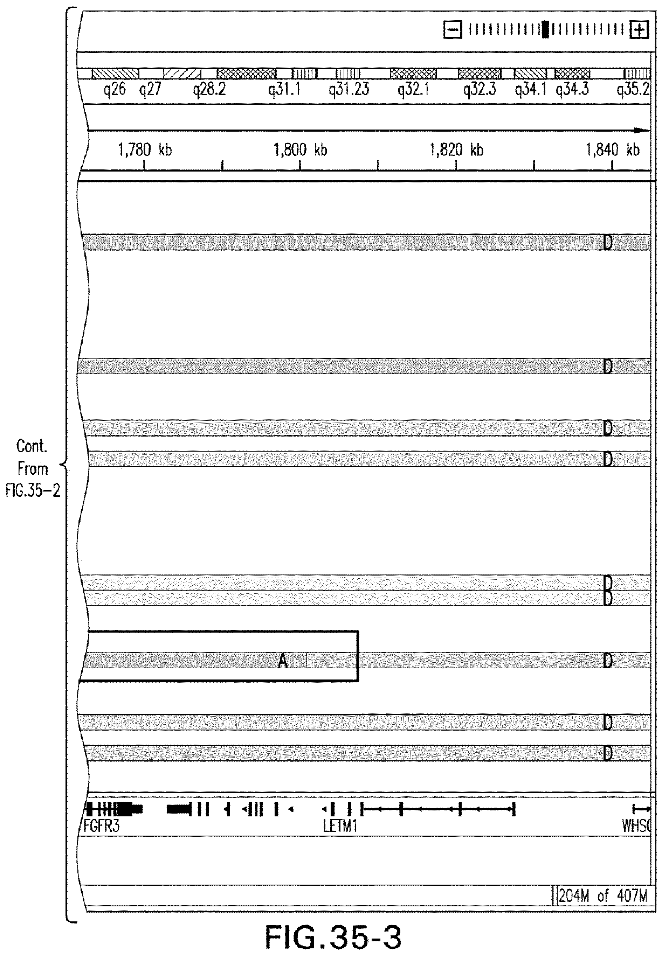

**FIG. 35** is a graphical representation of segmented CNVs data visualized using the Integrated Genomic Viewers software. One Head abd Neck Squamous Cell Carcinoma harbors FGFR3-TACC3 gene fusions (black box). Red indicates amplification (A), blue indicates deletion (D).

### DETAILED DESCRIPTION OF THE INVENTION

Glioblastoma multiformes (GBMs) are the most common form of brain tumors in adults accounting for 12-15% of intracranial tumors and 50-60% of primary brain tumors. GBM is among the most lethal forms of human cancer. The history of successful targeted therapy of cancer largely coincides with the inactivation of recurrent and oncogenic gene fusions in hematological malignancies and recently in some types of epithelial cancer. GBM is among the most lethal and incurable forms of human cancer. Targeted therapies against common genetic alterations in GBM have not changed the dismal clinical outcome of the disease, most likely because they have systematically failed to eradicate the truly addicting oncoprotein activities of GBM. Recurrent chromosomal rearrangements resulting in the creation of oncogenic gene fusions have not been found in GBM.

GBM is among the most difficult forms of cancer to treat in humans (*1*). So far, the therapeutic approaches that have been tested against potentially important oncogenic targets in GBM have met limited success (*2-4*). Recurrent chromosomal translocations leading to production of oncogenic fusion proteins are viewed as initiating and addicting events in the pathogenesis of human cancer, thus providing the most desirable molecular targets for cancer therapy (*5, 6*). Recurrent and oncogenic gene fusions have not been found in GBM. Chromosomal rearrangements are hallmarks of hematological malignancies but recently they have also been uncovered in subsets of solid tumors (breast, prostate, lung and colorectal carcinoma) (*7, 8*). Important and successful targeted therapeutic interventions for patients whose tumors carry these rearrangements have stemmed from the discovery of functional gene fusions, especially when the translocations involve kinase-coding genes (*BCR-ABL, EML4-ALK*) (*9, 10*).

A hallmark of GBM is rampant chromosomal instability (CIN), which leads to aneuploidy (*11*). CIN and aneuploidy are early events in the pathogenesis of cancer (*12*). It has been suggested that genetic alterations targeting mitotic fidelity might be responsible for missegregation of chromosomes during mitosis, resulting in aneuploidy (*13, 14*).

Fibroblast growth factor receptors (FGFR) are transmembrane receptors that bind to members of the fibroblast growth factor family of proteins. The structure of the FGFR.s consist of an extracellular ligand binding domain comprised of three Ig-like domains, a single transmembrane helix domain, and an intracellular domain with tyrosine kinase activity (Johnson, D.E., Williams, E. T. Structural and functional diversity in the FGF receptor multigene family. (1993) Adv. Cancer Res, 60:1-41).

Transforming acidic coiled-coiled protein (TACC) stabilize microtubules during mitosis by recruiting minispindles (Msps)/XMAP215 proteins to centrosomes. TACCs have been implicated in cancer.

From a medical perspective, the FGFR-TACC fusions provide the first "bonafide" oncogenically addictive gene fusions in GBM whose identification has long been overdue in this disease.

### DNA and AminoAcid Manipulation Methods and Purification Thereof

The practice of aspects of the present invention can employ, unless otherwise indicated, conventional techniques of cell biology, cell culture, molecular biology, transgenic biology, microbiology, recombinant DNA, and immunology, which are within the skill of the art. Such techniques are explained fully in the literature. *See,* e.g., Molecular Cloning A Laboratory Manual, 3rd Ed., ed. by Sambrook (2001), Fritsch and Maniatis (Cold Spring Harbor Laboratory Press: 1989); DNA Cloning, Volumes 1 and II (D. N. Glover ed., 1985); Oligonucleotide Synthesis (M. J. Gait ed., 1984); Mullis et al. U.S. Pat. No: 4,683,195; Nucleic Acid Hybridization (B. D. Hames & S. J. Higgins eds. 1984); Transcription and Translation (B. D. Hames & S. J. Higgins eds. 1984); Culture Of Animal Cells (R. I. Freshney, Alan R. Liss, Inc., 1987); Immobilized Cells and Enzymes (IRL Press, 1986); B. Perbal, A Practical Guide To Molecular Cloning (1984); the series, Methods In Enzymology (Academic Press, Inc., N.Y.), specifically, Methods In Enzymology. Vols. 154 and 155 (Wu et al. eds.); Gene Transfer Vectors For Mammalian Cells (J. H. Miller and M. P. Calos eds., 1987, Cold Spring Harbor Laboratory); Immunochemical Methods In Cell And Molecular Biology (Caner and Walker, eds. Academic Press, London, 1987); Handbook Of Experimental Immunology, Volumes I-IV (D. M. Weir and C. C. Blackwell, eds., 1986); Manipulating the Mouse Embryo, (Cold Spring Harbor Laboratory Press, Cold Spring Harbor, N.Y., 1986). All patents, patent applications and references cited herein are incorporated by reference in their entireties.

One skilled in the art can obtain a protein in several ways, which include, but are not limited to, isolating the protein via biochemical means or expressing a nucleotide sequence encoding the protein of interest by genetic engineering methods.

A protein is encoded by a nucleic acid (including, for example, genomic DNA, complementary DNA (cDNA), synthetic DNA, as well as any form of corresponding RNA). For example, it can be encoded by a recombinant nucleic acid of a gene. The proteins of the invention can be obtained from various sources and can be produced according to various techniques known in the art. For example, a nucleic acid that encodes a protein can be obtained by screening DNA libraries, or by amplification from a natural source. A protein can be a fragment or portion thereof. The nucleic acids encoding a protein can be produced via recombinant DNA technology and such recombinant nucleic acids can be prepared by conventional techniques, including chemical synthesis, genetic engineering, enzymatic techniques, or a combination thereof. For example, a fusion protein of the invention comprises a tyrosine kinase domain of an FGFR protein fused to a polypeptide that constitutively activates the tyrosine kinase domain of the FGFR protein. For example, a fusion protein of the invention comprises a transforming acidic coiled-coil (TACC) domain fused to a polypeptide with a tyrosine kinase domain, wherein the TACC domain constitutively activates the tyrosine kinase domain. An example of a FOFR1- TACC1 polypeptide has the amino acid sequence shown in SEQ ID NO: 150. An example of a FGFR3-TACC3 protein is the polypeptide encoded by the nucleic acid having the nucleotide sequence shown in SEQ ID NO: 94. Examples of a FGFR3-TACC3 polypeptide has the amino acid sequence shown in SEQ ID NO: 79, 158, 159, 160, or 161.

The Genbank ID for the FGFR3 gene is 2261. Three isoforms are listed for FGFGR3, e.g., having Genebank Accession Nos. NP_000133 (corresponding nucleotide sequence NM_000142); NP_001156685 (corresponding nucleotide sequence NM_001163213); NP_075254 (corresponding nucleotide sequence NM_022965).
SEQ ID NO: 90 is the FGFR3 Amino Acid Sequence, Transcript Variant 1 (NP_000133; 806 aa). 
1 MGAPACALAL CVAVAIVAGA SSESLGTEQR VVGRAAEVPG PEPGQQEQLV FGSGDAVELS
61 CPPPGGGPMG PTVWVKDGTG LVPSERVLVG PQRLQVLNAS HEDSGAYSCR QRLTQRVLCH
121 FSVRVTDAPS SGDDEDGEDE AEDTGVDTGA PYWTRPERMD KKLLAVPAAN TVRFRCPAAG
181 NPTPSISWLK NGREFRGEHR IGGIKLRHQQ WSLVMESVVP SDRGNYTCVV ENKFGSIRQT
241 YTLDVLERSP HRPILQAGLP ANQTAVLGSD VEFHCKVYSD AQPHIQWLKH VEVNGSKVGP
301 DGTPYVTVLK TAGANTTDKE LEVLSLHNVT FEDAGEYTCL AGNSIGFSHH SAWLVVLPAE
361 EELVEADEAG SVYAGILSYG VGFFLFILVV AAVTLCRLRS PPKKGLGSPT VHKISRFPLK
421 RQVSLESNAS MSSNTPLVRI ARLSSGEGPT LANVSELELP ADPKWELSRA RLTLGKPLGE
481 GCFGQVVMAE AIGIDKDRAA KPVTVAVKML KDDATDKDLS DLVSEMEMMK MIGKHKNIIN
541 LLGACTQGGP LYVLVEYAAK GNLREFLRAR RPPGLDYSFD TCKPPEEQLT FKDLVSCAYQ
601 VARGMEYLAS QKCIHRDLAA RNVLVTEDNV MKIADFGLAR DVHNLDYYKK TTNGRLPVKW
661 MAPEALFDRV YTHQSDVWSF GVLLWEIFTL GGSPYPGIPV EELFKLLKEG HRMDKPANCT
721 HDLYMIMREC WHAAPSQRPT FKQLVEDLDR VLTVTSTDEY LDLSAPFEQY SPGGQDTPSS
781 SSSGDDSVFA HDLLPPAPPS SGGSRT
SEQ ID NO: 91 is the FGFR3 Nucleotide Sequence, Transcript Variant 1 (NM_000142; 4304 bp).

1 gtcgcgggca gctggcgccg cgcggtcctg ctctgccggt cgcacggacg caccggcggg
61 ccgccggccg gagggacggg gcgggagctg ggcccgcgga cagcgagccg gagcgggagc
121 cgcgcgtagc gagccgggct ccggcgctcg ccagtctccc gagcggcgcc cgcctcccgc
181 cggtgcccgc gccgggccgt ggggggcagc atgcccgcgc gcgctgcctg aggacgccgc
241 ggcccccgcc cccgccatgg gcgcccctgc ctgcgccctc gcgctctgcg tggccgtggc
301 catcgtggcc ggcgcctcct cggagtcctt ggggacggag cagcgcgtcg tggggcgagc
361 ggcagaagtc ccgggcccag agcccggcca gcaggagcag ttggtcttcg gcagcgggga
421 tgctgtggag ctgagctgtc ccccgcccgg gggtggtccc atggggccca ctgtctgggt
481 caaggatggc acagggctgg tgccctcgga gcgtgtcctg gtggggcccc agcggctgca
541 ggtgctgaat gcctcccacg aggactccgg ggcctacagc tgccggcagc ggctcacgca
601 gcgcgtactg tgccacttca gtgtgcgggt gacagacgct ccatcctcgg gagatgacga
661 agacggggag gacgaggctg aggacacagg tgtggacaca ggggcccctt actggacacg
721 gcccgagcgg atggacaaga agctgctggc cgtgccggcc gccaacaccg tccgcttccg
781 ctgcccagcc gctggcaacc ccactccctc catctcctgg ctgaagaacg gcagggagtt
841 ccgcggcgag caccgcattg gaggcatcaa gctgcggcat cagcagtgga gcctggtcat
901 ggaaagcgtg gtgccctcgg accgcggcaa ctacacctgc gtcgtggaga acaagtttgg
961 cagcatccgg cagacgtaca cgctggacgt gctggagcgc tccccgcacc ggcccatcct
1021 gcaggcgggg ctgccggcca accagacggc ggtgctgggc agcgacgtgg agttccactg
1081 caaggtgtac agtgacgcac agccccacat ccagtggctc aagcacgtgg aggtgaatgg
1141 cagcaaggtg ggcccggacg gcacacccta cgttaccgtg ctcaagacgg cgggcgctaa
1201 caccaccgac aaggagctag aggttctctc cttgcacaac gtcacctttg aggacgccgg
1261 ggagtacacc tgcctggcgg gcaattctat tgggttttct catcactctg cgtggctggt
1321 ggtgctgcca gccgaggagg agctggtgga ggctgacgag gcgggcagtg tgtatgcagg
1381 catcctcagc tacggggtgg gcttcttcct gttcatcctg gtggtggcgg ctgtgacgct
1441 ctgccgcctg cgcagccccc ccaagaaagg cctgggctcc cccaccgtgc acaagatctc
1501 ccgcttcccg ctcaagcgac aggtgtccct ggagtccaac gcgtccatga gctccaacac
1561 accactggtg cgcatcgcaa ggctgtcctc aggggagggc cccacgctgg ccaatgtctc
1621 cgagctcgag ctgcctgccg accccaaatg ggagctgtct cgggcccggc tgaccctggg
1681 caagcccctt ggggagggct gcttcggcca ggtggtcatg gcggaggcca tcggcattga
1741 caaggaccgg gccgccaagc ctgtcaccgt agccgtgaag atgctgaaag acgatgccac
1801 tgacaaggac ctgtcggacc tggtgtctga gatggagatg atgaagatga tcgggaaaca
1861 caaaaacatc atcaacctgc tgggcgcctg cacgcagggc gggcccctgt acgtgctggt
1921 ggagtacgcg gccaagggta acctgcggga gtttctgcgg gcgcggcggc ccccgggcct
1981 ggactactcc ttcgacacct gcaagccgcc cgaggagcag ctcaccttca aggacctggt
2041 gtcctgtgcc taccaggtgg cccggggcat ggagtacttg gcctcccaga agtgcatcca
2101 cagggacctg gctgcccgca atgtgctggt gaccgaggac aacgtgatga agatcgcaga
2161 cttcgggctg gcccgggacg tgcacaacct cgactactac aagaagacaa ccaacggccg
2221 gctgcccgtg aagtggatgg cgcctgaggc cttgtttgac cgagtctaca ctcaccagag
2281 tgacgtctgg tcctttgggg tcctgctctg ggagatcttc acgctggggg gctccccgta
2341 ccccggcatc cctgtggagg agctcttcaa gctgctgaag gagggccacc gcatggacaa
2401 gcccgccaac tgcacacacg acctgtacat gatcatgcgg gagtgctggc atgccgcgcc
2461 ctcccagagg cccaccttca agcagctggt ggaggacctg gaccgtgtcc ttaccgtgac
2521 gtccaccgac gagtacctgg acctgtcggc gcctttcgag cagtactccc cgggtggcca
2581 ggacaccccc agctccagct cctcagggga cgactccgtg tttgcccacg acctgctgcc
2641 cccggcccca cccagcagtg ggggctcgcg gacgtgaagg gccactggtc cccaacaatg
2701 tgagggtccc ctagcagccc accctgctgc tggtgcacag ccactccccg gcatgagact
2761 cagtgcagat ggagagacag ctacacagag ctttggtctg tgtgtgtgtg tgtgcgtgtg
2821 tgtgtgtgtg tgtgcacatc cgcgtgtgcc tgtgtgcgtg cgcatcttgc ctccaggtgc
2881 agaggtaccc tgggtgtccc cgctgctgtg caacggtctc ctgactggtg ctgcagcacc
2941 gaggggcctt tgttctgggg ggacccagtg cagaatgtaa gtgggcccac ccggtgggac
3001 ccccgtgggg cagggagctg ggcccgacat ggctccggcc tctgcctttg caccacggga
3061 catcacaggg tgggcctcgg cccctcccac acccaaagct gagcctgcag ggaagcccca
3121 catgtccagc accttgtgcc tggggtgtta gtggcaccgc ctccccacct ccaggctttc
3181 ccacttccca ccctgcccct cagagactga aattacgggt acctgaagat gggagccttt
3241 accttttatg caaaaggttt attccggaaa ctagtgtaca tttctataaa tagatgctgt
3301 gtatatggta tatatacata tatatatata acatatatgg aagaggaaaa ggctggtaca
3361 acggaggcct gcgaccctgg gggcacagga ggcaggcatg gccctgggcg gggcgtgggg
3421 gggcgtggag ggaggcccca gggggtctca cccatgcaag cagaggacca gggcctttc

3481 tggcaccgca gttttgtttt aaaactggac ctgtatattt gtaaagctat ttatgggccc
3541 ctggcactct tgttcccaca ccccaacact tccagcattt agctggccac atggcggaga
3601 gttttaattt ttaacttatt gacaaccgag aaggtttatc ccgccgatag agggacggcc
3661 aagaatgtac gtccagcctg ccccggagct ggaggatccc ctccaagcct aaaaggttgt
3721 taatagttgg aggtgattcc agtgaagata ttttatttcc tttgtccttt ttcaggagaa
3781 ttagatttct ataggatttt tctttaggag atttattttt tggacttcaa agcaagctgg
3841 tattttcata caaattcttc taattgctgt gtgtcccagg cagggagacg gtttccaggg
3901 aggggccggc cctgtgtgca ggttccgatg ttattagatg ttacaagttt atatatatct
3961 atatatataa tttattgagt ttttacaaga tgtatttgtt gtagacttaa cacttcttac
4021 gcaatgcttc tagagtttta tagcctggac tgctaccttt caaagcttgg agggaagccg
4081 tgaattcagt tggttcgttc tgtactgtta ctgggccctg agtctgggca gctgtccctt
4141 gcttgcctgc agggccatgg ctcagggtgg tctcttcttg gggcccagtg catggtggcc
4201 agaggtgtca cccaaaccgg caggtgcgat tttgttaacc cagcgacgaa ctttccgaaa
4261 aataaagaca cctggttgct aacctggaaa aaaaaaaaaa aaaa

The Genbank ID for the TACC3 gene is 10460. SEQ ID NO: 92 is the TACC3 Amino Acid Sequence (NP_006333) (838 aa). 
1 MSLQVLNDKN VSNEKNTENC DFLFSPPEVT GRSSVLRVSQ KENVPPKNLA KAMKVTFQTP
61 LRDPQTHRIL SPSMASKLEA PFTQDDTLGL ENSHPVWTQK ENQQLIKEVD AKTTHGILQK
121 PVEADTDLLG DASPAFGSGS SSESGPGALA DLDCSSSSQS PGSSENQMVS PGKVSGSPEQ
181 AVEENLSSYS LDRRVTPASE TLEDPCRTES QHKAETPHGA EEECKAETPH GAEEECRHGG
241 VCAPAAVATS PPGAIPKEAC GGAPLQGLPG EALGCPAGVG TPVPADGTQT LTCAHTSAPE
301 STAPTNHLVA GRAMTLSPQE EVAAGQMASS SRSGPVKLEF DVSDGATSKR APPPRRLGER
361 SGLKPPLRKA AVRQQKAPQE VEEDDGRSGA GEDPPMPASR GSYHLDWDKM DDPNFIPFGG
421 DTKSGCSEAQ PPESPETRLG QPAAEQLHAG PATEEPGPCL SQQLHSASAE DTPVVQLAAE
481 TPTAESKERA LNSASTSLPT SCPGSEPVPT HQQGQPALEL KEESFRDPAE VLGTGAEVDY
541 LEQFGTSSFK ESALRKQSLY LKFDPLLRDS PGRPVPVATE TSSMHGANET PSGRPREAKL
601 VEFDFLGALD IPVPGPPPGV PAPGGPPLST GPIVDLLQYS QKDLDAVVKA TQEENRELRS
661 RCEELHGKNL ELGKIMDRFE EVVYQAMEEV QKQKELSKAE IQKVLKEKDQ LTTDLNSMEK
721 SFSDLFKRFE KQKEVIEGYR KNEESLKKCV EDYLARITQE GQRYQALKAH AEEKLQLANE
781 EIAQVRSKAQ AEALALQASL RKEQMRIQSL EKTVEQKTKE NEELTRICDD LISKMEKI
SEQ ID NO: 93 is the TACC3 Nucleotide Sequence (NM_006342) (2847 bp): 
1 gcgtttgaaa ctccggcgcg ccggcggcca tcaagggcta gaagcgcgac ggcggtagca
61 gctaggcttg gcccccggcg tggagcagac gcggacccct ccttcctggc ggcggcggcg
121 cgggctcaga gcccggcaac gggcgggcgg gcagaatgag tctgcaggtc ttaaacgaca
181 aaaatgtcag caatgaaaaa aatacagaaa attgcgactt cctgttttcg ccaccagaag
241 ttaccggaag atcgtctgtt cttcgtgtgt cacagaaaga aaatgtgcca cccaagaacc
301 tggccaaagc tatgaaggtg acttttcaga cacctctgcg ggatccacag acgcacagga
361 ttctaagtcc tagcatggcc agcaaacttg aggctccttt cactcaggat gacacccttg
421 gactggaaaa ctcacacccg gtctggacac agaaagagaa ccaacagctc atcaaggaag
481 tggatgccaa aactactcat ggaattctac agaaaccagt ggaggctgac accgacctcc
541 tgggggatgc aagcccagcc tttgggagtg gcagctccag cgagtctggc ccaggtgccc
601 tggctgacct ggactgctca agctcttccc agagcccagg aagttctgag aaccaaatgg
661 tgtctccagg aaaagtgtct ggcagccctg agcaagccgt ggaggaaaac cttagttcct
721 attccttaga cagaagagtg acacccgcct ctgagaccct agaagaccct tgcaggacag
781 agtcccagca caaagcggag actccgcacg gagccgagga agaatgcaaa gcggagactc
841 cgcacggagc cgaggaggaa tgccggcacg gtggggtctg tgctcccgca gcagtggcca
901 cttcgcctcc tggtgcaatc cctaaggaag cctgcggagg agcacccctg cagggtctgc
961 ctggcgaagc cctgggctgc cctgcgggtg tgggcacccc cgtgccagca gatggcactc
1021 agacccttac ctgtgcacac acctctgctc ctgagagcac agccccaacc aaccacctgg
1081 tggctggcag ggccatgacc ctgagtcctc aggaagaagt ggctgcaggc caaatggcca
1141 gctcctcgag gagcggacct gtaaaactag aatttgatgt atctgatggc gccaccagca
1201 aaagggcacc cccaccaagg agactgggag agaggtccgg cctcaagcct cccttgagga
1261 aagcagcagt gaggcagcaa aaggccccgc aggaggtgga ggaggacgac ggtaggagcg
 
1321 gagcaggaga ggaccccccc atgccagctt ctcggggctc ttaccacctc gactgggaca
1381 aaatggatga cccaaacttc atcccgttcg gaggtgacac caagtctggt tgcagtgagg
1441 cccagccccc agaaagccct gagaccaggc tgggccagcc agcggctgaa cagttgcatg
1501 ctgggcctgc cacggaggag ccaggtccct gtctgagcca gcagctgcat tcagcctcag
1561 cggaggacac gcctgtggtg cagttggcag ccgagacccc aacagcagag agcaaggaga
1621 gagccttgaa ctctgccagc acctcgcttc ccacaagctg tccaggcagt gagccagtgc
1681 ccacccatca gcaggggcag cctgccttgg agctgaaaga ggagagcttc agagaccccg
1741 ctgaggttct aggcacgggc gcggaggtgg attacctgga gcagtttgga acttcctcgt
1801 ttaaggagtc ggccttgagg aagcagtcct tatacctcaa gttcgacccc ctcctgaggg
1861 acagtcctgg tagaccagtg cccgtggcca ccgagaccag cagcatgcac ggtgcaaatg
1921 agactccctc aggacgtccg cgggaagcca agcttgtgga gttcgatttc ttgggagcac
1981 tggacattcc tgtgccaggc ccacccccag gtgttcccgc gcctgggggc ccacccctgt
2041 ccaccggacc tatagtggac ctgctccagt acagccagaa ggacctggat gcagtggtaa
2101 aggcgacaca ggaggagaac cgggagctga ggagcaggtg tgaggagctc cacgggaaga
2161 acctggaact ggggaagatc atggacaggt tcgaagaggt tgtgtaccag gccatggagg
2221 aagttcagaa gcagaaggaa ctttccaaag ctgaaatcca gaaagttcta aaagaaaaag
2281 accaacttac cacagatctg aactccatgg agaagtcctt ctccgacctc ttcaagcgtt
2341 ttgagaaaca gaaagaggtg atcgagggct accgcaagaa cgaagagtca ctgaagaagt
2401 gcgtggagga ttacctggca aggatcaccc aggagggcca gaggtaccaa gccctgaagg
2461 cccacgcgga ggagaagctg cagctggcaa acgaggagat cgcccaggtc cggagcaagg
2521 cccaggcgga agcgttggcc ctccaggcca gcctgaggaa ggagcagatg cgcatccagt
2581 cgctggagaa gacagtggag cagaagacta aagagaacga ggagctgacc aggatctgcg
2641 acgacctcat ctccaagatg gagaagatct gacctccacg gagccgctgt ccccgccccc
2701 ctgctcccgt ctgtctgtcc tgtctgattc tcttaggtgt catgttcttt tttctgtctt
2761 gtcttcaact tttttaaaaa ctagattgct ttgaaaacat gactcaataa aagtttcctt
2821 tcaatttaaa cactgaaaaa aaaaaaa
SEQ ID NO: 94 is the nucleotide sequence of FGFR3-TACC3.

1 gtcgcgggca gctggcgccg cgcggtcctg ctctgccggt cgcacggacg caccggcggg
61 ccgccggccg gagggacggg gcgggagctg ggcccgcgga cagcgagccg gagcgggagc
121 cgcgcgtagc gagccgggct ccggcgctcg ccagtctccc gagcggcgcc cgcctcccgc
181 cggtgcccgc gccgggccgt ggggggcagc atgcccgcgc gcgctgcctg aggacgccgc
241 ggcccccgcc cccgccatgg gcgcccctgc ctgcgccctc gcgctctgcg tggccgtggc
301 catcgtggcc ggcgcctcct cggagtcctt ggggacggag cagcgcgtcg tggggcgagc
361 ggcagaagtc ccgggcccag agcccggcca gcaggagcag ttggtcttcg gcagcgggga
421 tgctgtggag ctgagctgtc ccccgcccgg gggtggtccc atggggccca ctgtctgggt
481 caaggatggc acagggctgg tgccctcgga gcgtgtcctg gtggggcccc agcggctgca
541 ggtgctgaat gcctcccacg aggactccgg ggcctacagc tgccggcagc ggctcacgca
601 gcgcgtactg tgccacttca gtgtgcgggt gacagacgct ccatcctcgg gagatgacga
661 agacggggag gacgaggctg aggacacagg tgtggacaca ggggcccctt actggacacg
721 gcccgagcgg atggacaaga agctgctggc cgtgccggcc gccaacaccg tccgcttccg
781 ctgcccagcc gctggcaacc ccactccctc catctcctgg ctgaagaacg gcagggagtt
841 ccgcggcgag caccgcattg gaggcatcaa gctgcggcat cagcagtgga gcctggtcat
901 ggaaagcgtg gtgccctcgg accgcggcaa ctacacctgc gtcgtggaga acaagtttgg
961 cagcatccgg cagacgtaca cgctggacgt gctggagcgc tccccgcacc ggcccatcct
1021 gcaggcgggg ctgccggcca accagacggc ggtgctgggc agcgacgtgg agttccactg
1081 caaggtgtac agtgacgcac agccccacat ccagtggctc aagcacgtgg aggtgaatgg
1141 cagcaaggtg ggcccggacg gcacacccta cgttaccgtg ctcaagacgg cgggcgctaa
1201 caccaccgac aaggagctag aggttctctc cttgcacaac gtcacctttg aggacgccgg
1261 ggagtacacc tgcctggcgg gcaattctat tgggttttct catcactctg cgtggctggt
1321 ggtgctgcca gccgaggagg agctggtgga ggctgacgag gcgggcagtg tgtatgcagg
1381 catcctcagc tacggggtgg gcttcttcct gttcatcctg gtggtggcgg ctgtgacgct
1441 ctgccgcctg cgcagccccc ccaagaaagg cctgggctcc cccaccgtgc acaagatctc
1501 ccgcttcccg ctcaagcgac aggtgtccct ggagtccaac gcgtccatga gctccaacac
1561 accactggtg cgcatcgcaa ggctgtcctc aggggagggc cccacgctgg ccaatgtctc
1621 cgagctcgag ctgcctgccg accccaaatg ggagctgtct cgggcccggc tgaccctggg
1681 caagcccctt ggggagggct gcttcggcca ggtggtcatg gcggaggcca tcggcattga
1741 caaggaccgg gccgccaagc ctgtcaccgt agccgtgaag atgctgaaag acgatgccac
1801 tgacaaggac ctgtcggacc tggtgtctga gatggagatg atgaagatga tcgggaaaca

1861 caaaaacatc atcaacctgc tgggcgcctg cacgcagggc gggcccctgt acgtgctggt
1921 ggagtacgcg gccaagggta acctgcggga gtttctgcgg gcgcggcggc ccccgggcct
1981 ggactactcc ttcgacacct gcaagccgcc cgaggagcag ctcaccttca aggacctggt
2041 gtcctgtgcc taccaggtgg cccggggcat ggagtacttg gcctcccaga agtgcatcca
2101 cagggacctg gctgcccgca atgtgctggt gaccgaggac aacgtgatga agatcgcaga
2161 cttcgggctg gcccgggacg tgcacaacct cgactactac aagaagacaa ccaacggccg
2221 gctgcccgtg aagtggatgg cgcctgaggc cttgtttgac cgagtctaca ctcaccagag
2281 tgacgtctgg tcctttgggg tcctgctctg ggagatcttc acgctggggg gctccccgta
2341 ccccggcatc cctgtggagg agctcttcaa gctgctgaag gagggccacc gcatggacaa
2401 gcccgccaac tgcacacacg acctgtacat gatcatgcgg gagtgctggc atgccgcgcc
2461 ctcccagagg cccaccttca agcagctggt ggaggacctg gaccgtgtcc ttaccgtgac
2521 gtccaccgac tttaaggagt cggccttgag gaagcagtcc ttatacctca agttcgaccc
2581 cctcctgagg gacagtcctg gtagaccagt gcccgtggcc accgagacca gcagcatgca
2641 cggtgcaaat gagactccct caggacgtcc gcgggaagcc aagcttgtgg agttcgattt
2701 cttgggagca ctggacattc ctgtgccagg cccaccccca ggtgttcccg cgcctggggg
2761 cccacccctg tccaccggac ctatagtgga cctgctccag tacagccaga aggacctgga
2821 tgcagtggta aaggcgacac aggaggagaa ccgggagctg aggagcaggt gtgaggagct
2881 ccacgggaag aacctggaac tggggaagat catggacagg ttcgaagagg ttgtgtacca
2941 ggccatggag gaagttcaga agcagaagga actttccaaa gctgaaatcc agaaagttct
3001 aaaagaaaaa gaccaactta ccacagatct gaactccatg gagaagtcct tctccgacct
3061 cttcaagcgt tttgagaaac agaaagaggt gatcgagggc taccgcaaga acgaagagtc
3121 actgaagaag tgcgtggagg attacctggc aaggatcacc caggagggcc agaggtacca
3181 agccctgaag gcccacgcgg aggagaagct gcagctggca aacgaggaga tcgcccaggt
3241 ccggagcaag gcccaggcgg aagcgttggc cctccaggcc agcctgagga aggagcagat
3301 gcgcatccag tcgctggaga agacagtgga gcagaagact aaagagaacg aggagctgac
3361 caggatctgc gacgacctca tctccaagat ggagaagatc tgacctccac ggagccgctg
3421 tccccgcccc cctgctcccg tctgtctgtc ctgtctgatt ctcttaggtg tcatgttctt
3481 ttttctgtct tgtcttcaac ttttttaaaa actagattgc tttgaaaaca tgactcaata
3541 aaagtttcct ttcaatttaa acactgaaaa aaaaaaaa

The Genbank ID for the FGFR1 gene is 2260. Eight isoforms are listed for FGFR1, e.g., having Genebank Accession Nos. NP_001167534 (corresponding nucleotide sequence NM_001174063); NP_001167535 (corresponding nucleotide sequence NM_001174064); NP_001167536 (corresponding nucleotide sequence NM_001174065); NP_001167537 (corresponding nucleotide sequence NM_001174066); NP_001 1 67538 (corresponding nucleotide sequence NM_001174067); NP_056934 (corresponding nucleotide sequence NM_015850); NP_075593 (corresponding nucleotide sequence NM_023105); NP_075594 (corresponding nucleotide sequence NM_023106); NP_075598 (corresponding nucleotide sequence NM_023110).
SEQ ID NO: 146 is the FGFR1 Amino Acid Sequence for isoform 10, having Genebank Accession No. NP_001167534 (820 aa): 
1 MWSWKCLLFW AVLVTATLCT ARPSPTLPEQ AQPWGAPVEV ESFLVHPGDL LQLRCRLRDD
61 VQSINWLRDG VQLAESNRTR ITGEEVEVQD SVPADSGLYA CVTSSPSGSD TTYFSVNVSD
121 ALPSSEDDDD DDDSSSEEKE TDNTKPNRMP VAPYWTSPEK MEKKLHAVPA AKTVKFKCPS
181 SGTPNPTLRW LKNGKEFKPD HRIGGYKVRY ATWSIIMDSV VPSDKGNYTC IVENEYGSIN
241 HTYQLDVVER SPHRPILQAG LPANKTVALG SNVEFMCKVY SDPQPHIQWL KHIEVNGSKI
301 GPDNLPYVQI LKTAGVNTTD KEMEVLHLRN VSFEDAGEYT CLAGNSIGLS HHSAWLTVLE
361 ALEERPAVMT SPLYLEIIIY CTGAFLISCM VGSVIVYKMK SGTKKSDFHS QMAVHKLAKS
421 IPLRRQVSAD SSASMNSGVL LVRPSRLSSS GTPMLAGVSE YELPEDPRWE LPRDRLVLGK
 
481 PLGEGCFGQV VLAEAIGLDK DKPNRVTKVA VKMLKSDATE KDLSDLISEM EMMKMIGKHK
541 NIINLLGACT QDGPLYVIVE YASKGNLREY LQARRPPGLE YCYNPSHNPE EQLSSKDLVS
601 CAYQVARGME YLASKKCIHR DLAARNVLVT EDNVMKIADF GLARDIHHID YYKKTTNGRL
661 PVKWMAPEAL FDRIYTHQSD VWSFGVLLWE IFTLGGSPYP GVPVEELFKL LKEGHRMDKP
721 SNCTNELYMM MRDCWHAVPS QRPTFKQLVE DLDRIVALTS NQEYLDLSMP LDQYSPSFPD
781 TRSSTCSSGE DSVFSHEPLP EEPCLPRHPA QLANGGLKRR
SEQ ID NO: 147 is the FGFR1 Nucleotide Sequence for isoform 10, having Genebank Accession No. NM_001174063 (5895 bp): 
1 agatgcaggg gcgcaaacgc caaaggagac caggctgtag gaagagaagg gcagagcgcc
61 ggacagctcg gcccgctccc cgtcctttgg ggccgcggct ggggaactac aaggcccagc
121 aggcagctgc agggggcgga ggcggaggag ggaccagcgc gggtgggagt gagagagcga
181 gccctcgcgc cccgccggcg catagcgctc ggagcgctct tgcggccaca ggcgcggcgt
241 cctcggcggc gggcggcagc tagcgggagc cgggacgccg gtgcagccgc agcgcgcgga
301 ggaacccggg tgtgccggga gctgggcggc cacgtccgga cgggaccgag acccctcgta
361 gcgcattgcg gcgacctcgc cttccccggc cgcgagcgcg ccgctgcttg aaaagccgcg
421 gaacccaagg acttttctcc ggtccgagct cggggcgccc cgcagggcgc acggtacccg
481 tgctgcagtc gggcacgccg cggcgccggg gcctccgcag ggcgatggag cccggtctgc
541 aaggaaagtg aggcgccgcc gctgcgttct ggaggagggg ggcacaaggt ctggagaccc
601 cgggtggcgg acgggagccc tccccccgcc ccgcctccgg ggcaccagct ccggctccat
661 tgttcccgcc cgggctggag gcgccgagca ccgagcgccg ccgggagtcg agcgccggcc
721 gcggagctct tgcgaccccg ccaggacccg aacagagccc gggggcggcg ggccggagcc
781 ggggacgcgg gcacacgccc gctcgcacaa gccacggcgg actctcccga ggcggaacct
841 ccacgccgag cgagggtcag tttgaaaagg aggatcgagc tcactgtgga gtatccatgg
901 agatgtggag ccttgtcacc aacctctaac tgcagaactg ggatgtggag ctggaagtgc
961 ctcctcttct gggctgtgct ggtcacagcc acactctgca ccgctaggcc gtccccgacc
1021 ttgcctgaac aagcccagcc ctggggagcc cctgtggaag tggagtcctt cctggtccac
1081 cccggtgacc tgctgcagct tcgctgtcgg ctgcgggacg atgtgcagag catcaactgg
1141 ctgcgggacg gggtgcagct ggcggaaagc aaccgcaccc gcatcacagg ggaggaggtg
1201 gaggtgcagg actccgtgcc cgcagactcc ggcctctatg cttgcgtaac cagcagcccc
1261 tcgggcagtg acaccaccta cttctccgtc aatgtttcag atgctctccc ctcctcggag
1321 gatgatgatg atgatgatga ctcctcttca gaggagaaag aaacagataa caccaaacca
1381 aaccgtatgc ccgtagctcc atattggaca tccccagaaa agatggaaaa gaaattgcat
1441 gcagtgccgg ctgccaagac agtgaagttc aaatgccctt ccagtgggac cccaaacccc
1501 acactgcgct ggttgaaaaa tggcaaagaa ttcaaacctg accacagaat tggaggctac
1561 aaggtccgtt atgccacctg gagcatcata atggactctg tggtgccctc tgacaagggc
1621 aactacacct gcattgtgga gaatgagtac ggcagcatca accacacata ccagctggat
1681 gtcgtggagc ggtcccctca ccggcccatc ctgcaagcag ggttgcccgc caacaaaaca
1741 gtggccctgg gtagcaacgt ggagttcatg tgtaaggtgt acagtgaccc gcagccgcac
1801 atccagtggc taaagcacat cgaggtgaat gggagcaaga ttggcccaga caacctgcct
1861 tatgtccaga tcttgaagac tgctggagtt aataccaccg acaaagagat ggaggtgctt
1921 cacttaagaa atgtctcctt tgaggacgca ggggagtata cgtgcttggc gggtaactct
1981 atcggactct cccatcactc tgcatggttg accgttctgg aagccctgga agagaggccg
2041 gcagtgatga cctcgcccct gtacctggag atcatcatct attgcacagg ggccttcctc
2101 atctcctgca tggtggggtc ggtcatcgtc tacaagatga agagtggtac caagaagagt
2161 gacttccaca gccagatggc tgtgcacaag ctggccaaga gcatccctct gcgcagacag
2221 gtgtctgctg actccagtgc atccatgaac tctggggttc ttctggttcg gccatcacgg
2281 ctctcctcca gtgggactcc catgctagca ggggtctctg agtatgagct tcccgaagac
2341 cctcgctggg agctgcctcg ggacagactg gtcttaggca aacccctggg agagggctgc
2401 tttgggcagg tggtgttggc agaggctatc gggctggaca aggacaaacc caaccgtgtg
2461 accaaagtgg ctgtgaagat gttgaagtcg gacgcaacag agaaagactt gtcagacctg
2521 atctcagaaa tggagatgat gaagatgatc gggaagcata agaatatcat caacctgctg
2581 ggggcctgca cgcaggatgg tcccttgtat gtcatcgtgg agtatgcctc caagggcaac
2641 ctgcgggagt acctgcaggc ccggaggccc ccagggctgg aatactgcta caaccccagc
2701 cacaacccag aggagcagct ctcctccaag gacctggtgt cctgcgccta ccaggtggcc
2761 cgaggcatgg agtatctggc ctccaagaag tgcatacacc gagacctggc agccaggaat
2821 gtcctggtga cagaggacaa tgtgatgaag atagcagact ttggcctcgc acgggacatt
2881 caccacatcg actactataa aaagacaacc aacggccgac tgcctgtgaa gtggatggca
 
2941 cccgaggcat tatttgaccg gatctacacc caccagagtg atgtgtggtc tttcggggtg
3001 ctcctgtggg agatcttcac tctgggcggc tccccatacc ccggtgtgcc tgtggaggaa
3061 cttttcaagc tgctgaagga gggtcaccgc atggacaagc ccagtaactg caccaacgag
3121 ctgtacatga tgatgcggga ctgctggcat gcagtgccct cacagagacc caccttcaag
3181 cagctggtgg aagacctgga ccgcatcgtg gccttgacct ccaaccagga gtacctggac
3241 ctgtccatgc ccctggacca gtactccccc agctttcccg acacccggag ctctacgtgc
3301 tcctcagggg aggattccgt cttctctcat gagccgctgc ccgaggagcc ctgcctgccc
3361 cgacacccag cccagcttgc caatggcgga ctcaaacgcc gctgactgcc acccacacgc
3421 cctccccaga ctccaccgtc agctgtaacc ctcacccaca gcccctgctg ggcccaccac
3481 ctgtccgtcc ctgtcccctt tcctgctggc aggagccggc tgcctaccag gggccttcct
3541 gtgtggcctg ccttcacccc actcagctca cctctccctc cacctcctct ccacctgctg
3601 gtgagaggtg caaagaggca gatctttgct gccagccact tcatcccctc ccagatgttg
3661 gaccaacacc cctccctgcc accaggcact gcctggaggg cagggagtgg gagccaatga
3721 acaggcatgc aagtgagagc ttcctgagct ttctcctgtc ggtttggtct gttttgcctt
3781 cacccataag cccctcgcac tctggtggca ggtgccttgt cctcagggct acagcagtag
3841 ggaggtcagt gcttcgtgcc tcgattgaag gtgacctctg ccccagatag gtggtgccag
3901 tggcttatta attccgatac tagtttgctt tgctgaccaa atgcctggta ccagaggatg
3961 gtgaggcgaa ggccaggttg ggggcagtgt tgtggccctg gggcccagcc ccaaactggg
4021 ggctctgtat atagctatga agaaaacaca aagtgtataa atctgagtat atatttacat
4081 gtctttttaa aagggtcgtt accagagatt tacccatcgg gtaagatgct cctggtggct
4141 gggaggcatc agttgctata tattaaaaac aaaaaagaaa aaaaaggaaa atgtttttaa
4201 aaaggtcata tattttttgc tacttttgct gttttatttt tttaaattat gttctaaacc
4261 tattttcagt ttaggtccct caataaaaat tgctgctgct tcatttatct atgggctgta
4321 tgaaaagggt gggaatgtcc actggaaaga agggacaccc acgggccctg gggctaggtc
4381 tgtcccgagg gcaccgcatg ctcccggcgc aggttccttg taacctcttc ttcctaggtc
4441 ctgcacccag acctcacgac gcacctcctg cctctccgct gcttttggaa agtcagaaaa
4501 agaagatgtc tgcttcgagg gcaggaaccc catccatgca gtagaggcgc tgggcagaga
4561 gtcaaggccc agcagccatc gaccatggat ggtttcctcc aaggaaaccg gtggggttgg
4621 gctggggagg gggcacctac ctaggaatag ccacggggta gagctacagt gattaagagg
4681 aaagcaaggg cgcggttgct cacgcctgta atcccagcac tttgggacac cgaggtgggc
4741 agatcacttc aggtcaggag tttgagacca gcctggccaa cttagtgaaa ccccatctct
4801 actaaaaatg caaaaattat ccaggcatgg tggcacacgc ctgtaatccc agctccacag
4861 gaggctgagg cagaatccct tgaagctggg aggcggaggt tgcagtgagc cgagattgcg
4921 ccattgcact ccagcctggg caacagagaa aacaaaaagg aaaacaaatg atgaaggtct
4981 gcagaaactg aaacccagac atgtgtctgc cccctctatg tgggcatggt tttgccagtg
5041 cttctaagtg caggagaaca tgtcacctga ggctagtttt gcattcaggt ccctggcttc
5101 gtttcttgtt ggtatgcctc cccagatcgt ccttcctgta tccatgtgac cagactgtat
5161 ttgttgggac tgtcgcagat cttggcttct tacagttctt cctgtccaaa ctccatcctg
5221 tccctcagga acggggggaa aattctccga atgtttttgg ttttttggct gcttggaatt
5281 tacttctgcc acctgctggt catcactgtc ctcactaagt ggattctggc tcccccgtac
5341 ctcatggctc aaactaccac tcctcagtcg ctatattaaa gcttatattt tgctggatta
5401 ctgctaaata caaaagaaag ttcaatatgt tttcatttct gtagggaaaa tgggattgct
5461 gctttaaatt tctgagctag ggattttttg gcagctgcag tgttggcgac tattgtaaaa
5521 ttctctttgt ttctctctgt aaatagcacc tgctaacatt acaatttgta tttatgttta
5581 aagaaggcat catttggtga acagaactag gaaatgaatt tttagctctt aaaagcattt
5641 gctttgagac cgcacaggag tgtctttcct tgtaaaacag tgatgataat ttctgccttg
5701 gccctacctt gaagcaatgt tgtgtgaagg gatgaagaat ctaaaagtct tcataagtcc
5761 ttgggagagg tgctagaaaa atataaggca ctatcataat tacagtgatg tccttgctgt
5821 tactactcaa atcacccaca aatttcccca aagactgcgc tagctgtcaa ataaaagaca
5881 gtgaaattga cctga
SEQ ID NO: 185 is the FGFR1 Amino Acid Sequence for isoform 1, having Genebank. Accession No. NP_075598 (822 aa): 
1 
61 
121 
181 
241 
 
301 
361 
421 
481 
541 
601 
661 
721 
781 
SEQ ID NO: 186 is the FGFR1 Nucleotide Sequence for isoform 1, having Genebank Accession No. NM_023110 (5917 bp):

1 agatgcaggg gcgcaaacgc caaaggagac caggctgtag gaagagaagg gcagagcgcc
61 ggacagctcg gcccgctccc cgtcctttgg ggccgcggct ggggaactac aaggcccagc
121 aggcagctgc agggggcgga ggcggaggag ggaccagcgc gggtgggagt gagagagcga
181 gccctcgcgc cccgccggcg catagcgctc ggagcgctct tgcggccaca ggcgcggcgt
241 cctcggcggc gggcggcagc tagcgggagc cgggacgccg gtgcagccgc agcgcgcgga
301 ggaacccggg tgtgccggga gctgggcggc cacgtccgga cgggaccgag acccctcgta
361 gcgcattgcg gcgacctcgc cttccccggc cgcgagcgcg ccgctgcttg aaaagccgcg
421 gaacccaagg acttttctcc ggtccgagct cggggcgccc cgcagggcgc acggtacccg
481 tgctgcagtc gggcacgccg cggcgccggg gcctccgcag ggcgatggag cccggtctgc
541 aaggaaagtg aggcgccgcc gctgcgttct ggaggagggg ggcacaaggt ctggagaccc
601 cgggtggcgg acgggagccc tccccccgcc ccgcctccgg ggcaccagct ccggctccat
661 tgttcccgcc cgggctggag gcgccgagca ccgagcgccg ccgggagtcg agcgccggcc
721 gcggagctct tgcgaccccg ccaggacccg aacagagccc gggggcggcg ggccggagcc
781 ggggacgcgg gcacacgccc gctcgcacaa gccacggcgg actctcccga ggcggaacct
841 ccacgccgag cgagggtcag tttgaaaagg aggatcgagc tcactgtgga gtatccatgg
901 agatgtggag ccttgtcacc aacctctaac tgcagaactg ggatgtggag ctggaagtgc
961 ctcctcttct gggctgtgct ggtcacagcc acactctgca ccgctaggcc gtccccgacc
1021 ttgcctgaac aagcccagcc ctggggagcc cctgtggaag tggagtcctt cctggtccac
1081 cccggtgacc tgctgcagct tcgctgtcgg ctgcgggacg atgtgcagag catcaactgg
1141 ctgcgggacg gggtgcagct ggcggaaagc aaccgcaccc gcatcacagg ggaggaggtg
1201 gaggtgcagg actccgtgcc cgcagactcc ggcctctatg cttgcgtaac cagcagcccc
1261 tcgggcagtg acaccaccta cttctccgtc aatgtttcag atgctctccc ctcctcggag
1321 gatgatgatg atgatgatga ctcctcttca gaggagaaag aaacagataa caccaaacca
1381 aaccgtatgc ccgtagctcc atattggaca tccccagaaa agatggaaaa gaaattgcat
1441 gcagtgccgg ctgccaagac agtgaagttc aaatgccctt ccagtgggac cccaaacccc
1501 acactgcgct ggttgaaaaa tggcaaagaa ttcaaacctg accacagaat tggaggctac
1561 aaggtccgtt atgccacctg gagcatcata atggactctg tggtgccctc tgacaagggc
1621 aactacacct gcattgtgga gaatgagtac ggcagcatca accacacata ccagctggat
1681 gtcgtggagc ggtcccctca ccggcccatc ctgcaagcag ggttgcccgc caacaaaaca
1741 gtggccctgg gtagcaacgt ggagttcatg tgtaaggtgt acagtgaccc gcagccgcac
1801 atccagtggc taaagcacat cgaggtgaat gggagcaaga ttggcccaga caacctgcct
1861 tatgtccaga tcttgaagac tgctggagtt aataccaccg acaaagagat ggaggtgctt
1921 cacttaagaa atgtctcctt tgaggacgca ggggagtata cgtgcttggc gggtaactct
1981 atcggactct cccatcactc tgcatggttg accgttctgg aagccctgga agagaggccg
2041 gcagtgatga cctcgcccct gtacctggag atcatcatct attgcacagg ggccttcctc
2101 atctcctgca tggtggggtc ggtcatcgtc tacaagatga agagtggtac caagaagagt
2161 gacttccaca gccagatggc tgtgcacaag ctggccaaga gcatccctct gcgcagacag
2221 gtaacagtgt ctgctgactc cagtgcatcc atgaactctg gggttcttct ggttcggcca
2281 tcacggctct cctccagtgg gactcccatg ctagcagggg tctctgagta tgagcttccc
2341 gaagaccctc gctgggagct gcctcgggac agactggtct taggcaaacc cctgggagag
2401 ggctgctttg ggcaggtggt gttggcagag gctatcgggc tggacaagga caaacccaac
2461 cgtgtgacca aagtggctgt gaagatgttg aagtcggacg caacagagaa agacttgtca
2521 gacctgatct cagaaatgga gatgatgaag atgatcggga agcataagaa tatcatcaac
2581 ctgctggggg cctgcacgca ggatggtccc ttgtatgtca tcgtggagta tgcctccaag
2641 ggcaacctgc gggagtacct gcaggcccgg aggcccccag ggctggaata ctgctacaac

2701 cccagccaca acccagagga gcagctctcc tccaaggacc tggtgtcctg cgcctaccag
2761 gtggcccgag gcatggagta tctggcctcc aagaagtgca tacaccgaga cctggcagcc
2821 aggaatgtcc tggtgacaga ggacaatgtg atgaagatag cagactttgg cctcgcacgg
2881 gacattcacc acatcgacta ctataaaaag acaaccaacg gccgactgcc tgtgaagtgg
2941 atggcacccg aggcattatt tgaccggatc tacacccacc agagtgatgt gtggtctttc
3001 ggggtgctcc tgtgggagat cttcactctg ggcggctccc cataccccgg tgtgcctgtg
3061 gaggaacttt tcaagctgct gaaggagggt caccgcatgg acaagcccag taactgcacc
3121 aacgagctgt acatgatgat gcgggactgc tggcatgcag tgccctcaca gagacccacc
3181 ttcaagcagc tggtggaaga cctggaccgc atcgtggcct tgacctccaa ccaggagtac
3241 ctggacctgt ccatgcccct ggaccagtac tcccccagct ttcccgacac ccggagctct
3301 acgtgctcct caggggagga ttccgtcttc tctcatgagc cgctgcccga ggagccctgc
3361 ctgccccgac acccagccca gcttgccaat ggcggactca aacgccgctg actgccaccc
3421 acacgccctc cccagactcc accgtcagct gtaaccctca cccacagccc ctgctgggcc
3481 caccacctgt ccgtccctgt cccctttcct gctggcagga gccggctgcc taccaggggc
3541 cttcctgtgt ggcctgcctt caccccactc agctcacctc tccctccacc tcctctccac
3601 ctgctggtga gaggtgcaaa gaggcagatc tttgctgcca gccacttcat cccctcccag
3661 atgttggacc aacacccctc cctgccacca ggcactgcct ggagggcagg gagtgggagc
3721 caatgaacag gcatgcaagt gagagcttcc tgagctttct cctgtcggtt tggtctgttt
3781 tgccttcacc cataagcccc tcgcactctg gtggcaggtg ccttgtcctc agggctacag
3841 cagtagggag gtcagtgctt cgtgcctcga ttgaaggtga cctctgcccc agataggtgg
3901 tgccagtggc ttattaattc cgatactagt ttgctttgct gaccaaatgc ctggtaccag
3961 aggatggtga ggcgaaggcc aggttggggg cagtgttgtg gccctggggc ccagccccaa
4021 actgggggct ctgtatatag ctatgaagaa aacacaaagt gtataaatct gagtatatat
4081 ttacatgtct ttttaaaagg gtcgttacca gagatttacc catcgggtaa gatgctcctg
4141 gtggctggga ggcatcagtt gctatatatt aaaaacaaaa aagaaaaaaa aggaaaatgt
4201 ttttaaaaag gtcatatatt ttttgctact tttgctgttt tattttttta aattatgttc
4261 taaacctatt ttcagtttag gtccctcaat aaaaattgct gctgcttcat ttatctatgg
4321 gctgtatgaa aagggtggga atgtccactg gaaagaaggg acacccacgg gccctggggc
4381 taggtctgtc ccgagggcac cgcatgctcc cggcgcaggt tccttgtaac ctcttcttcc
4441 taggtcctgc acccagacct cacgacgcac ctcctgcctc tccgctgctt ttggaaagtc
4501 agaaaaagaa gatgtctgct tcgagggcag gaaccccatc catgcagtag aggcgctggg
4561 cagagagtca aggcccagca gccatcgacc atggatggtt tcctccaagg aaaccggtgg
4621 ggttgggctg ggaggggggc acctacctag gaatagccac ggggtagagc tacagtgatt
4681 aagaggaaag caagggcgcg gttgctcacg cctgtaatcc cagcactttg ggacaccgag
4741 gtgggcagat cacttcaggt caggagtttg agaccagcct ggccaactta gtgaaacccc
4801 atctctacta aaaatgcaaa aattatccag gcatggtggc acacgcctgt aatcccagct
4861 ccacaggagg ctgaggcaga atcccttgaa gctgggaggc ggaggttgca gtgagccgag
4921 attgcgccat tgcactccag cctgggcaac agagaaaaca aaaaggaaaa caaatgatga
4981 aggtctgcag aaactgaaac ccagacatgt gtctgccccc tctatgtggg catggttttg
5041 ccagtgcttc taagtgcagg agaacatgtc acctgaggct agttttgcat tcaggtccct
5101 ggcttcgttt cttgttggta tgcctcccca gatcgtcctt cctgtatcca tgtgaccaga
5161 ctgtatttgt tgggactgtc gcagatcttg gcttcttaca gttcttcctg tccaaactcc
5221 atcctgtccc tcaggaacgg ggggaaaatt ctccgaatgt ttttggtttt ttggctgctt
5281 ggaatttact tctgccacct gctggtcatc actgtcctca ctaagtggat tctggctccc
5341 ccgtacctca tggctcaaac taccactcct cagtcgctat attaaagctt atattttgct
5401 ggattactgc taaatacaaa agaaagttca atatgttttc atttctgtag ggaaaatggg
5461 attgctgctt taaatttctg agctagggat tttttggcag ctgcagtgtt ggcgactatt
5521 gtaaaattct ctttgtttct ctctgtaaat agcacctgct aacattacaa tttgtattta
5581 tgtttaaaga aggcatcatt tggtgaacag aactaggaaa tgaattttta gctcttaaaa
5641 gcatttgctt tgagaccgca caggagtgtc tttccttgta aaacagtgat gataatttct
5701 gccttggccc taccttgaag caatgttgtg tgaagggatg aagaatctaa aagtcttcat
5761 aagtccttgg gagaggtgct agaaaaatat aaggcactat cataattaca gtgatgtcct
5821 tgctgttact actcaaatca cccacaaatt tccccaaaga ctgcgctagc tgtcaaataa
5881 aagacagtga aattgacctg aaaaaaaaaa aaaaaaa

The Genbank ID for the TACC1 gene is 6867. Three isoforms are listed for TACC1, e.g., having Genebank Accession Nos. NP_006274 (corresponding nucleotide sequence NM_001174063); NP_001167535 (corresponding nucleotide sequence NM_001174064);NP_001167536 (corresponding nucleotide sequence NM_001174065).
SEQ ID NO: 148 is the TACC1 Amino Acid Sequence for isoform 1, having Genebank Accession No. NP 006274 (805 aa): 
1 MAFSPWQILS PVQWAKWTWS AVRGGAAGED EAGGPEGDPE EEDSQAETKS LSFSSDSEGN
61 FETPEAETPI RSPFKESCDP SLGLAGPGAK SQESQEADEQ LVAEVVEKCS SKTCSKPSEN
121 EVPQQAIDSH SVKNFREEPE HDFSKISIVR PFSIETKDST DISAVLGTKA AHGCVTAVSG
181 KALPSSPPDA LQDEAMTEGS MGVTLEASAE ADLKAGNSCP ELVPSRRSKL RKPKPVPLRK
241 KAIGGEFSDT NAAVEGTPLP KASYHFSPEE LDENTSPLLG DARFQKSPPD LKETPGTLSS
301 DTNDSGVELG EESRSSPLKL EFDFTEDTGN IEARKALPRK LGRKLGSTLT PKIQKDGISK
361 SAGLEQPTDP VARDGPLSQT SSKPDPSQWE SPSFNPFGSH SVLQNSPPLS SEGSYHFDPD
421 NFDESMDPFK PTTTLTSSDF CSPTGNHVNE ILESPKKAKS RLITSGCKVK KHETQSLALD
481 ACSRDEGAVI SQISDISNRD GHATDEEKLA STSCGQKSAG AEVKGEPEED LEYFECSNVP
541 VSTINHAFSS SEAGIEKETC QKMEEDGSTV LGLLESSAEK APVSVSCGGE SPLDGICLSE
601 SDKTAVLTLI REEIITKEIE ANEWKKKYEE TRQEVLEMRK IVAEYEKTIA QMIEDEQRTS
661 MTSQKSFQQL TMEKEQALAD LNSVERSLSD LFRRYENLKG VLEGFKKNEE ALKKCAQDYL
721 ARVKQEEQRY QALKIHAEEK LDKANEEIAQ VRTKAKAESA ALHAGLRKEQ MKVESLERAL
781 QQKNQEIEEL TKICDELIAK LGKTD
SEQ ID NO: 149 is the TACC1 Nucleotide Sequence for isoform 1, having Genebank Accession No. NM_006283 (7802 bp): 
1 agctgatgcg cgccccgccg gccgggaggc gggagtccgc gagccgggag cgggagcagc
61 agaggtctag cagccgggcg ccgcgggccg ggggcctgag gaggccacag gacgggcgtc
121 ttcccggcta gtggagcccg gcgcggggcc cgctgcggcc gcaccgtgag ggaggaggc
181 cgaggaggac gcagcgccgg ctgccggcgg gaggaagcgc tccaccaggg cccccgacgg
241 cactcgttta accacatccg cgcctctgct ggaaacgctt gctggcgcct gtcaccggtt
301 ccctccattt tgaaagggaa aaaggctctc cccacccatt cccctgcccc taggagctgg
361 agccggagga gccgcgctca tggcgttcag cccgtggcag atcctgtccc ccgtgcagtg
421 ggcgaaatgg acgtggtctg cggtacgcgg cggggccgcc ggcgaggacg aggctggcgg
481 gcccgagggc gaccccgagg aggaggattc gcaagccgag accaaatcct tgagtttcag
541 ctcggattct gaaggtaatt ttgagactcc tgaagctgaa accccgatcc gatcaccttt
601 caaggagtcc tgtgatccat cactcggatt ggcaggacct ggggccaaaa gccaagaatc
661 acaagaagct gatgaacagc ttgtagcaga agtggttgaa aaatgttcat ctaagacttg
721 ttctaaacct tcagaaaatg aagtgccaca gcaggccatt gactctcact cagtcaagaa
781 tttcagagaa gaacctgaac atgattttag caaaatttcc atcgtgaggc cattttcaat
841 agaaacgaag gattccacgg atatctcggc agtcctcgga acaaaagcag ctcatggctg
901 tgtaactgca gtctcaggca aggctctgcc ttccagcccg ccagacgccc tccaggacga
961 ggcgatgaca gaaggcagca tgggggtcac cctcgaggcc tccgcagaag ctgatctaaa
1021 agctggcaac tcctgtccag agcttgtgcc cagcagaaga agcaagctga gaaagcccaa
1081 gcctgtcccc ctgaggaaga aagcaattgg aggagagttc tcagacacca acgctgctgt
1141 ggagggcaca cctctcccca aggcatccta tcacttcagt cctgaagagt tggatgagaa
1201 cacaagtcct ttgctaggag atgccaggtt ccagaagtct ccccctgacc ttaaagaaac
1261 tcccggcact ctcagtagtg acaccaacga ctcaggggtt gagctggggg aggagtcgag
1321 gagctcacct ctcaagcttg agtttgattt cacagaagat acaggaaaca tagaggccag
1381 gaaagccctt ccaaggaagc ttggcaggaa actgggtagc acactgactc ccaagataca
1441 aaaagatggc atcagtaagt cagcaggttt agaacagcct acagacccag tggcacgaga
1501 cgggcctctc tcccaaacat cttccaagcc agatcctagt cagtgggaaa gccccagctt
1561 caaccccttt gggagccact ctgttctgca gaactcccca cccctctctt ctgagggctc
1621 ctaccacttt gacccagata actttgacga atccatggat ccctttaaac caactacgac
1681 cttaacaagc agtgactttt gttctcccac tggtaatcac gttaatgaaa tcttagaatc
1741 acccaagaag gcaaagtcgc gtttaataac gagtggctgt aaggtgaaga agcatgaaac
1801 tcagtctctc gccctggatg catgttctcg ggatgaaggg gcagtgatct cccagatttc
1861 agacatttct aatagggatg gccatgctac tgatgaggag aaactggcat ccacgtcatg
1921 tggtcagaaa tcagctggtg ccgaggtgaa aggtgagcca gaggaagacc tggagtactt
 
1981 tgaatgttcc aatgttcctg tgtctaccat aaatcatgcg ttttcatcct cagaagcagg
2041 catagagaag gagacgtgcc agaagatgga agaagacggg tccactgtgc ttgggctgct
2101 ggagtcctct gcagagaagg cccctgtgtc ggtgtcctgt ggaggtgaga gccccctgga
2161 tgggatctgc ctcagcgaat cagacaagac agccgtgctc accttaataa gagaagagat
2221 aattactaaa gagattgaag caaatgaatg gaagaagaaa tacgaagaga cccggcaaga
2281 agttttggag atgaggaaaa ttgtagctga atatgaaaag actattgctc aaatgattga
2341 agatgaacaa aggacaagta tgacctctca gaagagcttc cagcaactga ccatggagaa
2401 ggaacaggcc ctggctgacc ttaactctgt ggaaaggtcc ctttctgatc tcttcaggag
2461 atatgagaac ctgaaaggtg ttctggaagg gttcaagaag aatgaagaag ccttgaagaa
2521 atgtgctcag gattacttag ccagagttaa acaagaggag cagcgatacc aggccctgaa
2581 aatccacgca gaagagaaac tggacaaagc caatgaagag attgctcagg ttcgaacaaa
2641 agcaaaggct gagagtgcag ctctccatgc tggactccgc aaagagcaga tgaaggtgga
2701 gtccctggaa agggccctgc agcagaagaa ccaagaaatt gaagaactga caaaaatctg
2761 tgatgagctg attgcaaagc tgggaaagac tgactgagac actccccctg ttagctcaac
2821 agatctgcat ttggctgctt ctcttgtgac cacaattatc ttgccttatc caggaataat
2881 tgcccctttg cagagaaaaa aaaaaactta aaaaaagcac atgcctactg ctgcctgtcc
2941 cgctttgctg ccaatgcaac agccctggaa gaaaccctag agggttgcat agtctagaaa
3001 ggagtgtgac ctgacagtgc tggagcctcc tagtttcccc ctatgaaggt tcccttaggc
3061 tgctgagttt gggtttgtga tttatcttta gtttgtttta aagtcatctt tactttccca
3121 aatgtgttaa atttgtaact cctctttggg gtcttctcca ccacctgtct gatttttttg
3181 tgatctgttt aatcttttaa ttttttagta tcagtggttt tatttaagga gacagtttgg
3241 cctattgtta cttccaattt ataatcaaga aggggctctg gatccccttt taaattacac
3301 acactctcac acacatacat gtatgtttat agatgctgct gctcttttcc ctgaagcata
3361 gtcaagtaag aactgctcta cagaaggaca tatttccttg gatgtgagac cctattttga
3421 aatagagtcc tgactcagaa caccaactta agaatttggg ggattaaaga tgtgaagacc
3481 acagtcttgg gttttcatat ctggagaaga ctatttgcca tgacgttttg ttgccctggt
3541 atttggacac tcctcagctt taatgggtgt ggccccttta gggttagtcc tcagactaat
3601 gatagtgtct gctttctgca tgaacggcaa tatgggactc cctccaagct agggtttggc
3661 aagtctgccc tagagtcatt tactctcctc tgcctccatt tgttaataca gaatcaacat
3721 ttagtcttca ttatcttttt tttttttttt gagacagagt ttcgatctat tttaagtatg
3781 tgaagaaaat ctacttgtaa aaggctcaga tcttaattaa aaggtaattg tagcacatta
3841 ccaattataa ggtgaagaaa tgtttttttc ccaagtgtga tgcattgttc ttcagatgtt
3901 gaaaagaaag caaaaaatac cttctaactt aagacagaat ttttaacaaa atgagcagta
3961 aaagtcacat gaaccactcc aaaaatcagt gcattttgca tatttttaaa caaagacagc
4021 ttgttgaata ctgagaagag gagtgcaagg agaaggtctg tactaacaaa gccaaattcc
4081 tcaagctctt actggactca gttcagagtg gtgggccatt aaccccaaca tggaattttt
4141 ccatataaat ctcaatgaat tccctttcat ttgaataggc aaacccaaat ccatgcaagt
4201 gttttaaagc actgtcctgt cttaatctta catgctgaaa gtcttcatgg tgatatgcac
4261 tatattcagt atacgtatgt tttcctactt ctcttgtaaa actgttgcat gatccaactt
4321 cagcaatgaa ttgtgcctag tggagaacct ctatagatct taaaaaatga attattcttt
4381 agcagtgtat tactcacatg ggtgcaatct ttagccccag ggaggtcaat aatgtctttt
4441 aaagccagaa gtcacatttt accaatatgc atttatcata attggtgctt aggctgtata
4501 ttcaagcctg ttgtcttaac attttgtata aaaaagaaca acagaaatta tctgtcattt
4561 gagaagtggc ttgacaatca tttgagcttt gaaagcagtc actgtggtgt aatatgaatg
4621 ctgtcctagt ggtcatagta ccaagggcac gtgtctcccc ttggtataac tgatttcctt
4681 tttagtcctc tactgctaaa taagttaatt ttgcattttg cagaaagaaa cattgattgc
4741 taaatctttt tgctgctgtg ttttggtgtt ttcatgttta cttgttttat attgatctgt
4801 tttaagtatg agaggcttat agtgccctcc attgtaaatc catagtcatc tttttaagct
4861 tattgtgttt aagaaagtag ctatgtgtta aacagaggtg atggcagccc ttccctagca
4921 cactggtgga agagacccct taagaacctg accccagtga atgaagctga tgcacaggga
4981 gcaccaaagg accttcgtta agtgataatt gtcctggcct ctcagccatg accgttatga
5041 ggaaatatcc cccattcgaa cttaacagat gcctcctctc caaagagaat taaaatcgta
5101 gcttgtacag atcaagagaa tatactgggc agaatgaagt atgtttgttt atttttcttt
5161 aaaaataaag gattttggaa ctctggagag taagaatata gtatagagtt tgcctcaaca
5221 catgtgaggg ccaaataacc tgctagctag gcagtaataa actctgttac agaagagaaa
5281 aagggccggg cacagtggct tattcctgta atcccaacac tgtggaaggc cgaggcagga
5341 ggatcacttg agtccaggag tttgaaacct acctaggcaa catggtgaaa ccttgtctct
5401 accaaaataa aaattagctg ggcatggtgg cacgtgcctg tggtcccagc tacttgggag
5461 gctgaggtgg gagcctggga ggtcaaggct gcagtgagcc atgatcatgc cactgcactc
5521 catcctgggt gacagcaaga tcttgtctca aaaaaaaaaa aaaaaaaaaa aaaaccagga
5581 gtgaaaaagg aaagtagaag gcagctgctg gcctagatgt tggtttggga atattaggtg
 
5641 atcctgttga gattctggat ccagagcaat ttctttagct tttgactttg ccaaagtgta
5701 gatagccttt atccagcagt attttaagtg gggaatgcaa cgtgaggcca actgaacaat
5761 tccccccgtg gctgcccaga tagtcacagt caaggttgga gagtctcctt ccagccagtg
5821 acctacccaa accttttgtt ctgtaaaact gctctggaaa taccgggaag cccagttttc
5881 tcacgtggtt tctagcttct tcagactcag cccaaattag gaagtgcaga agcacatgat
5941 ggtgaaaaac ctaggatttg gcagccttcc agaatggtat ggaatctgag ggaagattta
6001 tgtttcgttt tggaggatag ctcaagttga attttctttc cagccagtta ccctttcaac
6061 ctacccatac tttgtacaac tcttacacaa atacttagat atttattaga tagccctgaa
6121 ttcactctaa ttataaacag ggagtgtaaa ctgcccccag atgttcctgg gctgggtaaa
6181 agcagctgga gtgaagcact cattttccat aaaggtaaca aagggcagct cagtggttac
6241 tcaagctcaa aagggttttt ttaagagcaa gcattggtta agtctgtgta tactgagttg
6301 gaagtgattt cagcacattc ttttttagtg gagtgaaagt tctgaagccc ccttttaact
6361 tcctcttggt ttttcattat aattggtagc catctcatga actgtctctg actgttgtct
6421 ctttgtggtc atgtgattgt gagcttgctt tctgacttgc atttctgact ttatcctgtt
6481 gttaggaaga tagaaactag gttttgaaag attacatgat tcaagcgagg gattttaaag
6541 taaagatgta tttattctga agaatctaaa agataacaga ttatttgctt atgaaagaac
6601 aatatagtct gggaatccca gaatgtcaag ccaaaggtct aagaagtcat ctccttcaaa
6661 tactttaata aagaagtatt tcgaggagat atctgtccaa aaaggtttga ctggcctcca
6721 gattccagtt atttttaaaa agcaacttac cactaaatcc ttgagtctcc atagagtaac
6781 agtaaagaaa ctgatgtaac agactctcct ctcaaaggat ctcctctgga agagactatc
6841 agcggcagca ttctccaggg aagacccatc ccctagtgcc agagcttgca tcctggagac
6901 taaagattgc acttttttgt agttttttgt ccaaatgcaa tcccatttct gtgcctctta
6961 gcatgcagtt agatttggac aaacaagatt cctaaggaat gactttatta actataatat
7021 ggttacagct attatataaa tatatattct ggttatagtt ctaatatgga gatgttgtgt
7081 gcaatgctgg cctgtggtgg tctgtgtaat gctttaactt gtatggagga ggccaggctc
7141 agagctgaga tgtggcctga accttccctg tatcgatcct ttaatttaga actgtcaaga
7201 tgtcactttc tccccctctg ccttttagtg gtatctgaca tatactcaaa acagtaattt
7261 cctggtcaca tcattaactg ctaattctgt atttataaag aattttcaga tggacatgta
7321 caaatttgaa ctcaaaccat ccccagtcca gatacagggc agcgtgtagg tgaccacacc
7381 agagcctcag cctcggtcct tctcagccgt cgggatagga tccaggcatt tcttttaaat
7441 ctcagaggta gcagtaaact tttcagtatt gctgttagca agtgtgtgtt tgccaataga
7501 tacccattat actaatgtgc caagtaaatg ttcattgcac atctgcttcc actgtgttcc
7561 cacgggtgcc atgaagtgtg tgaggagccc ctcatctgga gggatgagtg ctgcgttgac
7621 tactgctatc aggattgtgt tgtgtggaat attcatctac ataaatttta tatgcacagt
7681 aatttccctt tttatatgtc aagtaactat ttgtaaaagt tatactcaca aattattata
7741 atgattacta atatattttt tccatgtttc attgcctgaa taaaaactgt ttaccactgt
7801 ta
SEQ ID NO: 150 is the amino acid sequence of the FGFR1-TACC1 fusion protein. 
MWSWKCLLFWAVLVTATLCTARPSPTLPEQDALPSSEDDDDDDDSSSEEKETDNTKPNPVAPYWTSPEKMEKKLH
AVPAAKTVKFKCPSSGTPNPTLRWLKNGKEFKPDHRIGGYKVRYATWSIIMDSVVPSDKGNYTCIVENEYGSINH
TYQLDVVERSPHRPILQAGLPANKTVALGSNVEFMCKVYSDPQPHIQWLKHIEVNGSKIGPDNLPYVQILKTAGV
NTTDKEMEVLHLRNVSFEDAGEYTCLAGNSIGLSHHSAWLTVLEALEERPAVMTSPLYLEIIIYCTGAFLISCMV
GSVIVYKMKSGTKKSDFHSQMAVHKLAKSIPLRRQVTVSADSSASMNSGVLLVRPSRLSSSGTPMLAGVSEYELP
EDPRWELPRDRLVLGKPLGEGCFGQVVLAEAIGLDKDKPNRVTKVAVKMLKSDATEKDLSDLISEMEMMKMIGKH
KNIINLLGACTQDGPLYVIVEYASKGNLREYLQARRPPGLEYCYNPSHNPEEQLSSKDLVSCAYQVARGMEYLAS
KKCIHRDLAARNVLVTEDNVMKIADFGLARDIHHIDYYKKTTNGRLPVKWMAPEALFDRIYTHQSDVWSFGVLLW
EIFTLGGSPYPGVPVEELFKLLKEGHRMDKPSNCTNELYMMMRDCWHAVPSQRPTFKQLVEDLDRIVALTSNQGL
LESSAEKAPVSVSCGGESPLDGICLSESDKTAVLTLIREEIITKEIEANEWKKKYEETRQEVLEMRKIVAEYEKT
IAQMIEDEQRTSMTSQKSFQQLTMEKEQALADLNSVERSLSDLFRRYENLKGVLEGFKKNEEALKKCAQDYLARV
KQEEQRYQALKIHAEEKLDKANEEIAQVRTKAKAESAALHAGLRKEQMKVESLERALQQKNQEIEELTKICDELI
AKLGKTD
SEQ ID NO: 151 is the nucleotide sequence that encodes the FGFR1-TACC1 fusion protein.

atgtggagctggaagtgcctcctcttctgggctgtgctggtcacagccacactctgcaccgctaggccgtccccg
accttgcctgaacaagcccagccctggggagcccctgtggaagtggagtccttcctggtccaccccggtgacctg
ctgcagcttcgctgtcggctgcgggacgatgtgcagagcatcaactggctgcgggacggggtgcagctggcgga
agcaaccgcacccgcatcacaggggaggaggtggaggtgcaggactccgtgcccgcagactccggcctctatgct
tgcgtaaccagcagcccctcgggcagtgacaccacctacttctccgtcaatgtttcagatgctctcccctcctcg
gaggatgatgatgatgatgatgactcctcttcagaggagaaagaaacagataacaccaaaccaaaccgtatgccc
gtagctccatattggacatccccagaaaagatggaaaagaaattgcatgcagtgccggctgccaagacagtgaag
ttcaaatgcccttccagtgggaccccaaaccccacactgcgctggttgaaaaatggcaaagaattcaaacctgac
cacagaattggaggctacaaggtccgttatgccacctggagcatcataatggactctgtggtgccctctgacaag
ggcaactacacctgcattgtggagaatgagtacggcagcatcaaccacacataccagctggatgtcgtggagcgg
tcccctcaccggcccatcctgcaagcagggttgcccgccaacaaaacagtggccctgggtagcaacgtggagttc
atgtgtaaggtgtacagtgacccgcagccgcacatccagtggctaaagcacatcgaggtgaatggagcaagatt
ggcccagacaacctgccttatgtccagatcttgaagactgctggagttaataccaccgacaaagagatggaggtg
cttcacttaagaaatgtctcctttgaggacgcaggggagtatacgtgcttggcgggtaactctatcggactctcc
catcactctgcatggttgaccgttctggaagccctggaagagaggccggcagtgatgacctcgcccctgtacctg
gagatcatcatctattgcacaggggccttcctcatctcctgcatggtggggtcggtcatcgtctacaagatgaag
agtggtaccaagaagagtgacttccacagccagatggctgtgcacaagctggccaagagcatccctctgcgcaga
caggtgtctgctgactccagtgcatccatgaactctggggttcttctggttcggccatcacggctctcctccagt
gggactcccatgctagcaggggtctctgagtatgagcttcccgaagaccctcgctgggagctgcctcgggacaga
ctggtcttaggcaaacccctgggagagggctgctttgggcaggtggtgttggcagaggctatcggctggacaag
gacaaacccaaccgtgtgaccaaagtggctgtgaagatgttgaagtcggacgcaacagagaaagacttgtcagac
ctgatctcagaaatggagatgatgaagatgatcgggaagcataagaatatcatcaacctgctgggggcctgcacg
caggatggtcccttgtatgtcatcgtggagtatgcctccaagggcaacctgcgggagtacctgcaggcccggagg
cccccagggctggaatactgctacaaccccagccacaacccagaggagcagctctcctccaaggacctggtgtcc
tgcgcctaccaggtggcccgaggcatggagtatctggcctccaagaagtgcatacaccgagacctggcagccagg
aatgtcctggtgacagaggacaatgtgatgaagatagcagactttggcctcgcacgggacattcaccacatcgac
tactataaaaagacaaccaacggccgactgcctgtgaagtggatggcacccgaggcattatttgaccggatctac
acccaccagagtgatgtgtggtctttcggggtgctcctgtgggagatcttcactctgggcggctccccatacccc
ggtgtgcctgtggaggaacttttcaagctgctgaaggagggtcaccgcatggacaagcccagtaactgcaccaac
gagctgtacatgatgatgcgggactgctggcatgcagtgccctcacagagacccaccttcaagcagctggtggaa
gacctggaccgcatcgtggccttgacctccaaccagtgggctgctggagtcctctgcagagaaggcccctgtgtc
ggtgtcctgtggaggtgagagccccctggatgggatctgcctcagcgaatcagacaagacagccgtgctcacctt
aataagagaagagataattactaaagagattgaagcaaatgaatggaagaagaaatacgaagagacccggcaaga
agttttggagatgaggaaaattgtagctgaatatgaaaagactattgctcaaatgattgaagatgaacaaaggac
aagtatgacctctcagaagagcttccagcaactgaccatggagaaggaacaggccctggctgaccttaactctgt
ggaaaggtccctttctgatctcttcaggagatatgagaacctgaaaggtgttctggaagggttcaagaagaatga
agaagccttgaagaaatgtgctcaggattacttagccagagttaaacaagaggagcagcgataccaggccctgaa
aatccacgcagaagagaaactggacaaagccaatgaagagattgctcaggttcgaacaaaagcaaaggctgagag
tgcagctctccatgctggactccgcaaagagcagatgaaggtggagtccctggaaagggccctgcagcagaagaa
ccaagaaattgaagaactgacaaaaatctgtgatgagctgattgcaaagctgggaaagactgac

The Genbank ID for the FGFR2 gene is 2263. Eight isoforms are listed for FGFR2, e.g., having Genebank Accession Nos. NP_000132 (corresponding nucleotide sequence NM_000141)., NP_001 138385 (corresponding nucleotide sequence NM_001144913); NP_001138386 (corresponding nucleotide sequence NM_001144914); NP_001138387 (corresponding nucleotide sequence NM_001144915); NP_001138388 (corresponding nucleotide sequence NM_001144916); NP_001138389 (corresponding nucleotide sequence NM_001144917); NP _001138390 (corresponding nucleotide sequence NM_001144918); NP_001138391 (corresponding nucleotide sequence NM_001144919), NP_075259 (corresponding nucleotide sequence NM_022970).
SEQ ID NO: 152 is the FGFR2 Amino Acid Sequence for isoform 1, having Genebank Accession No. NP_000132 (821 aa): 
1 
61 
121 
181 
241 
301 
361 
421 
481 
541 
601 
661 
721 
781 
SEQ ID NO: 153 is the FGFR2 Nucleotide Sequence for isoform 1, having Genebank Accession No. NM_000141 (4654 bp):

1 ggcggcggct ggaggagagc gcggtggaga gccgagcggg cgggcggcgg gtgcggagcg
61 ggcgagggag cgcgcgcggc cgccacaaag ctcgggcgcc gcggggctgc atgcggcgta
121 cctggcccgg cgcggcgact gctctccggg ctggcggggg ccggccgcga gccccggggg
181 ccccgaggcc gcagcttgcc tgcgcgctct gagccttcgc aactcgcgag caaagtttgg
241 tggaggcaac gccaagcctg agtcctttct tcctctcgtt ccccaaatcc gagggcagcc
301 cgcgggcgtc atgcccgcgc tcctccgcag cctggggtac gcgtgaagcc cgggaggctt
361 ggcgccggcg aagacccaag gaccactctt ctgcgtttgg agttgctccc cgcaaccccg
421 ggctcgtcgc tttctccatc ccgacccacg cggggcgcgg ggacaacaca ggtcgcggag
481 gagcgttgcc attcaagtga ctgcagcagc agcggcagcg cctcggttcc tgagcccacc
541 gcaggctgaa ggcattgcgc gtagtccatg cccgtagagg aagtgtgcag atgggattaa
601 cgtccacatg gagatatgga agaggaccgg ggattggtac cgtaaccatg gtcagctggg
661 gtcgtttcat ctgcctggtc gtggtcacca tggcaacctt gtccctggcc cggccctcct
721 tcagtttagt tgaggatacc acattagagc cagaagagcc accaaccaaa taccaaatct
781 ctcaaccaga agtgtacgtg gctgcgccag gggagtcgct agaggtgcgc tgcctgttga
841 aagatgccgc cgtgatcagt tggactaagg atggggtgca cttggggccc aacaatagga
901 cagtgcttat tggggagtac ttgcagataa agggcgccac gcctagagac tccggcctct
961 atgcttgtac tgccagtagg actgtagaca gtgaaacttg gtacttcatg gtgaatgtca
1021 cagatgccat ctcatccgga gatgatgagg atgacaccga tggtgcggaa gattttgtca
1081 gtgagaacag taacaacaag agagcaccat actggaccaa cacagaaaag atggaaaagc
1141 ggctccatgc tgtgcctgcg gccaacactg tcaagtttcg ctgcccagcc gggggaaacc
1201 caatgccaac catgcggtgg ctgaaaaacg ggaaggagtt taagcaggag catcgcattg
1261 gaggctacaa ggtacgaaac cagcactgga gcctcattat ggaaagtgtg gtcccatctg
1321 acaagggaaa ttatacctgt gtagtggaga atgaatacgg gtccatcaat cacacgtacc
1381 acctggatgt tgtggagcga tcgcctcacc ggcccatcct ccaagccgga ctgccggcaa
1441 atgcctccac agtggtcgga ggagacgtag agtttgtctg caaggtttac agtgatgccc
1501 agccccacat ccagtggatc aagcacgtgg aaaagaacgg cagtaaatac gggcccgacg
1561 ggctgcccta cctcaaggtt ctcaaggccg ccggtgttaa caccacggac aaagagattg
1621 aggttctcta tattcggaat gtaacttttg aggacgctgg ggaatatacg tgcttggcgg
1681 gtaattctat tgggatatcc tttcactctg catggttgac agttctgcca gcgcctggaa
1741 gagaaaagga gattacagct tccccagact acctggagat agccatttac tgcatagggg
1801 tcttcttaat cgcctgtatg gtggtaacag tcatcctgtg ccgaatgaag aacacgacca
1861 agaagccaga cttcagcagc cagccggctg tgcacaagct gaccaaacgt atccccctgc
1921 ggagacaggt aacagtttcg gctgagtcca gctcctccat gaactccaac accccgctgg
1981 tgaggataac aacacgcctc tcttcaacgg cagacacccc catgctggca ggggtctccg
2041 agtatgaact tccagaggac ccaaaatggg agtttccaag agataagctg acactgggca
2101 agcccctggg agaaggttgc tttgggcaag tggtcatggc ggaagcagtg ggaattgaca

2161 aagacaagcc caaggaggcg gtcaccgtgg ccgtgaagat gttgaaagat gatgccacag
2221 agaaagacct ttctgatctg gtgtcagaga tggagatgat gaagatgatt gggaaacaca
2281 agaatatcat aaatcttctt ggagcctgca cacaggatgg gcctctctat gtcatagttg
2341 agtatgcctc taaaggcaac ctccgagaat acctccgagc ccggaggcca cccgggatgg
2401 agtactccta tgacattaac cgtgttcctg aggagcagat gaccttcaag gacttggtgt
2461 catgcaccta ccagctggcc agaggcatgg agtacttggc ttcccaaaaa tgtattcatc
2521 gagatttagc agccagaaat gttttggtaa cagaaaacaa tgtgatgaaa atagcagact
2581 ttggactcgc cagagatatc aacaatatag actattacaa aaagaccacc aatgggcggc
2641 ttccagtcaa gtggatggct ccagaagccc tgtttgatag agtatacact catcagagtg
2701 atgtctggtc cttcggggtg ttaatgtggg agatcttcac tttagggggc tcgccctacc
2761 cagggattcc cgtggaggaa ctttttaagc tgctgaagga aggacacaga atggataagc
2821 cagccaactg caccaacgaa ctgtacatga tgatgaggga ctgttggcat gcagtgccct
2881 cccagagacc aacgttcaag cagttggtag aagacttgga tcgaattctc actctcacaa
2941 ccaatgagga atacttggac ctcagccaac ctctcgaaca gtattcacct agttaccctg
3001 acacaagaag ttcttgttct tcaggagatg attctgtttt ttctccagac cccatgcctt
3061 acgaaccatg ccttcctcag tatccacaca taaacggcag tgttaaaaca tgaatgactg
3121 tgtctgcctg tccccaaaca ggacagcact gggaacctag ctacactgag cagggagacc
3181 atgcctccca gagcttgttg tctccacttg tatatatgga tcagaggagt aaataattgg
3241 aaaagtaatc agcatatgtg taaagattta tacagttgaa aacttgtaat cttccccagg
3301 aggagaagaa ggtttctgga gcagtggact gccacaagcc accatgtaac ccctctcacc
3361 tgccgtgcgt actggctgtg gaccagtagg actcaaggtg gacgtgcgtt ctgccttcct
3421 tgttaatttt gtaataattg gagaagattt atgtcagcac acacttacag agcacaaatg
3481 cagtatatag gtgctggatg tatgtaaata tattcaaatt atgtataaat atatattata
3541 tatttacaag gagttatttt ttgtattgat tttaaatgga tgtcccaatg cacctagaaa
3601 attggtctct ctttttttaa tagctatttg ctaaatgctg ttcttacaca taatttctta
3661 attttcaccg agcagaggtg gaaaaatact tttgctttca gggaaaatgg tataacgtta
3721 atttattaat aaattggtaa tatacaaaac aattaatcat ttatagtttt ttttgtaatt
3781 taagtggcat ttctatgcag gcagcacagc agactagtta atctattgct tggacttaac
3841 tagttatcag atcctttgaa aagagaatat ttacaatata tgactaattt ggggaaaatg
3901 aagttttgat ttatttgtgt ttaaatgctg ctgtcagacg attgttctta gacctcctaa
3961 atgccccata ttaaaagaac tcattcatag gaaggtgttt cattttggtg tgcaaccctg
4021 tcattacgtc aacgcaacgt ctaactggac ttcccaagat aaatggtacc agcgtcctct
4081 taaaagatgc cttaatccat tccttgagga cagaccttag ttgaaatgat agcagaatgt
4141 gcttctctct ggcagctggc cttctgcttc tgagttgcac attaatcaga ttagcctgta
4201 ttctcttcag tgaattttga taatggcttc cagactcttt ggcgttggag acgcctgtta
4261 ggatcttcaa gtcccatcat agaaaattga aacacagagt tgttctgctg atagttttgg
4321 ggatacgtcc atctttttaa gggattgctt tcatctaatt ctggcaggac ctcaccaaaa
4381 gatccagcct catacctaca tcagacaaaa tatcgccgtt gttccttctg tactaaagta
4441 ttgtgttttg ctttggaaac acccactcac tttgcaatag ccgtgcaaga tgaatgcaga
4501 ttacactgat cttatgtgtt acaaaattgg agaaagtatt taataaaacc tgttaatttt
4561 tatactgaca ataaaaatgt ttctacagat attaatgtta acaagacaaa ataaatgtca
4621 cgcaacttat ttttttaata aaaaaaaaaa aaaa

The Genbank ID for the TACC2 gene is 10579. Four isoforms are listed for TACC2, e.g., having Genebank Accession Nos. NP_996744 (corresponding nucleotide sequence NM_206862); NP_996743 (corresponding nucleotide sequence NM_206861); NP_996742 (corresponding nucleotide sequence NM_206860); NP_008928 (corresponding nucleotide sequence NM_006997).
SEQ ID NO: 154 is the TACC2. Amino Acid Sequence for isoform a, having Genebank Accession No. NP_996744 (2948 aa): 1 MGNENSTSDN QRTLSAQTPR SAQPPGNSQN IKRKQQDTPG SPDHRDASSI GSVGLGGFCT 
61 ASESSASLDP CLVSPEVTEP RKDPQGARGP EGSLLPSPPP SQEREHPSSS MPFAECPPEG
121 CLASPAAAPE DGPQTQSPRR EPAPNAPGDI AAAFPAERDS STPYQEIAAV PSAGRERQPK
181 EEGQKSSFSF SSGIDQSPGM SPVPLREPMK APLCGEGDQP GGFESQEKEA AGGFPPAESR
241 QGVASVQVTP EAPAAAQQGT ESSAVLEKSP LKPMAPIPQD PAPRASDRER GQGEAPPQYL
301 TDDLEFLRAC HLPRSNSGAA PEAEVNAASQ ESCQQPVGAY LPHAELPWGL PSPALVPEAG
361 GSGKEALDTI DVQGHPQTGM RGTKPNQVVC VAAGGQPEGG LPVSPEPSLL TPTEEAHPAS
421 SLASFPAAQI PIAVEEPGSS SRESVSKAGM PVSADAAKEV VDAGLVGLER QVSDLGSKGE
481 HPEGDPGEVP APSPQERGEH LNTEQSHEVQ PGVPPPPLPK EQSHEVQPGA PPPPLPKAPS
541 ESARGPPGPT DGAKVHEDST SPAVAKEGSR SPGDSPGGKE EAPEPPDGGD PGNLQGEDSQ
601 AFSSKRDPEV GKDELSKPSS DAESRDHPSS HSAQPPRKGG AGHTDGPHSQ TAEADASGLP
661 HKLGEEDPVL PPVPDGAGEP TVPEGAIWEG SGLQPKCPDT LQSREGLGRM ESFLTLESEK
721 SDFPPTPVAE VAPKAQEGES TLEIRKMGSC DGEGLLTSPD QPRGPACDAS RQEFHAGVPH
781 PPQGENLAAD LGLTALILDQ DQQGIPSCPG EGWIRGAASE WPLLSSEKHL QPSQAQPETS
841 IFDVLKEQAQ PPENGKETSP SHPGFKDQGA DSSQIHVPVE PQEDNNLPTH GGQEQALGSE
901 LQSQLPKGTL SDTPTSSPTD MVWESSLTEE SELSAPTRQK LPALGEKRPE GACGDGQSSR
961 VSPPAADVLK DFSLAGNFSR KETCCTGQGP NKSQQALADA LEEGSQHEEA CQRHPGASEA
1021 ADGCSPLWGL SKREMASGNT GEAPPCQPDS VALLDAVPCL PALAPASPGV TPTQDAPETE
1081 ACDETQEGRQ QPVPAPQQKM ECWATSDAES PKLLASFPSA GEQGGEAGAA ETGGSAGAGD
1141 PGKQQAPEKP GEATLSCGLL QTEHCLTSGE EASTSALRES CQAEHPMASC QDALLPAREL
1201 GGIPRSTMDF STHQAVPDPK ELLLSGPPEV AAPDTPYLHV DSAAQRGAED SGVKAVSSAD
1261 PRAPGESPCP VGEPPLALEN AASLKLFAGS LAPLLQPGAA GGEIPAVQAS SGSPKARTTE
1321 GPVDSMPCLD RMPLLAKGKQ ATGEEKAATA PGAGAKASGE GMAGDAAGET EGSMERMGEP
1381 SQDPKQGTSG GVDTSSEQIA TLTGFPDFRE HIAKIFEKPV LGALATPGEK AGAGRSAVGK
1441 DLTRPLGPEK LLDGPPGVDV TLLPAPPARL QVEKKQQLAG EAEISHLALQ DPASDKLLGP
1501 AGLTWERNLP GAGVGKEMAG VPPTLREDER PEGPGAAWPG LEGQAYSQLE RSRQELASGL
1561 PSPAATQELP VERAAAFQVA PHSHGEEAVA QDRIPSGKQH QETSACDSPH GEDGPGDFAH
1621 TGVPGHVPRS TCAPSPQREV LTVPEANSEP WTLDTLGGER RPGVTAGILE MRNALGNQST
1681 PAPPTGEVAD TPLEPGKVAG AAGEAEGDIT LSTAETQACA SGDLPEAGTT RTFSVVAGDL
1741 VLPGSCQDPA CSDKAPGMEG TAALHGDSPA RPQQAKEQPG PERPIPAGDG KVCVSSPPEP
1801 DETHDPKLQH LAPEELHTDR ESPRPGPSML PSVPKKDAPR VMDKVTSDET RGAEGTESSP
1861 VADDIIQPAA PADLESPTLA ASSYHGDVVG QVSTDLIAQS ISPAAAHAGL PPSAAEHIVS
1921 PSAPAGDRVE ASTPSCPDPA KDLSRSSDSE EAFETPESTT PVKAPPAPPP PPPEVIPEPE
1981 VSTQPPPEEP GCGSETVPVP DGPRSDSVEG SPFRPPSHSF SAVFDEDKPI ASSGTYNLDF
2041 DNIELVDTFQ TLEPRASDAK NQEGKVNTRR KSTDSVPISK STLSRSLSLQ ASDFDGASSS
2101 GNPEAVALAP DAYSTGSSSA SSTLKRTKKP RPPSLKKKQT TKKPTETPPV KETQQEPDEE
2161 SLVPSGENLA SETKTESAKT EGPSPALLEE TPLEPAVGPK AACPLDSESA EGVVPPASGG
2221 GRVQNSPPVG RKTLPLTTAP EAGEVTPSDS GGQEDSPAKG LSVRLEFDYS EDKSSWDNQQ
2281 ENPPPTKKIG KKPVAKMPLR RPKMKKTPEK LDNTPASPPR SPAEPNDIPI AKGTYTFDID
2341 KWDDPNFNPF SSTSKMQESP KLPQQSYNFD PDTCDESVDP FKTSSKTPSS PSKSPASFEI
2401 PASAMEANGV DGDGLNKPAK KKKTPLKTDT FRVKKSPKRS PLSDPPSQDP TPAATPETPP
2461 VISAVVHATD EEKLAVTNQK WTCMTVDLEA DKQDYPQPSD LSTFVNETKF SSPTEELDYR
2521 NSYEIEYMEK IGSSLPQDDD APKKQALYLM FDTSQESPVK SSPVRMSESP TPCSGSSFEE
2581 TEALVNTAAK NQHPVPRGLA PNQESHLQVP EKSSQKELEA MGLGTPSEAI EITAPEGSFA
2641 SADALLSRLA HPVSLCGALD YLEPDLAEKN PPLFAQKLQE ELEFAIMRIE ALKLARQIAL
2701 ASRSHQDAKR EAAHPTDVSI SKTALYSRIG TAEVEKPAGL LFQQPDLDSA LQIARAEIIT
2761 KEREVSEWKD KYEESRREVM EMRKIVAEYE KTIAQMIEDE QREKSVSHQT VQQLVLEKEQ
2821 ALADLNSVEK SLADLFRRYE KMKEVLEGFR KNEEVLKRCA QEYLSRVKKE EQRYQALKVH
2881 AEEKLDRANA EIAQVRGKAQ QEQAAHQASL RKEQLRVDAL ERTLEQKNKE IEELTKICDE
2941 LIAKMGKS
SEQ ID NO: 155 is the TACC2 Nucleotide Sequence for isoform a, having Genebank Accession No. NM_ 206862 (9706 bp) 
1 gcctgctcca agggaaggat caggagagaa gaaacgcaaa tcccagaacc gtgccaacat
61 ataaaacccc acattaaggg ttgtacagtg cactgggatt tctcaagtca cccgcttggt
121 cctcttccaa gtatacttta cttcctttca ttcctctcta aaactttttt aaaaactttc
181 actcctgctc taaaagttat cttggtttct tactctacct tatgcccctt gggcgaattt
241 tttcctctga ggagggaaga atagagttgc tgctgcagac acatcagatt ccctactggt
 
301 aacagctgga gtgcgtcacc tctgacaaaa ttctggggac gctgggaaca ctgaatcaac
361 atgggcaatg agaacagcac ctcggacaac cagaggactt tatcagctca gactccaagg
421 tccgcgcagc cacccgggaa cagtcagaat ataaaaagga agcagcagga cacgcccgga
481 agccctgacc acagagacgc gtccagcatt ggcagcgttg ggcttggagg cttctgcacc
541 gcttctgaga gttctgccag cctggatcca tgccttgtgt ccccagaggt gactgagcca
601 aggaaggacc cacagggagc cagggggcca gaaggttctt tgctgcccag cccaccaccg
661 tcccaggagc gagagcaccc ctcgtcctcc atgccctttg ccgagtgtcc cccggaaggt
721 tgcttggcaa gtccagcagc ggcacctgaa gatggtcctc agactcagtc tcccaggagg
781 gaacctgccc caaatgcccc aggagacatc gcggcggcat ttcccgctga gagggacagc
841 tctactccat accaagagat tgctgccgtc cccagtgctg gaagagagag acagccgaag
901 gaagaaggac agaagtcctc cttctccttc tccagtggca tcgaccagtc acctggaatg
961 tcgccagtac ccctcagaga gccaatgaag gcaccgctgt gtggagaggg ggaccagcct
1021 ggtggttttg agtcccaaga gaaagaggct gcaggtggct ttccccctgc agagtccagg
1081 cagggggtgg cttctgtgca agtgacccct gaggcccctg ctgcagccca gcagggcaca
1141 gaaagctcag cggtcttgga gaagtccccc ctaaaaccca tggccccgat cccacaagat
1201 ccagccccaa gagcctcaga cagagaaaga ggccaagggg aggcgccgcc tcagtattta
1261 acagatgact tggaattcct cagggcctgc catctcccta ggagcaattc aggggctgcc
1321 ccagaagcag aagtgaatgc cgcttcccag gagagctgcc agcagccagt gggagcatat
1381 ctgccgcacg cagagctgcc ctggggcttg ccaagtcctg ccctggtgcc agaggctggg
1441 ggctctggga aggaggctct ggacaccatt gatgttcagg gtcacccaca gacagggatg
1501 cgaggaacca agcccaatca agttgtctgt gtggcagcag gcggccagcc cgaagggggt
1561 ttgcctgtga gccctgaacc ttccctgctc actccgactg aggaagcaca tccagcttca
1621 agcctcgctt cattcccagc tgctcagatt cctattgctg tagaagaacc tggatcatca
1681 tccagggaat cagtttccaa ggctgggatg ccagtttctg cagatgcagc caaagaggtg
1741 gtggatgcag ggttggtggg actggagagg caggtgtcag atcttggaag caagggagag
1801 catccagaag gggaccctgg agaggttcct gccccatcac cccaggagag gggagagcac
1861 ttgaacacgg agcaaagcca tgaggtccaa ccaggagtac caccccctcc tcttcccaag
1921 gagcaaagcc atgaggtcca accaggagca ccaccccctc ctcttcccaa ggcaccaagt
1981 gaaagtgcca gagggccacc ggggccaacg gatggagcca aggtccatga agattccaca
2041 agcccagccg tggctaaaga aggaagcaga tcacctggtg acagccctgg aggaaaggag
2101 gaagccccag agccacctga tggtggagac ccagggaacc tgcaaggaga ggactctcag
2161 gctttcagca gcaagcgtga tccagaagta ggcaaagatg agctttcaaa gccaagcagt
2221 gatgcagaga gcagagacca tcccagctca cactcagcac agccacccag aaagggggt
2281 gctgggcaca cggacgggcc ccactctcag acagcagagg ctgatgcatc tggcctacca
2341 cacaagctgg gtgaggagga ccccgtcctg ccccctgtgc cagatggagc tggtgagccc
2401 actgttcccg aaggagccat ctgggagggg tcaggattgc agcccaaatg tcctgacacc
2461 cttcagagca gggaaggatt gggaagaatg gagtctttcc tgactttaga atcagagaaa
2521 tcagattttc caccaactcc tgttgcagag gttgcaccca aagcccagga aggtgagagc
2581 acattggaaa taaggaagat gggcagctgt gatggggagg gcttgctgac gtccccagat
2641 caaccccgcg ggccggcgtg tgatgcgtcg agacaggaat ttcatgctgg ggtgccacat
2701 cccccccagg gggagaactt ggcagcagac ctggggctca cggcactcat cctggaccaa
2761 gatcagcagg gaatcccatc ctgcccaggg gaaggctgga taagaggagc tgcatccgag
2821 tggcccctac tatcttctga gaagcatctc cagccatccc aggcacaacc agagacatcc
2881 atctttgacg tgctcaagga gcaggcccag ccacctgaaa atgggaaaga gacttctcca
2941 agccatccag gttttaagga ccagggagca gattcttccc aaatccatgt acctgtggaa
3001 cctcaggaag ataacaactt gcccactcat ggaggacagg agcaggcttt gggatcagaa
3061 cttcaaagtc agctccccaa aggcaccctg tctgatactc caacttcatc tcccactgac
3121 atggtttggg agagttctct gacagaagag tcagaattgt cagcaccaac gagacagaag
3181 ttgcctgcac taggggagaa gcggccagag ggagcatgcg gtgatggtca gtcctcgagg
3241 gtctcgcctc cagcagcaga tgtcttaaaa gacttttctc ttgcagggaa cttcagcaga
3301 aaggaaactt gctgcactgg gcaggggcca aacaagtctc aacaggcatt ggctgatgcc
3361 ttggaagaag gcagccagca tgaagaagca tgtcaaaggc atccaggagc ttctgaagca
3421 gctgatggtt gttccccact ctggggcttg agtaagaggg agatggcaag tggaaacaca
3481 ggggaggccc caccttgtca gcctgactca gtagctctcc tggatgcagt tccctgcctg
3541 ccagccctgg cgcccgccag ccccggagtc acacccaccc aggatgcccc agagacagag
3601 gcatgtgatg aaacccagga aggcaggcag caaccagtgc cggccccgca gcagaaaatg
3661 gagtgctggg ccacttcgga tgcagagtcc ccaaagcttc ttgcaagttt cccatcagct
3721 ggggagcaag gtggtgaagc cggggctgct gagactggtg gcagcgctgg tgcaggagac
3781 ccaggaaagc agcaggctcc ggagaaacct ggagaagcta ctttgagttg tggcctcctt
3841 cagactgagc actgccttac ctccggggag gaagcttcta cctctgccct acgtgagtcc
3901 tgccaagctg agcaccccat ggccagctgc caggatgcct tgctgccagc cagagagctg
 
3961 ggtgggattc ccaggagcac catggatttt tctacacacc aggctgtccc agacccaaag
4021 gagctcctgc tgtctgggcc accagaagtg gctgctcctg acacccctta cctgcatgtc
4081 gacagtgctg cccagagagg agcagaagac agtggagtga aagctgtttc ctctgcagac
4141 cccagagctc ctggcgaaag cccctgtcct gtaggggagc ccccacttgc cttggaaaat
4201 gctgcctcct tgaagctgtt tgctggctcc ctcgcccccc tgttgcaacc aggagctgca
4261 ggtggggaaa tccctgcagt gcaagccagc agtggtagtc ccaaagccag aaccactgag
4321 ggaccagtgg actccatgcc atgcctggac cggatgccac ttctggccaa gggcaagcag
4381 gcaacagggg aagagaaagc agcaacagct ccaggtgcag gtgccaaggc cagtggggag
4441 ggcatggcag gtgatgcagc aggagagaca gagggcagca tggagaggat gggagagcct
4501 tcccaggacc caaagcaggg cacatcaggt ggtgtggaca caagctctga gcaaatcgcc
4561 accctcactg gcttcccaga cttcagggag cacatcgcca agatcttcga gaagcctgtg
4621 ctcggagccc tggccacacc tggagaaaag gcaggagctg ggaggagtgc agtgggtaaa
4681 gacctcacca ggccattggg cccagagaag cttctagatg ggcctccagg agtggatgtc
4741 acccttctcc ctgcacctcc tgctcgactc caggtggaga agaagcaaca gttggctgga
4801 gaggctgaga tttcccatct ggctctgcaa gatccagctt cagacaagct tctgggtcca
4861 gcagggctga cctgggagcg gaacttgcca ggtgccggtg tggggaagga gatggcaggt
4921 gtcccaccca cactgaggga agacgagagg ccagaggggc ctggggcagc ctggccaggc
4981 ctggaaggcc aggcttactc acagctggag aggagcaggc aggaattagc ttcaggtctt
5041 ccttcaccag cagctactca ggagctccct gtggagagag ctgctgcctt ccaggtggct
5101 ccccatagcc atggagaaga ggccgtggcc caagacagaa ttccttctgg aaagcagcac
5161 caggaaacat ctgcctgcga cagtccacat ggagaagatg gtcccgggga ctttgctcac
5221 acaggggttc caggacatgt gccaaggtcc acgtgtgccc cttctcctca gagggaggtt
5281 ttgactgtgc ctgaggccaa cagtgagccc tggacccttg acacgcttgg gggtgaaagg
5341 agacccggag tcactgctgg catcttggaa atgcgaaatg ccctgggcaa ccagagcacc
5401 cctgcaccac caactggaga agtggcagac actcccctgg agcctggcaa ggtggcaggc
5461 gctgctgggg aagcagaggg tgacatcacc ctgagcacag ctgagacaca ggcatgtgcg
5521 tccggtgatc tgcctgaagc aggtactacg aggacattct ccgttgtggc aggtgacttg
5581 gtgctgccag gaagctgtca ggacccagcc tgctctgaca aggctccggg gatggagggt
5641 acagctgccc ttcatgggga cagcccagcc aggccccagc aggctaagga gcagccaggg
5701 cctgagcgcc ccattccagc tggggatggg aaggtgtgcg tctcctcacc tccagagcct
5761 gacgaaactc acgacccgaa gctgcaacat ttggctccag aagagctcca cactgacaga
5821 gagagcccca ggcctggccc atccatgtta ccttcggttc ctaagaagga tgctccaaga
5881 gtcatggata aagtcacttc agatgagacc agaggtgcgg aaggaacaga aagttcacct
5941 gtggcagatg atatcatcca gcccgctgcc cccgcagacc tggaaagccc aaccttagct
6001 gcctcttcct accacggtga tgttgttggc caggtctcta cggatctgat agcccagagc
6061 atctccccag ctgctgccca tgcgggtctt cctccctcgg ctgcagaaca catagtttcg
6121 ccatctgccc cagctggtga cagagtagaa gcttccactc cctcctgccc agatccggcc
6181 aaggacctca gcaggagttc cgattctgaa gaggcatttg agaccccgga gtcaacgacc
6241 cctgtcaaag ctccgccagc tccaccccca ccaccccccg aagtcatccc agaacccgag
6301 gtcagcacac agccaccccc ggaagaacca ggatgtggtt ctgagacagt ccctgtccct
6361 gatggcccac ggagcgactc ggtggaagga agtcccttcc gtccccagtc acactccttc
6421 tctgccgtct tcgatgaaga caagccgata gccagcagtg ggacttacaa cttggacttt
6481 gacaacattg agcttgtgga tacctttcag accttggagc ctcgtgcctc agacgctaag
6541 aatcaggagg gcaaagtgaa cacacggagg aagtccacgg attccgtccc catctctaag
6601 tctacactgt cccggtcgct cagcctgcaa gccagtgact ttgatggtgc ttcttcctca
6661 ggcaatcccg aggccgtggc ccttgcccca gatgcatata gcacgggttc cagcagtgct
6721 tctagtaccc ttaagcgaac taaaaaaccg aggccgcctt ccttaaaaaa gaaacagacc
6781 accaagaaac ccacagagac ccccccagtg aaggagacgc aacaggagcc agatgaagag
6841 agccttgtcc ccagtgggga gaatctagca tctgagacga aaacggaatc tgccaagacg
6901 gaaggtccta gcccagcctt attggaggag acgccccttg agcccgctgt ggggcccaaa
6961 gctgcctgcc ctctggactc agagagtgca gaaggggttg tccccccggc ttctggaggt
7021 ggcagagtgc agaactcacc ccctgtcggg aggaaaacgc tgcctcttac cacggccccg
7081 gaggcagggg aggtaacccc atcggatagc ggggggcaag aggactctcc agccaaaggg
7141 ctctccgtaa ggctggagtt tgactattct gaggacaaga gtagttggga caaccagcag
7201 gaaaaccccc ctcctaccaa aaagataggc aaaaagccag ttgccaaaat gcccctgagg
7261 aggccaaaga tgaaaaagac acccgagaaa cttgacaaca ctcctgcctc acctcccaga
7321 tcccctgctg aacccaatga catccccatt gctaaaggta cttacacctt tgatattgac
7381 aagtgggatg accccaattt taaccctttt tcttccacct caaaaatgca ggagtctccc
7441 aaactgcccc aacaatcata caactttgac ccagacacct gtgatgagtc cgttgacccc
7501 tttaagacat cctctaagac ccccagctca ccttctaaat ccccagcctc ctttgagatc
7561 ccagccagtg ctatggaagc caatggagtg gacggggatg ggctaaacaa gcccgccaag
 
7621 aagaagaaga cgcccctaaa gactgacaca tttagggtga aaaagtcgcc aaaacggtct
7681 cctctctctg atccaccttc ccaggacccc accccagctg ctacaccaga aacaccacca
7741 gtgatctctg cggtggtcca cgccacagat gaggaaaagc tggcggtcac caaccagaag
7801 tggacgtgca tgacagtgga cctagaggct gacaaacagg actacccgca gccctcggac
7861 ctgtccacct ttgtaaacga gaccaaattc agttcaccca ctgaggagtt ggattacaga
7921 aactcctatg aaattgaata tatggagaaa attggctcct ccttacctca ggacgacgat
7981 gccccgaaga agcaggcctt gtaccttatg tttgacactt ctcaggagag ccctgtcaag
8041 tcatctcccg tccgcatgtc agagtccccg acgccgtgtt cagggtcaag ttttgaagag
8101 actgaagccc ttgtgaacac tgctgcgaaa aaccagcatc ctgtcccacg aggactggcc
8161 cctaaccaag agtcacactt gcaggtgcca gagaaatcct cccagaagga gctggaggcc
8221 atgggcttgg gcaccccttc agaagcgatt gaaattacag ctcccgaggg ctcctttgcc
8281 tctgctgacg ccctcctcag caggctagct caccccgtct ctctctgtgg tgcacttgac
8341 tatctggagc ccgacttagc agaaaagaac cccccactat tcgctcagaa actccaggag
8401 gagttagagt ttgccatcat gcggatagaa gccctgaagc tggccaggca gattgctttg
8461 gcttcccgca gccaccagga tgccaagaga gaggctgctc acccaacaga cgtctccatc
8521 tccaaaacag ccttgtactc ccgcatcggg accgctgagg tggagaaacc tgcaggcctt
8581 ctgttccagc agcccgacct ggactctgcc ctccagatcg ccagagcaga gatcataacc
8641 aaggagagag aggtctcaga atggaaagat aaatatgaag aaagcaggcg ggaagtgatg
8701 gaaatgagga aaatagtggc cgagtatgag aagaccatcg ctcagatgat agaggacgaa
8761 cagagagaga agtcagtctc ccaccagacg gtgcagcagc tggttctgga gaaggagcaa
8821 gccctggccg acctgaactc cgtggagaag tctctggccg acctcttcag aagatatgag
8881 aagatgaagg aggtcctaga aggcttccgc aagaatgaag aggtgttgaa gagatgtgcg
8941 caggagtacc tgtcccgggt gaagaaggag gagcagaggt accaggccct gaaggtgcac
9001 gcggaggaga aactggacag ggccaatgct gagattgctc aggttcgagg caaggcccag
9061 caggagcaag ccgcccacca ggccagcctg cggaaggagc agctgcgagt ggacgccctg
9121 gaaaggacgc tggagcagaa gaataaagaa atagaagaac tcaccaagat ttgtgacgaa
9181 ctgattgcca aaatggggaa aagctaactc tgaaccgaat gttttggact taactgttgc
9241 gtgcaatatg accgtcggca cactgctgtt cctccagttc catggacagg ttctgttttc
9301 actttttcgt atgcactact gtatttcctt tctaaataaa attgatttga ttgtatgcag
9361 tactaaggag actatcagaa tttcttgcta ttggtttgca ttttcctagt ataattcata
9421 gcaagttgac ctcagagttc ctgtatcagg gagattgtct gattctctaa taaaagacac
9481 attgctgacc ttggccttgc cctttgtaca caagttccca gggtgagcag cttttggatt
9541 taatatgaac atgtacagcg tgcataggga ctcttgcctt aaggagtgta aacttgatct
9601 gcatttgctg atttgttttt aaaaaaacaa gaaatgcatg tttcaaataa aattctctat
9661 tgtaaataaa atttttcttt tggatcttgg caaaaaaaaa aaaaaa
SEQ ID NO: 158 is the amino acid sequence of the FGFR3-TACC3-1 fusion protein. The bolded text corresponds to the FGF3 protein: 
MGAPACALALCVAVAIVAGASSESLGTEQRVVGRAAEVPGPEPGQQEQLVFGSGDAVELSCPPPGGGPMGPTVWV
KDGTGLVPSERVLVGPQRLQVLNASHEDSGAYSCRQRLTQRVLCHFSVRVTDAPSSGDDEDGEDEAEDTGVDTGA
PYWTRPERMDKKLLAVPAANTVRFRCPAAGNPTPSISWLKNGREFRGEHRIGGIKLRHQQWSLVMESVVPSDRGN
YTCVVENKFGSIRQTYTLDVLERSPHRPILQAGLPANQTAVLGSDVEFHCKVYSDAQPHIQWLKHVEVNGSKVGP
DGTPYVTVLKTAGANTTDKELEVLSLHNVTFEDAGEYTCLAGNSIGFSHHSAWLVVLPAEEELVEADEAGSVYAG
ILSYGVGFFLFILVVAAVTLCRLRSPPKKGLGSPTVHKISRFPLKRQVSLESNASMSSNTPLVRIARLSSGEGPT
LANVSELELPADPKWELSRARLTLGKPLGEGCFGQVVMAEAIGIDKDRAAKPVTVAVKMLKDDATDKDLSDLVSE
MEMMKMIGKHKNIINLLGACTQGGPLYVLVEYAAKGNLREFLRARRPPGLDYSFDTCKPPEEQLTFKDLVSCAYQ
VARGMEYLASQKCIHRDLAARNVLVTEDNVMKIADFGLARDVHNLDYYKKTTNGRLPVKWMAPEALFDRVYTHQS
DVWSFGVLLWEIFTLGGSPYPGIPVEELFKLLKEGHRMDKPANCTHDLYMIMRECWHAAPSQRPTFKQLVEDLDR
VLTVTSTDFKESALRKQSLYLKFDPLLRDSPGRPVPVATETSSMHGANETPSGRPREAKLVEFDFLGALDIPVPG
PPPGVPAPGGPPLSTGPIVDLLQYSQKDLDAVVKATQEENRELRSRCEELHGKNLELGKIMDRFEEVVYQAMEEV
QKQKELSKAEIQKVLKEKDQLTTDLNSMEKSFSDLFKRFEKQKEVIEGYRKNEESLKKCVEDYLARITQEGQRYQ
ALKAHAEEKLQLANEEIAQVRSKAQAEALALQASLRKEQMRIQSLEKTVEQKTKENEELTRICDDLISKMEKI
SEQ ID NO: 159 is the amino acid sequence of the FGFR3-TACC3-2 fusion protein. The bolded text corresponds to the FGF3 protein: 
MGAPACALALCVAVAIVAGASSESLGTEQRVVGRAAEVPGPEPGQQEQLVFGSGDAVELSCPPPGGGPMGPTVWV
KDGTGLVPSERVLVGPQRLQVLNASHEDSGAYSCRQRLTQRVLCHFSVRVTDAPSSGDDEDGEDEAEDTGVDTGA
PYWTRPERMDKKLLAVPAANTVRFRCPAAGNPTPSISWLKNGREFRGEHRIGGIKLRHQQWSLVMESVVPSDRGN
YTCVVENKFGSIRQTYTLDVLERSPHRPILQAGLPANQTAVLGSDVEFHCKVYSDAQPHIQWLKHVEVNGSKVGP
DGTPYVTVLKTAGANTTDKELEVLSLHNVTFEDAGEYTCLAGNSIGFSHHSAWLVVLPAEEELVEADEAGSVYAG
ILSYGVGFFLFILVVAAVTLCRLRSPPKKGLGSPTVHKISRFPLKRQVSLESNASMSSNTPLVRIARLSSGEGPT
LANVSELELPADPKWELSRARLTLGKPLGEGCFGQVVMAEAIGIDKDRAAKPVTVAVKMLKDDATDKDLSDLVSE
MEMMKMIGKHKNIINLLGACTQGGPLYVLVEYAAKGNLREFLRARRPPGLDYSFDTCKPPEEQLTFKDLVSCAYQ
VARGMEYLASQKCIHRDLAARNVLVTEDNVMKIADFGLARDVHNLDYYKKTTNGRLPVKWMAPEALFDRVYTHQS
DVWSFGVLLWEIFTLGGSPYPGIPVEELFKLLKEGHRMDKPANCTHDLYMIMRECWHAAPSQRPTFKQLVEDLDR
VLTVTSTDVSAGSGLVPPAYAPPPAVPGHPSGRPREAKLVEFDFLGALDIPVPGPPPGVPAPGGPPLSTGPIVDL
LQYSQKDLDAVVKATQEENRELRSRCEELHGKNLELGKIMDRFEEVVYQAMEEVQKQKELSKAEIQKVLKEKDQL
TTDLNSMEKSFSDLFKRFEKQKEVIEGYRKNEESLKKCVEDYLARITQEGQRYQALKAHAEEKLQLANEEIAQVR
SKAQAEALALQASLRKEQMRIQSLEKTVEQKTKENEELTRICDDLISKMEKI
SEQ ID NO: 160 is the amino acid sequence of the FGFR3-TACC3-3 fusion protein. The bolded text corresponds to the FGF3 protein: 
MGAPACALALCVAVAIVAGASSESLGTEQRVVGRAAEVPGPEPGQQEQLVFGSGDAVELSCPPPGGGPMGPTVWV
KDGTGLVPSERVLVGPQRLQVLNASHEDSGAYSCRQRLTQRVLCHFSVRVTDAPSSGDDEDGEDEAEDTGVDTGA
PYWTRPERMDKKLLAVPAANTVRFRCPAAGNPTPSISWLKNGREFRGEHRIGGIKLRHQQWSLVMESVVPSDRGN
YTCVVENKFGSIRQTYTLDVLERSPHRPILQAGLPANQTAVLGSDVEFHCKVYSDAQPHIQWLKHVEVNGSKVGP
DGTPYVTVLKTAGANTTDKELEVLSLHNVTFEDAGEYTCLAGNSIGFSHHSAWLVVLPAEEELVEADEAGSVYAG
ILSYGVGFFLFILVVAAVTLCRLRSPPKKGLGSPTVHKISRFPLKRQVSLESNASMSSNTPLVRIARLSSGEGPT
LANVSELELPADPKWELSRARLTLGKPLGEGCFGQVVMAEAIGIDKDRAAKPVTVAVKMLKDDATDKDLSDLVSE
MEMMKMIGKHKNIINLLGACTQGGPLYVLVEYAAKGNLREFLRARRPPGLDYSFDTCKPPEEQLTFKDLVSCAYQ
VARGMEYLASQKCIHRDLAARNVLVTEDNVMKIADFGLARDVHNLDYYKKTTNGRLPVKWMAPEALFDRVYTHQS
DVWSFGVLLWEIFTLGGSPYPGIPVEELFKLLKEGHRMDKPANCTHDLYMIMRECWHAAPSQRPTFKQLVEDLDR
VLTVTSTDVPGPPPGVPAPGGPPLSTGPIVDLLQYSQKDLDAVVKATQEENRELRSRCEELHGKNLELGKIMDRF
EEVVYQAMEEVQKQKELSKAEIQKVLKEKDQLTTDLNSMEKSFSDLFKRFEKQKEVIEGYRKNEESLKKCVEDYL
ARITQEGQRYQALKAHAEEKLQLANEEIAQVRSKAQAEALALQASLRKEQMRIQSLEKTVEQKTKENEELTRICD
DLISKMEKI
SEQ ID NO: 161 is the amino acid sequence of the FGFR3-TACC3-4 fusion protein. The bolded text corresponds to the FGF3 protein:

MGAPACALALCVAVAIVAGASSESLGTEQRVVGRAAEVPGPEPGQQEQLVFGSGDAVELSCPPPGGGPMGPTVWV
KDGTGLVPSERVLVGPQRLQVLNASHEDSGAYSCRQRLTQRVLCHFSVRVTDAPSSGDDEDGEDEAEDTGVDTGA
PYWTRPERMDKKLLAVPAANTVRFRCPAAGNPTPSISWLKNGREFRGEHRIGGIKLRHQQWSLVMESVVPSDRGN
YTCVVENKFGSIRQTYTLDVLERSPHRPILQAGLPANQTAVLGSDVEFHCKVYSDAQPHIQWLKHVEVNGSKVGP
DGTPYVTVLKTAGANTTDKELEVLSLHNVTFEDAGEYTCLAGNSIGFSHHSAWLVVLPAEEELVEADEAGSVYAG
ILSYGVGFFLFILVVAAVTLCRLRSPPKKGLGSPTVHKISRFPLKRQVSLESNASMSSNTPLVRIARLSSGEGPT
LANVSELELPADPKWELSRARLTLGKPLGEGCFGQVVMAEAIGIDKDRAAKPVTVAVKMLKDDATDKDLSDLVSE
MEMMKMIGKHKNIINLLGACTQGGPLYVLVEYAAKGNLREFLRARRPPGLDYSFDTCKPPEEQLTFKDLVSCAYQ
VARGMEYLASQKCIHRDLAARNVLVTEDNVMKIADFGLARDVHNLDYYKKTTNGRLPVKWMAPEALFDRVYTHQS
DVWSFGVLLWEIFTLGGSPYPGIPVEELFKLLKEGHRMDKPANCTHDLYMIMRECWHAAPSQRPTFKQLVEDLDR
VLTVTSTDVKATQEENRELRSRCEELHGKNLELGKIMDRFEEVVYQAMEEVQKQKELSKAEIQKVLKEKDQLTTD
LNSMEKSFSDLFKRFEKQKEVIEGYRKNEESLKKCVEDYLARITQEGQRYQALKAHAEEKLQLANEEIAQVRSKA
QAEALALQASLRKEQMRIQSLEKTVEQKTKENEELTRICDDLISKMEKI

The Genbank ID for the FGFR4 gene is 2264. Three isoforms are listed for FGFR4, e.g., having Genebank Accession Nos. NP_002002 (corresponding nucleotide sequence NM_002011); NP_075252 (corresponding nucleotide sequence NM_022963);: NP_998812 (corresponding nucleotide sequence NM_213647).

As used herein, a "FGFR fusion molecule" can be a nucleic acid (e.g., synthetic, purified, and/or recombinant) which encodes a polypeptide corresponding to a fusion protein comprising a tyrosine kinase domain of an FGFR protein fused to a polypeptide that constitutively activates the tyrosine kinase domain of the FGFR protein, or a nucleic acid encoding a fusion protein comprising a transforming acidic coiled-coil (TACC) domain fused to a polypeptide with a tyrosine kinase domain, wherein the TACC domain constitutively activates the tyrosine kinase domain. It can also be a fusion protein comprising a tyrosine kinase domain of an FGFR protein fused to a polypeptide that constitutively activates the tyrosine kinase domain of the FGFR protein, or a fusion protein comprising a transforming acidic coiled--coil (TACC) domain fused to a polypeptide with a tyrosine kinase domain, wherein the TACC domain constitutively activates the tyrosine kinase domain. For example, a FGFR fusion molecule can include a FGFR1-TACC1 (e.g., comprising the amino acid sequence shown in SEQ ID NO: 150, or comprising the nucleic acid sequence shown in SEQ ID NO: 88), FGFR2-TACC2, FGFR3-TACC3 (e.g., comprising the amino acid sequence shown in SEQ ID NOS: 79, 158-160, or 161, or comprising the nucleic acid sequence shown in SEQ ID NOS: 80-82, 84, 94-144, or 145), or other FGFR-TACC fusion proteins (e.g., an N-terminal fragment of FGFR4 containing its tyrosine kinase domain fused to a fragment of TACC1, TACC2, or TACC3). For example, a FGFR fusion molecule can include a FGFR1-containing fusion comprising the amino acid sequence corresponding to Genebank Accession no. NP_001167534, NP_001167535, NP_001167536, NP_001167537, NP_001167538, NP_056934, NP_075593, NP_075594, or NP_075598; or a FGFR1-containing fusion comprising the nucleotide sequence corresponding to Genebank Accession no. NM_001174063, NM_001174064, NM_00111,4065, NM_001174066, NM_001174067, NM_015850, NM_023105NM_023106, or NM_023110. For example, a FGFR fusion molecule can include a FGFR2-containing fusion comprising the amino acid sequence corresponding to Genebank Accession no. NP_000132, NP_001138385, NP_001138.386, NP_001138387, NP_001138388, NP_001138389, NP_001138390, NP_001138391, or NP_075259; or a FGFR2-containing fusion comprising the nucleotide sequence corresponding to Genebank Accession no. NM_000141, NM_001144913, NM_001144914, NM 001144915, NM_001144916, NM_001144917, NM_001144918, NM_001144919, or NM_022970. For example, a FGFR fusion molecule can include a FGFR3-containing fusion comprising the amino acid sequence corresponding to Genebank Accession no. NP_000133, NP_001156685, or NP_075254; or a FGFR3-containing fusion comprising the nucleotide sequence corresponding to Genebank Accession no. NM_000142, NM_001163213, or NM_022965. For example, a FGFR fusion molecule can include a FGFR4-containing fusion comprising the amino acid sequence corresponding to Genebank Accession no. NP_002002, NP_075252, or NP_998812; or a FGFR4-containing fusion comprising the nucleotide sequence corresponding to Genebank Accession no. NM002011, NM_022963, or NM_21 3647. A FGFR fusion molecule can also include a tyrosine kinase domain of an FGFR protein fused to a protein encoded by any one of the genes listed in Table 7. A FGFR fusion molecule can include a variant of the above described examples, such as a fragment thereof.

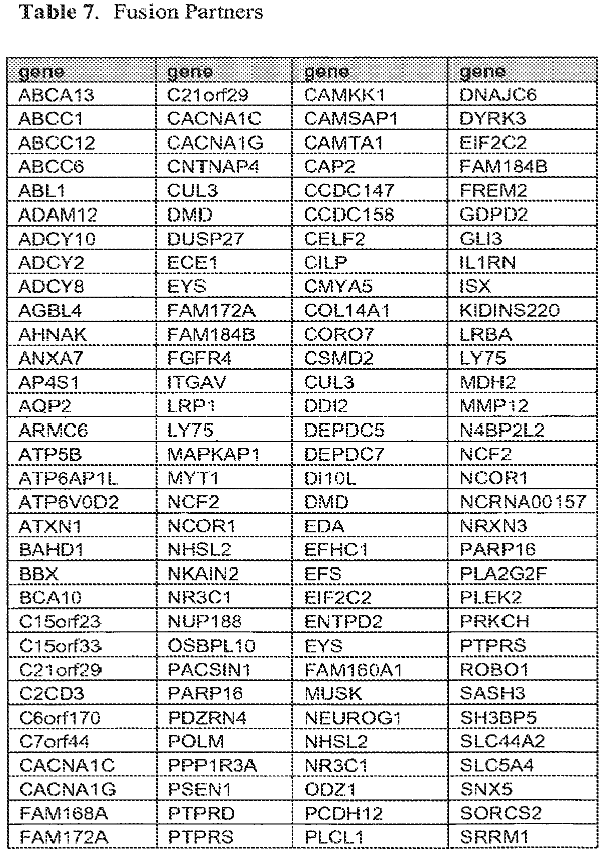

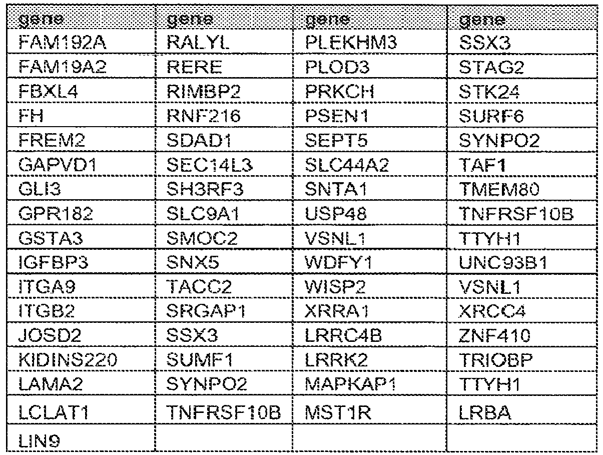

The nucleic acid can be any type of nucleic acid, including genomic DNA, complementary DNA (cDNA), recombinant DNA, synthetic or semi-synthetic DNA, as well as any form of corresponding RNA. A cDNA is a form of DNA artificially synthesized from a messenger RNA template and is used to produce gene clones. A synthetic DNA is free of modifications that can be found in cellular nucleic acids, including, but not limited to, histones and methylation. For example, a nucleic acid encoding a FGFR fusion molecule can comprise a recombinant nucleic acid encoding such a protein. The nucleic acid can be a anonnaturally occurring nucleic acid created artificially (such as by assembling, cutting, ligating or amplifying sequences). It can be double-stranded or single-stranded.

The invention further provides for nucleic acids that are complementary to a FGFR fusion molecule. Complementary nucleic acids can hybridize to the nucleic acid sequence described above under stringent hybridization conditions. Non-limiting examples of stringent hybridization conditions include temperatures above 30°C, above 35°C, in excess of 42°C, and/or salinity of less than about 500 mM, or less than 200 mM. Hybridization conditions can be adjusted by the skilled artisan via modifying the temperature, salinity and/or the concentration of other reagents such as SDS or SSC.

According to the invention, protein variants can include amino acid sequence modifications. For example, amino acid sequence modifications fall into one or more of three classes: substitutional, insertional or deletional variants. Insertions can include amino and/or carboxyl terminal fusions as well as intrasequence insertions of single or multiple amino acid residues. Insertions ordinarily will be smaller insertions than those of amino or carboxyl terminal fusions, for example, on the order of one to four residues. Deletions are characterized by the removal of one or more amino acid residues from the protein sequence. These variants ordinarily are prepared by site-specific mutagenesis of nucleotides in the DNA encoding the protein, thereby producing DNA encoding the variant, and thereafter expressing the DNA in recombinant cell culture.

In one embodiment, a FGFR fusion molecule comprises a protein or polypeptide encoded by a nucleic acid sequence encoding a FGFR fusion molecule, such as the sequences shown in SEQ ID NOS: 80-82, 84, 94-144, or 145. In some embodiments, the nucleic acid sequence encoding a FGFR fusion molecule is about 70%, about 75%, about 80%, about 85%, about 90%, about 93%, about 95%, about 97%, about 98%, or about 99% identical to SEQ ID NOS: 80-82, 84, 94-144, or 145. In another embodiment, the polypeptide can be modified, such as by glycosylations and/or acetylations and/or chemical reaction or coupling, and can contain one or several non-natural or synthetic amino acids. An example of a FGFR fusion molecule is the polypeptide having the amino acid sequence shown in SEQ ID NOS: 79, 88, 150, 158-160, or 161. In some embodiments, the FGFR fusion molecule that is a polypeptide is about 70%, about 75%, about 80%, about 85%, about 90%, about 93%, about 95%, about 97%, about 98%, or about 99% identical to SEQ ID NOS: 79, 88, 150, 158-160, or 161. In another embodiment, a FGFR fusion molecule can be a fragment of a FGFR fusion protein. For example, the FGFR fusion molecule can encompass any portion of at least about 8 consecutive amino acids of SEQ ID NOS: 79, 88, 150, 158-160, or 161. The fragment can comprise at least about 10 amino acids, a least about 20 amino acids, at least about 30 amino acids, at least about 40 amino acids, a least about 50 amino acids, at least about 60 amino acids, or at least about 75 amino acids of SEQ ID NOS: 79, 88, 150, 158-160, or 161. Fragments include all possible amino acid lengths between about 8 and 100 about amino acids, for example, lengths between about 10 and 100 amino acids, between about 15 and 100 amino acids, between about 20 and 100 amino acids, between about 35 and 100 amino acids, between about 40 and 100 amino acids, between about 50 and 100 amino acids, between about 70 and 100 amino acids, between about 75 and 100 amino acids, or between about 80 and 100 amino acids. Fragments include all possible amino acid lengths between about 100 and 800 amino acids, for example, lengths between about 125 and 800 amino acids, between about 150 and 800 amino acids, between about 175 and 800 amino acids, between about 200 and 800 amino acids, between about 225 and 800 amino acids, between about 250 and 800 amino acids, between about 275 and 800 amino acids, between about 300 and 800 amino acids, between about 325 and 800 amino acids, between about 350 and 800 amino acids, between about 375 and 800 amino acids, between about 400 and 800 amino acids, between about 425 and 800 amino acids, between about 450 and 800 amino acids, between about 475 and 800 amino acids, between about 500 and 800 amino acids, between about 52.5 and 800 amino acids, between about 550 and 800 amino acids, between about 575 and 800 amino acids, between about 600 and 800 amino acids, between about 625 and 800 amino acids, between about 650 and 800 amino acids, between about 675 and 800 amino acids, between about 700 and 800 amino acids, between about 725 and 800 amino acids, between about 750 and 800 amino acids, or between about 775 and 800 amino acids.

Chemical Synthesis. Nucleic acid sequences encoding a FGFR fusion molecule can be synthesized, in whole or in part, using chemical methods known in the art. Alternatively, a polypeptide can be produced using chemical methods to synthesize its amino acid sequence, such as by direct peptide synthesis using solid-phase techniques. Protein synthesis can either be performed using manual techniques or by automation. Automated synthesis can be achieved, for example, using Applied Biosystems 43 1A Peptide Synthesizer (Perkin Elmer).

Optionally, polypeptides fragments can be separately synthesized and combined using chemical methods to produce a foil-length molecule. For example, these methods can be utilized to synthesize a fusion protein of the invention. In one embodiment, the fusion protein comprises a tyrosine kinase domain of an FGFR protein fused to a polypeptide that constitutively activates the tyrosine kinase domain of the FGFR protein. In another embodiment, a fusion protein comprises a transforming acidic coiled-coil (TACC) domain fused to a polypeptide with a tyrosine kinase domain, wherein the TACC domain constitutively activates the tyrosine kinase domain. An example of a fusion protein is the FGFR1-TACC1 polypeptide, which comprises the amino acid sequence shown in SEQ ID NO: 150. An example of a fusion protein is the FGFR3-TACC3 polypeptide, which has the amino acid sequence comprising SEQ ID NO: 79, 158, 159, 160, or 161.

Obtaining, Purifying and Detecting FGFR fusion molecules. A polypeptide encoded by a nucleic acid, such as a nucleic acid encoding a FGFR fusion molecule, or a variant thereof, can be obtained by purification from human cells expressing a protein or polypeptide encoded by such a nucleic acid. Non-limiting purification methods include size exclusion chromatography, ammonium sulfate fractionation, ion exchange chromatography, affinity chromatography, and preparative gel electrophoresis.

A synthetic polypeptide can be substantially purified via high performance liquid chromatography (HPLC), such as ion exchange chromatography (TEX-HPLC). The composition of a synthetic polypeptide, such as a FGFR fusion molecule, can be confirmed by amino acid analysis or sequencing.

Other constructions can also be used to join a nucleic acid sequence encoding a polypeptide/protein of the claimed invention to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Including cleavable linker sequences (i.e., those specific for Factor Xa or enterokinase (Invitrogen, San Diego, Calif.)) between the purification domain and a polypeptide encoded by a nucleic acid of the invention also can be used to facilitate purification. For example, the skilled artisan can use an expression vector encoding 6 histidine residues that precede a thioredoxin or an enterokinase cleavage site in conjunction with a nucleic acid of interest. The histidine residues facilitate purification by immobilized metal ion affinity chromatography, while the enterokinase cleavage site provides a means for purifying the polypeptide encoded by, for example, an FGFR1-TACC1, FGFR2-TACC2, FGFR3-TACC3, other FGFR-TACC, FGFR-containing, or TACC containing nucleic acid.

Host cells which contain a nucleic acid encoding a FGFR fusion molecule, and which subsequently express the same, can be identified by various procedures known to those of skill in the art. These procedures include, but are not limited to, DNA-DNA or DNA-RNA hybridizations and protein bioassay or immunoassay techniques which include membrane, solution, or chip-based technologies for the detection and/or quantification of nucleic acid or protein. For example, the presence of a nucleic acid encoding a FGFR fusion molecule can be detected by DNA-DNA or DNA-RNA hybridization or amplification using probes or fragments of nucleic acids encoding the same. In one embodiment, a nucleic acid fragment of a FGFR fusion molecule can encompass any portion of at least about 8 consecutive nucleotides of SEQ ID NOS: 80-82, 84, 94-144, or 145. In another embodiment, the fragment can comprise at least about 10 consecutive nucleotides, at least about 15 consecutive nucleotides, at least about 20 conseutive nucleotides, or at least about 30 consecutive nucleotides of SEQ ID NOS: 80-82, 84, 94-144, or 145. Fragments can include all possible nucleotide lengths between about 8 and about 100 nucleotides, for example, lengths between about 15 and about 100 nucleotides, or between about 20 and about 100 nucleotides. Nucleic acid amplification-based assays involve the use of oligonucleotides selected from sequences encoding a FGFR fusion molecule nucleic acid, or FGFR fusion molecule nucleic acid to detect transformants which contain a nucleic acid encoding a protein or polypeptide of the same.

Protocols are known in the art for detecting and measuring the expression of a polypeptide encoded by a nucleic acid, such as a nucleic acid encoding a FGFR. fusion molecule, using either polyclonal or monoclonal antibodies specific for the polypeptide. Non-limiting examples include enzyme-linked immunosorbent assay (ELISA), radioimmunoassay (RIA), and fluorescence activated cell sorting (FACS). A two-site, monoclonal-based immunoassay using monoclonal antibodies reactive to two non-interfering epitopes on a polypeptide encoded by a nucleic acid, such as a nucleic acid encoding a FGFR fusion molecule, can be used, or a competitive binding assay can be employed.

Labeling and conjugation techniques are known by those skilled in the art and can be used in various nucleic acid and amino acid assays. Methods for producing labeled hybridization or PCR probes for detecting sequences related to nucleic acid sequences encoding a protein, such as FGFR fusion molecule, include, but are not limited to, oligolabeling, nick translation, end-labeling, or PCR amplification using a labeled nucleotide. Alternatively, nucleic acid sequences, such as nucleic acids encoding a FGFR fusion molecule, can be cloned into a vector for the production of an mRNA probe. Such vectors are known in the art, are commercially available, and can be used to synthesize RNA probes *in vitro* by addition of labeled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, and/or magnetic particles.

A fragment can be a fragment of a protein, such as a FGFR fusion protein. For example, a fragment of a FGFR fusion molecule can encompass any portion of at least about 8 consecutive amino acids of SEQ ID NOS: 79, 88, 150, 158-160, or 161. The fragment can comprise at least about 10 consecutive amino acids, at least about 20 consecutive amino acids, at least about 30 consecutive amino acids, at least about 40 consecutive amino acids, a least about 50 consecutive amino acids, at least about 60 consecutive amino acids, at least about 70 consecutive amino acids, at least about 75 consecutive amino acids, at least about 80 consecutive amino acids, at least about 85 consecutive amino acids, at least about 90 consecutive amino acids, at least about 95 consecutive amino acids, at least about 100 consecutive amino acids, at least about 200 consecutive amino acids, at least about 300 consecutive amino acids, at least about 400 consecutive amino acids, at least about 500 consecutive amino acids, at least about 600 consecutive amino acids, at least about 700 consecutive amino acids, or at least about 800 consecutive amino acids of SEQ ID NOS: 79, 88, 150, 158-160, or 161. Fragments include all possible amino acid lengths between about 8 and 100 about amino acids, for example, lengths between about 10 and about 100 amino acids, between about 15 and about 100 amino acids, between about 20 and about 1 00 amino acids, between about 35 and about 100 amino acids, between about 40 and about 100 amino acids, between about 50 and about 100 amino acids, between about 70 and about 100 amino acids, between about 75 and about 100 amino acids, or between about 80 and about 100 amino acids.

### Cell Transfection

Host cells transformed with a nucleic acid sequence of interest can be cultured under conditions suitable for the expression and recovery of the protein from cell culture. The polypeptide produced by a transformed cell can be secreted or contained intracellularly depending on the sequence and/or the vector used. Expression vectors containing a nucleic acid sequence, such as a nucleic acid encoding a FGFR. fusion molecule, can be designed to contain signal sequences which direct secretion of soluble polypeptide molecules encoded by the nucleic acid. Cell transfection and culturing methods are described in more detail below.

A eukaryotic expression vector can be used to transfect cells in order to produce proteins encoded by nucleotide sequences of the vector, e.g. those encoding a FGFR fusion molecule. Mammalian cells can contain an expression vector (for example, one that contains a nucleic acid encoding a fusion protein comprising a tyrosine kinase domain of an FGFR protein fused to a polypeptide that constitutively activates the tyrosine kinase domain of the FGFR protein, or a nucleic acid encoding a fusion protein comprises a transforming acidic coiled-coil (TACC) domain fused to a polypeptide with a tyrosine kinase domain, wherein the TACC domain constitutively activates the tyrosine kinase domain) via introducing the expression vector into an appropriate host cell via methods known in the art.

A host cell strain can be chosen for its ability to modulate the expression of the inserted sequences or to process the expressed polypeptide encoded by a nucleic acid, in the desired fashion. Such modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function. Different host cells which have specific cellular machinery and characteristic mechanisms for post-translational activities (e.g., CHO, HeLa, MDCK, HEK293, and WI38), are available from the American Type Culture Collection (ATCC; 10801 University Boulevard, Manassas, Va. 20110-2209) and can be chosen to ensure the correct modification and processing of the foreign protein.

An exogenous nucleic acid can be introduced into a cell via a variety of techniques known in the art, such as lipofection, microinjection, calcium phosphate or calcium chloride precipitation, DEAE-dextran-mediated transfection, or electroporation. Electroporation is carried out at approximate voltage and capacitance to result in entry of the DNA construct(s) into cells of interest (such as glioma cells (cell line SF188), neuroblastoma cells (cell lines IMR-32, SK-N-SH, SH-F and SH-N), astrocytes and the like). Other transfection methods also include modified calcium phosphate precipitation, polybrene precipitation, liposome fusion, and receptor-mediated gene delivery.

Cells that will be genetically engineered can be primary and secondary cells obtained from various tissues, and include cell types which can be maintained and propagated in culture. Non-limiting examples of primary and secondary cells include epithelial cells, neural cells, endothelial cells, glial cells, fibroblasts, muscle cells (such as myoblasts) keratinocytes, formed elements of the blood (e.g., lymphocytes, bone marrow cells), and precursors of these somatic cell types.

Vertebrate tissue can be obtained by methods known to one skilled in the art, such a punch biopsy or other surgical methods of obtaining a tissue source of the primary cell type of interest. In one embodiment, a punch biopsy or removal (e.g., by aspiration) can be used to obtain a source of cancer cells (for example, glioma cells, neuroblastoma cells, and the like). A mixture of primary cells can be obtained from the tissue, using methods readily practiced in the art, such as explanting or enzymatic digestion (for examples using enzymes such as pronase, trypsin, collagenase, elastase dispase, and chymotrypsin). Biopsy methods have also been described in United States Patent No. 7,419,661 and PCT application publication WO 2001/32840, and each are hereby incorporated by reference.

Primary cells can be acquired from the individual to whom the genetically engineered primary or secondary cells are administered. However, primary cells can also be obtained from a donor, other than the recipient, of the same species. The cells can also be obtained from another species (for example, rabbit, cat, mouse, rat, sheep, goat, dog, horse, cow, bird, or pig). Primary cells can also include cells from a purified vertebrate tissue source grown attached to a tissue culture substrate (for example, flask or dish) or grown in a suspension; cells present in an explant derived from tissue; both of the aforementioned cell types plated for the first time; and cell culture suspensions derived from these plated cells. Secondary cells can be plated primary cells that are removed from the culture substrate and replated, or passaged, in addition to cells from the subsequent passages. Secondary cells can be passaged one or more times. These primary or secondary cells can contain expression vectors having a gene that encodes a FGFR fusion molecule.

### Cell Culturing

Various culturing parameters can be used with respect to the host cell being cultured. Appropriate culture conditions for mammalian cells are well known in the art (Cleveland WL, et al., J Immunol Methods, 1983, 56(2): 221-234) or can be determined by the skilled artisan (see, for example, Animal Cell Culture: A Practical Approach 2nd Ed., Rickwood, D. and Hames, B. D., eds. (Oxford University Press: New York, 1992)). Cell culturing conditions can vary according to the type of host cell selected. Commercially available medium can be utilized. Non-limiting examples of medium include, for example, Minimal Essential Medium (MEM, Sigma, St. Louis, Mo.); Dulbecco's Modified Eagles Medium (DMEM, Sigma); Ham's F10 Medium (Sigma); HyClone cell culture medium (HyClone, Logan, Utah); RPMT-1640 Medium (Sigma); and chemically-defined (CD) media, which are formulated for various cell types, e.g., CD-CHO Medium (Invitrogen, Carlsbad, Calif.).

The cell culture media can be supplemented as necessary with supplementary components or ingredients, including optional components, in appropriate concentrations or amounts, as necessary or desired. Cell culture medium solutions provide at least one component from one or more of the following categories: (1) an energy source, usually in the form of a carbohydrate such as glucose; (2) all essential amino acids, and usually the basic set of twenty amino acids plus cysteine; (3) vitamins and/or other organic compounds required at low concentrations; (4) free fatty acids or lipids, for example linoleic acid; and (5) trace elements, where trace elements are defined as inorganic compounds or naturally occurring elements that can be required at very low concentrations, usually in the micromolar range.

The medium also can be supplemented electively with one or more components from any of the following categories: (1) salts, for example, magnesium, calcium, and phosphate; (2) hormones and other growth factors such as, serum, insulin, transferrin, and epidermal growth factor; (3) protein and tissue hydrolysates, for example peptone or peptone mixtures which can be obtained from purified gelatin, plant material, or animal byproducts; (4) nucleosides and bases such as, adenosine, thymidine, and hypoxanthine; (5) buffers, such as HEPES; (6) antibiotics, such as gentamycin or ampicillin; (7) cell protective agents, for example pluronic polyol; and (8) galactose. In one embodiment, soluble factors can be added to the culturing medium.

The mammalian cell culture that can be used with the present invention is prepared in a medium suitable for the type of cell being cultured. In one embodiment, the cell culture medium can be any one of those previously discussed (for example, MEM) that is supplemented with serum from a mammalian source (for example, fetal bovine serum (FBS)). In another embodiment, the medium can be a conditioned medium to sustain the growth of host cells.

Three-dimensional cultures can be formed from agar (such as Gey's Agar), hydrogels (such as matrigel, agarose, and the like; Lee et al., (2004) Biomaterials 25: 2461-2466) or polymers that are cross-linked. These polymers can comprise natural polymers and their derivatives, synthetic polymers and their derivatives, or a combination thereof. Natural polymers can be anionic polymers, cationic polymers, amphipathic polymers, or neutral polymers. Non-limiting examples of anionic polymers can include hyaluronic acid, alginic acid (alginate), carageenan, chondroitin sulfate, dextran sulfate, and pectin. Some examples of cationic polymers, include but are not limited to, chitosan or polylysine. (Peppas et al., (2006) Adv Mater. 18: 1345-60; Hoffman, A. S., (2002) Adv Drug Deliv Rev. 43: 3-12; Hoffman, A. S., (2001) Ann NY Acad Sci 944: 62-73). Examples of amphipathic polymers can include, but are not limited to collagen, gelatin, fibrin, and carboxymethyl chitin. Non-limiting examples of neutral polymers can include dextran, agarose, or pullulan. (Peppas et al., (2006) Adv Mater. 18: 1345-60; Hoffman, A. S., (2002) Adv Drug Deliv Rev. 43: 3-12; Hoffman, A. S., (2001) Ann NY Acad Sci 944: 62-73).

Cells to be cultured can harbor introduced expression vectors, such as plasmids. The expression vector constructs can be introduced via transformation, microinjection, transfection, lipofection, electroporation, or infection. The expression vectors can contain coding sequences, or portions thereof, encoding the proteins for expression and production. Expression vectors containing sequences encoding the produced proteins and polypeptides, as well as the appropriate transcriptional and translational control elements, can be generated using methods well known to and practiced by those skilled in the art. These methods include synthetic techniques, *in vitro* recombinant DNA techniques, and *in vivo* genetic recombination which are described in J. Sambrook et al., 2001, Molecular Cloning, A Laboratory Manual, Cold Spring Harbor Press, Plainview, N.Y. and in F. M. Ausubel et al., 1989, Current Protocols in Molecular Biology, John Wiley & Sons, New York, N.Y.

### FGFR Fusion Molecule Inhibitors

The invention provides methods for use of compounds that decrease the expression level or activity of a FGFR fusion molecule in a subject. In addition, the invention provides methods for using compounds for the treatment of a gene-fusion associated cancer. In one embodiment, the gene-fusion associated cancer is an epithelial cancer. In one embodiment, the gene-fusion associated cancer comprises glioblastoma multiforme, breast cancer, lung cancer, prostate cancer, or colorectal carcinoma.

As used herein, a "FGFR fusion molecule inhibitor" refers to a compound that interacts with a FGFR fusion molecule of the invention and modulates its activity and/or its expression. For example, the compound can decrease the activity or expression of a FGFR fusion molecule. The compound can be an antagonist of a FGFR fusion molecule (e.g., a FGFR fusion molecule inhibitor). Some non-limiting examples of FGFR fusion molecule inhibitors include peptides (such as peptide fragments comprising a FGFR fusion molecule, or antibodies or fragments thereof), small molecules, and nucleic acids (such as siRNA or antisense RNA specific for a nucleic acid comprising a FGFR fusion molecule). Antagonists of a FGFR fusion molecule decrease the amount or the duration of the activity of an FGFR fusion protein. In one embodiment, the fusion protein comprises a tyrosine kinase domain of an FGFR protein fused to a polypeptide that constitutively activates the tyrosine kinase domain of the FGFR protein (e.g., FGFR1-TACC1, FGFR2-TACC2, FGFR3-TACC3 or other FGFR-TACC), or a fusion protein comprises a transforming acidic coiled-coil (TACC) domain fused to a polypeptide with a tyrosine kinase domain, wherein the TACC domain constitutively activates the tyrosine kinase domain. Antagonists include proteins, nucleic acids, antibodies, small molecules, or any other molecule which decrease the activity of a FGFR fusion molecule.

The term "modulate," as it appears herein, refers to a change in the activity or expression of a FGFR fusion molecule. For example, modulation can cause a decrease in protein activity, binding characteristics, or any other biological, functional, or immunological properties of a FGFR fusion molecule, such as an FGFR fusion protein.

In one embodiment, a FGFR fusion molecule inhibitor can be a peptide fragment of a FGFR fusion protein that binds to the protein itself.

For example, the FGFR fusion polypeptide can encompass any portion of at least about 8 consecutive amino acids of SEQ ID NOS: 79, 88, 150, 158-160, or 161. The fragment can comprise at least about 10 consecutive amino acids, at least about 20 consecutive amino acids, at least about 30 consecutive amino acids, at least about 40 consecutive amino acids, a least about 50 consecutive amino acids, at least about 60 consecutive amino acids, at least about 70 consecutive amino acids, at least about 75 consecutive amino acids, at least about 80 consecutive amino acids, at least about 85 consecutive amino acids, at least about 90 consecutive amino acids, at least about 95 consecutive amino acids, at least about 100 consecutive amino acids, at least about 200 consecutive amino acids, at least about 300 consecutive amino acids, at least about 400 consecutive amino acids, at least about 500 consecutive amino acids, at least about 600 consecutive amino acids, at least about 700 consecutive amino acids, or at least about 800 consecutive amino acids of SEQ ID NOS: 79, 88, 150, 158-160, or 161. Fragments include all possible amino acid lengths between about 8 and 100 about amino acids, for example, lengths between about 10 and about 100 amino acids, between about 15 and about 100 amino acids, between about 20 and about 100 amino acids, between about 35 and about 100 amino acids, between about 40 and about 100 amino acids, between about 50 and about 100 amino acids, between about 70 and about 100 amino acids, between about 75 and about 100 amino acids, or between about 80 and about 100 amino acids. These peptide fragments can be obtained commercially or synthesized via liquid phase or solid phase synthesis methods (Atherton et al., (1989) Solid Phase Peptide Synthesis: a Practical Approach. IRL Press, Oxford, England). The FGFR fusion peptide fragments can be isolated from a natural source, genetically engineered, or chemically prepared. These methods are well known in the art.

A FGFR fusion molecule inhibitor can be a protein, such as an antibody (monoclonal, polyclonal, humanized, chimeric, or fully human), or a binding fragment thereof, directed against a FGFR fusion moleculeof the invention. An antibody fragment can be a form of an antibody other than the full- length form and includes portions or components that exist within full-length antibodies, in addition to antibody fragments that have been engineered. Antibody fragments can include, but are not limited to, single chain Fv (scFv), diabodies, Fv, and (Fab')₂, triabodies, Fc, Fab, CDR1, CDR2, CDR3, combinations of CDR's, variable regions, tetrabodies, bifunctional hybrid antibodies, framework regions, constant regions, and the like (*see*, Maynard et al., (2000) Ann. Rev. Biomed. Eng. 2:339-76; Hudson (1998) Curr. Opin. Biotechnol. 9:395-402). Antibodies can be obtained commercially, custom generated, or synthesized against an antigen of interest according to methods established in the art (see United States Patent Nos. 6,914,128, 5,780,597, 5,811,523; Roland E. Kontermann and Stefan Dübel (editors), Antibody Engineering. Vol. 1 & II, (2010) 2nd ed., Springer; Antony S. Dimitrov (editor), Therapeutic Antibodies: Methods and Protocols (Methods in Molecular Biology). (2009), Humana Press; Benny Lo (editor) Antibody Engineering: Methods and Protocols (Methods in Molecular Biology), (2004) Humana Press, each of which are hereby incorporated by reference in their entireties). For example, antibodies directed to a FGFR fusion molecule can be obtained commercially from Abeam, Santa Cruz Biotechnology, Abgent, R&D Systems, Novus Biologicals, etc. Human antibodies directed to a FGFR fusion molecule (such as monoclonal, humanized, fully human, or chimeric antibodies) can be useful antibody therapeutics for use in humans. In one embodiment, an antibody or binding fragment thereof is directed against SEQ ID NOS: 79, 88, 150, 158-160, or 161.

Inhibition of RNA encoding a FGFR fusion molecule can effectively modulate the expression of a FGFR fusion molecule. Inhibitors are selected from the group comprising: siRNA; interfering RNA or RNAi; dsRNA; RNA Polymerase III transcribed DNAs; ribozymes; and antisense nucleic acids, which can be RNA, DNA, or an artificial nucleic acid.

Antisense oligonucleotides, including antisense DNA, RNA, and DNA/RNA molecules, act to directly block the translation of mRNA by binding to targeted mRNA and preventing protein translation. For example, antisense oligonucleotides of at least about 15 bases and complementary to unique regions of the DNA sequence encoding a FGFR fusion molecule can be synthesized, e.g., by conventional phosphodiester techniques (Dallas et al., (2006) Med. Sci. Monit.12(4):RA67-74; Kalota et al., (2006) Handb. Exp. Pharmacol. 173:173-96; Lutzelburger et al., (2006) Handb. Exp. Pharmacol. 173:243-59). Antisense nucleotide sequences include, but are not limited to: morpholinos, 2'-O-methyl polynucleotides, DNA, RNA and the like.

siRNA comprises a double stranded structure containing from about 15 to about 50 base pairs, for example from about 21 to about 25 base pairs, and having a nucleotide sequence identical or nearly identical to an expressed target gene or RNA within the cell. The siRNA comprise a sense RNA strand and a complementary antisense RNA strand annealed together by standard Watson-Crick base-pairing interactions. The sense strand comprises a nucleic acid sequence which is substantially identical to a nucleic acid sequence contained within the target miRNA molecule. "Substantially identical" to a target sequence contained within the target mRNA refers to a nucleic acid sequence that differs from the target sequence by about 3% or less. The sense and antisense strands of the siRNA can comprise two complementary, single-stranded RNA molecules, or can comprise a single molecule in which two complementary portions are base-paired and are covalently linked by a single-stranded "hairpin" area. See also, McMnaus and Sharp (2002) Nat Rev Genetics, 3:737-47, and Sen and Blau (2006) FASEB J., 20:1293-99, the entire disclosures of which are herein incorporated by reference.

The siRNA can be altered RNA that differs from naturally-occurring RNA by the addition, deletion, substitution and/or alteration of one or more nucleotides. Such alterations can include addition of non-nucleotide material, such as to the end(s) of the siRNA, or to one or more internal nucleotides of the siRNA, or modifications that make the siRNA resistant to nuclease digestion, or the substitution of one or more nucleotides in the siRNA with deoxyribo-nucleotides. One or both strands of the siRNA can also comprise a 3' overhang. As used herein, a 3' overhang refers to at least one unpaired nucleotide extending from the 3'-end of a duplexed RNA strand. For example, the siRNA can comprise at least one 3' overhang of from 1 to about 6 nucleotides (which includes ribonucleotides or deoxyribonucleotides) in length, or from 1 to about 5 nucleotides in length, or from 1 to about 4 nucleotides in length, or from about 2 to about 4 nucleotides in length. For example, each strand of the siRNA can comprise 3' overhangs of dithymidylic acid ("TT") or diuridylic acid ("uu").

siRNA can be produced chemically or biologically, or can be expressed from a recombinant plasmid or viral vector (for example, see U.S. Patent No. 7,294,504 and U.S. Patent No. 7,422,896, the entire disclosures of which are herein incorporated by reference). Exemplary methods for producing and testing dsRNA or siRNA molecules are described in U.S. Patent Application Publication No. 2002/0173478 to Oewirtz, U.S. Patent No. 8,071,559 to Hannon et al., and in U.S. Patent No. 7,148,342 to Tolentino et al., the entire disclosures of which are herein incorporated by reference.

In one embodiment, an siRNA directed to a human nucleic acid sequence comprising a FGFR fusion molecule can be generated against any one of SEQ ID NOS: 80-82, 84, 94-144, or 145. In another embodiment, an siRNA directed to a human nucleic acid sequence comprising a breakpoint of anFGFR. fusion molecule can be generated against any one of SEQ ID NOS: 1-77, 80-82, 84-144, or 145. In one embodiment, the hairpin sequences targeting the *FGFR3* gene comprise SEQ ID NOS: 183, 184, or 185.

RNA polymerase III transcribed DNAs contain promoters, such as the U6 promoter. These DNAs can be transcribed to produce small hairpin RNAs in the cell that can function as siRNA or linear RNAs, which can function as antisense RNA. The FGFR fusion molecule inhibitor can comprise ribonucleotides, deoxyribonucleotides, synthetic nucleotides, or any suitable combination such that the target RNA and/or gene is inhibited. In addition, these forms of nucleic acid can be single, double, triple, or quadruple stranded, (see for example Bass (2001) Nature, 411:428-429; Elbashir et al., (2001) Nature, 411:494 498; U.S. Patent No. 6,509,154; U.S. Patent Application Publication No. 2003/0027783; and PCT Publication Nos. WO 00/044895, WO 99/032619, WO 00/01846, WO 01/029058, WO 00/044914).

FGFR fusion molecule inhibitor can be a small molecule that binds to a FGFR fusion protein described herein and disrupts its function. Small molecules are a diverse group of synthetic and natural substances generally having low molecular weights. They can be isolated from natural sources (for example, plants, fungi, microbes and the like), are obtained commercially and/or available as libraries or collections, or synthesized. Candidate small molecules that inhibit a FGFR fusion protein can be identified via *in silica* screening or high-through-put (HTP) screening of combinatorial libraries according to methods established in the art (e.g., see Potyrailo et al., (2011) ACS Comb Sci. 13(6):579-633; Mensch et al., (2009) J Pharm Sci. 98(12):4429-68; Schnur (2008) Curr Opin Drug Discov Devel. 11(3):375-80; and Jhoti (2007) Ernst Schering Found Symp Proc. (3):169-85, each of which are hereby incorporated by reference in their entireties.) Most conventional pharmaceuticals, such as aspirin, penicillin, and many chemotherapeutics, are small molecules, can be obtained commercially, can be chemically synthesized, or can be obtained from random or combinatorial libraries as described below (*see,* e.g., Werner et al., (2006) Brief Fund. Genomic Proteomic 5(1):32-6).

Non-limiting examples of FGFR fusion molecule inhibitors include the FGFR inhibitors AZD4547 (*see* Gavine et al., (2012) Cancer Res, 72(8); 2045-56; *see also* PCT Application Publication No. WO 2008/075068, each of which are hereby incorporated by reference in their entireties); NVP-BGJ398 (*see* Guagnano et al., (2011) J. Med. Chem., 54:7066-7083; *see also* U.S. Patent Application Publication No. 2008-0312248 A1, each of which are hereby incorporated by reference in their entireties); PD173074 (*see* Guagnano et al., (2011) J. Med. Chem., 54:7066-7083; see also Mohammadi et al., (1998) EMBO J., 17:5896-5904, each of which are hereby incorporated by reference in their entireties); NF449 (EMD Millipore (Billerica, MA) Cat. No. 480420; *see also* Krejci, (2010) the Journal of Biological Chemistry, 285(27):20644-20653, which is hereby incorporated by reference in its entirety); LY2874455 (Active Biochem; *see* Zhao et al. (2011) Mol Cancer Ther. (11):2200-10; *see also* PCT Application Publication No. WO 2010129509, each of which are hereby incorporated by reference in their entireties); TKI258 (Dovitinib); BIBF-1120 (Intedanib-Vargatef); BMS-582664 (Brivanib alaninate); AZD-2171 (Cediranib); TSU-68 (Orantinib); AB-1010 (Masiiinib); AP-24534 (Ponatinib); and E-7080 (by Eisai). A non-limiting example of an FGFR fusion molecule inhibitor includes the TACC inhibitor KHS101 (Wurdak et al., (2010) PNAS, 107(38): 16542-47, which is hereby incorporated by reference in its entirety).

Structures of FGFR fusion molecule inhibitors useful for the invention include, but are not limited to: the FGFR inhibitor AZD4547, 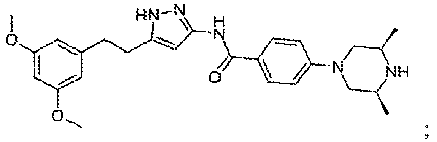 the FGFR inhibitor NVP-BGJ398, 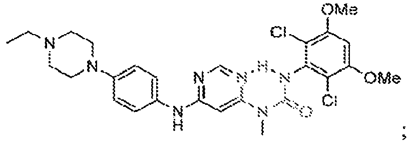 the FGFR inhibitor PD173074, 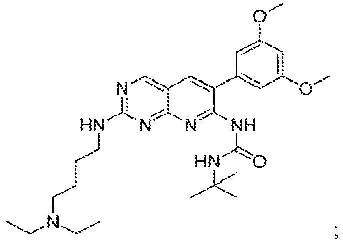 the FGFR inhibitor LY2874455 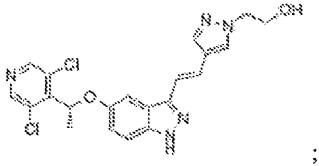 and the FGFR inhibitor NF449 (EMD Millipore (Billerica, MA) Cat. No. 480420.),

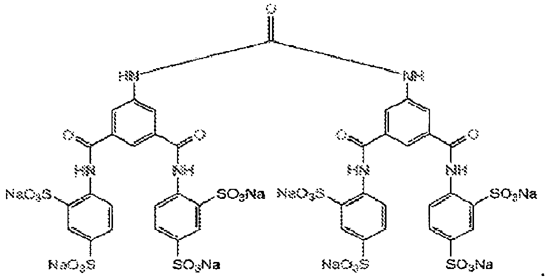

Other FGFR inhibitors include, but are not limited to:

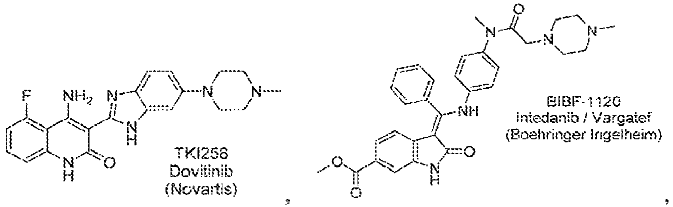

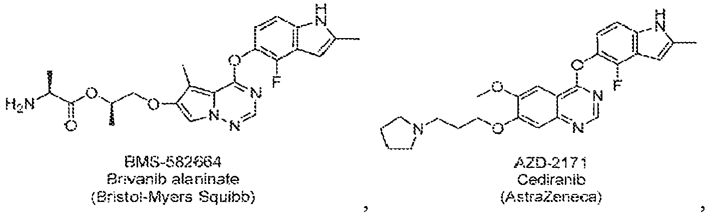

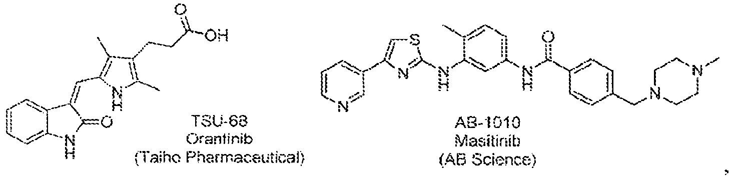

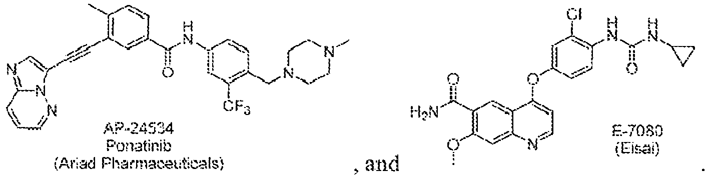

A structure of an FGFR fusion molecule inhibitor useful for the invention include, but is not limited to the TACC inhibitor KHS101,

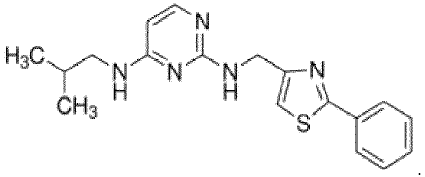

### Assessment and Therapuetic Treatment

The invention provides a method of decreasing the growth of a solid tumor in a subject. The tumor is associated with, but not limited to, glioblastoma multiforme, breast cancer, lung cancer, prostate cancer, or colorectal carcinoma. In one embodiment, the method comprises detecting the presence of a FGFR fusion molecule in a sample obtained from a subject. In some embodiments, the sample is incubated with an agent that binds to an FGFR fusion molecule, such as an antibody, a probe, a nucleic acid primer, and the like. In further embodiments, the method comprises administering to the subject an effective amount of a FGFR fusion molecule inhibitor, wherein the inhibitor decreases the size of the solid tumor.

The invention also provides a method for treating or preventing a gene-fusion associated cancer in a subject. In one embodiment, the gene-fusion associated cancer comprises an epithelial cancer. In one embodiment, the gene-fusion associated cancer comprises glioblastoma multiforme, breast cancer, lung cancer, prostate cancer, or colorectal carcinoma. In some embodiments, the epithelial cancer comprises bladder urothelial carcinoma, breast carcinoma, colorectal cancer, prostate carcinoma, lung squamous cell carcinoma, head and neck squamous cell carcinoma, or a combination of the epithelial cancers decribed. In one embodiment, the method comprises detecting the presence of a FGFR fusion molecule in a sample obtained from a subject, the presence of the fusion being indicative of a gene-fusion associated cancer, and, administering to the subject in need a therapeutic treatment against a gene-fusion associated cancer. In some embodiments, the sample is incubated with an agent that binds to an FGFR fusion molecule, such as an antibody, a probe, a nucleic acid primer, and the like.

The invention also provides a method for decreasing in a subject in need thereof the expression level or activity of a fusion protein comprising the tyrosine kinase domain of an FGFR protein fused to a polypeptide that constitutively activates the tyrosine kinase domain of the FGFR protein. In some embodiments, the method comprises obtaining a biological sample from the subject. In some embodiments, the sample is incubated with an agent that binds to an FGFR fusion molecule, such as an antibody, a probe, a nucleic acid primer, and the like. In some embodiments, the method comprises administering to the subject a therapeutic amount of a composition comprising an admixture of a pharmaceutically acceptable carrier an inhibitor of the fusion protein of the invention. In another embodiment, the method further comprises determining the fusion protein expression level or activity. In another embodiment, the method further comprises detecting whether the fusion protein expression level or activity is decreased as compared to the fusion protein expression level or activity prior to administration of the composition, thereby decreasing the expression level or activity of the fusion protein. In some embodiments, the fusion protein is an FGFR-TACC fusion protein.

The administering step in each of the claimed methods can comprise a drug administration, such as FGFR fusion molecule inhibitor (for example, a pharmaceutical composition comprising an antibody that specifically binds to a FGFR fusion molecule or a fragment thereof; a small molecule that specifically binds to a FGFR protein; a small molecule that specifically binds to a TACC protein; an antisense RNA or antisense DNA that decreases expression of a FGFR fusion molecule; a siRNA that specifically targets a gene encoding a FGFR fusion molecule; or a combination thereof). In one embodiment, the therapeutic molecule to be administered comprises a polypeptide of a FGFR fusion molecule, comprising at least about 75%, at least about 80%, at least about 85%, at least about 90%, at least about 93%, at least about 95%, at least about 97%, at least about 98%, at least about 99%, or 100% of the amino acid sequence of SEQ ID NOS: 79, 88, 150, 158-160, or 161, and exhibits the function of decreasing expression of such a protein, thus treating a gene fusion-associated cancer. In another embodiment, administration of the therapeutic molecule decreases the size of the solid tumor associated with glioblastoma multiforme, breast cancer, lung cancer, prostate cancer, or colorectal carcinoma.

In another embodiment, the therapeutic molecule to be administered comprises an siRNA directed to a human nucleic acid sequence comprising a FGFR fusion molecule. In one embodiment, the siRNA is directed to any one of SEQ ID NOS: 80-82, 84, 94-144, or 145. In another embodiment, the siRNA is directed to any one of SEQ ID NOS: 1-77, 80-82, 84-144, or 145. In a further embodiment, the therapeutic molecule to be administered comprises an antibody or binding fragment thereof, which is directed against SEQ ID NOS: 79, 88, 150, 158-160, or 161. In some embodiments, the therapeutic molecule to be administered comprises a small molecule that specifically binds to a FGFR protein, such as AZD4547, NVP-BGJ398, PD173074, NF449, TK1258, BIBF-1120, BMS-582664, AZD-2171, TSU68, AB1010, AP24534, E--7080, or LY2874455. In other embodiments, the therapeutic molecule to be administered comprises a small molecule that specifically binds to a TACC protein, such as KHS101.

In one embodiment, the invention provides for the detection of a chromosomal rearrangement at given chromosomal coordinates. In another embodiment, the detection or determination comprises nucleic acid sequencing, selective hybridization, selective amplification, gene expression analysis, or a combination thereof. In another embodiment, the detection or determination comprises protein expression analysis, for example by western blot analysis, ELISA, or other antibody detection methods.

In one embodiment, the biological sample comprises neuronal cells, serum, bone marrow, blood, peripheral blood, lymph nodes, cerebro-spinal fluid, urine, a saliva sample, a buccal swab, a serum sample, a sputum sample, a lacrimal secretion sample, a semen sample, a vaginal secretion sample, a fetal tissue sample, or a combination thereof.

A FGFR fusion molecule, for example, a fusion between FGFR1, FGFR2, FGFR3, or any other FGFR, and TACC1, TACC2, TACC3 or any other TACC, can be determined at the level of the DNA, RNA, or polypeptide. Optionally, detection can be determined by performing an oligonucleotide ligation assay, a confirmation based assay, a hybridization assay, a sequencing assay, an allele-specific amplification assay, a microsequencing assay, a melting curve analysis, a denaturing high performance liquid chromatography (DHPLC) assay (for example, *see* Jones et al, (2000) Hum Genet., 106(6):663-8), or a combination thereof. In one embodiment, the detection is performed by sequencing all or part of a FGFR fusion molecule (e.g., a FGFR1-TACC1, FGFR2-TACC2, FGFR3-TACC3 or other FGFR-TACC nucleic acid, or a FGFR1, TACC1, FGFR2, TACC2, FGFR3, TACC3 or other FGFR or TACC nucleic acid), or by selective hybridization or amplification of all or part of a FGFR fusion molecule (e.g., a FGFR1-TACC1, FGFR2-TACC2, FGFR3-TACC3 or other FGFR-TACC nucleic acid, or a FGFR1, TACC1, FGFR2, TACC2, FGFR3, TACC3 or other FGFR or TACC nucleic acid). A FGFR fusion molecule specific amplification (e.g., a FGFR1-TACC1, FGFR2-TACC2, FGFR3-TACC3 or other FGFR-TACC nucleic acid specific amplification) can be carried out before the fusion identification step.

The invention provides for a method of detecting a chromosomal alteration in a subject afflicted with a gene-fusion associated cancer. In one embodiment, the chromosomal alteration is an in-frame fused transcript described herein, for example an FGFR fusion molecule. In some embodiments, the chromosomal alteration is a chromosomal translocation, for example an FGFR fusion molecule. An alteration in a chromosome region occupied by a FGFR fusion molecule, such as a FGFR1-TACC1, FGFR2-TACC2, FGFR3-TACC3 or other FGFR-TACC nucleic acid, can be any form of mutation(s), deletion(s), rearrangements) and/or insertions in the coding and/or non-coding region of the locus, alone or in various combinations). Mutations can include point mutations. Insertions can encompass the addition of one or several residues in a coding or non-coding portion of the gene locus. Insertions can comprise an addition of between 1 and 50 base pairs in the gene locus. Deletions can encompass any region of one, two or more residues in a coding or non-coding portion of the gene locus, such as from two residues up to the entire gene or locus. Deletions can affect smaller regions, such as domains (introns) or repeated sequences or fragments of less than about 50 consecutive base pairs, although larger deletions can occur as well. Rearrangement includes inversion of sequences. The alteration in a chromosome region occupied by a FGFR fusion molecule, e.g., a FGFR1-TACC1, FGFR2-TACC2, FGFR3-TACC3 or other FGFR-TACC nucleic acid, can result in amino acid substitutions, RNA splicing or processing, product instability, the creation of stop codons, production of oncogenic fusion proteins, frame-shift mutations, and/or truncated polypeptide production. The alteration can result in the production of a FGFR fusion molecule, for example, one encoded by a FGFR1-TACC1, FGFR2-TACC2, FGFR3-TACC3 or other FGFR-TACC nucleic acid, with altered function, stability, targeting or structure. The alteration can also cause a reduction, or even an increase in protein expression. In one embodiment, the alteration in the chromosome region occupied by a FGFR fusion molecule can comprise a chromosomal rearrangement resulting in the production of a FGFR fusion molecule, such as a FGFRI-TACC1, FGFR2-TACC2, FGFR3-TACC3 or other FGFR-TACC fusion. This alteration can be determined at the level of the DNA, RNA, or polypeptide. In another embodiment, the detection or determination comprises nucleic acid sequencing, selective hybridization, selective amplification, gene expression analysis, or a combination thereof. In another embodiment, the detection or determination comprises protein expression analysis, for example by western blot analysis, ELISA, or other antibody detection methods. In one embodiment, the coordinates comprising FGFR1 translocations comprise chr8:38,268,656-38,325,363. In another embodiment, the coordinates comprising FGFR2 translocations comprise chr10:123,237,844-123,357,972. In a further embodiment, the coordinates comprising FGFR3 translocations comprise chr4:1,795,039-1,810,599. In yet another embodiment, the coordinates comprising FGFR4 translocations comprise chr5:176,513,921-176,525,126. In one embodiment, the coordinates comprising TACC1 translocations comprise chr8:38,644,722-38,710,546. In another embodiment, the coordinates comprising TACC2 translocations comprise chr10:123,748,689-124,014,057. In a further embodiment, the coordinates comprising TACC3 translocations comprise chr4:1,723,217-1,746,905.

The present invention provides a method for treating a gene-fusion associated cancer in a subject in need thereof. In one embodiment, the method comprises obtaining a sample from the subject to determine the level of expression of an FGFR fusion molecule in the subject. In some embodiments, the sample is incubated with an agent that binds to an FGFR fusion molecule, such as an antibody, a probe, a nucleic acid primer, and the like. In another embodiment, the detection or determination comprises nucleic acid sequencing, selective hybridization, selective amplification, gene expression analysis, or a combination thereof. In another embodiment, the detection or determination comprises protein expression analysis, for example by western blot analysis, ELISA, or other antibody detection methods. In some embodiments, the method further comprises assessing whether to administer a FGFR fusion molecule inhibitor based on the expression pattern of the subject. In further embodiments, the method comprises administering a FGFR fusion molecule inhibitor to the subject. In one embodiment, the gene-fusion associated cancer comprises an epithelial cancer. In one embodiment, the gene-fusion associated cancer comprises glioblastoma multiforme, breast cancer, lung cancer, prostate cancer, or colorectal carcinoma. In some embodiments, the epithelial cancer comprises bladder urothelial carcinoma, breast carcinoma, colorectal cancer, prostate carcinoma, lung squamous cell carcinoma, head and neck squamous cell carcinoma, or a combination of the epithelial cancers decribed.

In one embodiment, the invention provides for a method of detecting the presence of altered RNA expression of an FGFR fusion molecule in a subject, for example one afflicted with a gene-fusion associated cancer. In another embodiment, the invention provides for a method of detecting the presence of an FGFR fusion molecule in a subject. In some embodiments, the method comprises obtaining a sample from the subject to determine whether the subject expresses an FGFR fusion molecule. In some embodiments, the sample is incubated with an agent that binds to an FGFR fusion molecule, such as an antibody, a probe, a nucleic acid primer, and the like. In other embodiments, the detection or determination comprises nucleic acid sequencing, selective hybridization, selective amplification, gene expression analysis, or a combination thereof. In another embodiment, the detection or determination comprises protein expression analysis, for example by western blot analysis, ELISA, or other antibody detection methods. In some embodiments, the method further comprises assessing whether to administer a FGFR fusion molecule inhibitor based on the expression pattern of the subject. In further embodiments, the method comprises administering a FGFR fusion molecule inhibitor to the subject. Altered RNA expression includes the presence of an altered RNA sequence, the presence of an altered RNA splicing or processing, or the presence of an altered quantity of RNA. These can be detected by various techniques known in the art, including sequencing all or part of the RNA or by selective hybridization or selective amplification of all or part of the RNA. In a further embodiment, the method can comprise detecting the presence or expression of a FGFR. fusion molecule, such as one encoded by a FGFR1-TACC1, FGFR2-TACC2, FGFR3-TACC3 or other FGFR-TACC nucleic acid. Altered polypeptide expression includes the presence of an altered polypeptide sequence, the presence of an altered quantity of polypeptide, or the presence of an altered tissue distribution. These can be detected by various techniques known in the art, including by sequencing and/or binding to specific ligands (such as antibodies). In one embodiment, the detecting comprises using a northern blot; real time PCR and primers directed to SEQ ID NOS: 80-82, 84, 94-144, or 145; a ribonuclease protection assay; a hybridization, amplification, or sequencing technique to detect an FGFR fusion molecule, such as one comprising SEQ ID NOS: 80-82, 84, 94-144, or 145; or a combination thereof. In another embodiment, the PCR primers comprise SEQ ID NOS: 162, 163, 164, or 165. In a further embodiment, primers used for the screening of FGFR fusion molecules, such as FGFR-TACC fusions, comprise SEQ ID NOS: 166, 167, 168, or 169. In some embodiments, primers used for genomic detection of an FGFR3-TACC3 fusion comprise SEQ ID NOS: 170 and 171.

Various techniques known in the art can be used to detect or quantify altered gene or RNA expression or nucleic acid sequences, which include, but are not limited to, hybridization, sequencing, amplification, and/or binding to specific ligands (such as antibodies). Other suitable methods include allele-specific oligonucleotide (ASO), oligonucleotide ligation, allele-specific amplification, Southern blot (for DNAs), Northern blot (for RNAs), single-stranded conformation analysis (SSCA), PFGE, fluorescent in situ hybridization (FISH), gel migration, clamped denaturing gel electrophoresis, denaturing HLPC, melting curve analysis, heteroduplex analysis, RNase protection, chemical or enzymatic mismatch cleavage, ELISA, radio-immunoassays (RIA) and immuno-enzymatic assays (IEMA).

Some of these approaches (such as SSCA and constant gradient gel electrophoresis (CGGE)) are based on a change in electrophoretic mobility of the nucleic acids, as a result of the presence of an altered sequence. According to these techniques, the altered sequence is visualized by a shift in mobility on gels. The fragments can then be sequenced to confirm the alteration. Some other approaches are based on specific hybridization between nucleic acids from the subject and a probe specific for wild type or altered gene or RNA. The probe can be in suspension or immobilized on a substrate. The probe can be labeled to facilitate detection of hybrids. Some of these approaches are suited for assessing a polypeptide sequence or expression level, such as Northern blot, ELISA and RIA. These latter require the use of a ligand specific for the polypeptide, for example, the use of a specific antibody.

Hybridization, Hybridization detection methods are based on the formation of specific hybrids between complementary nucleic acid sequences that serve to detect nucleic acid sequence alterations). A detection technique involves the use of a nucleic acid probe specific for a wild type or altered gene or RNA, followed by the detection of the presence of a hybrid. The probe can be in suspension or immobilized on a substrate or support (for example, as in nucleic acid array or chips technologies). The probe can be labeled to facilitate detection of hybrids. In one embodiment, the probe according to the invention can comprise a nucleic acid directed to SEQ ID NOS: 80-82, 84, 94-144, or 145. For example, a sample from the subject can be contacted with a nucleic acid probe specific for a gene encoding a FGFR fusion molecule, and the formation of a hybrid can be subsequently assessed. In one embodiment, the method comprises contacting simultaneously the sample with a set of probes that are specific for an FGFR fusion molecule. Also, various samples from various subjects can be investigated in parallel.

According to the invention, a probe can be a polynucleotide sequence which is complementary to and specifically hybridizes with a, or a target portion of a, gene or RNA corresponding to a FGFR fusion molecule. Useful probes are those that are complementary to the gene, RNA, or target portion thereof. Probes can comprise single-stranded nucleic acids of between 8 to 1000 nucleotides in length, for instance between 10 and 800, between 15 and 700, or between 20 and 500. Longer probes can be used as well. A useful probe of the invention is a single stranded nucleic acid molecule of between 8 to 500 nucleotides in length, which can specifically hybridize to a region of a gene or RNA that corresponds to a FGFR fusion molecule.

The sequence of the probes can be derived from the sequences of the FGFR fusion genes provided herein. Nucleotide substitutions can be performed, as well as chemical modifications of the probe. Such chemical modifications can be accomplished to increase the stability of hybrids (e.g., intercalating groups) or to label the probe. Some examples of labels include, without limitation, radioactivity, fluorescence, luminescence, and enzymatic labeling.

A guide to the hybridization of nucleic acids is found in e.g., Sambrook, ed., Molecular Cloning: A LaboratoryManual , (3rd Ed.), Vols. 1-3, Cold Spring Harbor Laboratory, 1989; Current Protocols In Molecular Biology, Ausubel, ed. John Wiley & Sons, Inc., New York, 2001; Laboratory Techniques In Biochemistry And Molecular Biology: HybridizationWith Nucleic Acid Probes, Part I. Theoryand Nucleic Acid Preparation, Tijssen, ed. Elsevier, N.Y., 1993.

Sequencing. Sequencing can be carried out using techniques well known in the art, using automatic sequencers. The sequencing can be performed on the complete FGFR fusion molecule or on specific domains thereof.

Amplification. Amplification is based on the formation of specific hybrids between complementary nucleic acid sequences that serve to initiate nucleic acid reproduction. Amplification can be performed according to various techniques known in the art, such as by polymerase chain reaction (PCR), ligase chain reaction (LCR), strand displacement amplification (SDA) and nucleic acid sequence based amplification (NASBA). These techniques can be performed using commercially available reagents and protocols. Useful techniques in the art encompass real-time PCR, allele-specific PCR, or PCR based single-strand conformational polymorphism (SSCP). Amplification usually requires the use of specific nucleic acid primers, to initiate the reaction. For example, nucleic acid primers useful for amplifying sequences corresponding to a FGFR fusion molecule are able to specifically hybridize with a portion of the gene locus that flanks a target region of the locus. In one embodiment, amplification comprises using forward and reverse PCR primers directed to SEQ ID NOS: 80-82, 84, 94-144, or 145. Nucleic acid primers useful for amplifying sequences from a FGFR fusion molecule (e.g., a FGFR1-TACC1, FGFR2-TACC2, FGFR3-TACC3 or other FGFR-TACC nucleic acid); the primers specifically hybridize with a portion of an FGFR fusion molecule. In certain subjects, the presence of an FGFR fusion molecule corresponds to a subject with a gene fusion-associated cancer. In one embodiment, amplification can comprise using forward and reverse PCR primers comprising nucleotide sequences of SEQ ID NOS: 80-82, 84, 94-144, or 145.

Non-limiting amplification methods include, e.g., polymerase chain reaction, PCR (PCR Protocols, _A Guide To Methods And Applications, ed. Innis, Academic Press, N.Y., 1990 and PCR Strategies, 1995, ed. Innis, Academic Press, Inc., N.Y.); ligase chain reaction (LCR) (Wu (1989) Genomics 4:560; Landegren (1988) Science 241:1077; Barringer (1990) Gene 89:117); transcription amplification (K woh (1989) PNAS 86:1173); and, self-sustained sequence replication (Guatelli (1990) PNAS 87:1874); Q Beta replicase amplification (Smith (1997) J. Clin. Microbiol. 35:1477-1491), automated Q-beta replicase amplification assay (Burg (1996) Mol. Cell. Probes 16):257-271) and other RNA polymerase mediated techniques (e.g., NASH-A, Cangene, Mississauga, Ontario; *see also* Berger (1987) Methods Enzymol. 152:307-316; U.S. Pat. Nos. 4,683,195 and 4,683,202; and Sooknanan (1995) Biotechnology 13:563-564). All the references stated above are incorporated by reference in their entireties.

The invention provides for a nucleic acid primer, wherein the primer can be complementary to and hybridize specifically to a portion of a FGFR fusion molecule, such as a FGFR1-TACC1, FGFR2-TACC2, FGFR3-TACC3 or other FGFR-TACC nucleic acid (e.g., DNA or RNA) in certain subjects having a gene fusion-associated cancer. In one embodiment, the gene-fusion associated cancer comprises glioblastoma multiforme, breast cancer, lung cancer, prostate cancer, or colorectal carcinoma. Primers of the invention can be specific for fusion sequences in a FGFR1-TACC1, FGFR2-TACC2, FGFR3-TACC3 or other FGFR-TACC nucleic acid (DNA or RNA). By using such primers, the detection of an amplification product indicates the presence of a fusion of a FGFR1 and TACC1 , FGFR2 and TACC2, FGFR3 and TACC3 or other FGFR and TACC nucleic acid. Examples of primers of this invention can be single -stranded nucleic acid molecules of about 5 to 60 nucleotides in length, or about 8 to about 25 nucleotides in length. The sequence can be derived directly from the sequence of a FGFR fusion molecule, e.g. FGFR1-TACC1, FGFR2-TACC2, FGFR3-TACC3 or other FGFR-TACC nucleic acid. Perfect complementarity is useful to ensure high specificity; however, certain mismatch can be tolerated. For example, a nucleic acid primer or a pair of nucleic acid primers as described above can be used in a method for detecting the presence of a gene fusion-associated cancer in a subject. In one embodiment, primers can be used to detect an FGFR fusion molecule, such as a primer comprising SEQ ID NOS: 80-82, 84, 94-144, or 145; or a combination thereof. In another embodiment, the PCR primers comprise SEQ ID NOS: 162, 163, 164, or 165. In a further embodiment, primers used for the screening of FGFR fusion molecules, such as FGFR-TACC fusions, comprise SEQ ID NOS: 166, 167, 168, or 169. In some embodiments, primers used for genomic detection of an FGFR3-TACC3 fusion comprise SEQ ID NOS: 170 and 171.

Specific Ligand Binding. As discussed herein, a nucleic acid encoding a FGFR fusion molecule or expression of a FGFR. fusion molecule, can also be detected by screening for alteration(s) in a sequence or expression level of a polypeptide encoded by the same. Different types of ligands can be used, such as specific antibodies. In one embodiment, the sample is contacted with an antibody specific for a polypeptide encoded by a FGFR fusion molecule and the formation of an immune complex is subsequently determined. Various methods for detecting an immune complex can be used, such as ELISA, radioimmunoassays (RIA) and immuno-enzymatic assays (IEMA).

For example, an antibody can be a polyclonal antibody, a monoclonal antibody, as well as fragments or derivatives thereof having substantially the same antigen .specificity. Fragments include Fab, Fab'2, or CDR regions. Derivatives include single-chain antibodies, humanized antibodies, or poly-functional antibodies. An antibody specific for a polypeptide encoded by a FGFR fusion molecule can be an antibody that selectively binds such a polypeptide. In one embodiment, the antibody is raised against a polypeptide encoded by a FGFR fusion molecule (such as FGFR1-TACC1, FGFR2-TACC2, FGFR3-TACC3 or other FGFR-TACC fusion) or an epitope-containing fragment thereof. Although non-specific binding towards other antigens can occur, binding to the target polypeptide occurs with a higher affinity and can be reliably discriminated from non-specific binding. In one embodiment, the method can comprise contacting a sample from the subject with an antibody specific for a FGFR fusion molecule, and determining the presence of an immune complex. Optionally, the sample can be contacted to a support coated with antibody specific for a FGFR fusion molecule. In one embodiment, the sample can be contacted simultaneously, or in parallel, or sequentially, with various antibodies specific for different forms of a FGFR fusion molecule, e.g., FGFR1-TACC1, FGFR2-TACC2, FGFR3-TACC3 or other FGFR-TACC fusion.

The invention also provides for a diagnostic kit comprising products and reagents for detecting in a sample from a subject the presence of a FGFR fusion molecule. The kit can be useful for determining whether a sample from a subject exhibits increased or reduced expression of a FGFR fusion molecule. For example, the diagnostic kit according to the present invention comprises any primer, any pair of primers, any nucleic acid probe and/or any ligand, or any antibody directed specifically to a FGFR fusion molecule. The diagnostic kit according to the present invention can further comprise reagents and/or protocols for performing a hybridization, amplification, or antigen-antibody immune reaction. In one embodiment, the kit can comprise nucleic acid primers that specifically hybridize to and can prime a polymerase reaction from a FGFR fusion molecule comprising SEQ ID NOS: 80-82, 84, 94-144, or 145, or a combination thereof. In one embodiment, primers can be used to detect a FGFR. fusion molecule, such as a primer directed to SEQ ID NOS: 80-82, 84, 94-144, or 145; or a combination thereof. In another embodiment, the PCR primer comprises SEQ ID NOS: 162, 163, 164, 165, 166, 167, 168, or 169. In a further embodiment, primers used for the screening of FGFR fusion molecules, such as FGFR-TACC fusions, comprise SEQ ID NOS: 166, 167, 168, or 169. In some embodiments, primers used for genomic detection of an FGFR3-TACC3 fusion comprise SEQ ID NOS: 170 and 171. In some embodiments, the kit comprises an antibody that specifically binds to a FGFR fusion molecule comprising SEQ ID NOS: 79, 85-89, 150, 158-160, or 161, wherein the antibody will recognize the protein only when a FGFR fusion molecule is present

The diagnosis methods can be performed *in vitro, ex vivo,* or *in vivo.* These methods utilize a sample from the subject in order to assess the status of a FGFR fusion molecule. The sample can be any biological sample derived from a subject, which contains nucleic acids or polypeptides. Examples of such samples include, but are not limited to, fluids, tissues, cell samples, organs, and tissue biopsies. Non-limiting examples of samples include blood, liver, plasma, serum, saliva, urine, or seminal fluid. The sample can be collected according to conventional techniques and used directly for diagnosis or stored. The sample can be treated prior to performing the method, in order to render or improve availability of nucleic acids or polypeptides for testing. Treatments include, for instance, lysis (e.g., mechanical, physical, or chemical), centrifugation. The nucleic acids and/or polypeptides can be pre-purified or enriched by conventional techniques, and/or reduced in complexity. Nucleic acids and polypeptides can also be treated with enzymes or other chemical or physical treatments to produce fragments thereof. In one embodiment, the sample is contacted with reagents, such as probes, primers, or ligands, in order to assess the presence of a FGFR fusion molecule. Contacting can be performed in any suitable device, such as a plate, tube, well, or glass. In some embodiments, the contacting is performed on a substrate coated with the reagent, such as a nucleic acid array or a specific ligand array. The substrate can be a solid or semi-solid substrate such as any support comprising glass, plastic, nylon, paper, metal, or polymers. The substrate can be of various forms and sizes, such as a slide, a membrane, a bead, a column, or a gel. The contacting can be made under any condition suitable for a complex to be formed between the reagent and the nucleic acids or polypeptides of the sample.

### Nucleic Acid Delivery Methods

Delivery of nucleic acids into viable cells can be effected *ex vivo, in situ,* or in vivo by use of vectors, such as viral vectors (e.g., lentivirus, adenovirus, adeno-associated virus, or a retrovirus), or ex vivo by use of physical DNA transfer methods (e.g., liposomes or chemical treatments). Non-limiting techniques suitable for the transfer of nucleic acid into mammalian cells *in vitro* include the use of liposomes, electroporation, microinjection, cell fusion, DEAE-dextran, and the calcium phosphate precipitation method (See, for example, Anderson, Nature, 1998) supplement to 392(6679):25(), Introduction of a nucleic acid or a gene encoding a polypeptide of the invention can also be accomplished with extrachromosomal substrates (transient expression) or artificial chromosomes (stable expression). Cells can also be cultured ex vivo in the presence of therapeutic compositions of the present invention in order to proliferate or to produce a desired effect on or activity in such cells. Treated cells can then be introduced in vivo for therapeutic purposes.

Nucleic acids can be inserted into vectors and used as gene therapy vectors. A number of viruses have been used as gene transfer vectors, including papovaviruses, e.g., SV40 (Madzak et al., (1992) J Gen Virol. 73( Pt 6):1533-6), adenovirus (Berkner (1992) Curr Top Microbiol Immunol. 158:39-66; Berkner (1988) Biotechniques, 6(7):616-29; Gorziglia and Kapikian (1992) J Virol. 66(7):4407-12; Quantin et al., (1992) Proc Natl Acad Sci U S A. 89(7):2581-4; Rosenfeld et al., (1992) Cell. 68(1):143-55; Wilkinson et al., (1992) Nucleic Acids Res. 20(9):2233-9; Stratford-Perricaudet et al., (1990) Hum Gene Ther. 1(3):241-56), vaccinia virus (Moss (1992) Curr Opin Biotechnol. 3(5):518-22), adeno-associated virus (Muzyczka, (1992) Curr Top Microbiol Immunol. 158:97-129; Ohi et al., (1990) Gene. 89(2):279-82), herpesviruses including HSV and EBV (Margolskee (1992) Curr Top Microbiol Immunol. 158:67-95; Johnson et al., (1992) Brain Res Mol Brain Res.12(1-3):95-102; Fink et al., (1992) Hum Gene Ther. 3(1):11-9; Breakefield and Geller (1987) Mol Neurobiol. 1(4):339-71; Freese et al., (1990) Biochem Pharmacol. 40(10):2189-99), and retroviruses of avian (Bandyopadhyay and Temin (1984) Mol Cell Biol. 4(4):749-54; Petropoulos et al., (1992) J Virol. 66(6):3391-7), murine (Miller et al. (1992) Mol Cell Biol. 12(7):3262-72; Miller et al., (1985) J Virol. 55(3):521-6; Sorge et al., (1984) Mol Cell Biol. 4(9):1730-7; Mann and Baltimore (1985) J Virol. 54(2):401-7; Miller et al., (1988) J Virol. 62(11):4337-45), and human origin (Shimada et al., (1991) J Clin Invest. 88(3):1043-7; Helseth et al., (1990) J Virol. 64(12):6314-8; Page et al., (1990) J Virol. 64(11):5270-6; Buchschacher and Panganiban (1992) J Virol. 66(5):2731-9).

Non-limiting examples of *in vivo* gene transfer techniques include transfection with viral (e.g., retroviral) vectors (*see* U.S. Pat. No. 5,252,479, which is incorporated by reference in its entirety) and viral coat protein-liposome mediated transfection (Dzau et al., (1993) Trends in Biotechnology 11:205-210), incorporated entirely by reference). For example, naked DNA vaccines are generally known in the art; *see* Brower, (1998) Nature Biotechnology, 16:1304-1305, which is incorporated by reference in its entirety. Gene therapy vectors can be delivered to a subject by, for example, intravenous injection, local administration (*see*, e.g., U.S. Pat. No.5,328,470) or by stereotactic injection (*see*, e.g., Chen, et al., (1994) Proc. Natl. Acad. Sci. USA 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells that produce the gene delivery system.

For reviews of nucleic acid delivery protocols and methods *see* Anderson et al. (1992) Science 256:808-813; U.S. Pat. Nos. 5,252,479, 5,747,469, 6,017,524, 6,143,290, 6,410,010 6,511,847; and U.S. Application Publication No. 2002/0077313, which are all hereby incorporated by reference in their entireties. For additional reviews, *see* Friedmann (1989) Science, 244:1275-1281; Verma, Scientific American: 68-84 (1990); Miller (1992) Nature, 357: 455--460; Kikuchi et al. (2008) J Dermatol Sci. 50(2):87-98; Isaka et al. (2007) Expert Opin Drug Deliv. 4(5):561-71; Jager et al.(2007) Curr Gene Ther. 7(4):272-83; Waehler et al.(2007) Nat Rev Genet. 8(8):573-87; Jensen et al. (2007) Ann Med. 39(2):108-15; Herweijer et al. (2007) Gene Ther. 14(2):99-107; Eliyahu et al. (2005) Molecules 10(1):34-64; and Altaras et al. (2005) Adv Biochem Eng Biotechnol. 99:193-260, all of which are hereby incorporated by reference in their entireties.

A FGFR fusion nucleic acid can also be delivered in a controlled release system. For example, the FGFR fusion molecule can be administered using intravenous infusion, an implantable osmotic pump, a transdermal patch, liposomes, or other modes of administration. In one embodiment, a pump can be used (*see* Sefton (1987) Biomed. Eng. 14:201; Buchwald et al. (1980) Surgery 88:507; Saudek et al. (1989) N. Engl. J. Med. 321:574). In another embodiment, polymeric materials can be used (*see* Medical Applications of Controlled Release, Langer and Wise (eds.), CRC Pres., Boca Raton, Fla. (1974); Controlled Drug Bioavailability. Drug Product Design and Performance. Smolen and Ball (eds.), Wiley, New York (1984); Ranger and Peppas, (1983) J. Macromol. Sci. Rev. Macromol. Chem. 23:61; *see also* Levy et al. (1985) Science 228:190; During et al. (1989) Ann. Neurol. 25:351; Howard et al. (1989) J. Neurosurg. 71:105). In yet another embodiment, a controlled release system can be placed in proximity of the therapeutic target thus requiring only a fraction of the systemic dose (*see,* e.g., Goodson, in Medical Applications of Controlled Release, supra, vol. 2, pp. 115-138 (1984)). Other controlled release systems are discussed in the review by Langer (Science (1990) 249:1527-1533).

### Pharmaceutical Compositions and Administration for Therapy

An inhibitor of the invention can be incorporated into pharmaceutical compositions suitable for administration, for example the inhibitor and a pharmaceutically acceptable carrier

A FGFR fusion molecule or inhibitor of the invention can be administered to the subject once (e.g., as a single injection or deposition). Alternatively, a FGFR fusion molecule or inhibitor can be administered once or twice daily to a subject in need thereof for a period of from about two to about twenty-eight days, or from about seven to about ten days. A FGFR fusion molecule or inhibitor can also be administered once or twice daily to a subject for a period of 1, 2, 3, 4, 5, 6, 7, 8, 9, 10, 11, 12 tunes per year, or a combination thereof. Furthermore, a FGFR fusion molecule or inhibitor of the invention can be co-administrated with another therapeutic. Where a dosage regimen comprises multiple administrations, the effective amount of the FGFR fusion molecule or inhibitor administered to the subject can comprise the total amount of gene product administered over the entire dosage regimen.

A FGFR fusion molecule or inhibitor can be administered to a subject by any means suitable for delivering the FGFR fusion molecule or inhibitor to cells of the subject, such as cancer cells, e.g., glioblastoma multiforme, breast cancer, lung cancer, prostate cancer, or colorectal carcinoma. For example, a FGFR. fusion molecule or inhibitor can be administered by methods suitable to transfect cells. Transfection methods for eukaryotic cells are well known in the art, and include direct injection of the nucleic acid into the nucleus or pronucleus of a cell; electroporation; liposome transfer or transfer mediated by lipophilic materials; receptor mediated nucleic acid delivery, bioballistic or particle acceleration; calcium phosphate precipitation, and transfection mediated by viral vectors.

The compositions of this invention can be formulated and administered to reduce the symptoms associated with a gene fusion-associated cancer, e.g., glioblastoma multiforme, breast cancer, lung cancer, prostate cancer, or colorectal carcinoma, by any means that produces contact of the active ingredient with the agent's site of action in the body of a subject, such as a human or animal (e.g., a dog, cat, or horse). They can be administered by any conventional means available for use in conjunction with pharmaceuticals, either as individual therapeutic active ingredients or in a combination of therapeutic active ingredients. They can be administered alone, but are generally administered with a pharmaceutical carrier selected on the basis of the chosen route of administration and standard pharmaceutical practice.

A therapeutically effective dose of FGFR fusion molecule or inhibitor can depend upon a number of factors known to those or ordinary skill in the art. The dose(s) of the FGFR fusion molecule inhibitor can vary, for example, depending upon the identity, size, and condition of the subject or sample being treated, further depending upon the route by which the composition is to be administered, if applicable, and the effect which the practitioner desires the a FGFR fusion molecule inhibitor to have upon the nucleic acid or polypeptide of the invention. These amounts can be readily determined by a skilled artisan. Any of the therapeutic applications described herein can be applied to any subject in need of such therapy, including, for example, a mammal such as a dog, a cat, a cow, a horse, a rabbit, a monkey, a pig, a sheep, a goat, or a human.

Pharmaceutical compositions for use in accordance with the invention can be formulated in conventional manner using one or more physiologically acceptable carriers or excipients. The therapeutic compositions of the invention can be formulated for a variety of routes of administration, including systemic and topical or localized administration. Techniques and formulations generally can be found in Remmington's Pharmaceutical Sciences, Meade Publishing Co., Easton, Pa (20th Ed., 2000), the entire disclosure of which is herein incorporated by reference. For systemic administration, an injection is useful , including intramuscular, intravenous, intraperitoneal, and subcutaneous. For injection, the therapeutic compositions of the invention can be formulated in liquid solutions, for example in physiologically compatible buffers such as Hank's solution or Ringer's solution. In addition, the therapeutic compositions can be formulated in solid form and redissolved or suspended immediately prior to use. Lyophilized forms are also included. Pharmaceutical compositions of the present invention are characterized as being at least sterile and pyrogen-free. These pharmaceutical formulations include formulations for human and veterinary use.

According to the invention, a pharmaceutically acceptable carrier can comprise any and all solvents, dispersion media, coatings, antibacterial and antifungal agents, isotonic and absorption delaying agents, and the like, compatible with pharmaceutical administration. The use of such media and agents for pharmaceutically active substances is well known in the art. Any conventional media or agent that is compatible with the active compound can be used. Supplementary active compounds can also be incorporated into the compositions.

A pharmaceutical composition containing FGFR fusion molecule inhibitor can be administered in conjunction with a pharmaceutically acceptable carrier, for any of the therapeutic effects discussed herein. Such pharmaceutical compositions can comprise, for example antibodies directed to a FGFR fusion molecule, or a variant thereof, or antagonists of a FGFR fusion molecule. The compositions can be administered alone or in combination with at least one other agent, such as a stabilizing compound, which can be administered in any sterile, biocompatible pharmaceutical carrier including, but not limited to, saline, buffered saline, dextrose, and water. The compositions can be administered to a patient alone, or in combination with other agents, drugs or hormones.

Sterile injectable solutions can be prepared by incorporating the FGFR fusion molecule inhibitor (e.g., a polypeptide or antibody) in the required amount in an appropriate solvent with one or a combination of ingredients enumerated herein, as required, followed by filtered sterilization. Generally, dispersions are prepared by incorporating the active compound into a sterile vehicle which contains a basic dispersion medium and the required other ingredients from those enumerated herein. In the case of sterile powders for the preparation of sterile injectable solutions, examples of useful preparation methods are vacuum drying and freeze-drying which yields a powder of the active ingredient plus any additional desired ingredient from a previously sterile-filtered solution thereof.

In some embodiments, the FGFR fusion molecule inhibitor can be applied via transdermal delivery systems, which slowly releases the active compound for percutaneous absorption. Permeation enhancers can be used to facilitate transdermal penetration of the active factors in the conditioned media. Transdermal patches are described in for example, U.S. Pat. No. 5,407,713; U.S. Pat. No. 5,352,456; U.S. Pat. No. 5,332,213; U.S. Pat. No. 5,336,168; U.S. Pat. No. 5,290,561; U.S. Pat. No. 5,254,346; U.S. Pat. No. 5,164,189; U.S. Pat. No. 5,163,899; U.S. Pat. No. 5,088,977; U.S. Pat. No. 5,087,240; U.S. Pat. No. 5,008,110; and U.S. Pat. No. 4,921,475.

"Subcutaneous" administration can refer to administration just beneath the skin (i.e., beneath the dermis). Generally, the subcutaneous tissue is a layer of fat and connective tissue that houses larger blood vessels and nerves. The size of this layer varies throughout the body and from person to person. The interface between the subcutaneous and muscle layers can be encompassed by subcutaneous administration. This mode of administration can be feasible where the subcutaneous layer is sufficiently thin so that the factors present in the compositions can migrate or diffuse from the locus of administration. Thus, where intradermal administration is utilized, the bolus of composition administered is localized proximate to the subcutaneous layer.

Administration of the cell aggregates (such as DP or DS aggregates) is not restricted to a single route, but can encompass administration by multiple routes. For instance, exemplary administrations by multiple routes include, among others, a combination of intradermal and intramuscular administration, or intradermal and subcutaneous administration. Multiple administrations can be sequential or concurrent. Other modes of application by multiple routes will be apparent to the skilled artisan.

In other embodiments, this implantation method will be a one-time treatment for some subjects. In further embodiments of the invention, multiple cell therapy implantations will be required. In some embodiments, the cells used for implantation will generally be subject-specific genetically engineered cells. In another embodiment, cells obtained from a different species or another individual of the same species can be used. Thus, using such cells can require administering an immunosuppressant to prevent rejection of the implanted cells. Such methods have also been described in United States Patent No. 7,419,66 land PCT application publication WO 2001/32840, and are hereby incorporated by reference.

A pharmaceutical composition of the invention is formulated to be compatible with its intended route of administration. Examples of routes of administration include parenteral, e.g., intravenous, intradermal, subcutaneous, oral (e.g., inhalation or ingestion), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid, buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic,

Pharmaceutical compositions suitable for injectable use include sterile aqueous solutions (where water soluble) or dispersions and sterile powders for the extemporaneous preparation of sterile injectable solutions or dispersions. For intravenous administration, suitable carriers include physiological saline, bacteriostatic water, or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, a pharmaceutically acceptable polyol like glycerol, propylene glycol, liquid polyetheylene glycol, and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it can be useful to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the inhibitor (e.g., a polypeptide or antibody or small molecule) of the invention in the required amount in an appropriate solvent with one or a combination of ingredients enumerated herein, as required, followed by filtered sterilization. Generally, dispersions are prepared by incorporating the active compound into a sterile vehicle which contains a basic dispersion medium and the required other ingredients from those enumerated herein. In the case of sterile powders for the preparation of sterile injectable solutions, examples of useful preparation methods are vacuum drying and freeze -drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier and subsequently swallowed.

Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or sterotes; a glidant such as colloidal silicon dioxide; a sweetening agent such as sucrose or saccharin:, or a flavoring agent such as peppermint, methyl salicylate, or orange flavoring.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

In some embodiments, the effective amount of the administered FGFR fusion molecule inhibitor is at least about 0.0001 µg/kg body weight, at least about 0.00025 µg/kg body weight, at least about 0.0005 µg/kg body weight, at least about 0.00075 µg/kg body weight, at least about 0.001 µg/kg body weight, at least about 0.0025 µg/kg body weight, at least about 0.005 µg/kg body weight, at least about 0.0075 µg/kg body weight, at least about 0.01 µg/kg body weight, at least about 0.025 µg/kg body weight, at least about 0.05 µg/kg body weight, at least about 0.075 µg/kg body weight, at least about 0.1 µg/kg body weight, at least about 0.25 µg/kg body weight, at least about 0.5 µg/kg body weight, at least about 0.75 µg/kg body weight, at least about 1 µg/kg body weight, at least about 5 µg/kg body weight, at least about 10 µg/kg body weight, at least about 25 µg/kg body weight, at least about 50 µg/kg body weight, at least about 75 µg/kg body weight, at least about 100 µg/kg body weight, at least about 150 µg/kg body weight, at least about 200 µg/kg body weight, at least about 250 µg/kg body weight, at least about 300 µg/kg body weight, at least about 350 µg/kg body weight, at least about 400 µg/kg body weight, at least about 450 µg/kg body weight, at least about 500 µg/kg body weight, at least about 550 µg/kg body weight, at least about 600 µg/kg body weight, at least about 650 µg/kg body weight, at least about 700 µg/kg body weight, at least about 750 µg/kg body weight, at least about 800 µg/kg body weight, at least about 850 µg/kg body weight, at least about 900 µg/kg body weight, at least about 950 µg/kg body weight, at least about 1,000 µg/kg body weight, at least about 2,000 µg/kg body weight, at least about 3,000 µg/kg body weight, at least about 4,000 µg/kg body weight, at least about 5,000 µg/kg body weight, at least about 6,000 µg/kg body weight, at least about 7,000 µg/kg body weight, at least about 8,000 µg/kg body weight, at least about 9,500 µg/kg body weight, or at least about 10,000 µg/kg body weight.

Unless otherwise defined, all technical and scientific terms used herein have the same meaning as commonly understood by one of ordinary skill in the art to which this invention belongs. Exemplary methods and materials are described below, although methods and materials similar or equivalent to those described herein can also be used in the practice or testing of the present invention.

All publications and other references mentioned herein are incorporated by reference in their entirety, as if each individual publication or reference were specifically and individually indicated to be incorporated by reference. Publications and references cited herein are not admitted to be prior art.

### EXAMPLES

Examples are provided below to facilitate a more complete understanding of the invention. The following examples illustrate the exemplary modes of making and practicing the invention. However, the scope of the invention is not limited to specific embodiments disclosed in these Examples, which are for purposes of illustration only, since alternative methods can be utilized to obtain similar results.

### Example 1: Transforming and recurrent fusions of GFR and TACC gene in glioblastoma

The history of successful targeted therapy of cancer largely coincides with the inactivation of recurrent, oncogenic and addicting gene fusions in hematological malignancies and recently in some types of epithelial cancer. Glioblastoma multiforme (GBM) is among the most lethal forms of human cancer. Here, an integrated gene fusion discovery pipeline was developed for the detection of in-frame fused transcripts from RNA-seq and genomic fusions from whole exome sequences. The application of the pipeline to human GBM unraveled recurrent chromosomal translocations, which fuse in-frame the tyrosine kinase domain of FGFR genes (FGFR1 or FGFR3) to the TACC domain of TACC1 or TACC3, respectively. The frequency of FCFR-TACC fusions is 3 of 97 GBM (3.1%). The FGFR-TACC fusion protein displays strong oncogenic activity when introduced into astrocytes or transduced by lentivirus-mediated stereotactic delivery to the adult mouse brain. The FGFR-TACC fusion protein mis-localizes over the mitotic spindle pole, has constitutive tyrosine kinase activity and dysregulates the mitotic cycle with delayed mitotic progression. The impaired mitotic fidelity triggers chromatid cohesion defects, defective spindle checkpoint activation, chromosomal mis-segregation, and rampant, aneuploidy. Inhibition of FGFR kinase corrects the aneuploidy and oral administration of a specific FGFR tyrosine kinase inhibitor under clinical investigation arrests tumor growth and prolongs survival of mice harboring intracranial FGFR3-TACC3-initiated glioma. FGFR-TACC fusions identify a subset of GBM patients who may benefit from targeted inhibition of the tyrosine kinase activity of FGFR.

Glioblastoma multiforme (GBM) is among the most difficult forms of cancer to treat in humans (Ohgaki and Kleihues, 2005). So far, the targeted therapeutic approaches that have been tested against potentially important oncogenic drivers in GBM have met limited success (Lo, 2010; Reardon et al., 2010; van den Bent et al., 2009). Recurrent chromosomal translocations leading to production of oncogenic fusion proteins are viewed as initiating and addicting events in the pathogenesis of human cancer, thus providing the most desirable molecular targets for cancer therapy (Ablain et al., 2011; Mitelman et al., 2007). Chromosomal rearrangements resulting in recurrent and oncogenic gene fusions are hallmarks of hematological malignancies and recently they have also been uncovered in subsets of solid tumors (breast, prostate, lung and colorectal carcinoma), but they have not been found in GBM (Bass et al., 2011; Prensner and Chinnaiyan, 2009). Important and successful targeted therapeutic interventions for patients whose tumors carry these rearrangements have stemmed from the discovery of functional gene fusions, especially when the translocations involve kinase-coding genes (*BCR-ABL, EML4-ALK*) (Druker, 2009; Gerber and Minna, 2010).

A hallmark of GBM is rampant chromosomal instability (CIN), which leads to aneuploidy (Furnari et al., 2007). CIN and aneuploidy are early events in the pathogenesis of cancer (Cahill et al., 1999). It has been suggested that genetic alterations targeting mitotic fidelity might be responsible for mis-segregation of chromosomes during mitosis, resulting in aneuploidy (Gordon et al., 2012; Solomon et al., 2011). Here, the first cases of recurrent and oncogenic gene fusions in human GBM are described. The resulting fusion protein localizes to mitotic cells, disrupts the normal control of chromosome segregation and induces aneuploidy. A therapeutic strategy with FGFR. tyrosine kinase inhibitors is also reported for the targeted therapy of GBM patients harboring these chromosomal rearrangements.

***Identification of recurrent fusions of FGFR and TACC genes.*** To identify genomic rearrangements in GBM that generate functional fusion proteins and are recurrent, gene pairs discovered as in-frame fused transcripts from the analysis of massively parallel, paired-end sequencing of expressed transcripts (RNA-seq) would also emerge as fused gene pairs from the genomic analysis of human GBM. Towards this aim, two complementary gene fusion discovery methods were devised and were applied to two GBM cohorts. The first, TX-Fuse, is an algorithm for the discovery of candidate fusion transcripts from RNA-seq (**Figure 8**). The second, Exome-Fuse, detects fusion genes from whole exome DNA sequences (**Figure 8**). As first step for the detection of fused transcripts, RNA-seq data was generated from short-term cultures of glioma stem-like cells (GSCs) freshly isolated from nine patients carrying primary GBM. The culture of primary GBM tumors under serum-free conditions selects cells that retain phenotypes and genotypes closely mirroring primary tumor profiles as compared to serum-cultured glioma cell lines that have largely lost their developmental identities (Lee et al., 2006). Therefore, without being bound by theory, if glioma cells carry gene fusions causally responsible for the most aggressive hallmarks of GBM, they should be selected in GSCs. RNA-seq generated an average of 60.3 million paired reads for each GSC culture, of which over 80% were mapped to the reference transcriptome and genome. TX-Fuse detects two main sources of evidence: split reads and split inserts (see Experimental Procedures). The application of TX-Fuse to the RNA-seq dataset from nine GSCs led to the discovery of five candidate rearrangements (all of which were intrachromosomal) that give rise to in-frame fusion transcripts (Table 1).

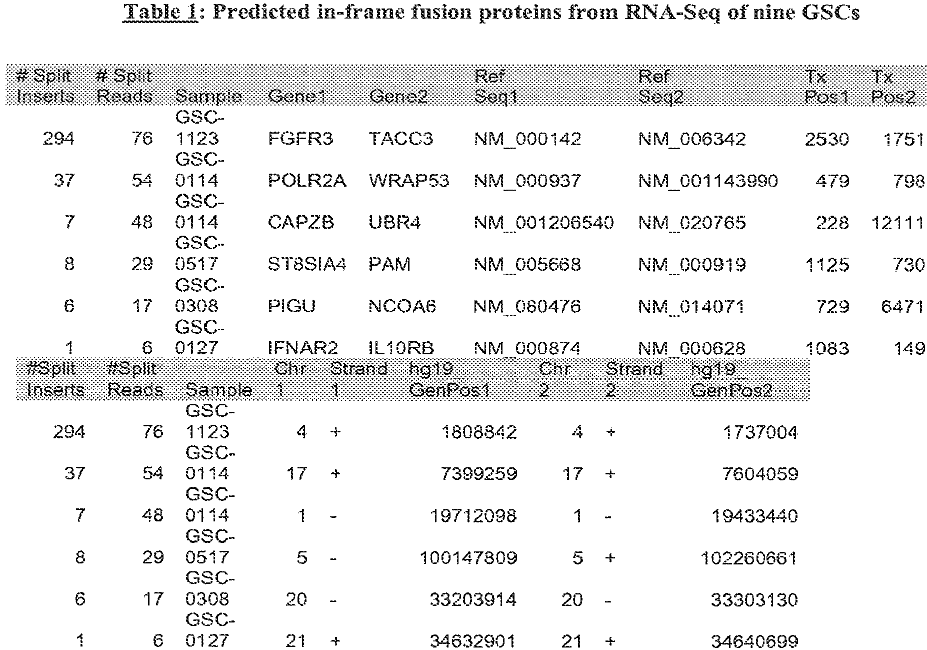

Next, genomic rearrangements leading to gene fusions were identified in GBM by applying Exome-Fuse to a dataset of paired-end exome DNA sequences from 84 GBM samples from TCGA (Table 2).

This analysis detected 147 paired gene fusions, thus producing an average of 1.75 gene fusion events per tumor (Table 3).

The FGFR and TACC families of genes were markedly enriched among those recurrently involved in genomic fusions, with eight tumors harboring FGFR rearrangements and seven tumors harboring fusions that implicate TACC genes (**Figure 1A**). The comparative analysis of the TX-Fuse and Exon-Fuse outputs revealed that FGFR3-TACC3 was the only fusion pair identified as either an in-frame transcript by TX-Fuse and genomic fusions by Exome-Fuse (Tables 1, 2 and 3).

**Table 2** shows fusion breakpoint information of recurrent gene fusions identified by Exome-fuse analysis of 84 GBM from TCGA. As multiple junctions may exist in each fusion candidate, information for all breakpoints is displayed. Column definitions include: sample = TCGA sample ID, virtForSplitReads/virtRevSplitReads/virtTotSplitReads = # forward/reverse/total split reads, splitinserts = # split inserts, dirA/dirB = forward (1) or reverse (0) direction of split read portion mapping to gene A/B, dirAB_matepair = direction of mate pair of split read, cosmicA+B = # recorded mutations of gene A+B in COSMIC.

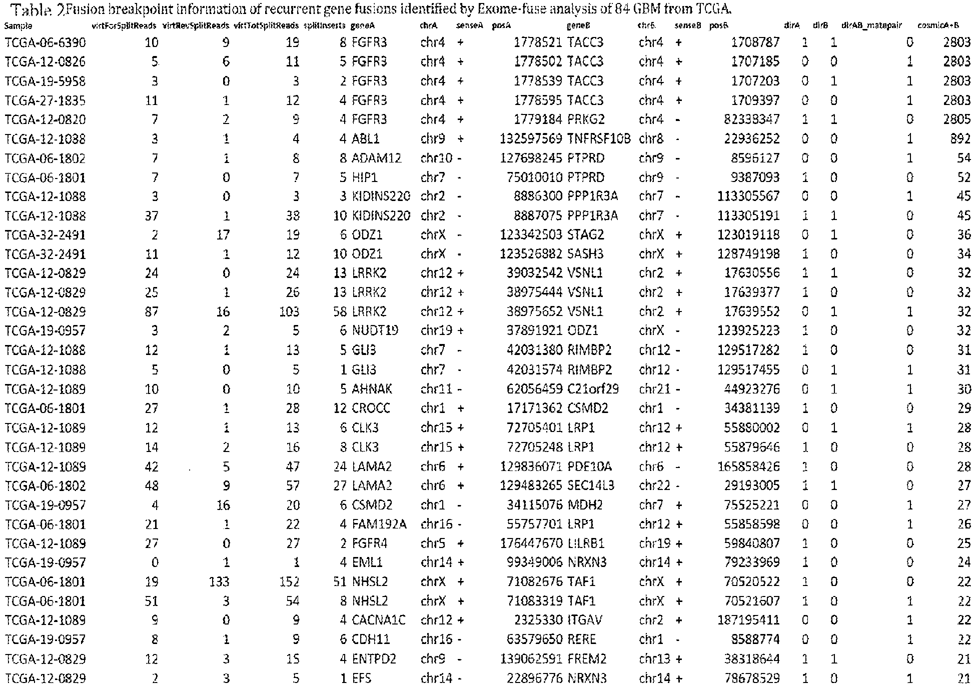

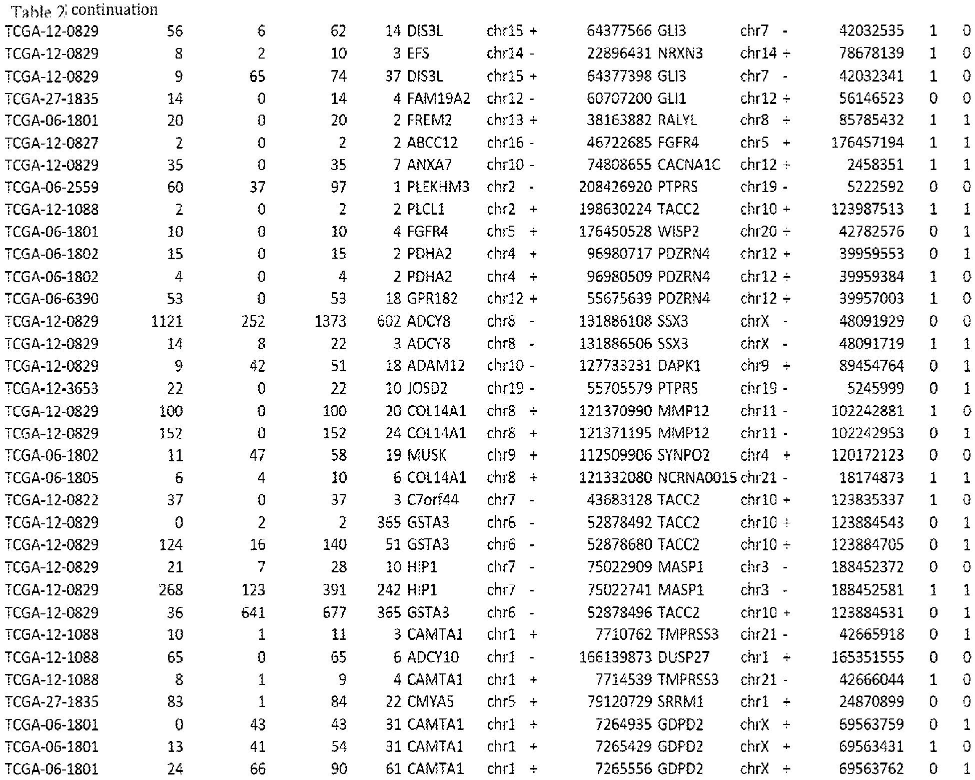

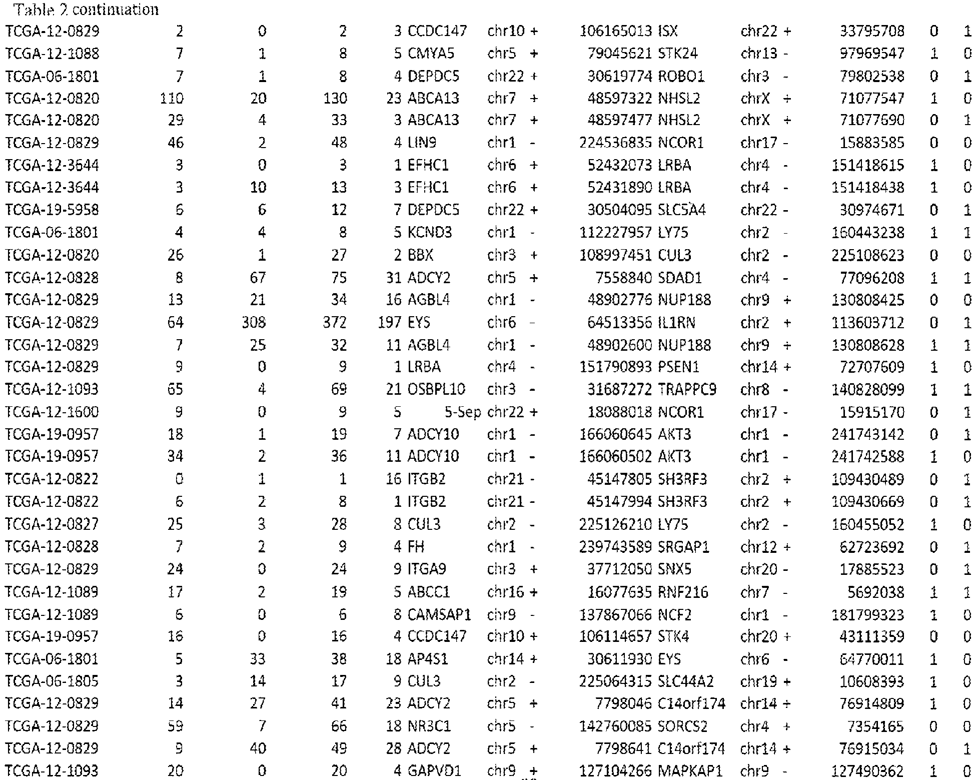

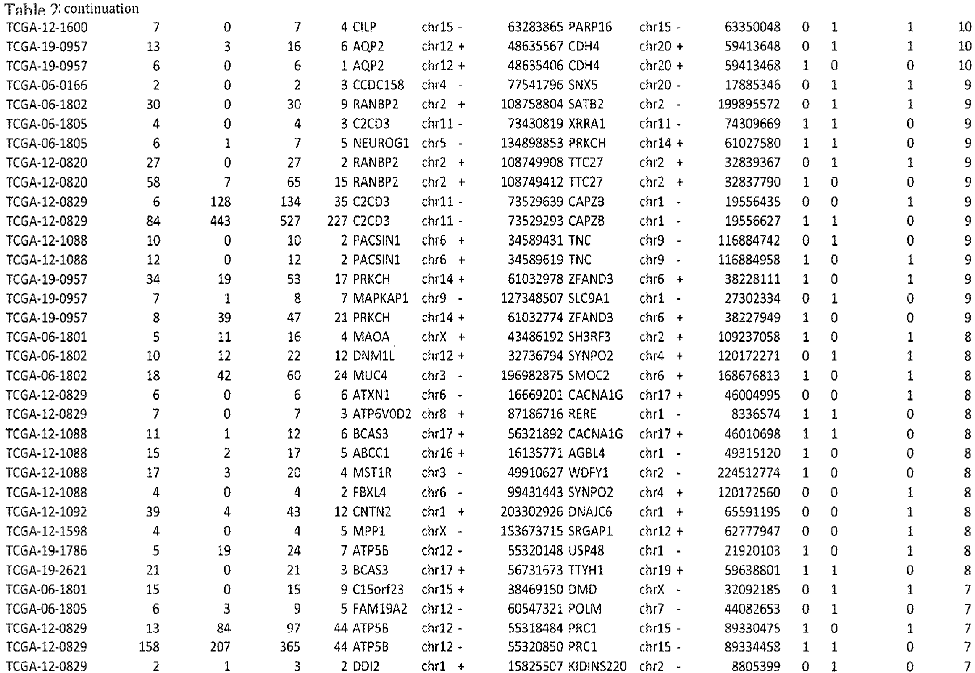

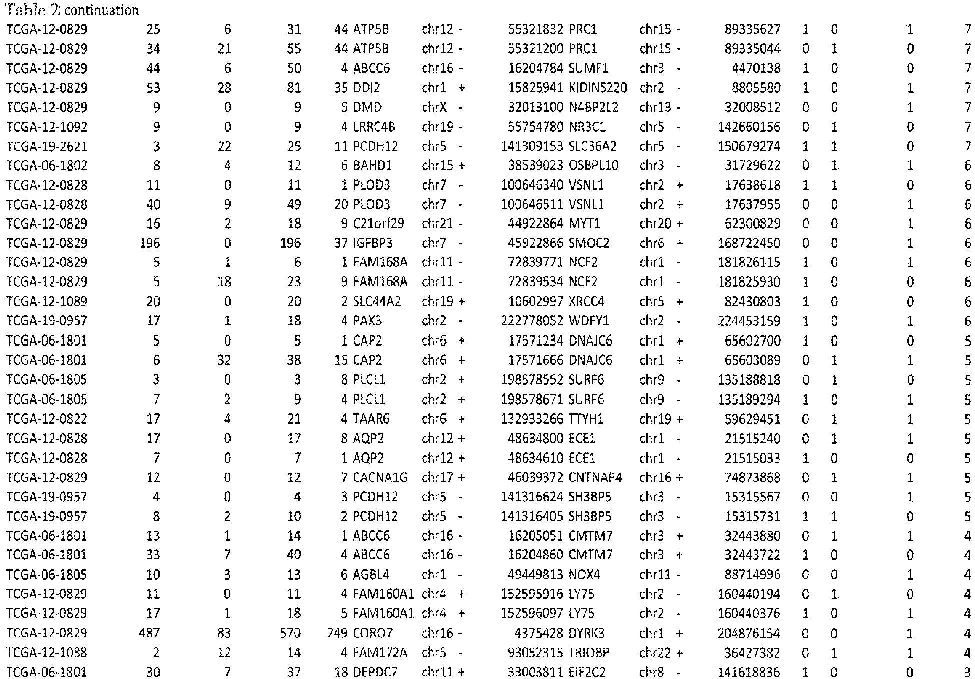

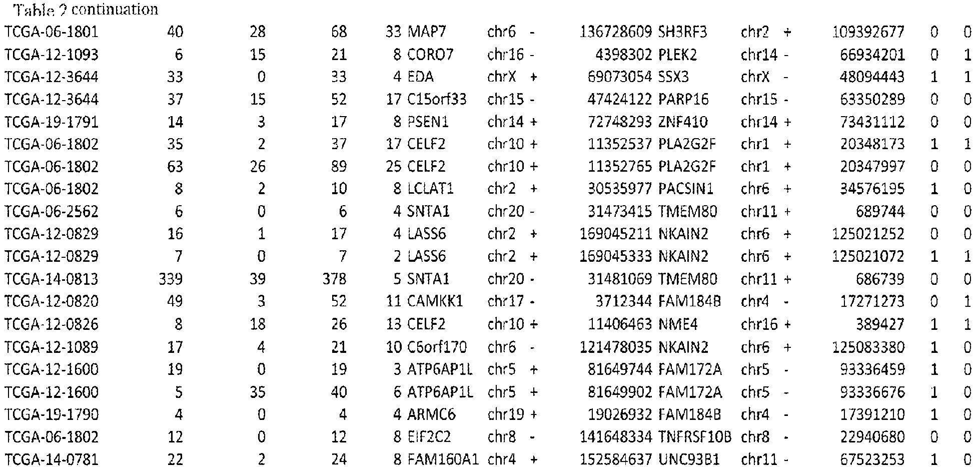

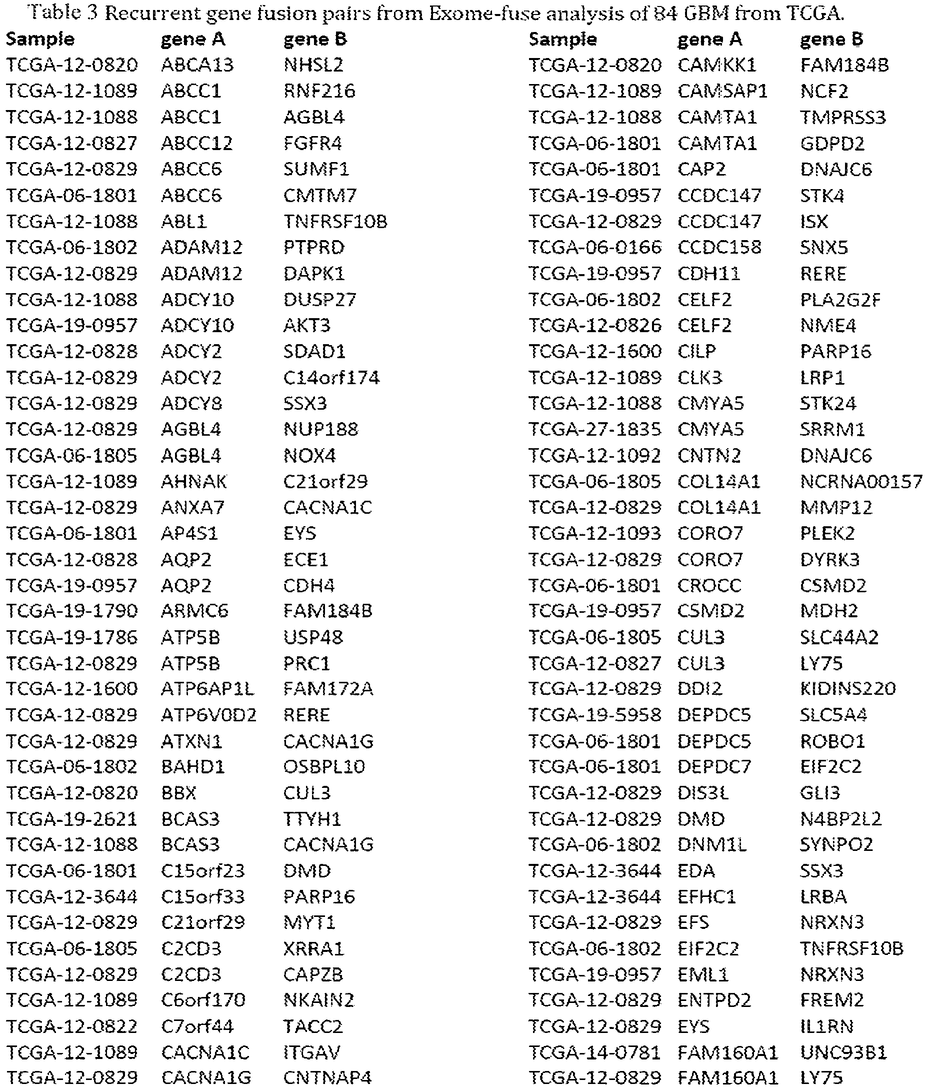

**Table 3** above shows recurrent gene fusion pairs from Exome-fuse analysis of 84 GBM from TCGA. Fusion candidates have been nominated if they have at least two split inserts and at least two split reads. To further filter the list on recurrence, any fusion candidate was kept in which one of the genes is involved in at least two fusions across different samples.

To experimentally validate the computational predictions that emerged from TX-Fuse, the PCR products spanning the fusion breakpoint were sequenced and validated each of the five in-frame fusion predictions (**Figures 1** and **9**). In Figure 1B, the prediction is shown and in **Figure 1C**, the cDNA sequence validation for the fusion with the highest read support involving FGFR", fused in-frame with TACC3) in GSC-1123 is shown. The same FGFR3-TACC3 fusion transcript was also detected in the primary GBM-1123 tumor specimen from which the GSC-1123 culture was established (**Figure 1C**). The amplified cDNA contained an open reading frame for a protein of 1,048 amino acids resulting from the fusion of a FGFR3 amino-terminal portion of residues 1-758 with a TACC3 carboxy-terminal portion of residues 549-838 (**Figure 1D**). FGFR3 is a member of the FGFR receptor tyrosine kinase (TK) family that transduces intracellular signals after binding to FGF ligands (Turner and Grose, 2010). TACC3 belongs to the evolutionarily conserved TACC gene family, which also includes TACC1 and TACC2. The distinctive feature of TACC proteins is the presence of a coiled-coil domain at the C-terminus, known as the TACC domain. Through the TACC domain, TACC proteins localize to the mitotic spindle during metaphase and stabilize the microtubule spindle network (Hood and Royle, 2011; Peset and Vernos, 2008). In the predicted fusion protein the intracellular TK domain of FGFR3 is fused upstream of the TACC domain of TACC3 (**Figure 1D**).

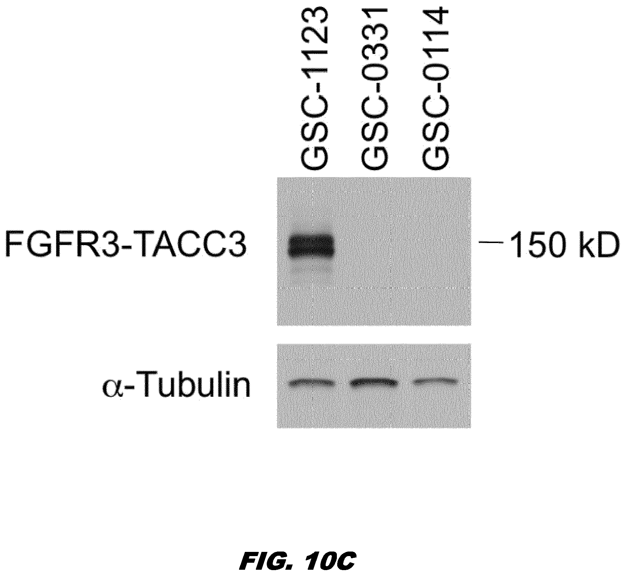

Exon-specific gene expression analysis from the RNA-seq coverage in GSC-1123 demonstrated that the FGFR3 and TACC3 exons implicated in the fusion are highly overexpressed compared with the mRNA sequences not included in the fusion event (**Figure 10A**). Quantitative RT-PCR showed that the expression of the fused FGFR3-TACC3 exons is significantly higher in GSC-1123 than other GSCs and the normal brain (80 to 130-fold, **Figure 10B**). Without being bound by theory, functionally significant genetic rearrangements may result in marked overexpression (outlier) of the genes implicated in the fusion events (Tomlins et al., 2007; Tomlins et al., 2005). The FGFR3-TACC3 fusion protein was also abundantly expressed in GSC-1123 and in the primary tumor GBM-1123, as shown by Western blot and immunohistochemistry (**Figures 10C** **and** **10D**). On a Western Blot, the FGFR3-TACC3 fusion protein migrated at a size of ~150 kD and immunoprecipitation followed by mass spectrometry revealed the presence of FGFR3 and TACC3 peptides consistent with the cDNA translation prediction (Figure 10E). Using PCR, the genomic breakpoint coordinates were mapped to chromosome 4 (#1,808,966 for FGFR3 and #1,737,080 for TACC3, genome build GRCh37/hg19) falling within FGFR3 exon 17 and TACC3 intron 7, which gives rise to a transcript in which the 5' FGFR3 exon 16 is spliced to the 3' TACC3 exon 8. The DNA junctions of FGFR3 and TACC3 show microhomology within a 10-base region, an observation consistent with results previously reported for other chromosomal rearrangements in human cancer (Bass et al., 2011; Stephens et al., 2009) **(****Figure 1E****).**

The experimental validation of the inferred genomic fusions was focused on FGFR3-TACC3. Exome-Fuse identified FGFR3-TACC3 gene fusions in four GBM samples with breakpoints spanning invariably within intron 16 of FGFR3 (which is downstream to the coding region for the TK domain) and intron 7-10 of TACC3 (which is upstream to the TACC domain) **(****Figure 2A****,** Tables 4 and 5). Among the four positive TCGA GBM specimens, two were available from TCGA centers for molecular analysis (TCGA-27-1835 and TCGA-06-6390) and, by Sanger sequencing, each of them were confirmed to carry an in-frame fusion transcript that is consistent with the predicted genomic breakpoints **(****Figures 2B** and **2C****).** Thus, the frames of the FGFR3-TACC3 fusion proteins invariably result in juxtaposing the TK domain of FGFR3 upstream of the TACC domain of TACC3. Consistent with the abundant expression of FGFR3-TACC3 in GSC-1123 and GBM-1123, the mRNA expression analysis of the TCGA tumors revealed that the four FGFR3-TACC3-positive GBM display marked co-outiier expression of FGFR3 and TACC3 (**Figure 2D**). Recurrent gene fusions can be associated with local copy number variations (CNV) of the breakpoint regions (Wang et al., 2009). Accordingly, the analysis of SNP arrays in the TCGA dataset revealed the presence of microamplification events of the FGFR3 and TACC3 genes in all four FGFR3-TACC3-positive GBM (**Figure 2E**).

**Table 4. List of split inserts supporting the identification of FGFR3-TACC3 fusion genes in four GBM samples from the ATLAS-TCGA exome collection**

| TCGA sample ID | gene1 | gene 1 length | read ID | % identity | length | mis-match | gap |
|---|---|---|---|---|---|---|---|
| TCGA-06-6390 | FGFR3 | 76 | C01PRACXX110628:1:1301:1934:116558 | 100 | 76 | 0 | 0 |
| TCGA-06-6390 | FGFR3 | 76 | C01RDACXX110628:3 :2305:4872:47008 | 98.68 | 76 | 1 | 0 |
| TCGA-06-6390 | FGFR3 | 76 | D03U9ACXX110625:6:1203:16178:138219 | 100 | 76 | 0 | 0 |
| TCGA-06-6390 | TACC3 | 76 | C01PRACXX110628:2:1102:13552:120312 | 100 | 76 | 0 | 0 |
| TCGA-06-6390 | TACC3 | 76 | C01PRACXX110628:8:2308:6515:60354 | 100 | 76 | 0 | 0 |
| TCGA-06-6390 | TACC3 | 76 | C01RDACXX110628:6:1305:16843:57213 | 98.68 | 76 | 1 | 0 |
| TCGA-06-6390 | TACC3 | 75 | D03U9ACXX110625:2:1202:19578:90281 | 100 | 75 | 0 | 0 |
| TCGA-06-6390 | TACC3 | 76 | D03U9ACXX110625:4:2306:2694:174970 | 100 | 76 | 0 | 0 |
| TCGA-12-0826 | FGFR3 | 72 | 61C59AAXX100217:4:21:17613:20886 | 98.61 | 72 | 0 | 1 |
| TCGA-12-0826 | TACC3 | 75 | 42MJNAAXX090813:5:30:1412:1280#0 | 100 | 75 | 0 | 0 |
| TCGA-12-0826 | TACC3 | 76 | 61C59AAXX100217:4:2:4279:6949 | 100 | 76 | 0 | 0 |
| TCGA-12-0826 | FGFR3 | 76 | 42MJNAAXX090813:5:37:435:1250#0 | 100 | 76 | 0 | 0 |
| TCGA-12-0826 | FGFR3 | 51 | 61C59AAXX100217:5:89:7727:2557 | 98.04 | 51 | 1 | 0 |
| TCGA-19-5958 | TACC3 | 62 | D03U9ACXX110625:4:2206:9451:114168 | 90.32 | 62 | 6 | 0 |
| TCGA-19-5958 | TACC3 | 74 | D03U9ACXX110625:1:2204:20064:21192 | 95.95 | 74 | 3 | 0 |
| TCGA-27-1835 | FGFR3 | 76 | C00HWABXX110325:7:2202:17680:110666 | 100 | 76 | 0 | 0 |
| TCGA-27-1835 | TACC3 | 76 | C00HWABXX110325:7:1104:10731:5183 | 100 | 76 | 0 | 0 |
| TCGA-27-1835 | TACC3 | 60 | B09V2ABXX110408:2:2201:5811:24541 | 100 | 60 | 0 | 0 |
| TCGA-27-1835 | TACC3 | 61 | B097UABXX110405:4:2102:15742:63594 | 91.8 | 61 | 5 | 0 |

| TCGA sample ID | read start | read end | hg18 genome start | hg18 genome end | e-value | bit score | read 1 fasta |
|---|---|---|---|---|---|---|---|
| TCGA-06-6390 | 1 | 76 | 1778372 | 1778447 | 8E-40 | 151 | GTGCTGGCATGCCGCGCCCTCCCAGAGGCCCACCTTCAAG CAGCTGGTGGAGGACCTGGACCGTGTCCTTACCGTG |
| TCGA-06-6390 | 1 | 76 | 1778364 | 1778439 | 2E-37 | 143 | ATGCGGGAGTGCTGGCATGACGCGCCCTCCCAGAGGCCC ACCTTCAAGCAGCTGGTGGAGGACCTGGACCGTGTCC |
| TCGA-06-6390 | 1 | 76 | 1778413 | 1778488 | 8E-40 | 151 | AGCTGGTGGAGGACCTGGACCGTGTCCTTACCGTGACGTC CACCGACGTGAGTGCTGGCTCTGGCCTGGTGCCACC |
| TCGA-06-6390 | 1 | 76 | 1708918 | 1708843 | 8E-40 | 151 | CCCTTAAAACAACTCGTTCCCTCAGACCACACAAGAC AGTTCAAGAGGGACTCAAGGACTTACAGGAATGTCCA |
| TCGA-06-6390 | 1 | 76 | 1708956 | 1708881 | 8E-40 | 151 | AACCAAAGGCTCAGACCCCCAGGAATAGAAAATATAGGC CCTTAAAACAACTCGTTCCCTCAGACCACACACAAGA |
| TCGA-06-6390 | 1 | 76 | 1708865 | 1708790 | 2E-37 | 143 | TCAAGGACTTACAGGAATGTCCAGTGCTCCCAAGAAATC GAACTCCACAAGCTTGGCTTCCCGCGCACGTCCTGAG |
| TCGA-06-6390 | 1 | 75 | 1708861 | 1708787 | 3E-39 | 149 | GGACTTACAGGAATGTCCAGTGCTCCCAAGAAATCGAAC TCCACAAGCTTGGCTTCCCGCGGACGTCCTGAGGGAT |
| TCGA-06-6390 | 1 | 76 | 1708896 | 1708821 | 8E-40 | 151 | CAGACCACACACAAGACAGTTCAAGAGGGACTCAAGGAC TTACAGGAATGTCCAGTGCTCCCAAGAAATCGAACTC |
| TCGA-12-0826 | 1 | 71 | 1778439 | 1778510 | 2E-34 | 133 | CTTACCGTGACGTCCACCGACGTGAGTGCTGGCTCTGGCC TGGTGCCACCCGCCTATGCCCCTCCCCTGCCCTTAG |
| TCGA-12-0826 | 2 | 76 | 1707299 | 1707225 | 3E-39 | 149 | AAACTTGAGGTATAAGGACTGCTTCCTCAAGGCCGACTCC TTAAACTGGGGACAAGAGGGCAAGTGATCAGGTCTG |
| TCGA-12-0826 | 1 | 76 | 1707299 | 1707224 | 8E-40 | 151 | AACTTGAGGTATAAGGACTGCTTCCTCAAGGCCGACTCCT TAAACTGGGGACAAGAGGGCAAGTGATCAGGTCTGA |
| TCGA-12-0826 | 1 | 76 | 1778346 | 1778421 | 8E-40 | 151 | GCCCGCAGGTACATGATCATGCGGGAGTGCTGGCATGCC GCGCCCTCCCAGAGGCCCACCTTCAAGCAGCTGGTGG |
| TCGA-12-0826 | 1 | 51 | 1778443 | 1778493 | 4E-24 | 93.7 | ACCGTGACGTCCACCGACGTGAGTGCTGGCTCTGGCCTGG TGCGACCCGCCGATCTCTCTCCCCTGTCCTTTTCCT |
| TCGA-19-5958 | 1 | 62 | 1707141 | 1707202 | 4E-17 | 75.8 | TGGGAGGGTGCGGGGGGCCGGGGGGGGGAGTGTGCAGG TGAGCTCCCTGGCCCTTGGCCCCCTGCCCTCTGGGGGG |
| TCGA-19-5958 | 1 | 74 | 1707097 | 1707170 | 5E-33 | 123 | CTGGGAATGGTGGTGTCTCGGGCAGGGTTGTGGGTGACC GGGGGTGGGAGGGTGCGGGGGACCGGGGGGGGGAGGG |
| TCGA-27-1835 | 1 | 76 | 1778338 | 1778413 | 8E-40 | 151 | AGCGCCCTGCCCGCAGGTACATGATCATGCGGAGTGCT GGCATGCCGCGCCCTCCCAGAGGCCCACCTTCAAGCA |
| TCGA-27-1835 | 1 | 76 | 1709492 | 1709417 | 8E-40 | 151 | GCCAACGCCATGCCCAGGCCGGAGAGTCCCGGGAGGCT GCTGGTGGGCAGCTGACTGCGGGACACTGGGTGGAA |
| TCGA-27-1835 | 1 | 60 | 1709504 | 1709445 | 3E-30 | 119 | AGGCCACCAGAGGCCAACGCCATGCCCAGGCCGGAGAGT CCCGGGGAGGCTGCTGGTGGGAGGCGAACGCGGGGA |
| TCGA-27-1835 | 1 | 61 | 1709482 | 1709422 | 6E-19 | 81.8 | TGCCCAGGCCGGAGAGTCCCGGGCGGCTGCTGGGGGG AGCTGACTGGGGGGCACTGGGGGGAGACCCGGGCC |

| TCGA sampleID | gene2 | gene2 length | read_ID | % identity | length | mis-match | gap |
|---|---|---|---|---|---|---|---|
| TCGA-06-6390 | TACC3 | 76 | C01PRACXX110628:1:1301:1934:116558 | 100 | 76 | 0 | 0 |
| TCGA-06-6390 | TACC3 | 76 | C01RDACXX110628:3 :2305:4872:47008 | 100 | 76 | 0 | 0 |
| TCGA-06-6390 | TACC3 | 76 | D03U9ACXX110625:6:1203:16178:138219 | 100 | 76 | 0 | 0 |
| TCGA-06-6390 | FGFR3 | 76 | C01PRACXX110628:2:1102:13552:120312 | 100 | 76 | 0 | 0 |
| TCGA-06-6390 | FGFR3 | 76 | C01PRACXX110628:8:2308:6515:60354 | 100 | 76 | 0 | 0 |
| TCGA-06-6390 | FGFR3 | 76 | C01RDACXX110628:6:1305:16843:57213 | 96.05 | 76 | 3 | 0 |
| TCGA-06-6390 | FGFR3 | 76 | D03U9ACXX110625:2:1202:19578:90281 | 100 | 76 | 0 | 0 |
| TCGA-06-6390 | FGFR3 | 76 | D03U9ACXX110625:4:2306:2694:174970 | 100 | 76 | 0 | 0 |
| TCGA-12-0826 | TACC3 | 72 | 61C59AAXX100217:4:21:17613:20886 | 95.83 | 72 | 3 | 0 |
| TCGA-12-0826 | FGFR3 | 76 | 42MJNAAXX090813:5:30:1412:1280#0 | 98.68 | 76 | 1 | 0 |
| TCGA-12-0826 | FGFR3 | 76 | 61C59AAXX100217:4:2:4279:6949 | 98.68 | 76 | 0 | 1 |
| TCGA-12-0826 | TACC3 | 67 | 42MJNAAXX090813:5:37:435:1250#0 | 98.51 | 67 | 1 | 0 |
| TCGA-12-0826 | TACC3 | 75 | 61C59AAXX100217:5:89:7727:2557 | 97.33 | 75 | 2 | 0 |
| TCGA-19-5958 | FGFR3 | 76 | D03U9ACXX110625:4:2206:9451:114168 | 98.68 | 76 | 1 | 0 |
| TCGA-19-5958 | FGFR3 | 76 | D03U9ACXX110625:1:2204:20064:21192 | 100 | 76 | 0 | 0 |
| TCGA-27-1835 | TACC3 | 76 | C00HWABXX110325:7:2202:17680:110666 | 96.05 | 76 | 3 | 0 |
| TCGA-27-1835 | FGFR3 | 76 | C00HWABXX110325:7:1104:10731:5183 | 96.05 | 76 | 3 | 0 |
| TCGA-27-1835 | FGFR3 | 76 | B09V2ABXX110408:2:2201:5811:24541 | 100 | 76 | 0 | 0 |
| TCGA-27-1835 | FGFR3 | 76 | B097UABXX110405:4:2102:15742:63594 | 100 | 76 | 0 | 0 |

| TCGA sampleID | read start | read end | hg18 genome start | hg18 genome end | e-value | bit score | read 2 fasta |
|---|---|---|---|---|---|---|---|
| TCGA-06-6390 | 1 | 76 | 1708922 | 1708847 | 8E-40 | 151 | TAGGCCCTTAAAACAACTCGTTCCCTCAGACCACACACAAGACAGTT CAAGAGGGACTCAAGGACTTACAGGAATG |
| TCGA-06-6390 | 1 | 76 | 1708867 | 1708792 | 8E-40 | 151 | ACTCAAGGACTTACAGGAATGTCCAGTGCTCCCAAGAAATCGAACTC CACAAGCTTGGCTTCCCGCGGACGTCCTG |
| TCGA-06-6390 | 1 | 76 | 1708921 | 1708846 | 8E-40 | 151 | AGGCCCTTAAAACAACTCGTTCCCTCAGACCACACACAAGACAGTTC AAGAGGGACTCAAGGACTTACAGGAATGT |
| TCGA-06-6390 | 1 | 76 | 1778387 | 1778462 | 8E-40 | 151 | GCCCTCCCAGAGGCCCACCTTCAAGCAGCTGGTGGAGGACCTGGACC GTGTCCTTACCGTGACGTCCACCGACGTG |
| TCGA-06-6390 | 1 | 76 | 1778382 | 1778457 | 8E-40 | 151 | GCCGCGCCCTCCCAGAGGCCCACCTTCAAGCAGCTGGTGGAGGACCT GGACCGTGTCCTTACCGTGACGTCCACCG |
| TCGA-06-6390 | 1 | 76 | 1778417 | 1778492 | lE-32 | 127 | GGTGGAGGACCTGGACCGTGACCTTACCGGGACGTCCACCGACGGG AGTGCTGGCTCTGGCCTGGTGCCACCCGCC |
| TCGA-06-6390 | 1 | 76 | 1778447 | 1778522 | 8E-40 | 151 | GACGTCCACCGACGTGAGTGCTGGCTCTGGCCTGGTGCCACCCGCCT ATGCCCCTCCCCCTGCCGTCCCCGGCCAT |
| TCGA-06-6390 | 1 | 76 | 1778435 | 1778510 | 8E-40 | 151 | TGTCCTTACCGTGACGTCCACCGACGTGAGTGCTGGCTCTGGCCTGG TGCCACCCGCCTATGCCCCTCCCCCTGCC |
| TCGA-12-0826 | 1 | 72 | 1707362 | 1707291 | 3E-30 | 119 | TACCTGCTGGTCTCGGTGGCCACGGGCACTGGTCTACCAGGGCTGTC CCTCCGGAGGGGGTCAAACTTGAGGGATA |
| TCGA-12-0826 | 1 | 76 | 1778427 | 1778502 | 2E-37 | 143 | CTGGACCGTGTCCTTACCGTGACGTCCACCGACGTGAGTGCTGGCTC TGGCCTGGTGCCACCCGCCCATGCCCCTC |
| TCGA-12-0826 | 1 | 75 | 1778435 | 1778510 | 8E-37 | 141 | TGTCCTTACCGTGACGTCCACCGACGTGAGTGCTGGCTCTGGCCTGG TGCCACCCGCCTATGCCCCTCCCCTGCCC |
| TCGA-12-0826 | 1 | 67 | 1707635 | 1707569 | 5E-32 | 125 | AAAAGATTTAAGTTTAGATCTTTAATATACCTAGAACGGTGGCTGTA ACCAGCAAGGCAGGAGCCCTTTGTGTTGG |
| TCGA-12-0826 | 2 | 76 | 1707306 | 1707232 | 5E-36 | 133 | TGGGTCAAACTTGAGGTATAAGGACTGCTTCCTCAAGGCCGACTCCT TATACTGGGGACAAGAGGGCAAGTGATCA |
| TCGA-19-5958 | 1 | 76 | 1778462 | 1778537 | 2E-37 | 143 | GAGTGCTGGCTCTGGCCTGGTGCCACCCGCCTATGCCCCTCCCCCTG GCGTCCCCGGCCATCCTGCCCCCCAGAGT |
| TCGA-19-5958 | 1 | 76 | 1778462 | 1778537 | 2E-41 | 151 | GAGTGCTGGCTCTGGCCTGGTGCCACCCGCCTATGCCCCTCCCCCTG CCGTCCCCGGCCATCCTGCCCCCCAGAGT |
| TCGA-27-1835 | 1 | 76 | 1709492 | 1709417 | IE-32 | 127 | GCCAACGCCATGCCCAGGCCGGAGAGTCCCGGGAGGCTGCTGGTG GGGAGCTGACTTCGGGGACACTGGGGGGAA |
| TCGA-27-1835 | 1 | 76 | 1778363 | 1778438 | IE-32 | 127 | CATGCGGGAGTGCTGGCATGGCGCGCCCTCCCAGCGGCCCACCTTCA AGCAGCTGGTGGGGGACCTGGACCGTGTC |
| TCGA-27-1835 | 1 | 76 | 1778458 | 1778533 | 8E-40 | 151 | ACGTGAGTGCTGGCTCTGGCCTGGTGCCACCCGCCTATGCCCCTCCC CCTGCCGTCCCCGGCCATCCTGCCCCCCA |
| TCGA-27-1835 | 1 | 76 | 1778388 | 1778463 | 8E-40 | 151 | CCCTCCCAGAGGCCCACCTTCAAGCAGCTGGTGGAGGACCTGGACCG TGTCCTTACCGTGACGTCCACCGACGTGA |

**Table 5: List of split reads supporting the identification of FGFR3-TACC3 fusion genes in four GBM samples from the ATLAS-TCGA exome collection**

| sample | Gene split 1 | readiID | directionsplit | hg18 start split1 | hg18 stop split1 |
|---|---|---|---|---|---|
| TCGA-06-6390 | TACC3 | D03U9ACXX110625:2:1202:19578:90281 | R | 1778521 | 1778521 |
| TCGA-06-6390 | FGFR3 | C01PRACXX110628:3:1104:10052:66371 | F | 1778520 | 1778521 |
| TCGA-06-6390 | FGFR3 | C01PRACXX110628:5:1108:3119:22892 | F | 1778520 | 1778521 |
| TCGA-06-6390 | FGFR3 | D03U9ACXX110625:8:2304:13007:108632 | F | 1778520 | 1778521 |
| TCGA-06-6390 | FGFR3 | C01PRACXX110628:5:2108:1999:91559 | F | 1778518 | 1778521 |
| TCGA-06-6390 | FGFR3 | C01RDACXX110628:3:1308:1446:66311 | F | 1778515 | 1778521 |
| TCGA-06-6390 | TACC3 | D03U9ACXX110625:5:2205:12523:196352 | R | 1778514 | 1778521 |
| TCGA-06-6390 | TACC3 | C01PRACXX110628:5:2103:6815:17943 | R | 1778514 | 1778521 |
| TCGA-06-6390 | FGFR3 | C01PRACXX110628:3:1204:10831:2928 | F | 1778512 | 1778521 |
| TCGA-06-6390 | FGFR3 | C01PRACXX110628:5:2204:6732: 191360 | F | 1778512 | 1778521 |
| TCGA-06-6390 | FGFR3 | C01PRACXX110628:8:1308:2911:26590 | F | 1778511 | 1778521 |
| TCGA-06-6390 | FGFR3 | C01PRACXX110628:8:2207:4586:84017 | F | 1778509 | 1778521 |
| TCGA-06-6390 | TACC3 | C01PRACXX110628:7:2205:11825:39734 | R | 1778501 | 1778521 |
| TCGA-06-6390 | TACC3 | C01PRACXX110628:6:1106:12159:179499 | R | 1778494 | 1778521 |
| TCGA-06-6390 | TACC3 | D03U9ACXX110625:4:2202:12501:40389 | R | 1778491 | 1778521 |
| TCGA-06-6390 | FGFR3 | C01RDACXX110628:3:1305:3044:13238 | F | 1778473 | 1778521 |
| TCGA-06-6390 | TACC3 | D03U9ACXX110625:5:2205:12523:196352 | R | 1778470 | 1778521 |
| TCGA-06-6390 | TACC3 | C01PRACXX110628:7:2205:11825:39734 | R | 1778469 | 1778521 |
| TCGA-06-6390 | FGFR3 | D03U9ACXX110625:7:2106:4492: 173350 | F | 1778464 | 1778521 |
| TCGA-06-6390 | TACC3 | C01PRACXX110628:5:2103:6815:17943 | R | 1778452 | 1778521 |
| TCGA-12-0826 | TACC3 | 61C59AAXX100217:4:93:15133:6133 | R | 1778495 | 1778502 |
| TCGA-12-0826 | TACC3 | 61C59AAXX100217:5:107:10675:16040 | R | 1778495 | 1778502 |
| TCGA-12-0826 | FGFR3 | 61C59AAXX100217:5:108:1809:11295 | F | 1778494 | 1778502 |
| TCGA-12-0826 | FGFR3 | 61C59AAXX100217:5:82:13129:10637 | F | 1778490 | 1778502 |
| TCGA-12-0826 | FGFR3 | 42MJNAAXX090813:6:80:691:1877#0 | F | 1778481 | 1778502 |
| TCGA-12-0826 | TACC3 | 61C59AAXX100217:3:75:10586:12881 | R | 1778470 | 1778502 |
| TCGA-12-0826 | TACC3 | 61C59AAXX100217:4:114:5844:3161 | R | 1778470 | 1778502 |
| TCGA-12-0826 | TACC3 | 42MJNAAXX090813:5:70:888:108#0 | R | 1778466 | 1778502 |
| TCGA-12-0826 | TACC3 | 61C59AAXX100217:3:55:4966:15975 | R | 1778451 | 1778502 |
| TCGA-12-0826 | FGFR3 | 42MJNAAXX090813:5:23:156:1150#0 | F | 1778447 | 1778502 |
| TCGA-12-0826 | FGFR3 | 61C59AAXX100217:4:21:17613:20886 | F | 1778439 | 1778502 |
| TCGA-12-0826 | FGFR3 | 61C59AAXX100217:4:2:4279:6949 | F | 1778435 | 1778502 |
| TCGA-19-5958 | TACC3 | C01RDACXX110628:6:1102:11157:101962 | R | 1778533 | 1778539 |
| TCGA-19-5958 | TACC3 | C01REACXX110629:2:2104:5009:98392 | R | 1778517 | 1778539 |
| TCGA-19-5958 | TACC3 | C01PRACXX110628:7:2103:12434:91988 | R | 1778501 | 1778539 |
| TCGA-27-1835 | TACC3 | B06UCABXX110322:6:1103:9262:46754 | R | 1778586 | 1778595 |
| TCGA-27-1835 | FGFR3 | C00HWABXX110325:4:1201:20980:90877 | F | 1778567 | 1778595 |
| TCGA-27-1835 | TACC3 | B06UCABXX110322:5:1108:14043:83287 | R | 1778564 | 1778595 |
| TCGA-27-1835 | TACC3 | B097UABXX110405:4:2204:19445:88453 | R | 1778558 | 1778595 |
| TCGA-27-1835 | TACC3 | B097UABXX110405:4:2201:20658:44401 | R | 1778557 | 1778595 |
| TCGA-27-1835 | TACC3 | B097UABXX110405:2:2104:15688:71022 | R | 1778555 | 1778595 |
| TCGA-27-1835 | TACC3 | C00HWABXX110325:6:2102:20394:42427 | R | 1778543 | 1778595 |
| TCGA-27-1835 | TACC3 | B09V2ABXX110408:6:1203:18187:141862 | R | 1778543 | 1778595 |
| TCGA-27-1835 | TACC3 | B09V2ABXX110408:8:1205:4774:81604 | R | 1778537 | 1778595 |
| TCGA-27-1835 | TACC3 | C00HWABXX110325:2:1107:16168:23614 | R | 1778535 | 1778595 |
| TCGA-27-1835 | TACC3 | COOHWABXX110325:7:2107:1225:167363 | R | 1778530 | 1778595 |
| TCGA-27-1835 | TACC3 | B097UABXX110405:2:2104:15688:71022 | R | 1778523 | 1778595 |

| sample | length 1 | mis-match 1 | gap 1 | seqsplit | |
|---|---|---|---|---|---|
| TCGA-06-6390 | 1 | 0 | 0 | GGACTTACAGGAATGTCCAGTGCTCCCAAGAAATCGAACTCCACAAG CTTGGCTTCCCGCGGACGTCCTGAGGGA***T | |
| TCGA-06-6390 | 2 | 0 | 0 | CA***TCCCTCAGGACGTCCGCGGGAAGCCAAGCTTGTGGAGTTCGAT TTCTTGGGAGCACTGGACATTCCTGTAAGTC | |
| TCGA-06-6390 | 2 | 0 | 0 | CA***TCCCTCAGGACGTCCGCGGAAGCCAAGCTTGTGGAGTTCGAT TTCTTGGGAGCACTGGACATTCCTGTAAGTC | |
| TCGA-06-6390 | 2 | 0 | 0 | CA***TCCCTCAGGACGTCCGCGGAAGCCAAGCTTGTGGAGTTCGAT TTCTTGGGAGCACTGGACATTCCTGTAAGTC | |
| TCGA-06-6390 | 4 | 0 | 0 | GCCA***TCCCTCAGGACGTCCGCGGGAAGCCAAGCTTGTGGAGTTCG ATTTCTTGGGAGCACTGGACATTCCTGTAAG | |
| TCGA-06-6390 | 7 | 0 | 0 | CCGGCCA***TCCCTCAGGACGTCCGCGGGAAGCCAAGCTTGTGGAGT TCGATTTCTTGGGAGCACTGGACATTCCTGT | |
| TCGA-06-6390 | 8 | 1 | 0 | CAGGAATGTCCAGTGCTACCAAGAAATCGAACTCCACAAGCTTGGGT TCCCGCGGACGTCCTCCGGGA***TGGCCGTG | |
| TCGA-06-6390 | 8 | 0 | 0 | CAGGAATGTCCAGTGCTCCCAAGAAATCGAACTCCACAAGCTTGGCTT CCCGCGGACGTCCTGAGGGA***TGGCCGGG | |
| TCGA-06-6390 | 10 | 0 | 0 | TCCCCGGCCA***TCCCTCAGGACGTCCGCGGAAGCCAAGCTTGTGG AGTTCGATTTCTTGGGAGCACTGGACATTCC | |
| TCGA-06-6390 | 10 | 0 | 0 | TCCCCGGCCA***TCCCTCAGGANGTCCGCGGAAGCCAAGCTTGTGG AGTTCGATTTCTTGGGAGCACTGGACATTCC | |
| TCGA-06-6390 | 11 | 0 | 0 | GTCCCCGGCCA***TCCCTCAGGACGTCCGCGGAAGCCAAGCTTGTG GAGTTCGATTTCTTGGGAGCACTGGACATTC | |
| TCGA-06-6390 | 13 | 0 | 0 | CCGTCCCCGGCCA***TCCCTCAGGACGTCCGCGGGAAGCCAAGCTTG TGGAGTTCGATTTCTTGGGAGCACTGGACAT | |
| TCGA-06-6390 | 21 | 0 | 0 | TGCTCCCAAGAAATCGAACTCCACAAGCTTGGCTTCCCGCGGACGTCC TGAGGGA***TGGCCGGGGACGGCAGGGGGA | |
| TCGA-06-6390 | 28 | 0 | 0 | AAGAAATCGAACTCCACAAGCTTGGCTTCCCGCGGACGTCCTGAGGG A***TGGCCGGGGACGGCAGGGGGAGGGGCAT | |
| TCGA-06-6390 | 31 | 1 | 0 | AAATCGAACTCCACAAGCTTGGCTTCCCGCGGACGTCCTGAGGGA*** TGGCCGGGGGCGGCAGGGGGAGGGGCATAGG | |
| TCGA-06-6390 | 49 | 0 | 0 | CTGGCCTGGTGCCACCCGCCTATGCCCCTCCCCCTGCCGTCCCCGGCC A***TCCCTCAGGACGTCCGCGGGAAGCCAA | |
| TCGA-06-6390 | 52 | 4 | 0 | TCGTCCCGCGGACTTCCTGATGGA***TCGCCGGGGACGGCAGGGGGA GGGGCATAGGCGTGTGGCACCAGGCCAGCTC | |
| TCGA-06-6390 | 53 | 1 | 0 | CTTCCCGCGGACGTCCTGAGGGA***TGGCCGGGGACGGNAGGGGGA GGGGCATAGGCGGGTGGCACCAGGCCAGAGCC | |
| TCGA-06-6390 | 58 | 0 | 0 | GTGCTGGCTCTGGCCTGGTGCCACCCGCCTATGCCCCTCCCCCTGCCG TCCCCGGCCA***TCCCTCAGGACGTCCGCG | |
| TCGA-06-6390 | 70 | 0 | 0 | GAGGGA***TGGCCGGGGACGGCAGGGGGAGGGCATAGGCGGGTGG CACCAGGCCAGAGCCAGCACTCACGTCGGTGG | |
| TCGA-12-0826 | 8 | 0 | 0 | GGACAAGAGGGCAAGTGATCAGGTCTGACTGCCATCCCCTAACACAC ACAGGGGGGCTAAGGGCAGGG***GAGGGGCA | |
| TCGA-12-0826 | 8 | 0 | 0 | GGACAAGAGGGCAAGTGATCAGGTCTGACTGCCATCCCCTAACACAC ACAGGGGGGCTAAGGGCAGGG***GAGGGGCA | |
| TCGA-12-0826 | 9 | 0 | 0 | ATGCCCCTC***CCCTGCCCTTAGCCCCCCTGTGTGTGTTAGGGGATGG CAGTCAGACCTGATCACTTGCCCTCTTGTC | |
| TCGA-12-0826 | 13 | 0 | 0 | GCCTATGCCCCTC***CCCTGCCCTTAGCCCCCCTGTGTGTGTTAGGGG ATGGCAGTCAGACCTGATCACTTGCCCTCT | |
| TCGA-12-0826 | 22 | 0 | 0 | GTGCCACCCGCCTATGCCCCTC***CCCTGCCCTTAGCCCCCCTGTGTG TGTTAGGGGATGGCAGTCAGACCTGATCAC | |
| TCGA-12-0826 | 33 | 1 | 0 | TGACTGCCATCCCCTAACACACACAGGGGGGCTAAGGCAGGG***GA GGGGCATAGGCGGGGGGCACCAGGCCAGAGC | |
| TCGA-12-0826 | 33 | 1 | 0 | TGACTGCCATCCCCTAACACACACAGGGGGGCTAAGGCAGGG***GA GGGGCATAGGCGGGGGGCACCAGGCCAGAGC | |
| TCGA-12-0826 | 37 | 3 | 0 | TGCCATCCCCTAACACACACAGGGGGGCTAAGGCAGGG***GAGGG GCATAGGCGGGGGCACCAGGACAGAGGCAGC | |
| TCGA-12-0826 | 52 | 5 | 0 | CACACAGGGGGCTAAGGGCAGGG***GAGGGCATAGGCGGGGGG ACCAGGCCCGAGCCAGCACTCACGTCGGGGGG | |
| TCGA-12-0826 | 56 | 0 | 0 | GACGTCCACCGACGTGAGTGCTGGCTCTGGCCTGGTGCCACCCGCCTA TGCCCCTC***CCCTGCCCTTAGACCCCCTG | |
| TCGA-12-0826 | 64 | 0 | 0 | CTTACCGTGACGTCCACCGACGTGAGTGCTGGCTCTGGCCTGGTGCCA CCCGCCTATGCCCCTC***CCCTGCCCTTAG | |
| TCGA-12-0826 | 68 | 0 | 0 | TGTCCTTACCGTGACGTCCACCGACGTGAGTGCTGGCTCTGGCCTGGT GCCACCCGCCTATGCCCCTC***CCCTGCCC | |
| TCGA-19-5958 | 7 | 0 | 0 | CGGGGGTGGAGTGTGCGGGTGACCGGGGGTGGAGTGTGCAGGTGA CCTCCCTGGCCCTTAGCCCCCT***GCACTCT | |
| TCGA-19-5958 | 23 | 0 | 0 | CGGGTGACCGGGGGAGGGAGTGTGCAGGGACCTCCCTGGCCCTTAG CCCCCT***GCACTCTGGGGGCAGGATGGCC | |
| TCGA-19-5958 | 39 | 0 | 0 | GGAGTGTGCAGGTGACCTCCCTGGCCCTTAGCCCCCT***GCACTCTGG GGGGCAGGATGGCCGGGGACGGCAGGGGGA | |
| TCGA-27-1835 | 10 | 1 | 0 | GGGGAGGCTGCTGGTGGGCAGCTGACTGCGGGACACTGGAGGAA GCCTGGACCCTCAGCGAACT***TCGCCCAGCC | |
| TCGA-27-1835 | 29 | 0 | 0 | ACAGCCTGGGCACAGAGGTGGCTGTGCGA***AGGTCGCTGAGGGTCC AGGCTTCCACCCAGTGTCCCCGCAGTCAGCT | |
| TCGA-27-1835 | 32 | 0 | 0 | TGACTGCGGGGACACTGGGTGGAAGCCTGGACCCTCAGCGACCT***T CGCACAGCCACCTCTGTGCCCAGGCTGTGCC | |
| TCGA-27-1835 | 38 | 0 | 0 | CGGGGACACTGGGTGGAAGCCTGGACCCTCAGCGACCT***TCGCACA GCCACCTCTGTGCCCAGGCTGTGCCCCAGAA | |
| TCGA-27-1835 | 39 | 2 | 0 | GGGGACACTGGGTGGAAGCCTGGACCCTCAGCGACCT***TCGCACAG CCACCTCTGTGGCCAGGCTGTGCCACAGAAG | |
| TCGA-27-1835 | 41 | 0 | 0 | GGACACTGGGTGGAAGCCTGGACCCTCAGCGACCT***TCGCACAGCC ACCTCTGTGCCCAGGCTGTGCCCCAGAAGGC | |
| TCGA-27-1835 | 53 | 3 | 0 | GAAGCCTGGACCCTCAGCGACCT***TCGCACAGCCACCTCTGTGCCCC GGCTGTGCCCCAGCCGGCCCGCCCCACACC | |
| TCGA-27-1835 | 53 | 0 | 0 | GAAGCCTGGACCCTCAGCGACCT***TCGCACAGCCACCTCTGTGCCC AGGCTGTGCCCCAGAAGGCCCGCCCCACACC | |
| TCGA-27-1835 | 59 | 0 | 0 | TGGACCCTCAGCGACCT***TCGCACAGCCACCTCTGTGCCCAGGCTGT GCCCCAGAAGGCCCGCCCCACACCTCAGCA | |
| TCGA-27-1835 | 61 | 0 | 0 | GACCCTCAGCGACCT***TCGCACAGCCACCTCTGTGCCCAGGCTGTGC CCCAGAAGGCCCGCCCCACACCTCAGCACT | |
| TCGA-27-1835 | 66 | 0 | 0 | TCAGCGACCT***TCGCACAGCCACCTCTGTGCCCAGGCTGTGCCCCAG AAGGCCCGCCCCACACCTCAGCACTCTGGG | |
| TCGA-27-1835 | 73 | 0 | 0 | CCT***TCGCACAGCCACCTCTGTGCCCAGGCTGTGCCCCAGAAGGCCC GCCCCACACCTCAGCACTCTGGGGGGCAGG | |

| sample | gene split2 | readi ID | direction split | hg18 start split2 | hg18 stop split2 |
|---|---|---|---|---|---|
| TCGA-06-6390 | FGFR3 | D03U9ACXX110625:2:1202:19578:90281 | R | 1708787 | 1708861 |
| TCGA-06-6390 | TACC3 | C01PRACXX110628:3:1104:10052:66371 | F | 1708787 | 1708860 |
| TCGA-06-6390 | TACC3 | C01PRACXX110628:5:1108:3119:22892 | F | 1708787 | 1708860 |
| TCGA-06-6390 | TACC3 | D03U9ACXX110625:8:2304:13007:108632 | F | 1708787 | 1708860 |
| TCGA-06-6390 | TACC3 | C01PRACXX110628:5:2108:1999:91559 | F | 1708787 | 1708858 |
| TCGA-06-6390 | TACC3 | C01RDACXX110628:3:1308:1446:66311 | F | 1708787 | 1708855 |
| TCGA-06-6390 | FGFR3 | D03U9ACXX110625:5:2205:12523:196352 | R | 1708787 | 1708854 |
| TCGA-06-6390 | FGFR3 | C01PRACXX110628:5:2103:6815:17943 | R | 1708787 | 1708854 |
| TCGA-06-6390 | TACC3 | C01PRACXX110628:3:1204:10831:2928 | F | 1708787 | 1708852 |
| TCGA-06-6390 | TACC3 | C01PRACXX110628:5:2204:6732: 191360 | F | 1708787 | 1708852 |
| TCGA-06-6390 | TACC3 | C01PRACXX110628:8:1308:2911:26590 | F | 1708787 | 1708851 |
| TCGA-06-6390 | TACC3 | C01PRACXX110628:8:2207:4586:84017 | F | 1708787 | 1708849 |
| TCGA-06-6390 | FGFR3 | C01PRACXX110628:7:2205:11825:39734 | R | 1708787 | 1708841 |
| TCGA-06-6390 | FGFR3 | C01PRACXX110628:6:1106:12159:179499 | R | 1708787 | 1708834 |
| TCGA-06-6390 | FGFR3 | D03U9ACXX110625:4:2202:12501:40389 | R | 1708787 | 1708831 |
| TCGA-06-6390 | TACC3 | C01RDACXX110628:3:1305:3044:13238 | F | 1708787 | 1708813 |
| TCGA-06-6390 | FGFR3 | D03U9ACXX110625:5:2205:12523:196352 | R | 1708787 | 1708810 |
| TCGA-06-6390 | FGFR3 | C01PRACXX110628:7:2205:11825:39734 | R | 1708787 | 1708809 |
| TCGA-06-6390 | TACC3 | D03U9ACXX110625:7:2106:4492:173350 | F | 1708787 | 1708804 |
| TCGA-06-6390 | FGFR3 | C01PRACXX110628:5:2103:6815:17943 | R | 1708787 | 1708792 |
| TCGA-12-0826 | FGFR3 | 61C59AAXX100217:4:93:15133:6133 | R | 1707185 | 1707253 |
| TCGA-12-0826 | FGFR3 | 61C59AAXX100217:5:107:10675:16040 | R | 1707185 | 1707253 |
| TCGA-12-0826 | TACC3 | 61C59AAXX100217:5:108:1809:11295 | F | 1707185 | 1707252 |
| TCGA-12-0826 | TACC3 | 61C59AAXX100217:5:82:13129:10637 | F | 1707185 | 1707248 |
| TCGA-12-0826 | TACC3 | 42MJNAAXX090813:6:80:691:1877#0 | F | 1707185 | 1707239 |
| TCGA-12-0826 | FGFR3 | 61C59AAXX100217:3:75:10586:12881 | R | 1707185 | 1707228 |
| TCGA-12-0826 | FGFR3 | 61C59AAXX100217:4:114:5844:3161 | R | 1707185 | 1707228 |
| TCGA-12-0826 | FGFR3 | 42MJNAAXX090813:5:70:888:108#0 | R | 1707185 | 1707224 |
| TCGA-12-0826 | FGFR3 | 61C59AAXX100217:3:55:4966:15975 | R | 1707185 | 1707209 |
| TCGA-12-0826 | TACC3 | 42MJNAAXX090813:5:23:156:1150#0 | F | 1707185 | 1707205 |
| TCGA-12-0826 | TACC3 | 61C59AAXX100217:4:21:17613:20886 | F | 1707185 | 1707197 |
| TCGA-12-0826 | TACC3 | 61C59AAXX100217:4:2:4279:6949 | F | 1707185 | 1707193 |
| TCGA-19-5958 | FGFR3 | C01RDACXX110628:6:1102:11157:101962 | R | 1707202 | 1707270 |
| TCGA-19-5958 | FGFR3 | C01REACXX110629:2:2104:5009:98392 | R | 1707202 | 1707254 |
| TCGA-19-5958 | FGFR3 | C01PRACXX110628:7:2103:12434:91988 | R | 1707202 | 1707238 |
| TCGA-27-1835 | FGFR3 | B06UCABXX110322:6:1103:9262:46754 | R | 1709397 | 1709462 |
| TCGA-27-1835 | TACC3 | C00HWABXX110325:4:1201:20980:90877 | F | 1709397 | 1709443 |
| TCGA-27-1835 | FGFR3 | B06UCABXX110322:5:1108:14043:83287 | R | 1709397 | 1709440 |
| TCGA-27-1835 | FGFR3 | B097UABXX110405:4:2204:19445:88453 | R | 1709397 | 1709434 |
| TCGA-27-1835 | FGFR3 | B097UABXX110405:4:2201:20658:44401 | R | 1709397 | 1709433 |
| TCGA-27-1835 | FGFR3 | B097UABXX110405:2:2104:15688:71022 | R | 1709397 | 1709431 |
| TCGA-27-1835 | FGFR3 | COOHWABXX110325:6:2102:20394:42427 | R | 1709397 | 1709419 |
| TCGA-27-1835 | FGFR3 | B09V2ABXX110408:6:1203:18187:141862 | R | 1709397 | 1709419 |
| TCGA-27-1835 | FGFR3 | B09V2ABXX110408:8:1205:4774:81604 | R | 1709397 | 1709413 |
| TCGA-27-1835 | FGFR3 | C00HWABXX110325:2:1107:16168:23614 | R | 1709397 | 1709411 |
| TCGA-27-1835 | FGFR3 | C00HWABXX110325:7:2107:1225:167363 | R | 1709397 | 1709406 |
| TCGA-27-1835 | FGFR3 | B097UABXX110405:2:2104:15688:71022 | R | 1709397 | 1709399 |

| sample | length 2 | mis-match 2 | gap 2 | seq mate | |
|---|---|---|---|---|---|
| TCGA-06-6390 | 75 | 0 | 0 | GACGTCCACCGACGTGAGTGCTGGCTCTGGCCTGGTGCC ACCCGCCTATGCCCCTCCCCCTGCCGTCCCCGGCCAT | |
| TCGA-06-6390 | 74 | 0 | 0 | CAAGAGGGACTCAAGGACTTACAGGAATGTCCAGTGCT CCCAAGAAATCGAACTCCACAAGCTTGGCTTCCCGCGG | |
| TCGA-06-6390 | 74 | 0 | 0 | CAAGAGGGACTCAAGGACTTACAGGAATGTCCAGTGCT CCCAAGAAATCGAACTCCACAAGCTTGGCTTCCCGCGG | |
| TCGA-06-6390 | 74 | 0 | 0 | ATAGGCCCTTAAAACAACTCGTTCCCTCAGACCACACAC AAGACAGTTCAAGAGGGACTCAAGGACTTACAGGAAT | |
| TCGA-06-6390 | 72 | 0 | 0 | TCAAGAGGGACTCAAGGACTTACAGGAATGTCCAGTGC TCCCAAGAAATCGAACTCCACAAGCTTGGCTTCCCGCG | |
| TCGA-06-6390 | 69 | 0 | 0 | ACCACACACAAGACAGTTCAAGAGGGACTCAAGGACTT ACAGGAATGTCCAGTGCTCCCAAGAAATCGAACTCCAC | |
| TCGA-06-6390 | 68 | 4 | 0 | GAGCTGGCCTGGTGCCACACGCCTATGCCCCTCCCCCTG CCGTCCCCGGCGATCCATCAGGAAGTCCGCGGACGA | |
| TCGA-06-6390 | 68 | 0 | 0 | CCACCGACGTGAGTGCTGGCTCTGGCCTGGTGCCACCCG CCTATGCCCCTCCCCCTGCCGTCCCCGGCCATCCCTC | |
| TCGA-06-63 90 | 66 | 0 | 0 | CAAGAGCCTCAGACAGTGCATGAGGACCCGAGACAGT GCGGCGAGGGAACAGCACAGCGGCCCCATGCCCCCAAC | |
| TCGA-06-63 90 | 66 | 1 | 0 | CAAGAGCCTCAGACAGTGCATGAGGACCCGAGACAGT GCGGCGAGGGAACAGCACAGGGGCCCATGCCCCCAAC | |
| TCGA-06-63 90 | 65 | 0 | 0 | CGTTCCCTCAGACCACACACAAGACAGTTCAAGAGGGA CTCAAGGACTTACAGGAATGTCCAGTGCTCCCAAGAGA | |
| TCGA-06-63 90 | 63 | 0 | 0 | CCAGGAATAGAAAATATAGGCCCTTAAAACAACTCGTT CCCTCAGACCACACACAAGACAGTTCAAGAGGGACTCA | |
| TCGA-06-63 90 | 55 | 0 | 0 | GGCTCTGGCCTGGTGCCACCCGCCTATGCCCCTCCCCCT NCCGTCCCCGGCCATCCCTCAGGACGTCCGCGGGAAG | |
| TCGA-06-63 90 | 48 | 0 | 0 | GCCCTGCCCGCAGGTACATGATCATGCGGGAGTGCTGGC ATGCCGCGCCCTCCCAGAGGCCCACCTTCAAGCAGCT | |
| TCGA-06-63 90 | 45 | 0 | 0 | CTGGCATGCCGCGCCCTCCCAGAGGCCCACCTTTAAGCA GCTGGTAGAGGGCCTGGACCGTGTCCTTACCGTGACG | |
| TCGA-06-63 90 | 27 | 0 | 0 | TAAAACAACTCGTTCCCTCAGACCACACACAAGACAGTT CAAGAGGGACTCAAGGACTTACAGGAATGTCCAGTGC | |
| TCGA-06-63 90 | 24 | 4 | 0 | CACGGCCATCCCGGAGGACGTCCGCGGAACCCAAGCT TGTGGAGTTCGATTTCTTGGTAGCACTGGACATTCCTG | |
| TCGA-06-63 90 | 23 | 0 | 0 | TCCCCCTGCCGTCCCCGGCCATCCCTCAGGACGTCCGCG GGAAGCCAAGCTTGTGGAGTTCGATTTCTTGGGAGCA | |
| TCGA-06-63 90 | 18 | 0 | 0 | AGACCACACACAAGACAGTTCAAGAGGGACTCAAGGAC TTACAGGAATGTCCAGTGCTCCCAAGAAATCGAACTCC | |
| TCGA-06-63 90 | 6 | 0 | 0 | CCCGGCCATCCCTCAGGACGTCCGCGGAAGCCAAGCTT GTGGAGTTCGATTTCTTGGGAGCACTGGACATTCCTG | |
| TCGA-12-0826 | 69 | 2 | 1 | GGCATGCCGCGCCCTCCCAGAGGCCCACCTTCAAGCAGC TGGTGGAGGACCTGGACCGTGTCCTTACCGTGACGTC | |
| TCGA-12-0826 | 69 | 2 | 1 | GGCATGCCGCGCCCTCCCAGAGGCCCACCTTCAAGCAGC TGGTGGAGGACCTGGACCGTGTCCTTACCGTGACGTC | |
| TCGA-12-0826 | 68 | 2 | 1 | CGGCGCACATACCTGCTGGTCTCGGTGGCCACGGGCACT GGTCTACCAGGACTGTCCCTCAGGAGGGGGTCAAACT | |
| TCGA-12-0826 | 64 | 2 | 1 | ATACCTGCTGGTCTCGGTGGCCACGGCACTGGTCTACC AGGACTGTCCCTCAGGAGGGGTCAAACTTGAGGTAT | |
| TCGA-12-0826 | 55 | 2 | 1 | AGGTATAAGGACTGCTTCCTCAAGGCCGACTCCTTAAAC TGGGGACAAGAGGGCAAGTGATCAGGTCTGACTGCCA | |
| TCGA-12-0826 | 44 | 2 | 1 | GGAGGACCTGGACTGTGTCCTTACCGTGACGTCCACCGA CGTGAGTGCTGGCTCTGGCCTGGTGCCACCCGCCTAT | |
| TCGA-12-0826 | 44 | 2 | 1 | GGAGGACCTGGACCGTGTCCTTACCGTGACGTCCACCGA CGTGAGTGCTGGCTCTGGCCTGGTGCCACCCGCCTAT | |
| TCGA-12-0826 | 40 | 2 | 1 | CAAGCAGCTGGTGGAGGACCTGGACCGTGTCCTTACCGT GACGTCCACCGACGTGAGTGCTGGCTCTGGCCTGGTG | |
| TCGA-12-0826 | 25 | 2 | 1 | ACCTTCAAGCAGCTGGTGGAGGACCTGGACCGTGTCCTT ACCGTGACGTCCACCGACGTGAGTGCTGGCTCTGGCC | |
| TCGA-12-0826 | 21 | 2 | 1 | CAAACTTGAGGTATAAGGACTGCTTCCTCAAGGCCGACT CCTTAAACTGGGGACAAGAGGGCAAGTGATCAGGTCT | |
| TCGA-12-0826 | 13 | 0 | 1 | TACCTGCTGGTCTCGGTGGCCACGGGCACTGGTCTACCA GGGCTGTCCCTCCGGAGGGGGTCAAACTTGAGGGATA | |
| TCGA-12-0826 | 9 | 0 | 1 | AACTTGAGGTATAAGGACTGCTTCCTCAAGGCCGACTCC TTAAACTGGGGACAAGAGGGCAAGTGATCAGGTCTGA | |
| TCGA-19-5958 | 69 | 1 | 0 | AGCTGGTGGAGGACCTGGACCGTGTCCTTACCGTGACGT CCACCGACGTGAGTGCTGGCTCTGGCCTGGTGCCACC | |
| TCGA-19-5958 | 53 | 3 | 0 | GCGCCCTCCCAGAGGCCCACCTTCAAGCAGCTGGTGGA GGACCTGGACCGTGTCCTTACCGTGACGTCCACCGACG | |
| TCGA-19-5958 | 37 | 1 | 0 | GCGGGAGTGCTGGCATGCCGCGCCCTCCCAGAGGCCCA CCTTCAAGCAGCTGGTGGAGGACCTGGACCGTGTCCTT | |
| TCGA-27-1835 | 66 | 2 | 0 | CCTCCACTGGGTCCTCAGGGGTGGGGTCCCTCCGGGGC TGGGCGGGGGAGGGACTGGCAGGCCTGCAGGGGGGTT | |
| TCGA-27-1835 | 47 | 0 | 0 | TCACGGCAGCAAGAACCACACTCACTGCTGCAAGGCCA CCAGAGGCCAACGCCATGCCCAGGCCGGAGAGTCCCGG | |
| TCGA-27-1835 | 44 | 0 | 0 | TACATGATCATGCGGAGGGCTGGCATGCCGCGCCCTCC CAGAGGCCCACCTTCAAGCAGCTGGTGGAGGGCCGGG | |
| TCGA-27-1835 | 38 | 0 | 0 | GGTGGGAAGCGGCGGGGCTCACTCCTGAGCGCCCTGCC CGCAGGGACATGATCATGCGGGGGTGCTGGCCTTGCGG | |
| TCGA-27-1835 | 37 | 0 | 0 | GCGCCCTCCCAGAGGCCCACCTTCAAGCAGCTGGTGGA GGACCTGGACCGTGTCCTTACCGTGACGTCCACCGACG | |
| TCGA-27-1835 | 35 | 0 | 0 | CCTGCCCCCCAGAGTGCTGAGGTGTGGGGCGGCCTTCT GGGGCACAGCCTGGGCACAGAGGTGGCTGTGCGAAGG | |
| TCGA-27-1835 | 23 | 0 | 0 | GCAGGTACATGATCATGCGGGAGTGCCGGCATTTCGGG ACCTTCCCTCGGGCCACCCTCTTCCGGTTGTTGTGGGC | |
| TCGA-27-1835 | 23 | 0 | 0 | GCAGGTACATGATCATGCGGGAGTGCTGGCATGCCGCG CCCTCCCCGAGGACCACCTTCCAGCAGCCGGGGGAGGG | |
| TCGA-27-1835 | 17 | 0 | 0 | CCCGAATAAGGTGGGAAGCGGCGGGGCTCACTCCTGAG CGCCCTGACCGCAGGTACATGAGCATGCGGGAGTGGCG | |
| TCGA-27-1835 | 15 | 0 | 0 | CGTGTCCTTACCGTGACGTCCACCGACGTGAGTGCTGGC TCTGGCCTGGTGCCACCCGCCTATGCCCCTCCCCCTG | |
| TCGA-27-1835 | 10 | 0 | 0 | ACATGATCATGCGGGAGTGCTGGCATGCCGCGCCCCCC AGAGGCCCACCTTCAAGCAGCTGGTGGAGGACCTGGA | |
| TCGA-27-1835 | 3 | 0 | 0 | GCCTTCTGGGCACAGCCTGGGCACAGAGGTGGCTGTG CGAAGGTCGCTGAGGGTCCAGGCTTCCACCCAGTGTCC | |

The FGFR3 and TACC3 genes are located 48-Kb apart on human chromosome 4p16. The other members of the FGFR and TACC families retain the close physical association of FGFR3 and TACC3, with FGFR1 and TACC1 paired on chromosome 8p11 and FGFR2 and TACC2 paired on chromosome 10q26. Without being bound by theory, the ancestral FGFR and TACC genes were physically linked and that this tandem gene cluster was duplicated at least twice to generate the FGFR1-TACC1, FGFR2-TACC2 and FGFR3-TACC3 pairs that mark mammalian evolution (Still et al., 1999). The highly conserved TK domains among FGFR genes and TACC domains among TACC genes together with their invariable fusion in the FGFR3-TACC3 rearrangements prompted to ask whether other intrachromosomal FGFR-TACC fusion combinations exist in human GBM.

cDNA from a panel of 88 primary GBM were screened using pairs of upstream PCR primers that bind the amino-terminal coding region of the TK domains of FGFR1, FGFR2 and FGFR3 and downstream primers that bind to the carboxy-terminal coding region of the TACC domains of TACC 1, TACC2 and TACC3 genes, respectively. The screening resulted in the identification of intrachromosomal FGFR-TACC fusions in two additional cases (one harboring FGFR1-TACC1 and one FGFR3--TACC3), corresponding to three of 97 total GBM (3.1%), including the GBM-1123 case. The FGFR1-TACC1 fusion breakpoint in GBM-51 joined in-frame exon 17 of FGFR1 to exon 7 of TACC1, resulting in a novel protein in which the TK domain of FGFR1 is fused upstream of the TACC domain of TACC 1 **(****Figure 2F****).** The same structure was conserved again in GBM-22 in which exon 16 of FGFR3 is joined in-frame to exon 10 of TACC3 (**Figure 2G**). None of the tumors harboring FGFR-TACC fusions had mutations in IDH1 or IDH2 genes, thus indicating that FGFR-TACC-positive GBM mark an independent subgroup of patients from those carrying IDH mutations (Table 6) (Yan et al., 2009). The constant linkage of the FGFR-TK to the TACC domain created in each of the seven GBM harboring FGFR-TACC rearrangements suggests that FGFR-TACC fusion proteins may generate important functional consequences for oncogenesis in the brain.

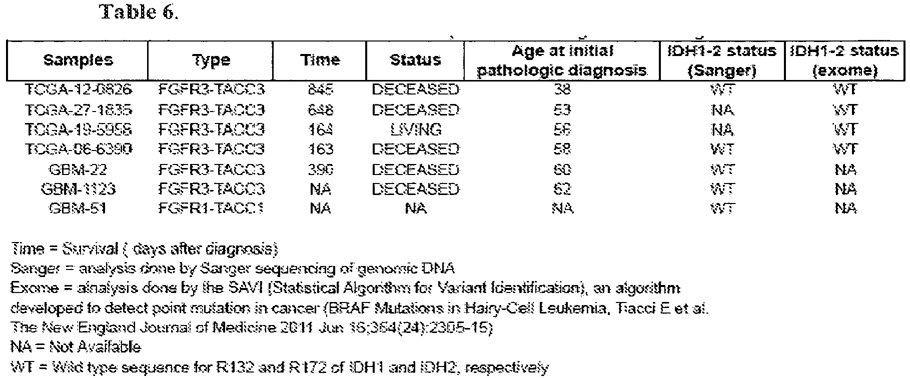

***Transforming activity of FGFR-TACC fusions.*** To test the functional importance of the FGFR-TACC fusions in GBM, the FGFR3-TACC3 cDNA was cloned from GSC-1123 and recombinant lentiviruses were prepared expressing FGFR3-TACC3, FGFR1 -TACC1, a kinase-dead FGFR3-TACC3 protein (FGFR3-TACC3-K508M), *-wild type* FGFR3 and *wild type* TACC3. Transduction of Rat1A fibroblasts and *Ink4A;Arf-*/*-* astrocytes with the FGFR3-TACC3 lentivirus resulted in the expression of the fusion protein at levels comparable to those present in GSC-1123 (**Figure 11**). Having reconstituted in nontransformed cells the endogenous level of the FGFR-TACC protein that accumulates in GBM cells, it was determined whether it was sufficient to initiate oncogenic transformation *in vitro* and *in vivo.* RatlA cells expressing FGFR3-TACC3 and FGFR1-TACC1 but not those expressing FGFR3-TACC3-K508M, FGFR3, TACC3 or the empty lentivirus acquired the ability to grow in anchorage-independent conditions in soft agar (**Figure 3A**). Transduction of the same lentiviruses in primary *Ink4A;Arf-*/*-* astrocytes followed by subcutaneous injection into immunodeficient mice revealed that only astrocytes expressing FGFR3-TACC3 and FGFR 1-TACC1 formed tumors. The tumors emerged in 100% of the mice injected with astrocytes expressing the fusion proteins and were glioma-like lesions with strong positivity for Ki67, phospho-histone H3, nestin, OFAP and Olig2 (**Figure 3B**).

Next, it was determined whether the FGFR3-TACC3 fusion protein is oncogenic when transduced to a small number of cells directly into the brain of immunocompetent animals. A recently described mouse glioma model was used in which brain tumors are initiated by lentiviral transduction of oncogenes and inactivation of p53 in the mouse brain (Marumoto et al., 2009). To target adult NSCs, the adult mouse hippocampus was stereotactically transduced with purified lentivirus expressing the FGFR3-TACC3 protein and shRNA against p53 (pTomo-FGFR3-TACC3-shp53). Seven of eight mice (87.5%) transduced with FGFR3-TACC3 succumbed from malignant brain tumors within 240 days (**Figure 3C**). None of the mice transduced with a lentivirus expressing the most frequent gain-of-function mutation in GBM (the constitutively active EGFRvIII, pTomo-EGFRvIII-shp53) or the pTomo-shp53 control lentivirus died or developed clinical signs of brain tumors **(****Figure 3C**). The FGFR3 -TACC3 tumors were high-grade glioma with strong propensity to invade the normal brain and stained positive for the glioma stem cell markers nestin and Olig2 and the glial marker GFAP. They were also highly positive for Ki67 and phospho-histone H3, thus displaying rapid tumor growth (**Figure 3D**). The expression of FGFR3-TACC3 in the xenograft and intracranial tumor models was comparable to the expression of the endogenous protein in the human GSCs and tumor (**Figure 11D, 11E and 11F****).**

These data show that FGFR-TACC fusion proteins possess transforming activity in two independent cellular models and this activity is not the result of the overexpression of individual FGFR and TACC genes. They also show that direct transduction of the FGFR3-TACC3 protein to the adult mouse brain leads to efficient development of malignant glioma.

***The FGFR-TACC fusions interfere with mitotic progression and induce chromosome missegregation and aneuploidy.*** To elucidate the mechanism by which the FGFR-TACC fusion drives oncogenesis, it was explored whether it activates downstream FGFR signaling. FGFR3 -TACC3 failed to hyperactivate the canonical signaling events downstream of FGFR (pERK and pAKT) in the presence or absence of the ligands FGF-1 , FGF-2 or FGF-8 (Wesche et al., 2011) (**Figures 12A****,** **12B** **and** **12C****).** However, FGFR3-TACC3 displayed constitutive phosphorylation of its TK domain and the adaptor protein FRS2, both of which were abolished by the specific inhibitor of FGFR-associated TK activity PD173074 (Mohammadi et al., 1998) or the K508M mutation (**Figure 4A**). Thus, FGFR3-TACC3 gains constitutive kinase activity that is essential for oncogenic transformation but the downstream signaling of this aberrant activity is distinct from the canonical signaling events downstream to FGFR. By driving the localization of the fusion protein, the TACC domain can create entirely novel TK-dependent functions. The TACC domain is essential for the localization of TACC proteins to the mitotic spindle (Hood and Royle, 2011; Peset and Vernos, 2008). Confocal imaging showed that FGFR3-TACC3 designed an arc-shaped structure bending over and encasing the metaphase spindle poles, frequently displaying asymmetry towards one of the two poles and relocated to the midbody as cells progressed into the late stages of mitosis (telophase and cytokinesis) (**Figure 4B** **and** **12D**). Conversely, the localization of TACC3 was restricted to spindle microtubules and did not relocalize to the midbody (**Figure 12E**). Wild type FGFR.3 lacked discrete localization patterns in mitosis (**Figure 12F**).

The mitotic localization of FGFR3-TACC3 indicates that it may impact the fidelity of mitosis and perturb the accurate delivery of the diploid chromosomal content to daughter cells, thus generating aneuploidy*.* Mitotic progression of individual cells was examined in vector-transduced and FGFR3-TACC3 expressing cells co-expressing histone H2B-GFP by time-lapse microscopy. The average time from nuclear envelope breakdown to anaphase onset was increased in cells expressing FGFR3-TACC3 in comparison with control cells. The mitotic delay was further exacerbated by difficulties in completing cytokinesis (**Figures 4C** **and** **4D**).

Next, it was determined whether the expressions of the FGFR-TACC fusion proteins induce defects of chromosomal segregation. Quantitative analyses of mitoses revealed that cells expressing FGFR3-TACC3 or FGFR1-TACC1 exhibit a three to five fold increase of chromosomal segregation errors than control cells. The most frequent mitotic aberrations triggered by the fusion proteins were misaligned chromosomes during metaphase, lagging chromosomes at anaphase and chromosome bridges that impaired cytokinesis and generated micronuclei in the daughter cells **(****Figures 4****.E, 4F and 13A).** Aberrations at the metaphase-anaphase transition frequently lead to the inability of mitotic cells to maintain a metaphase arrest after treatment with a spindle poison. Over 18% of cells expressing FGFR3-TACC3 displayed prematurely separated sister chromatids in contrast with less than 3% in control, FGFR3 or TACC3-expressing cells (**Figures 13B and 13C**). Accordingly, cells expressing the fusion protein were unable to efficiently arrest in metaphase after nocodazole treatment **(****Figure 13D****).**

The above findings indicate that expression of the FGFR3-TACC3 fusion protein may spark aneuploidy. Karyotype analysis revealed that FGFR3-TACC3 increased over 2.5 fold the percent of aneuploidy and led to the accumulation of cells with broad distribution of chromosome counts in comparison with cells transduced with empty vector, FGFR3 or TACC3 **(****Figure 5A****).** Accordingly, GSC-1 123 contained aneuploid modal number of chromosomes (49) and manifested a broad distribution of chromosome counts characterized by 60% of metaphase spreads that deviate from the mode (Table 7).

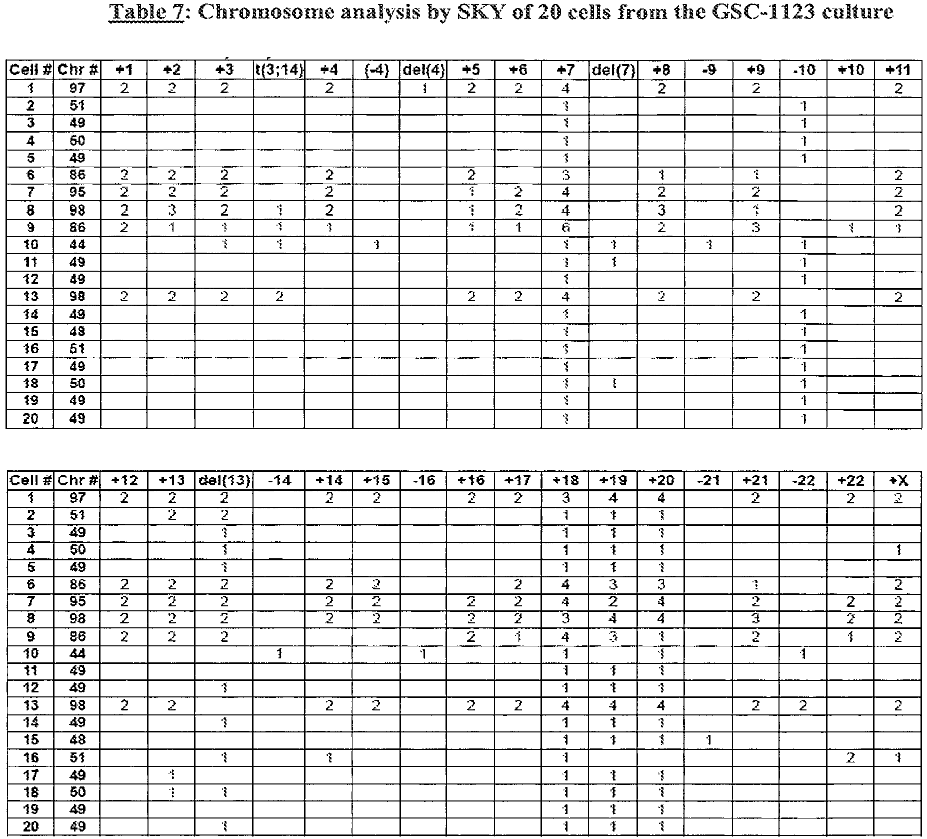

Next, it was determined whether aneuploidy is a direct consequence of FGFR3-TACC3 expression and is induced in human diploid neural cells. Primary human astrocytes analyzed six days after transduction with the FGFR3-TACC3 lentivirus exhibited a 5-fold increase of the rate of aneuploidy and a significantly wider distribution of chromosome counts (**Figures 5B, 5C** **and** **5D**). Consistent that aneuploidy is detrimental to cellular fitness, acute expression of FGFR3 -TACC3 compromised the proliferation capacity of human astrocytes. However, continuous culture of FGFR3-TACC3-expressing human astrocytes led to progressive gain of proliferative capacity that overrode that of control cells (**Figure 14A, 14B**). Thus, the acute expression of FGFR3-TACC3 in primary normal human cells from the central nervous system causes CIN and aneuploidy with an acute fitness cost manifested by slower proliferation.

It was also determined whether the CIN and aneuploidy caused by FGFR3-TACC3 requires the TK activity of FGFR3 and can be corrected. Treatment with PD 173074 rescued the aneuploidy caused by FGFR3-TACC3 by over 80%, restored the narrow distribution of chromosome counts typical of control cells and largely corrected the cohesion detect (**Figures 6A****,** **6B and 6C**). Together, these findings indicate that the CIN and aneuploidy caused by rearrangements of FGFR and TACC genes are reversible and suggest that specific FGFR kinase inhibition may be a valuable therapeutic strategy in tumor cells expressing FGFR-TACC fusion proteins.

***FGFR-TACC fusion proteins are new therapeutic targets in GBM**.* Driver genetic alterations trigger a state of oncogene addiction in the cancer cells harboring them that can be exploited therapeutically. To ask whether FGFR-TACC fusions confer addiction to FGFR-TK activity, cell growth was analyzed in the presence of PD173074, AZD4547 or BGJ398, the latter being two highly specific inhibitors of FCRFR-TK under clinical investigation (Gavine et al., 2012; Guagnano et al., 2011). Each of the three drugs inhibited growth of cells expressing FGFR3-TACC3 and FGFR1-TACC1 at concentrations <10 nM whereas they were ineffective at concentrations as high as 1 µM. in cells transduced with vector, FGFR3, TACC3 and the FGFR3-TACC3-K508M mutant (**Figures 7A****,** **14C** and **14D**). These findings underscore the elevated degree of specificity for FGFR kinase inhibition towards cells carrying the fusion protein. The growth of GSC-1123 cells, which naturally harbor the FGFR3-TACC3 translocation, was also abolished by nanomolar concentrations of FGFR-TKL inhibitors (**Figure 7B**). Targeting of the fusion gene by FGFR3 shRNA inhibited the growth of cells ectopically expressing FGFR3-TACC3 and GSC-1123 proportionally to the silencing efficiency of FGFR3-TACC3 (**Figures 7C** and **14E**).

Finally, it was determined whether treatment with PD173074 of mice bearing glioma xenografts of FGFR3-TACC3 transformed astrocytes inhibits tumor growth. Twelve days after injection of tumor cells, subcutaneous tumors were present in all animals. The mice were randomized in two cohorts and treated with PD173074 or vehicle. PD173074 elicited a potent growth inhibition of FGFR3-TACC3 glioma (**Figure 7D**). To confirm the efficacy of a clinically meaningful FGFR-TK inhibitor using a more anatomically relevant model, the AZD4547 FGFR inhibitor, a compound under clinical investigation (Gavine et al., 2012), was used against intracranial luciferase-expressing FGFR3-TACC3-driven glioma xenografts. After an engraftment period, tumor-bearing animals were treated with either AZD4547 or vehicle. Oral administration of AZD4547 markedly prolonged survival (**Figure 7E**). Taken together, the data provide a strong rationale for a clinical trial based on FGFR inhibitors in GBM harboring FGFR-TACC rearrangements.

### DISCUSSION

This work has established that recurrent, oncogenic and addicting gene fusions identify a subset of GBM patients. The functional characterization of FGFR-TACC fusions indicates that the constitutively active FGFR-TK and the TACC domain of the fusion protein are both essential for oncogenesis. The TACC-dependent mis-localization to mitotic cells of the FGFR kinase results in aberrant compartmentalization of a constitutively active TK to the mitotic spindle pole, thus providing a mechanistic explanation for the impaired mitotic fidelity, chromosome mis-segregation and aneuploidy instigated by the fusion protein.

Without being bound by theory, mutation of the genes that control chromosome segregation during mitosis can explain the high rate of CIN and aneuploidy, which is typical of most solid tumors including GBM. (Gordon et al., 2012). A few examples of mutational inactivation of candidate genes have been reported in human cancer (Solomon et al., 2011; Thompson et al., 2010). However, gain-of-function mutations causally implicated in the control of mitotic fidelity have not been described. This clashes with the classical observation from cell fusion experiments that the underlying mechanisms that cause CIN behave as dominant traits, indicating that the CTN phenotype results from gain-of-function events rather than gene inactivation (Lengauer et al., 1997, 1998). The FGFR-TACC gene fusion is a novel mechanism for the initiation of CIN and provides a clue to the nature of dominant mutations responsible for aneuploidy in human cancer.

The rapid emergence of mitotic defects and aneuploid cell populations triggered by the fusion protein in normal human astrocytes, combined with the correction of aneuploidy after short inhibition of FGFR-TK activity indicate that aneuploidy is a key event in tumor induction by the FGFR-TACC gene fusions. Induction of aneuploidy *per se* is detrimental to cellular fitness (Sheltzer and Amon, 2011). Full-blown tumorigenesis requires cooperation between aneuploidy and genetic lesions that confer growth advantage and protect cells against the detrimental effects of aneuploidy (Coschi and Dick, 2012; Holland and Cleveland, 2009; Weaver and Cleveland, 2009). Therefore, the potent tumor-initiating activity of FGFR-TACC fusions shows that the novel oncoproteins have growth-promoting signaling functions that complement the loss of mitotic fidelity with ensuing karyotypic alterations (Sheltzer and Amon, 2011).

Targeted therapies against common genetic alterations in GBM have not changed the dismal clinical outcome of the disease, most likely because they have systematically failed to eradicate the truly addicting oncoprotein activities of GBM. The highly specific antitumor effects and the correction of aneuploidy precipitated by FGFR-TK inhibition of FGFR-TACC-driven GBM provide a strong rationale for clinical trials based on FGFR inhibitors in patients harboring FGFR-TACC rearrangements. The computational gene fusion discovery pipeline reported here detected other GBM cases in which FGFR family genes are implicated in additional gene fusions beyond the FGFR-TACC rearrangements. Therefore, the frequency of 3.1% is likely to be an underestimate of the target GBM patient population that may benefit from FGFR-TK inhibition.

### EXPERIMENTAL PROCEDURES

***Cell culture* and *isolation* and** ***maintenance of GSCs**.* Rat1A, mouse astrocytes *Ink4A;Arf-*/*,* and human astrocytes were cultured in DMEM supplemented with 10% FBS. Isolation and culture of GSCs was performed as described (Carro et al., 2010). For treatment *in vitro* with PD173074, AZD4547 or BJG398, cells infected with vector control, FGFR3, TACC3, FGFR-TACC fusions or FGFR3-TACC3-K508M were seeded in 96-well plates and treated with increasing concentrations of FGFR inhibitors. After 72-120 h, growth rate was measured using the 3-(4,5-dimethythiazol-2-yl)-2,5-diphenyl tetrazolium bromide (MTT) assay. Data were expressed as mean±SD. Proliferation rate in GSC-1123 infected with FGFR3 shRNA lentivirus was determined by plating dissociated gliomaspheres at 2×10⁴ cells/well in twelve-well plates 5 days after infection. The number of viable cells was determined by trypan blue exclusion in triplicate cultures obtained from triplicate independent infections. Cell number was scored every other day.

### DNA, RNA preparation, genomic and real-time quantitative PCR (qRT-PCR).

The validation of fusion transcripts was performed using both genomic and RT-. PCR with forward and reverse primer combinations designed within the margins of the paired-end read sequences detected by RNA-seq. DNA, RNA preparation and qRT-PCR were performed as described (Carro et al., 2010; Zhao et al., 2008). To identify novel fusion transcripts within the GBM cohort, PCR primers pairs were designed to bind upstream to the TK domain of the FGFR genes and inside or downstream the Coiled Coil domain of the TACC genes. Expressed fusion transcript variants were subjected to direct sequencing to confirm sequence and frame. Primer sequences are included below.

***Subcutaneous xenografts and drug treatment.*** Rat1A or *Ink4A;Arf-*/*-* astrocytes (5×10⁵) transduced with different lentiviral constructs were suspended in 150 µl of PBS, together with 30 µl of Matrigel (BD Biosciences), and injected subcutaneously in the flank of athymic nude (Nu/Nu) mice (Charles River Laboratories, Wilmington, MA). For experiments with FGFR inhibitors, mice carrying -200-300 mm³ subcutaneous tumors derived from *Ink4A;Arf-*/*-* astrocytes were randomized to receive 50 mg/kg PD173074 in 0.05 M lactate buffer (pH 5) or an equal volume of lactate buffer by oral gavage. Treatment was administered for three cycles consisting of four consecutive days followed by two days of rest. Tumor diameters were measured with caliper, and tumor volumes estimated using the formula: 0.5 x length x width². Data are expressed as mean±SE. Mice were sacrificed when tumors in the control group reached the maximal size allowed.

***Orthotopic transplantation and drug treatment**. Ink4A;Arf-*/*-* astrocytes carrying a luciferase expressing vector were transduced with FGFR3-TACC3 lentivitus. 1×10³ cells in 2 µl of saline were injected in the caudate-putamen of 4-6 week old male athymic nude (Nu/Nu) mice using a stereotaxic frame (coordinates relative to bregma: 0.5 mm anterior; 1.1 mm lateral; 3.0 mm ventral) and a 26 gauge Hamilton syringe. Six days after injection, mice underwent bioluminescence imaging using a Xenogen CCD apparatus and were randomized to receive 50 mg/kg AZD4547 in 1% Tween 80 (treatment group) or DMSO in an equal volume of vehicle by oral gavage (control group). AZD4547 was administered daily for two cycles of 10 days with a two day interval. Mice were monitored daily and sacrificed when neurological symptoms appeared. Kaplan-Meier survival curve was generated using the DNA Statview software package (AbacusConcepts, Berkeley, CA). Log-rank analysis was performed on the Kaplan-Meier survival curve to determine statistical significance.

***Intracranial injections of lentiviruses.*** Intracranial injection of FGFR3-TACC3-shp53, EGFRvIII-shp53 or shp53 pTomo lentiviruses was performed in 4 week-old C57/BL/6J mice in accordance with guidelines of IACUC Committee. Briefly, 1.25 µl of purified lentiviral particles in PBS (1×10⁹/ml) were injected into the dentate gyrus using a stereotaxic frame (coordinates relative to bregma: 1.45 mm posterior; 1.65 mm lateral; 2.4 mm ventral) and a 26 gauge Hamilton syringe. Mice were monitored daily and sacrificed when neurological symptoms appeared. Mouse brain was analyzed histopathologically and by immunofluorescence staining.

***Histology and immunostaining.*** Tissue preparation and immunohistochemistry on brain tumors and immunofluorescence staining were performed as previously described (Carro et al., 2010; Zhao et al., 2009; Zhao et al., 2008). Antibodies used in immunostaining and immuno blotting are listed below.

***Cloning and Lentiviral production**.* Lentivirus preparation and infections were performed as described (Carro et al., 2010) and are detailed in Extended Experimental Procedures.

***Karyotype analysis.*** Cultured cells were colcemid (20 ng/ml) treated for 90 minutes before harvesting for karyotopic analysis as detailed in Extended Experimental procedures. At least one hundred cells in metaphase were examined for chromosome count. PMSCS was scored in cells where a majority of the sister chromosomes were no longer associated. Two-tailed unpaired t-tests with Welch's correction were performed for comparison of means analysis.

***Immunofluorescence and live-cell microscopy.*** Immunofluorescence microscopy was performed on cells fixed with 4% PFA in PHEM (60 mM Pipes, 27 mM Hepes, 10 mM EGTA, 4 mM MgSO₄, pH 7.0). Cells were permeabilized using 1% Triton X-100. Mitotic spindles were visualized by anti-α-tubulin antibody (Sigma). Secondary antibodies conjugated to Alexa Fluor-488/-594 (Molecular Probes) were used. All staining with multiple antibodies were performed in a sequential manner. DNA was stained by DAPI (Sigma). Fluorescence microscopy was performed on a Nikon A1R MP microscope.

***Identification of gene fusions from whole transcriptome (RNA-seq) and exome sequencing**.* RNA-Sequencing was performed from total RNA extracted from GSC cultures isolated from nine GBM patients using Illumina HiSeq 2000, producing roughly 60.3 million paired reads per sample. Using the global alignment software Burrows-Wheeler Aligner (BWA) (Li and Durbin, 2009) with modified Mott's trimming, an initial seed length of 32, maximum edit distance of 2 and a maximum gap number of 1, on average 43.1 million reads were mapped properly to the RefSeq transcriptome and, of the remaining, 8.6 million were mapped to the hg19 genome per sample. The remaining 14.3% of paired reads-including those that tailed to map to either transcriptome or genome with proper forward-reverse (F-R) orientation, within expected insert size, and with minimal soft clipping (unmapped portions at the ends of a read)- were considered to be appropriate for gene fusion analysis.

A novel computational pipeline was constructed called ***TX-Fuse*** that identifies two sources of evidence for the presence of a gene fusion: 1. *Split inserts,* in which each read of a mate pair maps entirely to one side of a breakpoint, and 2. Individual *split reads* that span a breakpoint. Split inserts are readily detected from BWA mapping. On the other hand, split reads demand precision alignment of smaller nucleotide stretches. To that end, the pipeline employs the local alignment package BLAST with word size of 20, identity cutoff of 95%, expectation cutoff of 10⁻⁴ , and soft filtering to map raw paired reads against the RefSeq transcriptome. From this procedure, a list of potential split reads were obtained that were filtered to ensure maintenance of coding frame in the predicted fusion transcript given the proper F-R orientation in the read pair. False positive candidates produced from paralogous gene pairs were also screened out using the Duplicated Genes Database and the EnsemblCompara GeneTrees (Vilella et al., 2009). Pseudogenes in the candidate list were annotated using the list from HUGO Gene Nomenclature Committee (HGNC) database (Seal et al., 2011) and given lower priority. For each remaining gene fusion candidate, a virtual reference was created based on the predicted fusion transcript and re-mapped all unmapped reads using BLAST with word size of 16, identity cutoff of 85%, query coverage greater than 85%, and expectation cutoff of 10⁻⁴ to obtain a final count of split reads and inserts. Moreover, sequencing depth per base of the virtual reference was calculated to corroborate that components of each gene participating in the gene fusion were highly expressed.

To establish the recurrence of the initial panel of gene fusion candidates, the gene fusion discovery pipeline was modified to produce ***EXome-Fuse**,* which probes for fusions within the available dataset of paired-read exome DNA sequencing of 84 matched GBM samples from TCGA. To increase sensitivity for gene fusion identification, reads unmapped by BWA were aligned to the gene pair participating in each fusion candidate using a BLAST word size of 24 for split inserts and 16 for split read and split insert discovery. Given that the breakpoint detected in DNA cannot directly indicate the resulting breakpoint in the transcribed RNA, no restriction was made on split insert orientation. For split reads, it was only required that the component of the split read mapped to the same gene as its mate maintained F-R directionality.

***Co-outlier expression and CNV analysis from TCGA GBM samples.*** Tomlins et al. (Tomlins et al., 2005) reported that outlier gene expression from microarray datasets identifies candidate oncogenic gene fusions. Wang et al. (Wang et al., 2009) suggested a "breakpoint principle" for intragenic copy number aberrations in fusion partners. The two principles (outlier expression and intragenic CNV) were combined to identify candidate gene fusions in GBM samples from Atlas-TCGA. Genomic and expression data sets were downloaded from TCGA public data portal as available on December 1, 2011, where a description of TCGA data types, platforms, and analyses is also available (2008). Specific data sources were (according to Data Levels and Data Types) as follows: Expression data, "Level 2" normalized signals per probe set (Affymetrix HT_HG-U133A) of 84 samples; Copy number data, "Level 1" raw signals per probe (Affymetrix Genome-Wide Human SNP *Array* 6.0) of the 4 *FGFR3-TACC5* gene fusion positive samples (tumor and matched normal control).

The gene expression analysis was performed first using R³. The median absolute deviation (MAD) was calculated and then a gene was labeled as an outlier according to the following formula: Z_{i,j}= 0.6745(x_{i,j} - mean(xᵢ) )/MADᵢ > 3.5 (Iglewicz and Hoaglin, 1993). Samples were identified as ECFS (expression candidate fusion sample) if both genes of interest (e. g. FGFR3 and TACC3) displayed outlier behavior (co-outliers). Next, ECFS were analyzed for CNV using pennCNV (Wang et al., 2007). Tumors samples were paired to their normal controls to obtain the log ratio values and the VEGA algorithm was used to obtain a more accurate segmentation (Morgatiella et al., 2010).

***Karyotypic Analysis.*** The colcemid treated cells were trypsinized, centrifuged for 7 minutes at 200 × g, and the cell pellet re-suspended in warmed hypotonic solution and incubated at 37°C for 13 minutes. The swollen cells were then centrifuged and the pellet resuspended in 8 ml of Carnoy's fixative (3:1 methanol:glacial acetic acid). The cell suspension was centrifuged and washed twice in Carnoy's fixative. After the last centrifugation, the cells were resuspended in 0.5 to 1 ml of freshly prepared fixative to produce an opalescent cell suspension. Drops of the final cell suspension were placed on clean slides and air-dried. Slides were stained with DAPI and metaphases were analyzed under a fluorescent microscope.

**Cloning and Lentiviral production.** Lentiviral expression vectors, pLOC-GFP (Open Biosystems) and pTomo-shp53, were used to clone FGFR3, TACC3, FGFR3-TACC3, FGFR3-TACC3-K508M, and FGFR1-TACC1. pTomo-shp53 was a gift of Inder Venna and Dinorah Friechnan-Morvinski (Salk Institute, San Diego). The FGFR3-TACC3-K508M mutant, was generated using the Phusion Site Direct Mutagenesis kit (NEB, USA). MISSION shRNAs clones (p.LKO. I lentiviral expression vectors) against FGFR3 were purchased from Sigma. The hairpin sequences targeting the *FGFR3* gene are-
5'-TGCGTCGTGGAGAACAAGTTT-3' (#TRCN0000000372; Sh#2) (SEQ ID NO: 182);
5'-GTTCCACTGCAAGGTGTACAG-3' (#TRCN0000430673; Sh#3) (SEQ ID NO: 183);
5'-GCACAACCTCGACTACTACAA-3' (#TRCN0000000374; Sh#4) (SEQ ID NO: 184).

**Genomic and mRN**A **RT-PCR.** Total RNA was extracted from cells by using RNeasy Mini Kit. (QIAGEN), following the manufacturer instructions. 500 ng of total RNA was retro-transcribed by using the Superscript III kit (invitrogen), following the manufacturer instructions. The cDNAs obtained after the retro-transcription was used as templates for qPCR. The reaction was performed with a Roclie480 thermal cycler, by using the Absolute Blue QPCR SYBR. Green Mix from Thermo Scientific. The relative amount of specific mRNA was normalized to 18S. Results are presented as the mean ± SD of triplicate amplifications.

Primers used are:
hFGFR3-RT-FW1: 5'-GTAACCTGCGGGAGTTTCTG-3' (SEQ ID NO: 162);
hFGFR3-RT-REV1: 5'-ACACCAGGTCCTTGAAGGTG-3' (SEQ ID NO: 1(3);
hTACC3-RT-FW2: S'-CCTGAGGGACAGTCCTGGTA-3' (SEQ ID NO: 164);
hTACC3-RT-REV2: 5'-AGTGCTCCCAAGAAATCGAA-3' (SEQ ID NO: 165);
hWRAP53-RT-FW1: 5'-AGAGGTGACCACCAATCAGC-3' (SEQ ID NO: 180);
hWRAP53-RT-REV1: 5'-CGTGTCCCACACAGAGACAG-3' (SEQ ID NO: 181).

Primers used for the screening of FGFR-TACC fusions are:
FGFR3-FWI: 5'-CGTGAAGATGCTGAAAGACGATG-3' (SEQ ID NO: 166);
TACC3-REV1: 5'- AAACGCTTGAAGAGGTCGGAG-3' (SEQ ID NO: 167);
FGFR1-FW1: 5'-ATGCTAGCAGGGGTCTCTGA-3' (SEQ ID NO: 168);
TACC1-REV1: 5'-CCCTTCCAGAACACCTTTCA-3' (SEQ ID NO: 169).

Primers used for genomic detection of FGFR3-TACC3 fusion in GBM-1123 and GSC-1123 are:
Genomic FGFR3-FW1: 5'-ATGATCATGCGGGAGTGC-3' (SEQ ID NO: 170);
genomicTACC3-REV1: 5'-GGGGGTCGAACTTGAGGTAT-3' (SEQ ID NO: 171).

Primers used to validate fusions detected by RNA-seq are:
POLR2A--FWI: 5'-CGCAGGCTTTTTGTAGTGAG-3' (SEQ ID NO: 172);
WRAP53-REV1: 5'-TGTAGGCGCGAAAGGAAG-3' (SEQ ID NO: 173);
PIGU-FW1: 5'-GAACTCATCCGGACCCCTAT-3' (SEQ ID NO: 174);
NCOA6-REV1: 5'-GCTTTCCCCATTGCACTTTA-3' (SEQ ID NO: 175);
ST8SIA4-FWI: 5'-GAGGAGAGAAGCACGTGGAG-3' (SEQ ID NO: 176);
PAM-REV 1: 5'-GGCAGACGTGTGAGGTGTAA-3' (SEQ ID NO: 177);
CAPZB-FW: 5'-GTGATCAGCAGCTGGACTGT-3' (SEQ ID NO: 178);
UBR4-REV1: 5'-GAGCCTGGGCATGGATCT-5' (SEQ ID NO: 179).

**Confocal microscopy imaging.** For immunofluorescence of fixed cells, images were recorded with a Z-optical spacing of 0.25 µm using a Nikon A1R MP and a 60X1.3 oil objective and analyzed using ImageJ software (National Institute of Health). For live-cell analyses, RatlA cells infected with pLNCX-H2B retrovirus and transduced with lentiviral vector or FGFR3-TACC3 fusion were seeded in glass bottom dishes in phenol red free DMEM and followed by time-lapse microscopy using the Nikon A1R MP biostation at 37°C and 5%, CO₂/95% air. Images with a Z-optical spacing of 1 µm were recorded every 4 min for 8 h. Images of unchallenged mitosis from early prophase until cytokinesis were processed using ImageJ software (National Institute of Health). The time-point of nuclear envelope breakdown (NEB) was defined as the first frame showing loss of smooth appearance of chromatin and anaphase was the first frame when chromosome movement towards the poles became apparent. Nuclear envelope reconstitution (NER) was defined as the first frame showing nuclei decondensation.

Box and whisker plots were calculated from image sequences from at least 50 recorded cells. Two-tailed unpaired t-tests with Welch's correction were performed for comparison of means analysis using StatView software (AbacusConcepts, Berkeley, CA).

**Immunofluorescence.** Antibodies and concentrations used in immunofluorescence staining are:

| | | | |
|---|---|---|---|
| Anti-Ki67 | Rabbit | 1:1000 | Vector Labs |
| Anti-pHH3 | Rabbit | 1:500 | Millipore |
| Anti-FGFR3 | Mouse | 1:1000 | Santa Cruz |
| Anti-Tacc3 | Goat | 1:1000 | USBiological |
| Anti-a-tubulin | Mouse | 1:1000 | Sigma |
| Anti-Nestin | Mouse | 1:1000 | BD Pharrningen |
| Anti-Olig2 | Rabbit | 1:200 | IBL |
| Anti-GFAP | Rabbit | 1:200 | Dako |
| Anti-ERK | Rabbit | 1:1000 | Cell Signaling |
| Anti-pERK | Rabbit | 1:1000 | Cell Signaling |
| AntiFRS | Rabbit | 1:250 | Santa Cruz |
| Anti-pFRS | Rabbit | 1:1000 | Cell Signaling |
| Anti-AKT | Rabbit | 1:1000 | Cell Signaling |
| Anti-pAKT473 | Rabbit | 1:1000 | Cell Signaling |

### REFERENCES

Ablain, J., Nasr, R., Bazarbachi, A., and de The, H. (2011). The Drug-Induced Degradation of Oncoproteins: An Unexpected Achilles' Heel of Cancer Cells? Cancer Discov. 1, 117-127.
Bass, A.J., Lawrence, M.S., Brace, L.E., Ramos, A.H., Drier, Y., Cibulskis, K., Sougnez, C., Voet, D., Saksena, G., Sivachenko, A., et al. (2011). Genomic sequencing of colorectal adenocarcinomas identifies a recurrent VTFI1A-TCF7L2 fusion. Nat. Genet. 43, 964-968.
Cahill, D.P., Kinzler, K.W., Vogelstein, B., and Lengauer, C. (1999). Genetic instability and darwinian selection in tumours. Trends Cell. Biol. 9, M57-60.
Carro, M.S., Lim, W.K., Alvarez, M.J., Bollo, R.J., Zhao, X., Snyder, E.Y., Sulman, E.P., Anne, S.L., Doetsch, F., Colman, H.. et al. (2010). The transcriptional network for mesenchymal transformation of brain tumours. Nature 463, 318-325.
Coschi, C.H., and Dick, F.A. (2012). Chromosome instability and deregulated proliferation: an unavoidable duo. Cell. Mol. Life Sci. 69, 2009-2024
Druker, B.J. (2009). Perspectives on the development of imatinib and the future of cancer research. Nat. Med. 15, 1149-1152.
Furnari, F.B., Fenton, T., Bachoo, R.M., Mukasa, A., Stommel, J.M., Stegh, A., Hahn, W.C., Ligon, K.L., Louis, D.N., Brennan, C., et al. (2007). Malignant astrocytic glioma: genetics, biology, and paths to treatment. Genes Dev. 21, 2683-2710.
Gavine, P.R., Mooney, L., Kilgour, E., Thomas, A.P., Al-Kadhimi, K., Beck, S., Rooney, C., Coleman, T., Baker, D., Mellor, M.J., et al. (2012). AZD4547: An Orally Bioavailable, Potent, and Selective Inhibitor of the Fibroblast Growth Factor Receptor Tyrosine Kinase Family. Cancer Res. 72, 2045-2056.
Gerber, D.E., and Minna, J.D. (2010). ALK. inhibition for non-small cell lung cancer: from discovery to therapy in record time. Cancer Cell 18, 548-551.
Gordon, D.J., Resio, B., and Pellman, D. (2012). Causes and consequences of aneuploidy in cancer. Nature reviews Genet. 13, 189-203.
Guagnano, V., Furet, P., Spanka, C., Bordas, V., Le Douget, M., Stamm, C., Brueggen, J., Jensen, M.R., Schnell, C., Schmid, H., et al. (2011). Discovery of 3-(2,6-dichloro-3,5-dimethoxy-phenyl)-1-{6-[4-(4-ethyl-piperazin-1-yl)~phenylamin o]-pyrimidin-4-yl}-1-methyl-urea (NVP-BGJ398), a potent and selective inhibitor of the fibroblast growth factor receptor family of receptor tyrosine kinase. J. Med. Chem. 54, 7066-7083.
Holland, A.J., and Cleveland, D.W. (2009). Boveri revisited: chromosomal instability, aneuploidy and tumorigenesis. Nat. Rev. Mol. Cell. Biol. 10, 478-487.
Hood, F.E., and Royle, S.J. (2011). Pulling it together: The mitotic function of TACC3. Bioarchitecture 1 105-109.
Lee, J., Kotliarova, S., Kotliarov, Y., Li, A., Su, Q., Donin, N.M., Pastorino, S., Purow, B.W., Christopher, N., Zhang, W., et al. (2006). Tumor stem cells derived from glioblastomas cultured in bFGF and EGF more closely mirror the phenotype and genotype of primary tumors than do serum-cultured cell lines. Cancer Cell 9, 391-403.
Lengauer, C., Kinzler, K.W., and Vogelstein, B. (1997). Genetic instability in colorectal cancers. Nature 386, 623-627.
Lengauer, C., Kinzler, K.W., and Vogelstein, B. (1998). Genetic instabilities in human cancers. Nature 396, 643-649.
Lo, H.W. (2010). EGFR-targeted therapy in malignant glioma: novel aspects and mechanisms of drug resistance. Corr. Mol. Pharmacol. 3, 37-52.
Marumoto, T., Tashiro, A., Friedmann-Morvinski, D., Scadeng, M., Soda, Y., Gage, F.H., and Verma, I.M. (2009). Development of a novel mouse glioma model using lentiviral vectors. Nat. Med. 15, 110-116.
Mitelman, F., Johansson, B., and Mertens, F. (2007). The impact of translocations and gene fusions on cancer causation. Nat. Rev. Cancer 7, 233-245.
Mohammadi, M., Froum, S., Hamby, J.M., Schroeder, M.C., Panek, R.L., Lu, G.H., Eliseenkova, A.V., Green, D., Schlessinger, J., and Hubbard, S.R. (1998). Crystal structure of an angiogenesis inhibitor bound to the FGF receptor tyrosine kinase domain. EMBO J. 17, 5896-5904.
Ohgaki, H., and Kleihues, P. (2005). Population-based studies on incidence, survival rates, and genetic alterations in astrocytic and oligodendroglial gliomas. J. Neuropathol. Exp. Neurol. 64, 479-489.
Peset, I, and Vernos, I. (2008). The TACC proteins: TACC-ling microtubule dynamics and centrosome function. Trends Cell. Biol. 18, 379-388.
Prensner, J.R., and Chinnaiyan, A.M. (2009). Oncogenic gene fusions in epithelial carcinomas. Curr Opin Genet. Dev. 19, 82-91.
Reardon, D.A., Desjardins, A., Vredenburgh, J.J., Gururangan, S., Friedman, A.H., Herndon, J.E., 2nd, Marcello, J., Norfleet, J.A., McLendon, R.E., Sampson, J.H., et al. (2010). Phase 2 trial of erlotinib plus sirolimus in adults with recurrent glioblastoma. J. Neurooncol. 96, 219-230.
Sheltzer, J.M., and Amon, A. (2011). The aneuploidy paradox: costs and benefits of an incorrect karyotype. Trends Genet. 27, 446-453,
Solomon, D.A., Kim, T., Diaz-Martinez, L.A., Fair, J., Elkahloun, A.G., Harris, B.T., Toretsky, J.A., Rosenberg, S.A., Shukla, N., Ladanyi, M., et al. (2011). Mutational inactivation of STAG2 causes aneuploidy in human cancer. Science 333, 1039-1043.
Stephens, P.J., McBride, D.J., Lin, M.L., Varela, I., Pleasance, E.D., Simpson, J.T., Stebbings, L.A., Leroy, C., Edkins, S., Mudie, L.J., et al. (2009). Complex landscapes of somatic rearrangement in human breast cancer genomes. Nature 462, 1005-1010.
Still, I.H., Vince, P., and Cowell, J.K. (1999). The third member of the transforming acidic coiled coil-containing gene family, TACC3, maps in 4p16, close to translocation breakpoints in multiple myeloma, and is upregulated in various cancer cell lines. Genomics 58, 165-170.
Thompson, S.L., Bakhoum, S.F., and Compton, D.A. (2010). Mechanisms of chromosomal instability. Curr. Biol. 20, R285-295.
Tomlins, S.A., Laxman, B., Dhanasekaran, S.M., Helgeson, B.E., Cao, X., Morris, D.S., Menon, A., ling, X., Cao, Q., Han, B., et al. (2007). Distinct classes of chromosomal rearrangements create oncogenic ETS gene fusions in prostate cancer. Nature 448, 595-599.
Tomlins, S.A., Rhodes, D.R., Perner, S., Dhanasekaran, S.M., Mehra, R., Sun, X.W., Varambally, S., Cao, 1., Tchinda, J., Kuefer, R., et al. (2005). Recurrent fusion of TMPRSS2 and ETS transcription factor genes in prostate cancer. Science 310, 644-648.
Turner, N., and Grose, R. (2010). Fibroblast growth factor signalling: from development to cancer. Nat. Rev. Cancer 10, 116-129.
van den Bent, M.J., Brandes, A.A., Rampling, R., Kouwenhoven, M.C., Kros, J.M., Carpentier, A.F., Clement, P.M., Frenay, M., Campone, M., Baurain, J.F., et al. (2009). Randomized phase II trial of erlotinib versus temozolomide or carmustine in recurrent glioblastoma: EORTC brain tumor group study 26034. J. Clin. Oncol. 27, 1268-1274.
Wang, X.S., Prensner, J.R., Chen, G., Cao, Q., Han, B., Dhanasekaran, S.M., Ponnala, R., Cao, X., Varaintially, S., Thomas, D.G., et al. (2009). An integrative approach to reveal driver gene fusions from paired-end sequencing data in cancer. Nat. Biotechnol. 27, 1005-1011.
Weaver, B.A., and Cleveland, D.W. (2009). The role of aneuploidy in promoting and suppressing tumors. J. Cell. Biol. 185, 935-937.
Wesche, J., Haglund, K., and Haugsten, E.M. (2011). Fibroblast growth factors and their receptors in cancer. Biochem. J. 437, 199-213.
Yan, H., Parsons, D.W., Jin, G., McLendon, R., Rasheed, B.A., Yuan, W., Kos, L, Batinic-Haberle, 1., Jones, S., Riggins, G.J., et al. (2009). IDH1 and IDH2 mutations in gliomas. New Engl, J. Med. 360, 765-773.
Zhao, X., D, D.A., Lim, W.K., Brahmachary, M., Carro, M.S., Ludwig, T., Cardo, C.C., Guillemot, F., Aldape, K., Califano, A., et al. (2009). The N-Myc-DLL3 cascade is suppressed by the ubiquitin ligase Huwel to inhibit proliferation and promote neurogenesis in the developing brain. Dev. Cell 17, 2 1 0-221.
Zhao, X., Heng, J.I., Guardavaccaro, D., Jiang, R., Pagano, M., Guillemot, F., Tavarone, A., and Lasorelia, A. (2008). The HECT-domain ubiquitin ligase Huwel controls neural differentiation and proliferation by destabilizing the N-Myc oncoprotein. Nat Cell Biol 10, 643-653.
(2008). Comprehensive genomic characterization defines human glioblastoma genes and core pathways. Nature 455, 1061-1068.
Iglewicz, B., and Hoaglin, D.C. (1993). How to detect and handle outliers (Milwaukee, Wis.: ASQC).
Li, H., and Durbin, R. (2009). Fast and accurate short read alignment with Burrows-Wheeler transform. Bioinformatics 25, 1754-1760.
Morganella, S., Cerulo, L., Viglietto, G., and Ceccarelli, M. (2010). VEGA: variational segmentation for copy number detection. Bioinformatics 26, 3020-3027.
Seal, R.L., Gordon, S.M., Lush, M.J., Wright, M.W., and Bruford, E.A. (2011). genenames.org: the HGNC resources in 2011, Nucleic Acids Res 39, D514-519.
Tomlins, S.A., Rhodes, D.R., Perner, S., Dhanasekaran, S.M., Mehra, R., Sun, X.W., Varambally, S., Cao, X., Tchinda, J., Kuefer, R., et al. (2005). Recurrent fusion of TMPRSS2 and ETS transcription factor genes in prostate cancer. Science 310, 644-648.
Vilella, A.J., Severin, J., Ureta-Vidal, A., Heng, L., Durbin, R., and Birney, E. (2009). EnsemblCompara GeneTrees: Complete, duplication-aware phylogenetic trees in vertebrates. Genome Res. 19, 327-335.
Wang, K., Li, M., Hadley, D., Liu, R., Glessner, J., Grant, S.F., Hakonarson, H., and Bucan, M. (2007). PennCNV: an integrated hidden Markov model designed for high-resolution copy number variation detection in whole-genome SNP genotyping data. Genome Res. 17, 1665-1674.
Wang, X.S., Prensner, J.R., Chen, G., Cao, Q., Han, B., Dhanasekaran, S.M., Ponnala, R., Cao, X., Varambally, S., Thomas, D.G., et al. (2009). An integrative approach to reveal driver gene fusions from paired-end sequencing data in cancer. Nat. BiotechnoL 27, 1005-1011.

### Example 2- Fusions in GBM

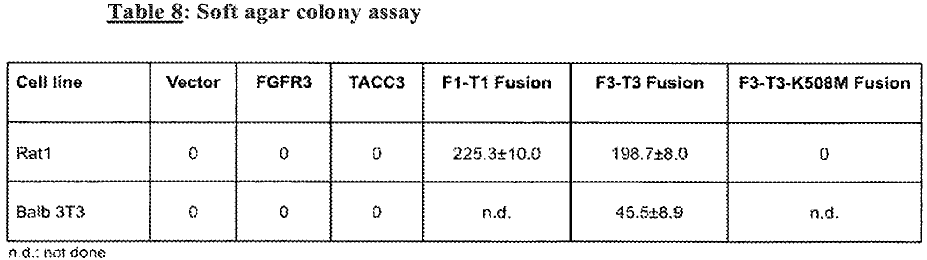

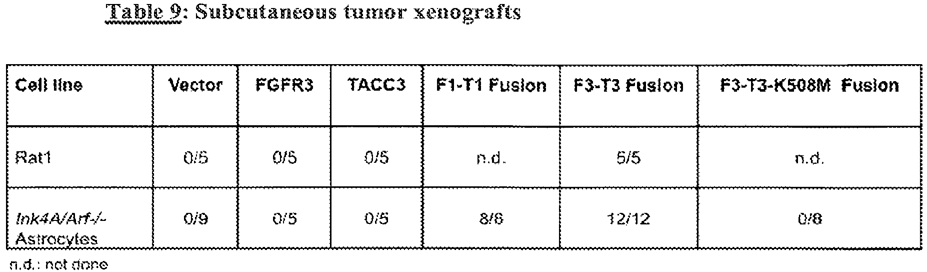

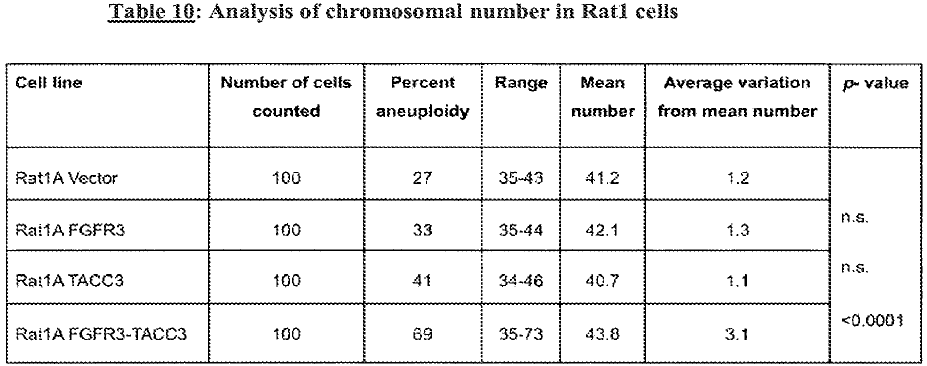

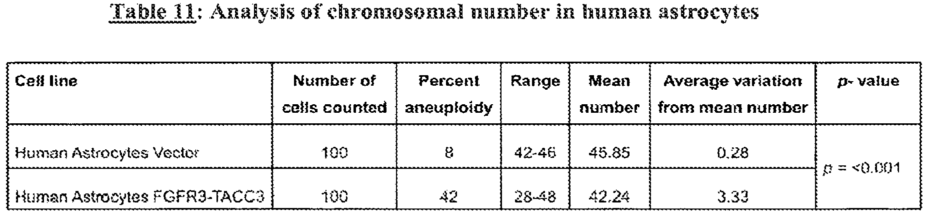

### Example 3 - Fusions in Other Cancers

The inventors previously reported in Example 1 that 3.1% of human glioblastoma harbor FGFR3-TACC3 and FGFR1-TACC1 gene fusions. Tumors harboring FGFR3-TACC3 gene fusions are identified by the presence of highly specific focal micro-amplification events of the rearranged portions of the FGFR3 and TACC3 genes (See FIG. 2E). Therefore, these micro-amplification events can be used as distinctive marks for the presence of FGFR3-TACC3 gene fusions. It was asked whether other types of human tumors also harbor FGFR3-TACC3 gene fusions from the analysis of Copy Number Variations (CNVs) of SNP arrays generated from the Atlas-TCGA project. This analysis was performed using segmented CNVs data visualized using the Integrated Genomic Viewers software. The analysis revealed that the following tumors, shown in the FIGS. 31-35, display focal micro-amplification events of FGFR3 and TACC3 that indicate the presence of FGFR3-TACC3 gene fusions (in FIGS. 31-35, red indicates amplification (A), blue indicates deletion (D); **FIG. 31****:** Bladder Urothelial Carcinoma; **FIG. 32****:** Breast Carcinoma; **FIG. 33****:** Colorectal Carcinoma; **FIG. 34****:** Lung Squamous Cell Carcinoma; **FIG. 35****:** Head and Neck Squamous Cell Carcinoma).

Taken together, these data indicate that the same FGFR3-TACC3 gene fusions reported for the first time in Glioblastoma also occur in several other types of human tumors. Therefore, as for Glioblastoma and other epithelial cancers (such as the human tumors discussed herein), the identification of FGFR-TACC gene fusions also provides a new diagnostic and therapeutic target for treatment with drugs that inhibit FGFR--TACC gene fusions.

## Claims

1. A diagnostic kit for determining whether a sample from a subject exhibits a presence of a FGFR fusion protein, the kit comprising at least one oligonucleotide that specifically hybridizes to a FGFR fusion protein, or a portion thereof, and wherein the FGFR fusion protein comprises the tyrosine kinase domain of a FGFR protein fused to the TACC domain of a TACC protein.

2. The kit of claim 1, wherein the oligonucleotides comprise a set of nucleic acid primers or in situ hybridization probes; and optionally wherein the primers prime a polymerase reaction only when a FGFR fusion is present; or wherein the oligonucleotide comprises SEQ ID NO: 162, 163, 164,165, 166, 167, 168, 169, or a combination thereof; or wherein the determining comprises gene sequencing, selective hybridization, selective amplification, gene expression analysis, or a combination thereof.

3. A diagnostic kit for determining whether a sample from a subject exhibits a presence of a FGFR fusion protein, the kit comprising an antibody that specifically binds to a FGFR fusion protein comprising SEQ ID NO: 79, 85, 86, 87, 88, 89, 150, 158, 159, 160, or 161, wherein the antibody will recognize the protein only when a FGFR fusion protein is present, and wherein the FGFR fusion protein comprises a tyrosine kinase domain of an FGFR protein fused to the TACC domain of a transforming acidic coiled-coil-containing (TACC) protein.

4. The kit of claim 3, wherein the antibody is directed to an FGFR fusion protein comprising SEQ ID NO: 79, 85, 86, 87, 88, 89, 150, 158, 159, 160, or 161.

5. A method for detecting the presence of a FGFR fusion protein in a human subject, wherein the FGFR fusion protein comprises the tyrosine kinase domain of a FGFR protein fused to the TACC domain of a TACC protein, the method comprising: (a) obtaining a biological sample from the human subject; and (b) detecting whether or not there is a FGFR fusion present in the subject.

6. The method of claim 5, wherein the detecting comprises measuring FGFR fusion protein levels by ELISA using an antibody directed to SEQ ID NO: 79, 85, 86, 87, 88, 89, 150, 158, 159, 160, or 161; western blot using an antibody directed to SEQ ID NO: 79, 85, 86, 87, 88, 89, 150, 158, 159, 160, or 161; mass spectroscopy, isoelectric focusing, or a combination thereof; or wherein the detecting of step (b) comprises detecting whether or not there is a nucleic acid sequence encoding a FGFR fusion protein in the subject; and optionally wherein the nucleic acid sequence comprises any one of SEQ ID NOS: 1-77, 80-84, or 95-145; or wherein the detecting comprises using hybridization, amplification, orsequencing techniques to detect a FGFR fusion protein; and optionally wherein the amplification uses primers comprising SEQ ID NO: 162, 163, 164,165, 166, 167, 168, or 169.
